(19) Europäisches Patentamt
European Patent Office
Office européen des brevets

(11) EP 3 036 236 B1

(12) **EUROPEAN PATENT SPECIFICATION**

(45) Date of publication and mention of the grant of the patent:
**07.06.2017 Bulletin 2017/23**

(21) Application number: **14806546.9**

(22) Date of filing: **15.08.2014**

(51) Int Cl.:
***C07D 487/04*** (2006.01) ***A61K 31/4985*** (2006.01)
***A61P 17/04*** (2006.01)

(86) International application number:
**PCT/EP2014/067494**

(87) International publication number:
**WO 2015/024878 (26.02.2015 Gazette 2015/08)**

(54) **NOVEL NEUROKININ 1 RECEPTOR ANTAGONIST COMPOUNDS II**

NEUARTIGE NEUROKININ-1-REZEPTORANTAGONISTENVERBINDUNGEN II

NOUVEAUX COMPOSÉS ANTAGONISTES II DU RÉCEPTEUR DE LA NEUROKININE 1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2013 PCT/CN2013/081889**

(43) Date of publication of application:
**29.06.2016 Bulletin 2016/26**

(73) Proprietor: **LEO PHARMA A/S**
**2750 Ballerup (DK)**

(72) Inventors:
- **BLADH, Haakon**
  **DK-2750 Ballerup (DK)**
- **FELDING, Jakob**
  **DK-2750 Ballerup (DK)**
- **ZHOU, Ding**
  **Shanghai, 201201 (CN)**
- **CAI, Zhen-wei**
  **Shanghai, 201201 (CN)**
- **SØRENSEN, Morten Dahl**
  **DK-2750 Ballerup (DK)**

(74) Representative: **Leo Pharma A/S**
**Industrieparken 55**
**2750 Ballerup (DK)**

(56) References cited:
**WO-A1-03/066635** **WO-A1-03/084955**
**WO-A1-2013/124286**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 036 236 B1

## Description

FIELD OF INVENTION

The present invention relates to novel heterocyclic compounds which are neurokinin 1 receptor antagonists, to intermediates for the preparation of said compounds, to their use in therapy such as in the prophylaxis or treatment of pruritic dermal diseases or conditions, and to pharmaceutical compositions comprising said compounds.

BACKGROUND OF THE INVENTION

Pruritus is a common symptom of skin diseases as well as a sign of an underlying systemic pathology. Pruritus is an unpleasant sensation in the skin that provokes a desire to scracth and may be acute (of short duration) such as the reaction to an insect bite or chronic (lasting for more than 6 weeks) such as in many inflammatory skin diseases. It is well known that patients with inflammatory skin diseases perceive pruritus as seriously compromising their quality of life.

Pruritus is mediated via free nerve endings of non-myelinated C-type nerve fibres in epidermis. These have been found to express neuropeptides, and the epidermal keratinocytes produce neuropeptides, receptors for neuropeptides, nerve growth factor, vanilloid receptors proteinase-activated receptor type 2 (PAR2) and voltage-gated ATP channels (MW Greaves, Curr. Allergy Asthma Rep. 10, 2010, pp. 236-242). Neuropeptides suchs as Substance P have been shown to increase the production and release of nerve growth factor from cultured keratinocytes, suggesting that interactions between the immune system and the nervous system are important in the development of inflammation including pruritus. Inflammatory skin diseases such as atopic dermatitis, contact dermatitis, psoriasis and urticaria are associated with increased production of cytokines, neurotrophins and neuropeptides that may exacerbate the pruritus (U. Raap et al., Curr. Opin. Allergy Clin. Immunol. 11, 2011, pp. 420-427). While neuropeptides such as Substance P are not considered crucial for the pathogenesis of inflammatory skin diseases such as atopic dermatitis, they do play an important role in the development and severity of the condition, for instance by provoking the itch-scratch cycle where scratching exacerbates the inflammatory symptoms of atopic dermatitis (J. Salomon and E. Baran, JEADV 22, 2008, pp. 223-228). Furthermore, an increase of dermal nerves and upregulation of receptors for neuropeptides, e.g. the neurokinin 1 receptor has been found in skin from psoriasis patients with pruritus as opposed to skin from psoriasis patients without pruritus (S-E. Chang et al., Br. J. Dermatol. 156, 2007, pp. 1272-1277).

It has also been found that other cell types resident in skin release mediators of pruritus. Thus, mast cells contain large amounts of histamine that are released on activation of the cells and induce pruritus by targeting histamine H1 receptors on nerve endings. Eosinophils which infiltrate inflamed skin in atopic dermatitis, urticaria and contact dermatitis produce and release neurotrophins such as nerve growth factor.

Current therapeutic treatments of pruritus include both topical and systemic medicaments. Topical treatments include emollients and barrier creams that are believed to act by improving the barrier function of the skin, corticosteroids that do not appear to be antipruritic in themselves but act by relieving the attendant skin inflammation. This may also be the case for calcineurin inhibitors such as tacrolimus which has been shown to reduce pruritus in atopic dermatitis patients. Histamine H1 antagonists have also shown effect against pruritus in atopic dermatitis patients, but exhibits systemic side effects in the form of drowsiness in a significant number of patients. Local anesthetics such as lidocain have been found to exhibit anti-pruritic properties (Patel and Yosipovitch, Expert. Opin. Pharmacother. 11(10), 2010, 1673-1682).

Systemic therapy of pruritus include oral antihistamines, antidepressants, neuroleptics and immunosuppressants such as cyclosporin. A neurokinin-1 receptor antagonist, aprepitant, which has been developed as an oral antiemetic drug for use to counteract the nausea and vomiting caused by chemotherapy or post surgery, has been found to relieve pruritus in patients suffering from Sézary syndrome by oral administration (Duval and Dubertret, New Engl. J. Med. 361, 2009, pp. 1415-1416) and in patients with atopic diathesis, prurigo nodularis and systemic pruritus (S. Stander et al., Plos One, 2010, p. 5).

WO01/25219 discloses piperazine derivatives which are antagonists of tachykinins, including substance P and other neurokinins.

WO02/32867 discloses piperidine derivatives which are antagonists of tachykinins, including substance P and other neurokinins

WO02/081457 discloses 1,4-diazepane-1-carboxylic acid derivatives process for their preparation and their use as tachykinin antagonists

WO2009002770 discloses 6.5 pyrrolopiperidine tachykinin receptor antagonists.

WO2013124286 discloses Novel Neurokinin 1 Receptor Antagonists.

As many of the antipruritic treatments available at present have side-effects that may limit their use, and as many dermal conditions are preferentially treated with topical medications, especially when the conditions is of mild to moderate severity, there is a continued need to develop neurokinin 1 receptor (NK1R) antagonists which are effective

in the treatment of itch on topical application, but which have reduced systemic effects on the central nervous system.

SUMMARY OF THE INVENTION

In one aspect, the present invention relates to compounds of general formula A

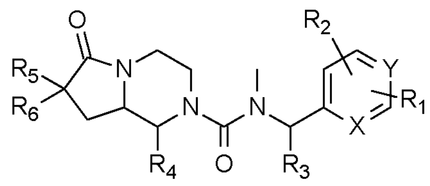

A

wherein

$R_1$ and $R_2$ independently are selected from the group consisting of $(C_{1-4})$alkyl, $(C_{1-4})$haloalkyl, $(C_{1-4})$alkoxy, $CD_3$ or halogen;

$R_3$ is selected from the group consisting of hydrogen, $(C_{1-4})$alkyl, $(C_{1-4})$haloalkyl and $(C_{1-4})$hydroxyalkyl;

$R_4$ is selected from the group consisting of phenyl, 5-membered heteroaryl and 6-membered heteroaryl, said phenyl and 6-membered heteroaryl being substituted with one ore more substituents independently selected from $R_7$, and said 5-membered heteroaryl optionally being substituted with one ore more substituents independently selected from $R_7$;

$R_5$ and $R_6$ are independently selected from the group consisting of hydrogen, $(C_{1-4})$alkyl, $(C_{1-4})$hydroxyalkyl and $(C_{1-4})$haloalkyl;

$R_7$ is selected from the group consisting of halogen, hydroxy, $(C_{1-4})$alkyl, $(C_{1-4})$alkoxy, aryloxy, aryl$(C_{1-4})$alkoxy, cyano, $(C_{1-4})$haloalkyl or $(C_{1-4})$hydroxyalkyl, wherein two adjacent $(C_{1-4})$alkyl's, together with the phenyl, 5-membered heteroaryl or 6-membered heteroaryl to which they are attached, may optionally form a 4-7 membered aliphatic ring;

X and Y are independently selected from the group consisting of CH and N;

or a pharmaceutically acceptable salt or solvate thereof;
with the proviso that when both X and Y are CH, $R_4$ is not 2-methylphenyl.

In another aspect the present invention relates to a compound according to general formula A for use in therapy.

In another aspect the present invention relates to a compound according to general formula A for use in the prevention, treatment or amelioration of pruritic skin conditions , e.g. acute pruritus in any condition; chronic pruritus on diseased skin, such as inflammatory, infectious, or autoimmune cutaneous diseases, genodermatoses, drug reactions, dermatoses of pregnancy and skin lymphomas, prurigo , lichen planus, atopic dermatitis, eczema, contact dermatitis, allergic dermatitis, nummular dermatitis, lichen simplex, psoriasis, Sézary syndrome, cutaneous lymphomas, bullous pemphigoid, alopecia areata, scabies, vitiligo, urticaria and drug-induced pruritus; pruritic diseases on non-diseased skin of systemic, neurological or psychosomatic/psychiatric origin, including endocrine and metabolic disorders, infections, haematological and lymphoproliferative diseases, solid neoplasms and drug-induced pruritus; mastocytosis; pruritus of unknown cause; pruritus with chronic secondary scratch lesions, such as prurigo nodularis, and all types of prurigo; or any other dermal disease or condition characterized by pruritus.

In another aspect the present invention relates to a pharmaceutical composition comprising, as a therapeutically active ingredient, a compound according to general formula A and a pharmaceutically acceptable carrier or vehicle.

In another aspect the present invention relates to intermediates for the preparation of a compound according to general formula A, selected from the list consisting of 1-(2,4-dimethylphenyl)hexahydropyrrolo[1,2-a]pyrazin-6(7H)-one, 7,7-diallyl-1-(2,4-dimethylphenyl)hexahydropyrrolo[1,2-a]pyrazin-6(7H)-one, 7,7-diallyl-1-(4-fluoro-2-methylphenyl)hexahydropyrrolo[1,2-a]pyrazin-6(7H)-one, 1-(4-methoxy-2-methylphenyl)hexahydropyrrolo[1,2-a]pyrazin-6(7H)-one, 1-(4-fluoro-2-methylphenyl)hexahydropyrrolo[1,2-a]pyrazin-6(7H)-one, 1-(4-methoxy-2-methylphenyl)hexahydropyrrolo[1,2-a]pyrazin-6(7H)-one, 7,7-diallyl-1-(4-methoxy-2-methylphenyl)hexahydropyrrolo[1,2-a]pyrazin-6(7H)-one, 1-(4-(benzyloxy)-2-methylphenyl)hexahydropyrrolo[1,2-a]pyrazin-6(7H)-one, 7,7-diallyl-1-(4-(benzyloxy)-2-methylphenyl)hexahydropyrrolo[1,2-a]pyrazin-6(7H)-one, tert-butyl7,7-diallyl-1-(2,5-dimethylphenyl)-6-oxo-hexahydropyrrolo[1,2-a]pyrazine-2(1H)-carboxylate, 7,7-diallyl-1-(2,3-dimethylphenyl)hexahydropyrrolo[1,2-a]pyrazin-6(7H)-one, 1-(2,3-dimethylphenyl)-hexahydropyrrolo[1,2-a]pyrazin-6(7H)-one, 1-(3-methylpyridin-2-yl)-hexahydropyrrolo[1,2-a]pyrazin-6(7H)-one, 1-(4-methylpyridin-3-yl)hexahydropyrrolo[1,2-a]pyrazin-6(7H)-one, 1-(3-methylpyridin-4-yl)hexahydropyrrolo[1,2-a]pyrazin-6(7H)-one, 1-(2-methylpyridin-3-yl)hexahydropyrrolo[1,2-a]pyrazin-6(7H)-one, 1-(4-methylthiophen-3-yl)hexahydropyrrolo[1,2-a]pyrazin-6(7H)-one, 1-(3-methylthiophen-2-yl)hexahydropyrrolo[1,2-a]pyrazin-6(7H)-one, 1-(thiophen-2-yl)hexahydropyrrolo[1,2-a]pyrazin-6(7H)-one, 1-(2-methylthiophen-3-yl)hexahydropyrrolo[1,2-a]pyrazin-6(7H)-one, 1-(2-methylthiazol-5-yl)hexahydropyrrolo[1,2-a]pyrazin-6(7H)-one, 1-(thiazol-5-yl)hexahydropyrrolo[1,2-a]pyrazin-6(7H)-one, 1-(1-benzyl-3-methyl-1H-pyrazol-4-yl)hexahydropyrrolo[1,2-a]pyrazin-6(7H)-one, 7,7-diallyl-1-(1-benzyl-3-methyl-1H-pyrazol-4-yl)hexahydropyrrolo[1,2-a]pyrazin-6(7H)-one, (1S,8aS)-1-(3-chloro-2-methylphenyl)hexahydropyrrolo[1,2-a]pyrazin-6(2H)-one, 1-(2-ethylphenyl)hexahydropyrrolo[1,2-a]pyrazin-6(7H)-one, 1-(2-chlorophenyl)hexahydropyrrolo[1,2-a]pyrazin-6(7H)-one, (1S, 8aS)-1-(2-vinylphenyl)hexahydropyrrolo[1,2-a]pyrazin-6(7H)-one, (8aS)-1-(3,4-dimethylthiophen-2-yl)hexahydropyrrolo[1,2-a]pyrazin-6(7H)-one, (1S,8aS)-1-(3-methoxy-2-methylphenyl)hexahydropyrrolo[1,2-a]pyrazin-6(7H)-one, (1S,8aS)-1-(2,3-dihydro-1H-inden-4-yl)hexahydropyrrolo[1,2-a]pyrazin-6(7H)-one, (1S,8aS)-7,7-diallyl-1-(2,3-dimethylphenyl)hexahydropyrrolo[1,2-a]pyrazin-6(7H)-one, 1-(2-chloro-4-fluorophenyl)hexahydropyrrolo[1,2-a]pyrazin-6(7H)-one, (1S,8aS)-1-(4-fluorophenyl)hexahydropyrrolo[1,2-a]pyrazin-6(7H)-one.

The compounds of formula A have been found to act as potent antagonists of NK1R and are therefore potentially useful in the treatment of any disease or condition where Substance P is involved in the disease pathology, in particular in the prevention, treatment or amelioration of a pruritic dermal disease or condition.

It has surprisingly been found that some compounds of formula A exhibit a high metabolic clearance, i.e. are quickly degraded upon systemic administration, which makes them uniquely suitable for topical application with a favourable safety profile.

It has surprisingly been found that some compounds of general formula A exhibit a favourable ratio between the in vitro efficacy of the compound and the in vitro efficacy of metabolites of the compound, thus providing a favourable safety profile of the compounds.

It has surprisingly been found that some compounds of general formula A exhibit a mode of action indicating a competitive, non-surmountable antagonism, suggesting a slow dissociating antagonist-receptor complex.

It has surprisingly been found that some compounds of general formula A both exhibit a mode of action indicating a competitive, non-surmountable antagonism as well as exhibiting a favourable ratio between the in vitro efficacy of the compound and the in vitro efficacy of metabolites of the compound.

## DETAILED DESCRIPTION OF THE INVENTION

### *Definitions*

The term "alkyl" is intended to indicate a radical obtained when one hydrogen atom is removed from a hydrocarbon. Said alkyl may be branched or straight and may comprise 1-6, such as 1-4 carbon atoms, such as 1-3 carbon atoms, such as 1-2 carbon atoms, such as 2-3 carbon atoms. The term includes the subclasses normal alkyl (*n*-alkyl), secondary and tertiary alkyl, such as methyl, ethyl, *n*-propyl, isopropyl, *n*-butyl, isobutyl, sec.-butyl, *tert*.-butyl, pentyl, isopentyl, neopentyl and hexyl.

The term "$(C_{1-4})$alkyl" is intended to indicate a radical obtained when one hydrogen atom is removed from a hydrocarbon. Said alkyl may be branched or straight and may comprise 1-4, such as 1-3 carbon atoms, such as 1-2 carbon atoms, such as 2-3 carbon atoms. The term includes the subclasses normal alkyl (*n*-alkyl), secondary and tertiary alkyl, such as methyl, ethyl, *n*-propyl, isopropyl, *n*-butyl, isobutyl, *sec.*-butyl and *tert.*-butyl.

The term "aryl" is intended to indicate a radical of aromatic carbocyclic rings comprising 6-10 carbon atoms, such as 6 carbon atoms. Representative examples of aryl include, but are not limited to phenyl and naphthyl.

The term "halogen" is intended to indicate a substituent from the $7^{th}$ main group of the periodic table, such as fluoro, chloro, bromo and iodo.

The term "haloalkyl" is intended to indicate an alkyl radical as defined above substituted with one or more halogen atoms, e.g trifluoromethyl, difluoromethyl, fluoromethyl or trifluoroethyl.

The term "$(C_{1-4})$haloalkyl" is intended to indicate an alkyl radical as defined above wherein the alkyl group consists of 1-4 carbon atoms and wherein the alkyl group is substituted with one or more halogen atoms, e.g trifluoromethyl, difluoromethyl, fluoromethyl or trifluoroethyl.

The term "heteroaryl" is intended to indicate radicals of monocyclic heteroaromatic rings comprising 5- or 6-membered rings which contain from 1-5 carbon atoms and from 1-4 heteroatoms selected from oxygen, sulphur and nitrogen. The heteroaryl radical may be connected to the parent molecular moiety through a carbon atom or a nitrogen

atom contained anywhere within the heteroaryl group. Representative examples of 5-membered heteroaryl groups include, but are not limited to, furanyl, imidazolyl, isothiazolyl, isoxazolyl, oxadiazolyl, oxazolyl, pyrazolyl, pyrrolyl, tetrazolyl, thiadiazolyl, thiazolyl, thienyl and triazolyl. Representative examples of 6-membered heteroaryl groups include, but are not limited to, pyrazinyl, pyridazinyl, pyridyl and pyrimidinyl.

The term "hydroxyalkyl" is intended to indicate an alkyl group as defined above substituted with one or more hydroxyl-groups, e.g. hydroxymethyl, hydroxyethyl, hydroxypropyl.

The term "($C_{1-4}$)hydroxyalkyl" is intended to indicate an alkyl group as defined above, wherein the alkyl group consists of 1-4 carbon atoms and wherein the alkyl group is substituted with one or more hydroxyl-groups, e.g. hydroxymethyl, hydroxyethyl, hydroxypropyl.

The term "alkoxy" is intended to indicate a radical of the formula -OR', wherein R' is alkyl as indicated above, e.g. methoxy, ethoxy, n-propoxy, isopropoxy, butoxy, etc.

The term "($C_{1-4}$)alkoxy" is intended to indicate a radical of the formula -OR', wherein R' is alkyl as indicated above and wherein the alkyl group consists of 1-4 carbon atoms, e.g. methoxy, ethoxy, n-propoxy, isopropoxy, butoxy, *tert*-butoxy.

The tem "aliphatic ring" is intended to indicate a non-aromatic ring. The aliphatic ring may contain unsaturated bonds.

Ther term "2-methylphenyl" is intended to indicate a radical of the formula

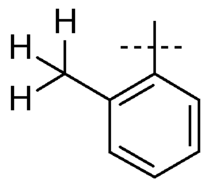

The term "oxo" is intended to indicate an oxygen atom which is connected to a carbon atom by a double bond, thereby forming a carbonyl group together with the carbon-atom to which it is connected.

The term "pharmaceutically acceptable salt" is intended to indicate salts prepared by reacting a compound of formula A or B with a suitable inorganic or organic acid, such as hydrochloric, hydrobromic, hydroiodic, sulfuric, nitric, phosphoric, formic, acetic, 2,2-dichloroaetic, adipic, ascorbic, L-aspartic, L-glutamic, galactaric, lactic, maleic, L-malic, phthalic, citric, propionic, benzoic, glutaric, gluconic, D-glucuronic, methanesulfonic, salicylic, succinic, malonic, tartaric, benzenesulfonic, ethane-1,2-disulfonic, 2-hydroxy ethanesulfonic acid, toluenesulfonic, sulfamic or fumaric acid.

The term "solvate" is intended to indicate a species formed by interaction between a compound, e.g. a compound of formula A, and a solvent, e.g. alcohol, glycerol or water, wherein said species are in a solid form. When water is the solvent, said species is referred to as a hydrate.

The term 'non-surmountable antagonist' or 'insurmountable antagonist' is intended to indicate an antagonist which produces shifts to the right of agonist concentration-response curves in the presence of increasing antagonist concentrations with depression of the maximal response to the agonist (ref. Kenakin, T. et. al., J. Pharm. Exp. Ther., (2006), 319, p. 710-723).

The term 'surmountable antagonist' is intended to indicate an antagonist which produces shifts to the right of agonist concentration-response curves in the presence of increasing antagonist concentrations with no concomitant diminution of the maximal response to the agonist. (ref. Kenakin, T. et. al., J. Pharm. Exp. Ther., (2006), 319, p. 710-723).

Chronic pruritus on diseased skin is intended to indicate pruritus in skin diseases which are accompanied by itch, such as inflammatory, infectious, or autoimmune cutaneous diseases, genodermatoses, drug reactions, dermatoses of pregnancy and skin lymphomas.

Genodermatoses are intended to include Darier's disease, Hailey-Hailey disease, ichthyoses, Sjögren-Larsson syndrome, EB pruriginosa.

Dermatoses of pregnancy are intended to include polymorphic eruption of pregnancy, pemphigoid gestationis, prurigo gestationis.

Skin lymphomas are intended to include cutaneous T-cell-lymphoma, cutaneous B-celllymphoma.

Pruritic diseases of systemic origin are intended to comprise diseases arising from diseases of organs other than the skin, such as liver, e.g. primary biliary cirrhosis; kidney,e.g. chronic renal failure; blood, e.g.Hodgkin's disease; and certain multifactorial, e.g.metabolic states or drugs.

Pruritic systemic endocrine and metabolic diseases are intended to comprise chronic renal failure, liver diseases with or without cholestasis, hyperthyroidism, malabsorption, perimenopausal pruritus.

Pruritic systemic infectious diseases are intended to comprise HIV-infection, helminthosis, Parasitosis.

Pruritic systemic haematological and lymphoproliferative diseases are intended to comprise iron deficiency, polycythaemia vera, Hodgkin's disease, Non-Hodgkin's lymphoma, plasmocytoma.

Chronic secondary scratch lesions are intended to indicate secondary acquired lesions induced by chronic scratching, such as for example lichen simplex chronicus, lichen Vidal, lichen amyloidosus, macular amyloidosus, and prurigo nodularis.

Embodiments of the present invention

In an embodiment the present invention relates to compounds of general formula A(i)

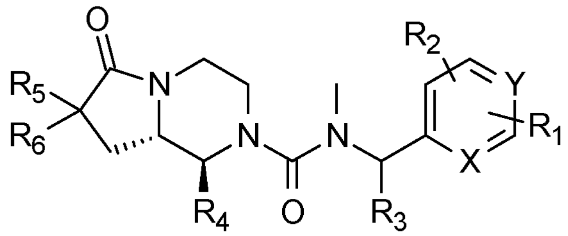

A(i)

wherein $R_1$, $R_2$, $R_3$, $R_4$, $R_5$, $R_6$, X and Y are as indicated above,
or a pharmaceutically acceptable salt or solvate thereof;
with the proviso that when both X and Y are CH, $R_4$ is not 2-methylphenyl.

In an embodiment the present invention relates to compounds of general formula A(ii)

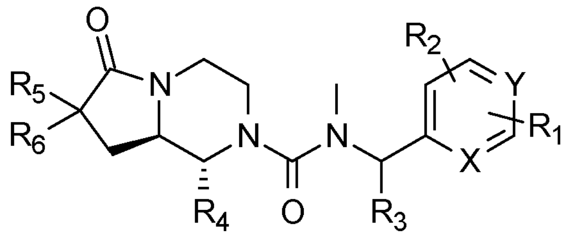

A(ii)

wherein $R_1$, $R_2$, $R_3$, $R_4$, $R_5$, $R_6$, X and Y are as indicated above,
or a pharmaceutically acceptable salt or solvate thereof;
with the proviso that when both X and Y are CH, $R_4$ is not 2-methylphenyl.

In an embodiment the present invention relates to compounds of general formula A, wherein $R_1$ and $R_2$ represent $(C_{1-4})$haloalkyl.

In an embodiment the present invention relates to compounds of general formula A, wherein $R_1$ represents $(C_{1-4})$haloalkyl and $R_2$ represents $(C_{1-4})$alkyl.

In an embodiment the present invention relates to compounds of general formula A, wherein $R_3$ is selected from the group consisting of hydrogen and $(C_{1-4})$alkyl.

In an embodiment the present invention relates to compounds of general formula A, wherein $R_5$ and $R_6$ independently are selected from the group consisting of hydrogen and $(C_{1-4})$hydroxyalkyl

In an embodiment the present invention relates to compounds of general formula A, wherein $R_7$ is selected from the group consisting of halogen and $(C_{1-4})$alkyl.

In an embodiment the present invention relates to compounds of general formula A, wherein X and Y represent CH.

In an embodiment the present invention relates to compounds of general formula A, wherein $R_4$ is phenyl, wherein said phenyl is substituted with two substituents independently selected from $R_7$.

In an embodiment the present invention relates to compounds of general formula A, selected from the group consisting of

N-((R)-1-(3,5-bis(trifluoromethyl)phenyl)ethyl)-1-(2,4-dimethylphenyl)-N-methyl-6-oxohexahydropyrrolo[1,2-a]pyrazine-2(1H)-carboxamide,
N-(3,5-bis(trifluoromethyl)benzyl)-1-(2,4-dimethylphenyl)-N-methyl-6-oxohexahydropyrrolo[1,2-a]pyrazine-2(1H)-carboxamide,
1-(2,4-dimethylphenyl)-N-methyl-N-((R)-1-(3-methyl-5-(trifluoromethyl)phenyl)ethyl)-6-oxohexahydropyrrolo[1,2-a]pyrazine-2(1H)-carboxamide,
N-((R)-1-(3,5-bis(trifluoromethyl)phenyl)ethyl)-1-(2,4-dimethylphenyl)-7,7-bis(2-hydroxyethyl)-N-methyl-6-oxohexahydropyrrolo[1,2-a]pyrazine-2(1H)-carboxamide, 1-(2,4-dimethylphenyl)-7,7-bis(2-hydroxyethyl)-N-methyl-N-((R)-1-(3-methyl-5-(trifluoromethyl)phenyl)ethyl)-6-oxo-hexahydropyrrolo[1,2-a]pyrazine-2(1H)-carboxamide,
N-(3,5-bis(trifluoromethyl)benzyl)-1-(2,4-dimethylphenyl)-7,7-bis(2-hydroxyethyl)-N-methyl-6-oxohexahydropyrrolo[1,2-a]pyrazine-2(1H)-carboxamide, *N*-((R)-1-(3-chloro-5-(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide,
1-(4-fluoro-2-methylphenyl)-7,7-bis(2-hydroxyethyl)-*N*-methyl-*N*-((R)-1-(3-methyl-5-(trifluoromethyl)phenyl)ethyl)-6-oxohexahydropyrrolo[1,2-a]pyrazine-2(1*H*)-carboxamide,
*N*-((R)-1-(3,5-bis(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide, N-((R)-1-(3,5-bis(trifluoromethyl)phenyl)ethyl)-7,7-bis(2-hydroxyethyl)-1-(4-methoxy-2-methylphenyl)-N-methyl-6-oxohexahydropyrrolo[1,2-a]pyrazine-2(1H)-carboxamide, (1S,8aS)-7,7-bis(2-hydroxyethyl)-1-(4-methoxy-2-methylphenyl)-N-methyl-N-((R)-1-(3-methyl-5-(trifluoromethyl)phenyl)ethyl)-6-oxohexahydropyrrolo[1,2-a]pyrazine-2(1H)-carboxamide,
*N*-((R)-1-(3,5-bis(trifluoromethyl)phenyl)ethyl)-1-(4-methoxy-2-methylphenyl)-*N*-methyl-6-oxohexahydropyrrolo[1,2-a]pyrazine-2(1*H*)-carboxamide, (1S,8aS)-N-((R)-1-(3,5-bis(trifluoromethyl)phenyl)ethyl)-1-(4-fluoro-2-methylphenyl)-N-methyl-6-oxohexahydropyrrolo[1,2-a]pyrazine-2(1H)-carboxamide, (1S,8aS)-1-(4-fluoro-2-methylphenyl)-N-methyl-N-((R)-1-(3-methyl-5-(trifluoromethyl) phenyl)ethyl)-6-oxohexahydropyrrolo[1,2-a]pyrazine-2(1H)-carboxamide,
(1S,8aS)-1-(4-fluoro-2-methylphenyl)-N-(1-(3-isopropyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3-ethyl-5-(trifluoromethyl)phenyl)ethyl)-1-(4-fluoro-2-methylphenyl)-N-methyl-6-oxohexahydropyrrolo[1,2-a]pyrazine-2(1H)-carboxamide, (1S,8aS)-N-((R)-1-(3-ethyl-5-(trifluoromethyl)phenyl)ethyl)-1-(4-fluoro-2-methylphenyl)-7,7-bis(2-hydroxyethyl)-N-methyl-6-oxohexahydropyrrolo[1,2-a]pyrazine-2(1H)-carboxamide,
(1S,8aS)-1-(4-fluoro-2-methylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide,
(1S,8aS)-1-(4-(benzyloxy)-2-methylphenyl)-N-((R)-1-(3,5-bis(trifluoromethyl)phenyl) ethyl)-N-methyl-6-oxohexahydropyrrolo[1,2-a]pyrazine-2(iH)-carboxamide, (1S,8aS)-N-((R)-1-(3,5-bis(trifluoromethyl)phenyl)ethyl)-1-(4-hydroxy-2-methylphenyl)-N-methyl-6-oxohexahydropyrrolo[1,2-a]pyrazine-2(1H)-carboxamide, (1S,8aS)-N-((R)-1-(3-ethyl-5-(trifluoromethyl)phenyl)ethyl)-1-(4-hydroxy-2-methylphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-7,7-bis(2-hydroxyethyl)-1-(4-methoxy-2-methylphenyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(4-hydroxy-2-methylphenyl)-7,7-bis(2-hydroxyethyl)-N-methyl-6-oxo-hexahydropyrrolo[1,2-a]pyrazine-2(1H)-carboxamide,
(1S,8aS)-N-((R)-1-(3-ethyl-5-(trifluoromethyl)phenyl)ethyl)-1-(4-hydroxy-2-methylphenyl)-7,7-bis(2-hydroxyethyl)-N-methyl-6-oxohexahydropyrrolo[1,2-a]pyrazine-2(1H)-carboxamide,
(1S,8aS)-N-((R)-1-(4-ethyl-6-(trifluoromethyl)pyridin-2-yl)ethyl)-N-methyl-6-oxo-1-o-tolylhexahydropyrrolo[1,2-a]pyrazine-2(1H)-carboxamide,
(1S,8aS)-7,7-bis(2-hydroxyethyl)-N-methyl-N-((4-methyl-6-(trifluoromethyl)pyridin-2-yl)methyl)-6-oxo-1-o-tolylhexahydropyrrolo[1,2-a]pyrazine-2(1H)-carboxamide, (1S,8aS)-N-((R)-1-(3-chloro-5-(trifluoromethyl)phenyl)ethyl)-1-(2,4-dimethylphenyl)-7,7-bis(2-hydroxyethyl)-N-methyl-6-oxo-hexahydropyrrolo[1,2-a]pyrazine-2(1H)-carboxamide,
(1S,8aS)-1-(2,4-dimethylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-7,7-bis(2-hydroxyethyl)-1-(2-methoxyphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2,5-dimethylphenyl)-7,7-bis(2-hydroxyethyl)-N-methyl-6-oxo-hexahydropyrrolo[1,2-a]pyrazine-2(1H)-carboxamide,
(1S,8aS)-1-(2,5-dimethylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(2,3-dimethylphenyl)-7,7-bis(2-hydroxyethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(2,3-dimethylphenyl)-N-methyl-6-oxo-hexahydropyrrolo[1,2-a]pyrazine-2(1H)-carboxamide, (1S,8aS)-1-(2,3-dimethylphenyl)-N-((R)-1-(3-ethyl-5-(trifluoromethyl)phenyl)ethyl)-N-methyl-6-oxo-hexahydropyrrolo[1,2-a]pyrazine-2(1H)-carboxamide, (1S,8aS)-1-(2,3-dimethylphenyl)-N-((R)-1-(3-isopropyl-5-trifluoromethyl)phenyl)ethyl)-N-methyl-6-oxo-hexahydropyrrolo[1,2-a]pyrazine-2(1H)-carboxamide,
(1R,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(4-methylpyridin-3-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(3-methylpyridin-4-yl)-6-oxohexahydropyrrolo[1,2-a]pyrazine-2(1H)-carboxamide, (1S,8aS)-N-((R)-1-(3,5-bis(trifluoromethyl)phenyl)ethyl)-N-methyl-1-(2-methylpyridin-3-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(4-ethylthiophen-3-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-N-methyl-1-(4-methylthiophen-3-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-1-(3-ethylthiophen-2-yl)-6-oxohexahydropyrrolo[1,2-a]pyrazine-2(1H)-carboxamide, (1R,8aS)-N-((R)-1-(3-ethyl-5-(trifluoromethyl)phenyl)ethyl)-N-methyl-1-(3-methylthiophen-2-yl)-6-oxohexahydropyrrolo[1,2-a]pyrazine-2(1H)-carboxamide, (1R,8aS)-N-methyl-N-((R)-1-(3-methyl-5-(trifluoromethyl)phenyl)ethyl)-1-(3-methylthiophen-2-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-6-oxo-1-(thiophen-2-yl)hexahydropyrrolo[1,2-a]pyrazine-2(1H)-carboxamide, (1R,8aS)-N-((R)-1-(3-ethyl-5-(trifluoromethyl)phenyl)ethyl)-N-methyl-6-oxo-1-(thiophen-2-yl)hexahydropyrrolo[1,2-a]pyrazine-2(1H)-carboxamide (1S,8aS)-N-methyl-N-((R)-1-(3-methyl-5-(trifluoromethyl)phenyl)ethyl)-1-(2-methylthiophen-3-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-N-methyl-1-(2-methylthiophen-3-yl)-6-oxohexahydropyrrolo[1,2-a]pyrazine-2(1H)-carboxamide, 1-(2-Methyl-thiophen-3-yl)-6-oxo-hexahydro-pyrrolo[1,2-a]pyrazine-2-carboxylic acid [1-(3,5-bis-trifluoromethyl-phenyl)-ethyl]-methyl-amide, N-((R)-1-(3,5-bis(trifluoromethyl)phenyl)ethyl)-N-methyl-1-(2-methylthiazol-5-yl)-6-oxohexahydropyrrolo[1,2-a]pyrazine-2(1H)-carboxamide, N-((R)-1-(3,5-bis(trifluoromethyl)phenyl)ethyl)-N-methyl-6-oxo-1-(thiazol-5-yl)-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(3-methyl-1H-pyrazol-4-yl)-6-oxohexahydropyrrolo[1,2-a]pyrazine-2(1H)-carboxamide (1S,8aS)-N-((R)-1-(3-ethyl-5-(trifluoromethyl)phenyl)ethyl)-N-methyl-1-(3-methyl-1H-pyrazol-4-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-7,7-bis(2-hydroxyethyl)-N-methyl-1-(3-methyl-1H-pyrazol-4-yl)-6-oxohexahydropyrrolo[1,2-a]pyrazine-2(1H)-carboxamide, (1S,8aS)-7,7-bis(2-hydroxyethyl)-N-methyl-1-(3-methyl-H-pyrazol-4-yl)-N-((R)-1-(3-methyl-5-(trifluoromethyl)phenyl)ethyl)-6-oxo-hexahydropyrrolo[1,2-a]pyrazine-2(1H)-carboxamide, (1S,8aS)-N-((R)-1-(3,5-bis(trifluoromethyl)phenyl)ethyl)-1-(1,3-dimethyl-1H-pyrazol-4-yl)-N-methyl-6-oxohexahydropyrrolo[1,2-a]pyrazine-2(1H)-carboxamide, (1S,8aS)-1-(2,3-dimethylphenyl)-N-((R)-1-(3-methyl-5-trifluoromethyl)phenyl)ethyl)-N-methyl-6-oxo-hexahydropyrrolo[1,2-a]pyrazine-2(1H)-carboxamide or a pharmaceutically acceptable salt or solvate thereof.

In an embodiment the present invention relates to compounds of general formula A, selected from the group consisting of (1S,8aS)-N-((R)-1-(3,5-bis(trifluoromethyl)phenyl)ethyl)-1-(3-chloro-2-methylphenyl)-N-methyl-6-oxohexahydropyrrolo[1,2-a]pyrazine-2(iH)-carboxamide, N-((R)-1-(3,5-bis(trifluoromethyl)phenyl)ethyl)-1-(2-ethylphenyl)-N-methyl-6-oxohexahydropyrrolo[1,2-a]pyrazine-2(1H)-carboxamide, N-((R)-1-(3-ethyl-5-(trifluoromethyl)phenyl)ethyl)-1-(2-ethylphenyl)-N-methyl-6-oxohexahydropyrrolo[1,2-a]pyrazine-2(1H)-carboxamide, N-((R)-1-(3,5-bis(trifluoromethyl)phenyl)ethyl)-1-(2-chlorophenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2-(hydroxymethyl)phenyl)-N-methyl-6-oxohexahydropyrrolo[1,2-a]pyrazine-2(1H)-carboxamide, (1R,8aS)-N-((R)-1-(3,5-bis(trifluoromethyl)phenyl)ethyl)-1-(3,4-dimethylthiophen-2-yl)-N-methyl-6-oxohexahydropyrrolo[1,2-a]pyrazine-2(1H)-carboxamide, (1R,8aS)-1-(3,4-dimethylthiophen-2-yl)-N-((R)-1-(3-ethyl-5-(trifluoromethyl)phenyl)ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(3-methoxy-2-methylphenyl)-N-methyl-6-oxohexahydropyrrolo[1,2-a]pyrazine-2(1H)-carboxamide, (1S,8aS)-N-((R)-1-(3-isopropyl-5-(trifluoromethyl)phenyl)ethyl)-1-(3-methoxy-2-methylphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2,3-dihydro-1H-inden-4-yl)-N-methyl-6-oxohexahydropyrrolo[1,2-a]pyrazine-2(1H)-carboxamide, (1S,8aS)-1-(2,3-dihydro-1H-inden-4-yl)-N-((R)-1-(3-ethyl-5-(trifluoromethyl)phenyl)ethyl)-N-methyl-6-oxohexahydropyrrolo[1,2-a]pyrazine-2(1H)-carboxamide, (1S,8aS)-1-(2,3-dihydro-1H-inden-4-yl)-N-((R)-1-(3-isopropyl-5-(trifluoromethyl)phenyl)ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2-fluoro-6-methylphenyl)-N-methyl-6-oxohexahydropyrrolo[1,2-a]pyrazine-2(1H)-carboxamide, N-((R)-1-(3,5-bis(trifluoromethyl)phenyl)ethyl)-1-(2-chloro-4-fluorophenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(4-fluorophenyl)-N-methyl-6-oxohexahydropyrrolo[1,2-a]pyrazine-2(1H)-carboxamide
or a pharmaceutically acceptable salt or solvate thereof.

In an embodiment the present invention relates to compounds of general formula A, selected from the group consisting of
N-((R)-1-(3,5-bis(trifluoromethyl)phenyl)ethyl)-1-(2,4-dimethylphenyl)-N-methyl-6-oxohexahydropyrrolo[1,2-a]pyrazine-2(1H)-carboxamide,
N-(3,5-bis(trifluoromethyl)benzyl)-1-(2,4-dimethylphenyl)-N-methyl-6-oxohexahydropyrrolo[1,2-a]pyrazine-2(1H)-carboxamide, 1-(2,4-dimethylphenyl)-N-methyl-N-((R)-1-(3-methyl-5-(trifluoromethyl)phenyl)ethyl)-6-oxohexahydropyrrolo[1,2-a]pyrazine-2(1H)-carboxamide,
N-((R)-1-(3,5-bis(trifluoromethyl)phenyl)ethyl)-1-(2,4-dimethylphenyl)-7,7-bis(2-hydroxyethyl)-N-methyl-6-oxohexahydropyrrolo[1,2-a]pyrazine-2(1H)-carboxamide, 1-(2,4-dimethylphenyl)-7,7-bis(2-hydroxyethyl)-N-methyl-N-((R)-1-(3-methyl-5-(trifluoromethyl)phenyl)ethyl)-6-oxo-hexahydropyrrolo[1,2-a]pyrazine-2(1H)-carboxamide, N-(3,5-bis(trifluoromethyl)benzyl)-1-(2,4-dimethylphenyl)-7,7-bis(2-hydroxyethyl)-N-methyl-6-oxohexahydropyrrolo[1,2-a]pyrazine-2(1H)-carboxamide, *N*-((R)-1-(3-chloro-5-(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide,
1-(4-fluoro-2-methylphenyl)-7,7-bis(2-hydroxyethyl)-*N*-methyl-*N*-((R)-1-(3-methyl-5-(trifluoromethyl)phenyl)ethyl)-6-oxohexahydropyrrolo[1,2-a]pyrazine-2(1*H*)-carboxamide,
*N*-((R)-1-(3,5-bis(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide, N-((R)-1-(3,5-bis(trifluoromethyl)phenyl)ethyl)-7,7-bis(2-hydroxyethyl)-1-(4-methoxy-2-methylphenyl)-N-methyl-6-oxohexahydropyrrolo[1,2-a]pyrazine-2(1H)-carboxamide, (1S,8aS)-7,7-bis(2-hydroxyethyl)-1-(4-methoxy-2-methylphenyl)-N-methyl-N-((R)-1-(3-methyl-5-(trifluoromethyl)phenyl)ethyl)-6-oxohexahydropyrrolo[1,2-a]pyrazine-2(1H)-carboxamide, *N*-((R)-1-(3,5-bis(trifluoromethyl)phenyl)ethyl)-1-(4-methoxy-2-methylphenyl)-*N*-methyl-6-oxohexahydropyrrolo[1,2-a]pyrazine-2(1*H*)-carboxamide, (1S,8aS)-N-((R)-1-(3,5-bis(trifluoromethyl)phenyl)ethyl)-1-(4-fluoro-2-methylphenyl)-N-methyl-6-oxohexahydropyrrolo[1,2-a]pyrazine-2(1H)-carboxamide, (1S,8aS)-1-(4-fluoro-2-methylphenyl)-N-methyl-N-((R)-1-(3-methyl-5-(trifluoromethyl) phenyl)ethyl)-6-oxohexahydropyrrolo[1,2-a]pyrazine-2(1H)-carboxamide, (1S,8aS)-1-(4-fluoro-2-methylphenyl)-N-(1-(3-isopropyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3-ethyl-5-(trifluoromethyl)phenyl)ethyl)-1-(4-fluoro-2-methylphenyl)-N-methyl-6-oxohexahydropyrrolo[1,2-a]pyrazine-2(1H)-carboxamide, (1S,8aS)-N-((R)-1-(3-ethyl-5-(trifluoromethyl)phenyl)ethyl)-1-(4-fluoro-2-methylphenyl)-7,7-bis(2-hydroxyethyl)-N-methyl-6-oxohexahydropyrrolo[1,2-a]pyrazine-2(1H)-carboxamide,
(1S,8aS)-1-(4-fluoro-2-methylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide,
(1S,8aS)-1-(4-(benzyloxy)-2-methylphenyl)-N-((R)-1-(3,5-bis(trifluoromethyl)phenyl) ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(4-hydroxy-2-methyl phenyl)-N-methyl-6-oxohexahydropyrrolo[1,2-a]pyrazine-2(1H)-carboxamide, (1S,8aS)-N-((R)-1-(3-ethyl-5-(trifluoromethyl)phenyl)ethyl)-1-(4-hydroxy-2-methylphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-7,7-bis(2-hydroxyethyl)-1-(4-methoxy-2-methylphenyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(4-hydroxy-2-methylphenyl)-7,7-bis(2-hydroxyethyl)-N-methyl-6-oxo-hexahydropyrrolo[1,2-a]pyrazine-2(1H)-carboxamide,
(1S,8aS)-N-((R)-1-(3-ethyl-5-(trifluoromethyl)phenyl)ethyl)-1-(4-hydroxy-2-methylphenyl)-7,7-bis(2-hydroxyethyl)-N-methyl-6-oxohexahydropyrrolo[1,2-a]pyrazine-2(1H)-carboxamide,
(1S,8aS)-N-((R)-1-(4-ethyl-6-(trifluoromethyl)pyridin-2-yl)ethyl)-N-methyl-6-oxo-1-o-tolylhexahydropyrrolo[1,2-a]pyrazine-2(1H)-carboxamide,
(1S,8aS)-7,7-bis(2-hydroxyethyl)-N-methyl-N-((4-methyl-6-(trifluoromethyl)pyridin-2-yl)methyl)-6-oxo-1-o-tolylhexahydropyrrolo[1,2-a]pyrazine-2(1H)-carboxamide, (1S,8aS)-N-((R)-1(3-chloro-5-(trifluoromethyl)phenyl)ethyl)-1-2,4-dimethylphenyl)-7,7-bis(2-hydroxyethyl)-N-methyl-6-oxo-hexahydropyrrolo[1,2-a]pyrazine-2(1H)-carboxamide,
(1S,8aS)-1-(2,4-dimethylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-7,7-bis(2-hydroxyethyl)-1-(2-methoxyphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2,5-dimethylphenyl)-7,7-bis(2-hydroxyethyl)-N-methyl-6-oxo-hexahydropyrrolo[1,2-a]pyrazine-2(1H)-carboxamide,
(1S,8aS)-1-(2,5-dimethylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(2,3-dimethylphenyl)-7,7-bis(2-hydroxyethyl)-N-methyl-6-oxohexahydropyrrolo[1,2-a]pyrazine-2(1H)-carboxamide,

(1S,8aS)-N-((R)-1-(3,5-bis(trifluoromethyl)phenyl)ethyl)-1-(2,3-dimethylphenyl)-N-methyl-6-oxo-hexahydropyrrolo[1,2-a]pyrazine-2(1H)-carboxamide, (1S,8aS)-1-(2,3-dimethylphenyl)-N-((R)-1-(3-ethyl-5-(trifluoromethyl)phenyl)ethyl)-N-methyl-6-oxo-hexahydropyrrolo[1,2-a]pyrazine-2(1H)-carboxamide, (1S,8aS)-1-(2,3-dimethylphenyl)-N-((R)-1-(3-isopropyl-5-trifluoromethyl)phenyl)ethyl)-N-methyl-6-oxo-hexahydropyrrolo[1,2-a]pyrazine-2(1H)-carboxamide, (1R,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(4-methylpyridin-3-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(3-methylpyridin-4-yl)-6-oxohexahydropyrrolo[1,2-a]pyrazine-2(1H)-carboxamide, (1S,8aS)-N-((R)-1-(3,5-bis(trifluoromethyl)phenyl)ethyl)-N-methyl-1-(2-methylpyridin-3-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(4-ethylthiophen-3-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-N-methyl-1-(4-methylthiophen-3-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-1-(3-ethylthiophen-2-yl)-6-oxohexahydropyrrolo[1,2-a]pyrazine-2(1H)-carboxamide, (1R,8aS)-N-((R)-1-(3-ethyl-5-(trifluoromethyl)phenyl)ethyl)-N-methyl-1-(3-methylthiophen-2-yl)-6-oxohexahydropyrrolo[1,2-a]pyrazine-2(iH)-carboxamide, (1R,8aS)-N-methyl-N-((R)-1-(3-methyl-5-(trifluoromethyl)phenyl)ethyl)-1-(3-methylthiophen-2-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-6-oxo-1-(thiophen-2-yl)hexahydropyrrolo[1,2-a]pyrazine-2(1H)-carboxamide, (1R,8aS)-N-((R)-1-(3-ethyl-5-(trifluoromethyl)phenyl)ethyl)-N-methyl-6-oxo-1-(thiophen-2-yl)hexahydropyrrolo[1,2-a]pyrazine-2(1H)-carboxamide (1S,8aS)-N-methyl-N-((R)-1-(3-methyl-5-(trifluoromethyl)phenyl)ethyl)-1-(2-methylthiophen-3-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-N-methyl-1-(2-methylthiophen-3-yl)-6-oxohexahydropyrrolo[1,2-a]pyrazine-2(1H)-carboxamide, (1S,8aS)-N-((R)-1-(3,5-bis(trifluoromethyl)phenyl)ethyl)-N-methyl-1-(2-methylthiophen-3-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-1-(2-methylthiazol-5-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-6-oxo-1-(thiazol-5-yl)-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(3-methyl-1H-pyrazol-4-yl)-6-oxohexahydropyrrolo[1,2-a]pyrazine-2(1H)-carboxamide (1S,8aS)-N-((R)-1-(3-ethyl-5-(trifluoromethyl)phenyl)ethyl)-N-methyl-1-(3-methyl-1H-pyrazol-4-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-7,7-bis(2-hydroxyethyl)-N-methyl-1-(3-methyl-1H-pyrazol-4-yl)-6-oxohexahydropyrrolo[1,2-a]pyrazine-2(1H)-carboxamide, (1S,8aS)-7,7-bis(2-hydroxyethyl)-N-methyl-1-(3-methyl-1H-pyrazol-4-yl)-N-((R)-1-(3-methyl-5-(trifluoromethyl)phenyl)ethyl)-6-oxo-hexahydropyrrolo[1,2-a]pyrazine-2(1H)-carboxamide, (1S,8aS)-N-((R)-1-(3,5-bis(trifluoromethyl)phenyl)ethyl)-1-(1,3-dimethyl-1H-pyrazol-4-yl)-N-methyl-6-oxohexahydropyrrolo[1,2-a]pyrazine-2(1H)-carboxamide, (1S,8aS)-1-(2,3-dimethylphenyl)-N-((R)-1-(3-methyl-5-trifluoromethyl)phenyl)ethyl)-N-methyl-6-oxo-hexahydropyrrolo[1,2-a]pyrazine-2(1H)-carboxamide, (1S,8aS)-N-((R)-1-(3,5-bis(trifluoromethyl)phenyl)ethyl)-1-(3-chloro-2-methylphenyl)-N-methyl-6-oxohexahydropyrrolo[1,2-a]pyrazine-2(1H)-carboxamide, N-((R)-1-(3,5-bis(trifluoromethyl)phenyl)ethyl)-1-(2-ethylphenyl)-N-methyl-6-oxohexahydropyrrolo[1,2-a]pyrazine-2(1H)-carboxamide, N-((R)-1-(3-ethyl-5-(trifluoromethyl)phenyl)ethyl)-1-(2-ethylphenyl)-N-methyl-6-oxohexahydropyrrolo[1,2-a]pyrazine-2(1H)-carboxamide, N-((R)-1-(3,5-bis(trifluoromethyl)phenyl)ethyl)-1-(2-chlorophenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2-(hydroxymethyl)phenyl)-N-methyl-6-oxohexahydropyrrolo[1,2-a]pyrazine-2(1H)-carboxamide, (1R,8aS)-N-((R)-1-(3,5-bis(trifluoromethyl)phenyl)ethyl)-1-(3,4-dimethylthiophen-2-yl)-N-methyl-6-oxohexahydropyrrolo[1,2-a]pyrazine-2(1H)-carboxamide, 1R,(8aS)-1-(3,4-dimethylthiophen-2-yl)-N-((R)-1-(3-ethyl-5-(trifluoromethyl)phenyl)ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(3-methoxy-2-methylphenyl)-N-methyl-6-oxohexahydropyrrolo[1,2-a]pyrazine-2(1H)-carboxamide, (1S,8aS)-N-((R)-1-(3-isopropyl-5-(trifluoromethyl)phenyl)ethyl)-1-(3-methoxy-2-methylphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2,3-dihydro-1H-inden-4-yl)-N-methyl-6-oxohexahydropyrrolo[1,2-a]pyrazine-2(1H)-carboxamide, (1S,8aS)-1-(2,3-dihydro-1H-inden-4-yl)-N-((R)-1-(3-ethyl-5-(trifluoromethyl)phenyl)ethyl)-N-methyl-6-oxo-hexahydropyrrolo[1,2-a]pyrazine-2(1H)-carboxamide, (1S,8aS)-1-(2,3-dihydro-1H-inden-4-yl)-N-((R)-1-(3-isopropyl-5-(trifluoromethyl)phenyl)ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2-fluoro-6-methylphenyl)-N-methyl-6-oxohexahydropyrrolo[1,2-a]pyrazine-2(1H)-carboxamide, N-((R)-1-(3,5-bis(trifluoromethyl)phenyl)ethyl)-1-(2-chloro-4-fluorophenyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(4-fluorophenyl)-N-methyl-6-oxohexahydropyrrolo[1,2-a]pyrazine-2(1H)-carboxamide
or a pharmaceutically acceptable salt or solvate thereof.

In an embodiment the present invention relates to compounds of general formula A, wherein $R_1$ and $R_2$ independently are selected from the group consisting of trifluoromethyl, methyl, ethyl, isopropyl and chloro.

In an embodiment the present invention relates to compounds of general formula A, wherein $R_1$ represents trifluoromethyl and wherein $R_2$ is selected from the group consisting of methyl, ethyl, isopropyl and chloro.

In an embodiment the present invention relates to compounds of general formula A, wherein $R_3$ is selected from the group consisting of hydrogen and methyl.

In an embodiment the present invention relates to compounds of general formula A, wherein $R_4$ is selected from the group consisting of phenyl, pyridyl, thiophenyl, thiazolyl and pyrazolyl.

In an embodiment the present invention relates to compounds of general formula A, wherein $R_5$ and $R_6$ independently are selected from the group consisting of hydrogen and 2-hydroxyethyl.

In an embodiment the present invention relates to compounds of general formula A, wherein $R_7$ is selected from the group consisting of methyl, fluoro, methoxy, benzyloxy and hydroxy.

In an embodiment the present invention relates to compounds of general formula A, wherein X and Y represent CH.

In an embodiment the present invention relates to compounds of general formula A, wherein X represents N and Y represents CH.

In an embodiment the present invention relates to compounds of general formula A, wherein $R_4$ is substituted with two substituents independently selected from $R_7$.

In an embodiment the present invention relates to compounds of general formula A, wherein $R_4$ is substituted with two adjacent substituents independently selected from $(C_{1-4})$alkyl, and wherein said substituents together with the phenyl, 5-membered or 6-membered heteroaryl to which they are attached form a 4-7 membered aliphatic ring. Representative examples of such bicyclic $R_4$'s include, but are not limited to indanyl and tetrahydronaphtyl.

In an embodiment the present invention relates to compounds of general formula A(iii),

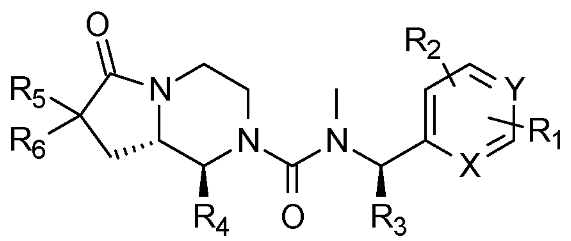

A(iii)

wherein $R_1$, $R_2$, $R_3$, $R_4$, $R_5$, $R_6$, X and Y are as indicated above,
or a pharmaceutically acceptable salt or solvate thereof,
with the proviso that when both X and Y are CH, $R_4$ is not 2-methylphenyl.

In an embodiment the present invention relates to compounds of general formula A, the compounds being selected from 1-(2,4-dimethylphenyl)-7,7-bis(2-hydroxyethyl)-N-methyl-N-((R)-1-(3-methyl-5-(trifluoromethyl)phenyl)ethyl)-6-oxo-hexahydropyrrolo[1,2-a]pyrazine-2(1H)-carboxamide or a pharmaceutically acceptable salt or solvate thereof.

In an embodiment the present invention relates to compounds of general formula A, the compounds being selected from (1S,8aS)-N-((R)-1-(3,5-bis(trifluoromethyl)phenyl)ethyl)-1-(2,3-dimethylphenyl)-7,7-bis(2-hydroxyethyl)-N-methyl-6-oxohexahydropyrrolo[1,2-a]pyrazine-2(1H)-carboxamide or a pharmaceutically acceptable salt or solvate thereof.

In an embodiment the present invention relates to compounds of general formula A, the compounds being selected from (1S,8aS)-N-((R)-1-(3,5-bis(trifluoromethyl)phenyl)ethyl)-1-(2,3-dimethylphenyl)-N-methyl-6-oxo-hexahydropyrrolo[1,2-a]pyrazine-2(1H)-carboxamide or a pharmaceutically acceptable salt or solvate thereof.

In an embodiment the present invention relates to compounds of general formula A, the compounds being selected from (1S,8aS)-1-(2,3-dimethylphenyl)-N-((R)-1-(3-methyl-5-trifluoromethyl)phenyl)ethyl)-N-methyl-6-oxo-hexahydropyrrolo[1,2-a]pyrazine-2(1H)-carboxamide or a pharmaceutically acceptable salt or solvate thereof.

In an embodiment the present invention relates to compounds of general formula A, the compounds being

selected from (1S,8aS)-N-((R)-1-(3,5-bis(trifluoromethyl)phenyl)ethyl)-N-methyl-1-(3-methylpyridin-4-yl)-6-oxohexahydropyrrolo[1,2-a]pyrazine-2(1H)-carboxamide or a pharmaceutically acceptable salt or solvate thereof.

In an embodiment the present invention relates to compounds of general formula A, the compounds being selected from (1S,8aS)-N-((R)-1-(3,5-bis(trifluoromethyl)phenyl)ethyl)-N-methyl-1-(4-methylpyridin-3-yl)-6-oxo-hexahydropyrrolo[1,2-a]pyrazine-2(1H)-carboxamide or a pharmaceutically acceptable salt or solvate thereof.

In an embodiment the present invention relates to compounds of general formula A, the compounds being selected from (1R,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide or a pharmaceutically acceptable salt or solvate thereof.

In an embodiment the present invention relates to compounds of general formula A, the compounds being selected from or (1S,8aS)-1-(2,3-dimethylphenyl)-N-((R)-1-(3-isopropyl-5-trifluoromethyl)phenyl)ethyl)-N-methyl-6-oxo-hexahydropyrrolo[1,2-a]pyrazine-2(1H)-carboxamide a pharmaceutically acceptable salt or solvate thereof.

In an embodiment the present invention relates to compounds of general formula A, the compounds being selected from (1S,8aS)-1-(2,3-dimethylphenyl)-N-((R)-1-(3-ethyl-5-(trifluoromethyl)phenyl)ethyl)-N-methyl-6-oxo-hexahydropyrrolo[1,2-a]pyrazine-2(1H)-carboxamide or a pharmaceutically acceptable salt or solvate thereof.

The compounds of formula A may be obtained in crystalline form either directly by concentration from an organic solvent or by crystallisation or recrystallisation from an organic solvent or mixture of said solvent and a cosolvent that may be organic or inorganic, such as water. The crystals may be isolated in essentially solvent-free form or as a solvate, such as a hydrate. The invention covers all crystalline modifications and forms and also mixtures thereof.

Compounds of formula A may comprise asymmetrically substituted (chiral) carbon atoms which give rise to the existence of isomeric forms, e.g. enantiomers and possibly diastereomers. The present invention relates to all such isomers, either in pure form or as mixtures thereof (e.g. racemates). Pure stereoisomeric forms of the compounds and the intermediates of this invention may be obtained by the application of procedures known in the art. The various isomeric forms may be separated by physical separation methods such as selective crystallization and chromatographic techniques, e.g. liquid chromatography using chiral stationary phases. Enantiomers may be separated from each other by the selective crystallization of their diastereomeric salts with optically active acids. Alternatively, enantiomers may be separated by chromatographic techniques using chiral stationary phases. Said pure stereoisomeric forms may also be derived from the corresponding pure stereoisomeric forms of the appropriate starting materials, provided that the reaction occurs stereoselectively or stereospecifically. Preferably, if a specific stereoisomer is desired, said compound will be synthesized by stereoselective or stereospecific methods of preparation. These methods will advantageously employ chiral pure starting materials.

In an embodiment the present invention relates to intermediates for the preparation of a compound according to general formula A, selected from the list consisting of 1-(2,4-dimethylphenyl)hexahydropyrrolo[1,2-a]pyrazin-6(7H)-one, 7,7-diallyl-1-(2,4-dimethylphenyl)hexahydropyrrolo[1,2-a]pyrazin-6(7H)-one, 7,7-diallyl-1-(4-fluoro-2-methylphenyl)hexahydropyrrolo[1,2-a]pyrazin-6(7H)-one, 1-(4-methoxy-2-methylphenyl)hexahydropyrrolo[1,2-a]pyrazin-6(7H)-one, 1-(4-fluoro-2-methylphenyl)hexahydropyrrolo[1,2-a]pyrazin-6(7H)-one, 1-(4-methoxy-2-methylphenyl)hexahydropyrrolo[1,2-a]pyrazin-6(7H)-one, 7,7-diallyl-1-(4-methoxy-2-methylphenyl)hexahydropyrrolo[1,2-a]pyrazin-6(7H)-one, 1-(4-(benzyloxy)-2-methylphenyl)hexahydropyrrolo[1,2-a]pyrazin-6(*7H*)-one, 7,7-diallyl-1-(4-(benzyloxy)-2-methylphenyl)hexahydropyrrolo[1,2-a]pyrazin-6(7H)-one, tert-butyl7,7-diallyl-1-(2,5-dimethylphenyl)-6-oxo-hexahydropyrrolo[1,2-a]pyrazine-2(1H)-carboxylate, 7,7-diallyl-1-(2,3-dimethylphenyl)hexahydropyrrolo[1,2-a]pyrazin-6(7H)-one, 1-(2,3-dimethylphenyl)-hexahydropyrrolo[1,2-a]pyrazin-6(7H)-one, 1-(3-methylpyridin-2-yl)-hexahydropyrrolo[1,2-a]pyrazin-6(7H)-one, 1-(4-methylpyridin-3-yl)hexahydropyrrolo[1,2-a]pyrazin-6(7H)-one, 1-(3-methylpyridin-4-yl)hexahydropyrrolo[1,2-a]pyrazin-6(7H)-one, 1-(2-methylpyridin-3-yl)hexahydropyrrolo[1,2-a]pyrazin-6(7H)-one, 1-(4-methylthiophen-3-yl)hexahydropyrrolo[1,2-a]pyrazin-6(7H)-one, 1-(3-methylthiophen-2-yl)hexahydropyrrolo[1,2-a]pyrazin-6(7H)-one, 1-(thiophen-2-yl)hexahydropyrrolo[1,2-a]pyrazin-6(7H)-one, 1-(2-methylthiophen-3-yl)hexahydropyrrolo[1,2-a]pyrazin-6(7H)-one, 1-(2-methylthiazol-5-yl)hexahydropyrrolo[1,2-a]pyrazin-6(7H)-one, 1-(thiazol-5-yl)hexahydropyrrolo[1,2-a]pyrazin-6(7H)-one, 1-(1-benzyl-3-methyl-1H-pyrazol-4-yl)hexahydropyrrolo[1,2-a]pyrazin-6(7H)-one, 7,7-diallyl-1-(1-benzyl-3-methyl-1H-pyrazol-4-yl)hexahydropyrrolo[1,2-a]pyrazin-6(7H)-one.

In an embodiment the present invention relates to intermediates for the preparation of a compound according to general formula A, selected from the list consisting of (1S,8aS)-1-(3-chloro-2-methylphenyl)hexahydropyrrolo[1,2-a]pyrazin-6(2H)-one, (1S,8aS)-1-(2-ethylphenyl)hexahydropyrrolo[1,2-a]pyrazin-6(7H)-one, (1S,8aS)-1-(2-chlorophenyl)hexahydropyrrolo[1,2-a]pyrazin-6(7H)-one, (1S, 8aS)-1-(2-vinylphenyl)hexahydropyrrolo[1,2-a]pyrazin-6(7H)-one, (8aS)-1-(3,4-dimethylthiophen-2-yl)hexahydropyrrolo[1,2-a]pyrazin-6(7H)-one, (1S,8aS)-1-(3-methoxy-2-methylphenyl)hexahydropyrrolo[1,2-a]pyrazin-6(7H)-one, (1S,8aS)-1-(2,3-dihydro-1H-inden-4-yl)hexahydropyrrolo[1,2-a]pyrazin-6(7H)-one, (1S,8aS)-7,7-diallyl-1-(2,3-dimethylphenyl)hexahydropyrrolo[1,2-a]pyrazin-6(7H)-one, 1-(2-chloro-4-fluorophenyl)hexahydropyrrolo[1,2-a]pyrazin-6(7H)-one, (1S,8aS)-1-(4-fluorophenyl)hexahydropyrrolo[1,2-a]pyrazin-6(7H)-one.

An embodiment of the invention is a compound according to general formula A for use in the prevention, treatment or amelioration of pruritic skin conditions ,wherein the pruritic skin condition is selected from prurigo , lichen

planus, atopic dermatitis, eczema, contact dermatitis, allergic dermatitis, nummular dermatitis, lichen simplex, psoriasis, Sézary syndrome, cutaneous lymphomas, urticaria, mastocytosis and pruritus with chronic secondary scratch lesions.

An embodiment of the invention is a pharmaceutical composition comprising, comprising as a therapeutically active ingredient, a compound according to general formula A and a pharmaceutically acceptable carrier or vehicle, together with one or more other therapeutically active compound(s).

An embodiment of the invention is a pharmaceutical composition comprising, comprising as a therapeutically active ingredient, a compound according to general formula A and a pharmaceutically acceptable carrier or vehicle, together with one or more other therapeutically active compound(s), suitable for topical administration.

An embodiment of the disclosure is a method of preventing, treating or ameliorating a condition involving pruritus of the skin, the method comprising applying, on the skin of a patient in need thereof, a therapeutically effective amount of a compound according to general formula A, optionally together with a pharmaceutically acceptable carrier or one or more excipients, optionally in combination with other therapeutically active compounds, wherein the condition is selected from the group consisting of acute pruritus in any condition; chronic pruritus on diseased skin, such as inflammatory, infectious, or autoimmune cutaneous diseases, genodermatoses, drug reactions, dermatoses of pregnancy and skin lymphomas, prurigo , lichen planus, atopic dermatitis, eczema, contact dermatitis, allergic dermatitis, nummular dermatitis, lichen simplex, psoriasis, Sézary syndrome, cutaneous lymphomas, bullous pemphigoid, alopecia areata, scabies, vitiligo, urticaria and drug-induced pruritus; pruritic diseases on non-diseased skin of systemic, neurological or psychosomatic/psychiatric origin, including endocrine and metabolic disorders, infections, haematological and lymphoproliferative diseases, solid neoplasms and drug-induced pruritus; mastocytosis; pruritus of unknown cause; pruritus with chronic secondary scratch lesions, such as prurigo nodularis, and all types of prurigo; or any other dermal disease or condition characterized by pruritus.

An embodiment of the invention relates to the use of a compound according to general formula A in the manufacture of a medicament for the prevention, treatment or amelioration of pruritic skin conditions , e.g. acute pruritus in any condition; chronic pruritus on diseased skin, such as inflammatory, infectious, or autoimmune cutaneous diseases, genodermatoses, drug reactions, dermatoses of pregnancy and skin lymphomas, prurigo , lichen planus, atopic dermatitis, eczema, contact dermatitis, allergic dermatitis, nummular dermatitis, lichen simplex, psoriasis, Sézary syndrome, cutaneous lymphomas, bullous pemphigoid, alopecia areata, scabies, vitiligo, urticaria and drug-induced pruritus; pruritic diseases on non-diseased skin of systemic, neurological or psychosomatic/psychiatric origin, including endocrine and metabolic disorders, infections, haematological and lymphoproliferative diseases, solid neoplasms and drug-induced pruritus; mastocytosis; pruritus of unknown cause; pruritus with chronic secondary scratch lesions, such as prurigo nodularis, and all types of prurigo; or any other dermal disease or condition characterized by pruritus.

An embodiment of the invention relates to a pharmaceutical composition comprising, as a therapeutically active ingredient, a compound according to general formula A and a pharmaceutically acceptable carrier or vehicle.

An embodiment of the invention relates to a pharmaceutical composition suitable for topical administration comprising, comprising as a therapeutically active ingredient, a compound according to general formula A and a pharmaceutically acceptable carrier or vehicle.

An embodiment of disclosure is a method of preventing, treating or ameliorating a condition involving pruritus of the skin, the method comprising applying, on the skin of a patient in need thereof, a therapeutically effective amount of a compound according to general formula A.

Besides being useful for human treatment, the compounds of the present invention may also be useful for veterinary treatment of animals including mammals such as horses, cattle, sheep, pigs, dogs, and cats.

PHARMACEUTICAL COMPOSITIONS OF THE INVENTION

For use in therapy, compounds of the present invention are typically in the form of a pharmaceutical composition. The invention therefore relates to a pharmaceutical composition comprising a compound of formula A, optionally together with one or more other therapeutically active compound(s), together with a pharmaceutically acceptable excipient or vehicle. The excipient must be "acceptable" in the sense of being compatible with the other ingredients of the composition and not deleterious to the recipient thereof.

Conveniently, the active ingredient comprises from 0.05-99.9% by weight of the formulation.

In the form of a dosage unit, the compound may be administered one or more times a day at appropriate intervals, always depending, however, on the condition of the patient, and in accordance with the prescription made by the medical practitioner. Conveniently, a dosage unit of a formulation contain between 0.1 mg and 1000 mg, preferably between 1 mg and 100 mg, such as 5-50 mg of a compound of formula A.

A suitable dosage of the compound of the invention will depend, inter alia, on the age and condition of the patient, the severity of the disease to be treated and other factors well known to the practising physician. The compound may be administered orally, parenterally or topically according to different dosing schedules, e.g. daily or with weekly intervals. In general a single dose will be in the range from 0.01 to 400 mg/kg body weight. The compound may be administered

as a bolus (i.e. the entire daily dosis is administered at once) or in divided doses two or more times a day.

In the context of topical treatment it may be more appropriate to refer to a "usage unit", which denotes a single dose which is capable of being administered to a patient, and which may be readily handled and packed, remaining as a physically and chemically stable unit dose comprising either the active material as such or a mixture of it with solid or liquid pharmaceutical diluents or carriers.

The term "usage unit" in connection with topical use means a unitary, i.e. a single dose capable of being administered topically to a patient in an application per square centimetre of the infected area of from 0.1 mg to 10 mg and preferably from 0.2 mg to 1 mg of the active ingredient in question.

It is also envisaged that in certain treatment regimes, administration with longer intervals, e.g. every other day, every week, or even with longer intervals may be beneficial.

If the treatment involves administration of another therapeutically active compound it is recommended to consult Goodman & Gilman’s The Pharmacological Basis of Therapeutics, 9th Ed., J.G. Hardman and L.E. Limbird (Eds.), McGraw-Hill 1995, for useful dosages of said compounds.

The administration of a compound of the present invention with one or more other active compounds may be either concomitantly or sequentially.

The formulations include e.g. those in a form suitable for oral (including sustained or timed release), rectal, parenteral (including subcutaneous, intraperitoneal, intramuscular, intraarticular and intravenous), transdermal, ophthalmic, topical, dermal, nasal, buccal or intradermal administration. Topical administration of the claimed formulation is particularly suitable.

The formulations may conveniently be presented in dosage unit form and may be prepared by any of the methods well known in the art of pharmacy, e.g. as disclosed in Remington, The Science and Practice of Pharmacy, 20th ed., 2000. All methods include the step of bringing the active ingredient into association with the carrier, which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid, such as ethanol or glycerol; or in the form of an oil-in-water emulsion or a water-in-oil emulsion. Such oils may be edible oils, such as e.g. cottonseed oil, sesame oil, coconut oil or peanut oil. Suitable dispersing or suspending agents for aqueous suspensions include synthetic or natural gums such as tragacanth, alginate, acacia, dextran, sodium carboxymethylcellulose, gelatin, methylcellulose, hydroxypropylmethylcellulose, hydroxypropylcellulose, carbomers and polyvinylpyrrolidone. The active ingredients may also be administered in the form of a bolus, electuary or paste.

A tablet may be made by compressing or moulding the active ingredient optionally with one or more accessory ingredients. Compressed tablets may be prepared by compressing, in a suitable machine, the active ingredient(s) in a free-flowing form such as a powder or granules, optionally mixed by a binder, such as e.g. lactose, glucose, starch, gelatine, acacia gum, tragacanth gum, sodium alginate, carboxymethylcellulose, methylcellulose, hydroxypropylmethylcellulose, polyethylene glycol, waxes or the like; a lubricant such as e.g. sodium oleate, sodium stearate, magnesium stearate, sodium benzoate, sodium acetate, sodium chloride or the like; a disintegrating agent such as e.g. starch, methylcellulose, agar, bentonite, croscarmellose sodium, sodium starch glycollate, crospovidone or the like or a dispersing agent, such as polysorbate 80. Moulded tablets may be made by moulding, in a suitable machine, a mixture of the powdered active ingredient and suitable carrier moistened with an inert liquid diluent.

Formulations for rectal administration may be in the form of suppositories in which the compound of the present invention is admixed with low melting water soluble or insoluble solids such as cocoa butter, hydrogenated vegetable oils, polyethylene glycol or fatty acids esters of polyethylene glycols, while elixirs may be prepared using myristyl palmitate.

Formulations suitable for parenteral administration conveniently comprise a sterile oily or aqueous preparation of the active ingredients, which is preferably isotonic with the blood of the recipient, e.g. isotonic saline, isotonic glucose solution or buffer solution. The formulation may be conveniently sterilised by for instance filtration through a bacteria retaining filter, addition of sterilising agent to the formulation, irradiation of the formulation or heating of the formulation. Liposomal formulations as disclosed in *e.g.* Encyclopedia of Pharmaceutical Technology, vol.9, 1994, are also suitable for parenteral administration.

Alternatively, the compounds of formula A may be presented as a sterile, solid preparation, e.g. a freeze-dried powder, which is readily dissolved in a sterile solvent immediately prior to use.

Transdermal formulations may be in the form of a plaster, patch, microneedles, liposomal or nanoparticulate delivery systems or other cutaneous formulations applied to the skin.

Formulations suitable for ophthalmic administration may be in the form of a sterile aqueous preparation of the active ingredients, which may be in microcrystalline form, for example, in the form of an aqueous microcrystalline suspension. Liposomal formulations or biodegradable polymer systems e.g. as disclosed in Encyclopedia of Pharmaceutical

Technology, vol.2, 1989, may also be used to present the active ingredient for ophthalmic administration.

Formulations suitable for topical, such as dermal, intradermal or ophthalmic administration include liquid or semi-liquid preparations such as liniments, lotions, gels, sprays, micro or nano-emulsions, oil-in-water or water-in-oil emulsions such as creams, ointments, pastes, applicants, foams, filmforming systems or microneedles; or solutions or suspensions such as drops. Compositions for ophthalmic treatment may preferably additionally contain a cyclodextrin.

For topical administration, the compound of formula A may typically be present in an amount of from 0.01 to 20% by weight of the composition, such as 0.1% to about 10 %, but may also be present in an amount of up to about 50% of the composition.

Formulations suitable for nasal or buccal administration include powder, self-propelling and spray formulations, such as aerosols and atomisers. Such formulations are disclosed in greater detail in e.g. Modern Pharmaceutics, 2nd ed., G.S. Banker and C.T. Rhodes (Eds.), page 427-432, Marcel Dekker, New York; Modern Pharmaceutics, 3th ed., G.S. Banker and C.T. Rhodes (Eds.), page 618-619 and 718-721, Marcel Dekker, New York and_Encyclopedia of Pharmaceutical Technology, vol. 10, J. Swarbrick and J.C. Boylan (Eds), page 191-221, Marcel Dekker, New York.

In addition to the aforementioned ingredients, the formulations of a compound of formula A may include one or more additional ingredients such as diluents, buffers, flavouring agents, colourant, surface active agents, thickeners, preservatives, e.g. methyl hydroxybenzoate (including anti-oxidants), emulsifying agents and the like.

When the active ingredient is administered in the form of salts with pharmaceutically acceptable non-toxic acids, preferred salts are for instance easily water-soluble or slightly soluble in water, in order to obtain a particular and appropriate rate of absorption.

The pharmaceutical composition may additionally comprise one or more other active components conventionally used in the treatment of dermal disease or conditions, e.g. selected from the group consisting of glucocorticoids, vitamin D and vitamin D analogues, antihistamines, phosphodiesterase 4 (PDE4) inhibitors, JAK inhibitors, methylxanthines, β-adrenergic agents, COX-2 inhibitors, salicylates, indomethacin, flufenamate, naproxen, timegadine, retinoids, zinc salts, salicylazosulfapyridine and calcineurin inhibitors.

The term "compound of formula A" as used herein is intended to include compounds of formula A(i), formula A(ii) and formula A(iii).

The invention is further described in the appended examples which are not in any way intended to limit the scope of the invention as claimed.

METHODS OF PREPARATION

The compounds of the present invention can be prepared in a number of ways well known to those skilled in the art of synthesis. The compounds of formula A may for example be prepared using the reactions and techniques outlined below together with methods known in the art of synthetic organic chemistry, or variations thereof as appreciated by those skilled in the art. Preferred methods include, but are not limited to, those described below. The reactions are carried out in solvents appropriate to the reagents and materials employed and suitable for the transformations being effected. Also, in the synthetic methods described below, it is to be understood that all proposed reaction conditions, including choice of solvent, reaction atmosphere, reaction temperature, duration of experiment and work-up procedures, are chosen to be conditions of standard for that reaction, which should be readily recognized by one skilled in the art of organic synthesis. Not all compounds falling into a given class may be compatible with some of the reaction conditions required in some of the methods described. Such restrictions to the substituents which are compatible with the reaction conditions will be readily apparent to one skilled in the art and alternative methods can be used.

Starting materials of general formula II are prepared according to standard procedures known to a chemist skilled in the art of organic synthesis. An aldehyde of formula I is treated with with (S)-2-methylpropane-2-sulfinamide and $Ti(O\text{-}iPr)_4$ in a suitable solvent (e.g. THF) under suitable conditions (e.g. 0 °C to RT) for a suitable period of time (e.g. 5-10 h) to form a compound of general formula II.

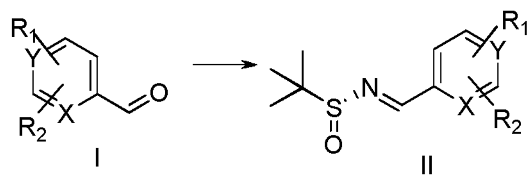

I

II

R1, R2 = H, Me, Cl, F, Br, CF3
X, Y = CH, N

Scheme 1

A compound of general formula III can be prepared as described in Scheme 2. A sulfinamide derivative of formula II is treated with methyl magnesium bromide in a suitable solvent (e.g. DCM) under suitable conditions (e.g. -50 °C to RT) for a suitable period of time (e.g. 2-5 h) to form compound III.

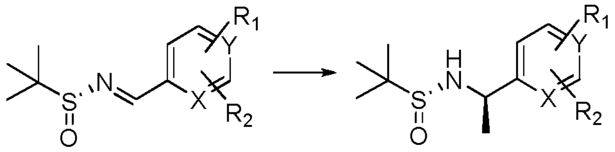

II III

Scheme 2

A compound of general formula IV can be prepared as described in Scheme 3. A sulfinamide derivative of general formula III is treated with a suitable base (e.g. LiHMDS) in a suitable solvent (e.g. THF), under suitable conditions (e.g. -70 °C to RT) for a suitable period of time (e.g. 1 h), followed by addition of iodomethane, for a suitable period of time (e.g. 12 h) to form a compound of general formula IV.

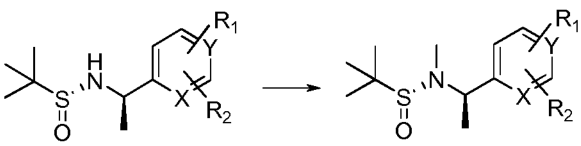

III IV

Scheme 3

A compound of general formula V can be prepared as described in Scheme 4. A compound of general formula IV is treated with hydrogen chloride in a suitable solvent (e.g. MeOH), for a suitable period of time (e.g. 1 h) at a suitable temperature (e.g. RT) to form a compound of general formula V.

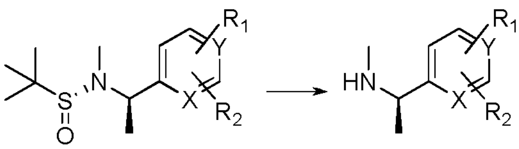

IV V

Scheme 4

A compound of general formula VIII can be prepared as described in Scheme 5. A diamine derivative of general formula VI is treated with a suitable acylating agent (e.g. benzyl chloroformate) in a suitable solvent (e.g. DCM), under suitable conditions (e.g. 0 °C to RT) for a suitable period of time (e.g. 3 h) to form a compound of general formula VII, which in turn is treated with pentynoic acid in the presence of suitable coupling agents (e.g. EDCI, HOBT), in a suitable solvent (e.g. DCM), under suitable conditions (e.g. 0 °C to RT) in the presence of a suitable base (e.g. TEA) for a suitable period of time (e.g. 12 h) to form a compound of general formula VIII.

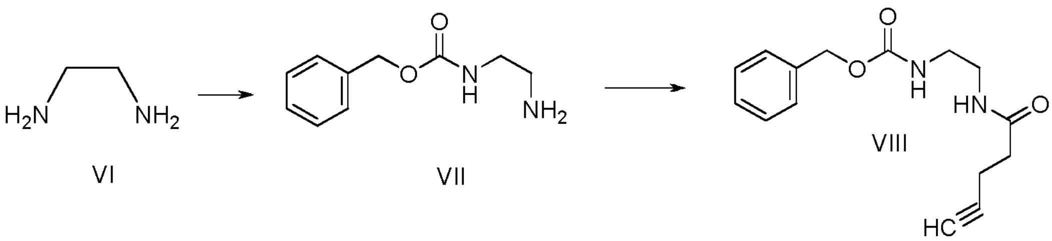

VI VII VIII

Scheme 5

A compound of general formula IX can be prepared as described in Scheme 6. A compound of general formula VIII is treated with 2-Iodotoluene in a suitable solvent (e.g. DMF), in the presence of a suitable base (e.g. TEA), under suitable coupling conditions (e.g. catalytic amounts of copper iodide and a palladium catalyst such as $PdCl_2(PPh_3)_2$), for a suitable period of time (e.g. 3 h) at a suitable temperature (e.g. RT) to form a compound of general formula IX.

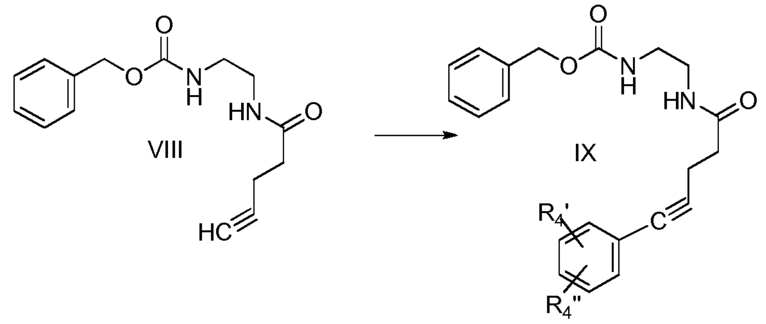

R4', R4'' = H, Me, F, OMe

Scheme 6

A compound of general formula X can be prepared as described in Scheme 7. A compound of general formula IX is treated with a suitable oxidizing agent (e.g. PIFA) in a suitable solvent (e.g. TFEA) for a suitable period of time (e.g. 3 h) at a suitable temperature (e.g. 0 °C to RT) to form a compound of general formula X.

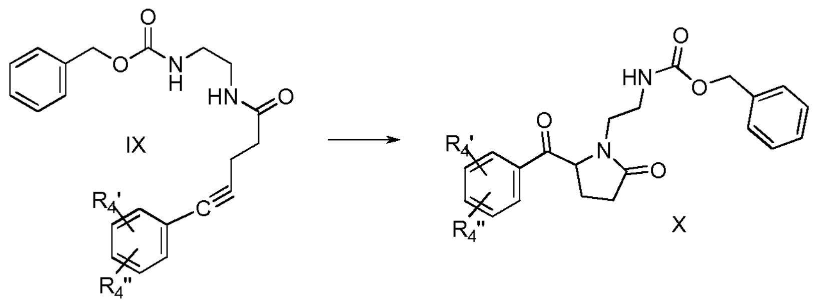

Scheme 7

A compound of general formula XI can be prepared as described in Scheme 8. A compound of general formula X is treated under suitable hydrogenation conditions (e.g. 5 Atm $H_2$) in a suitable acidic media (e.g. HCl/MeOH) for a suitable period of time (e.g. 24 h) and in the presence of a suitable catalyst (e.g. Pd/C 5%) to afford a compound of general formula XI.

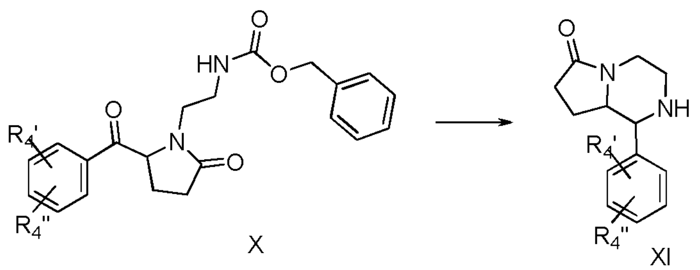

Scheme 8

An enantiomerically pure compound of formula XIII can be prepared as described in Scheme 9. A compound of general formula XII is treated with a suitable base (e.g. LiHMDS) in a suitable solvent (e.g. THF) for a suitable period of time (e.g. 0.5-1 h), followed by addition of a suitable reagent (e.g. 2-bromoacetonitrile), for a suitable period (e.g. 1-2

h) to afford a compound of general formula XIII.

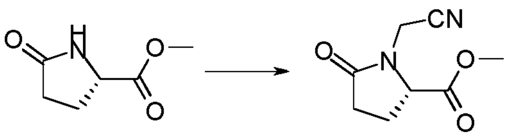

XII XIII

Scheme 9

An enantiomerically pure compound of general formula XIV can be prepared as described in Scheme 10. A compound of general formula XIII is treated under suitable hydrogenation conditions (e.g. 4 Atm $H_2$) in a suitable basic media (e.g. $NH_4OH$/MeOH) for a suitable period of time (e.g. 24 h) and in the presence of a suitable catalyst (e.g. Raney Ni) to afford a compound of general formula XIV.

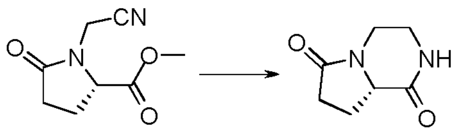

XIII XIV

Scheme 10

An enantiomerically pure compound of general formula XV can be prepared as described in Scheme 11. A compound of general formula XIV is treated with a suitable reagent (e.g. Boc anhydride) in the presence of a suitable base (e.g. TEA) in a suitable solvent (e.g. DCM) at a suitable temperature (e.g. RT) for a suitable period of time (e.g. 12 h) to give a compound of formula XV.

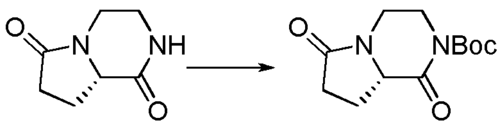

XIV XV

Scheme 11

An enantiomerically pure compound of general formula XVI can be prepared as described in Scheme 12. A compound of general formula XV is treated with a suitable reagent (e.g. Lithium reagent) in a suitable solvent (e.g. THF) at a suitable temperature (e.g. -70 °C) for a suitable period of time (e.g. 5-10 min) to give a compound of formula XVI.

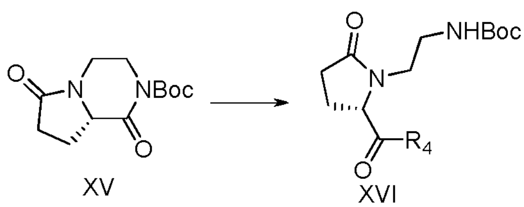

XV XVI

$R_4$ = substituted phenyl or heterocycle

Scheme 12

An enantiomerically pure compound of general formula XVII can be prepared as described in Scheme 13. A compound of general formula XVI is treated with a suitable reduction reagent (e.g. $LiAl(OtBu_3)H$) in a suitable solvent (e.g. THF) at a suitable temperature (e.g. RT) for a suitable period of time (e.g. 1.5 h) to give a compound of formula XVII.

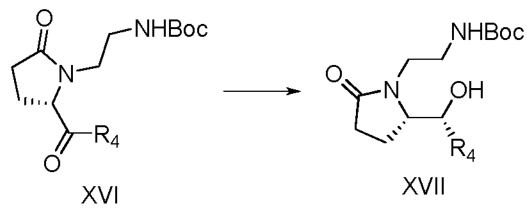

XVI XVII

Scheme 13

An enantiomerically pure compound of general formula XVIII can be prepared as described in Scheme 14. A compound of general formula XVII is treated with a suitable reagent (e.g. methanesulfonyl chloride) in the presence of a suitable base (e.g. TEA, DMAP) in a suitable solvent (e.g. DCM) at a suitable temperature (e.g. RT) for a suitable period of time (e.g. 20 h) to give a compound of formula XVIII.

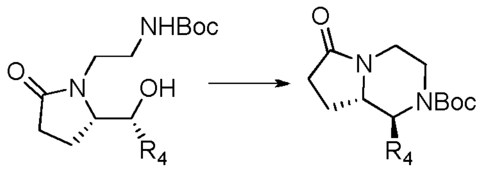

XVII XVIII

Scheme 14

An enantiomerically pure compound of general formula XIX can be prepared as described in Scheme 15. A compound of general formula XVIII is treated with a suitable acid (e.g. TFA) in a suitable solvent (e.g. DCM) to afford a compound of general formula XIX.

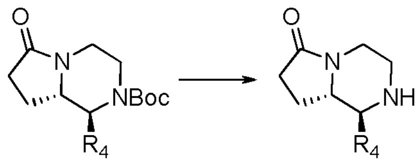

XVIII XIX

Scheme 15

A compound of general formula XX can be prepared as described in Scheme 16. A compound of general formula XI or XIX is treated with a suitable acylating agent (e.g. triphosgene) in a suitable solvent (e.g. EtOAc) in the presence of a compound of general formula V and of a suitable base (e.g. TEA), at a suitable temperature (e.g. 0 °C to RT) for a suitable period of time (e.g. 3 h) to afford a compound of general formula XX.

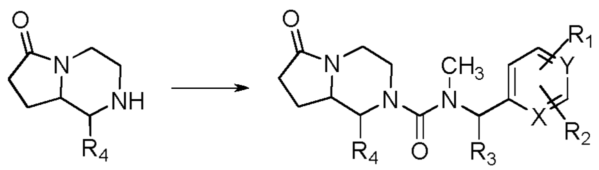

XI or XIX XX

$R_1$, $R_2$ = H, Me, Cl, F, $CF_3$
X, Y = CH, N
$R_3$ = H, Me
$R_4$ = substituted phenyl or Heteroaryl

Scheme 16

A compound of general formula XXII can be prepared as described in Scheme 17. A compound of general formula XXI or XVIII is treated with a suitable acylating or alkylating agent (methyl chloroformate (possibly followed by MeI), allyl bromide) in the presence of a suitable base (e.g. LiHMDS) in a suitable solvent (e.g. THF) at a suitable temperature (e.g. -78 °C to 0 °C) for a suitable period of time (e.g. 3 h). The resulting compound (XXII, R=Boc) can in turn be treated with a suitable acid (e.g. TFA) in a suitable solvent (e.g. DCM) to afford a compound of general formula XXII (R = H).

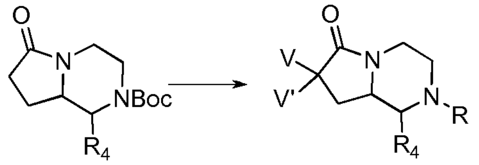

XXI or XVIII XXII

V , V' = CH3, allyl, COOCH3
R=H, Boc
$R_4$ = substituted phenyl or hetreocycle

Scheme 17

A compound of general formula XXIII can be prepared as described in Scheme 18. A compound of general formula XXII is treated as described for compound of general formula XI to give the urea derivative of general formula XX. Further elaboration of V, V' residues, using standard methods well described in the literature and well known to those skilled in the art (e.g. cleavage of an allylic double bond by ozonolysis to form the corresponding aldehyde and subsequent reduction of the aldehyde to form the corresponding alcohol, derivatization of the alcohol to form a mesylate) can lead to further derivatives also represented in the general formula XXIII.

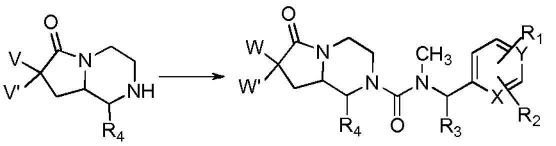

XXII XXIII

R1, R2 = H, Me, Cl, F, CF3
X, Y = CH, N
R3 = H, Me
$R_4$ = substituted phenyl or heterocycle
W , W' = H, $CH_3$, allyl, $COOCH_3$, $CH_2OH$, $CH_2CH_2OH$

Scheme 18

EXAMPLES

Proton Magnetic Resonance (NMR) spectra were recorded on a Bruker instrument at 400 MHz spectrometers at 25°C. Chemical shifts are reported in ppm ($\delta$) using the residual solvent line as internal standard. Peak multiplicities are expressed as follow: s, singlet; d, doublet; dd doublet of doublets, t, triplet; dt doublet of triplet; q, quartet; quin, quintet; m, multiplet; br s, broad singlet.

Total ion current (TIC) and DAD UV chromatographic traces together with MS and UV spectra associated with the peaks were taken also on a SHIMADZU LCMS2020 system equipped with a LCMS2020 mass spectrometer operating in positive and/or negative electrospray ionisation mode. [LC/MS/ESI (+/-): analyses performed using a Chromolith SpeedROD C18 column (50 x 4.6 mm, 2 $\mu$m particle size), column temperature 35°C, mobile phase: A = CH3CN/H2O/FA=10/90/0.05 and B = CH3CN/H2O/FA=90/10/0.05, Flow rate: 3.0 mL/min, Gradient: t=0.8 min 20% B, t=3.5 min 95% B, t=4.3 min 95% B, t=4.4 min 20% B.

Achiral purification were carried out using a SHIMADZU 6A system operated under follow chromatographic conditions, mobile phase: A = 0.1%FA in H2O and B = MeCN, flow rate 12 ml/min and column Shimapack-ODS(H) KIT (250*21.2mm,10um) @ room T.

For the chiral separation and the chiral quality control two different techniques were used: 1) Supercritical Fluid Choromatography (SFC): analytical chromatography was performed on a Berger SFC Analytix, while for the preparative

SFC, a Jasco preparative SFC system was used 2) High Performance Liquid Chromatography (HPLC): chiral Preparative HPLC was performed using a Waters 600 HPLC system and Agilent series 1100 instrument, while for analytical chromatography a SHIMADZU LC-2010A HPLC was used.

Abbreviations:

| | |
|---|---|
| AcOH : | Acetic acid |
| Bn: | Benzyl |
| Boc: | tert-butyloxycarbonyl |
| EtOAc: | ethyl acetate |
| CBz: | Carboxybenzyl |
| CH: | cyclohexane |
| Cy: | cyclohexane |
| DCM: | dichloromethane |
| DIBAL-H: | Diisobutylaluminium hydride |
| DMSO: | dimethylsulfoxide |
| DMAP: | 4-Dimethylaminopyridine |
| DMF: | N,N-dimethylformamide |
| DMPU: | 1,3-Dimethyl-3,4,5,6-tetrahydro-2(1H)-pyrimidinone |
| EDCI.HCl: | N-(3-Dimethylaminopropyl)-N'-ethylcarbodiimide hydrochloride |
| Et: | Ethyl |
| EtOH: | ethanol |
| HOBT.$H_2O$: | hydroxybenzotriazol monohydrate |
| HPLC: | High Performancee Liquid Chromatography |
| LHMDS: | lithium bis(trimethylsilyl)amide |
| Me: | Methyl |
| MeCN: | acetonitrile |
| MeOH: | methanol |
| MS: | Mass Spectrometry |
| Ms: | Mesyl |
| NMR: | Nuclear Magnetic Resonance |
| Pd(dppf)$Cl_2$: | [1,1'-Bis(diphenylphosphino)ferrocene]dichloropalladium(II) |
| PE: | Petroleum Ether |
| iPr: | isopropyl |
| PIFA | ((bis(trifluoroacetoxy)iodo)benzene |
| RT: | room temperature |
| ee: | enantiomeric eccess |
| Rt: | retention time |
| TEA: | triethylamine |
| TFA: | trifluoroacetic acid |
| TFEA: | 2,2,2-trifluoroethanol |
| THF: | tetrahydrofuran |
| TLC: | Thin Layer Chromatography |
| UPLC: | Ultra Performance Liquid Chromatography |

The bicyclic corestructure of the compounds of the present invention is numbered as follows:

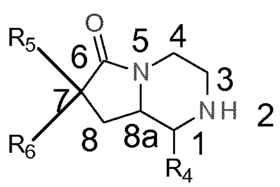

Intermediate 1

benzyl 2-(5-(2,4-dimethylphenyl)pent-4-ynamido)ethylcarbamate

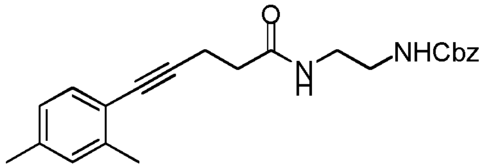

To a solution of benzyl 2-pent-4-ynamidoethylcarbamate (7 g, 25.5 mmol) in DMF (100 mL), was added 1-iodo-2,4-dimethylbenzene (6.7 g, 29 mmol), TEA (4.2 mL, 30.4 mmol), CuI (420 mg, 2.2 mmol) and $PdCl_2(PPh_3)_2$ (780 mg, 1.1 mmol) under $N_2$ atomosphare. The resulting mixture was stirred at 25 °C for 12 h and concentrated. The residue was dissolved in DCM (100 mL) and washed with 1 M HCl solution and brine. The organic layer was dried over anhydrous $Na_2SO_4$ and then concentrated in high vacuum. The residue was purified by silica gel chromatography (PE/EtOAc=2/3 to 1/10) to give the title compound (6.1 g, 63%); m/z (ES+): 379 $[M+H]^+$.

Intermediate 2

benzyl 2-(2-(2,4-dimethylbenzoyl)-5-oxopyrrolidin-1-yl)ethylcarbamate

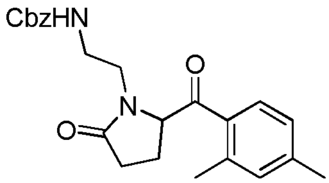

To a solution of benzyl 2-(5-(2,4-dimethylphenyl)pent-4-ynamido)ethylcarbamate (6.5 g, 16.1 mmol) in TFEA (250 mL) was cooled and stirred at 0°C, then a solution of PIFA (12.3 g, 28.7 mmol) in TFEA (150 mL) was added dropwise. The reaction was stirring at 0 °C for 2 h and then quenched by addition of sat. $Na_2CO_3$ solution. The resulting mixture was extracted with DCM (3 x 100 mL). The combined organic layers were washed with brine, dried over $Na_2SO_4$ and then concentrated in high vacuum. The residue was purified by silica gel chromatography (PE/EtOAc=2/1 to 1/10) to give the title compound (5 g, 79%); m/z (ES+): 395 $[M+H]^+$

Intermediate 3

1-(2,4-dimethylphenyl)hexahydropyrrolo[1,2-a]pyrazin-6(7H)-one (mixture of trans)

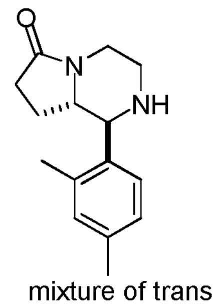

mixture of trans

To a solution of benzyl 2-(2-(2,4-dimethylbenzoyl)-5-oxopyrrolidin-1-yl)ethylcarbamate (7 g, 17.8 mmol) in 80

ML of MeOH was added 1 M HCl (35 mL) and Pd/C 5% (1 g). The reaction was stirred under $H_2$ (5 atm) atmosphare for 12 h. The reaction mixture was filtered through a celite pad and concentrated in high vacuum. The residue was dissolved in MeOH (30ml) and $NaBH_3(CN)$ (1.3 g, 20.5 mmol) was added. The reaction was stirred for 5h at room temperature and filtered. The filtrate was concentrated in high vacuum. The residue was purified by silica gel chromatography (PE/EtOAc=2/1 to 1/2) to give the title compound (2.3 g, 53%); m/z (ES+): 245 $[M+H]^+$

Intermediate 4

tert-butyl 1-(2,4-dimethylphenyl)-6-oxohexahydropyrrolo[1,2-a]pyrazine-2(1H)-carboxylate (mixture of trans)

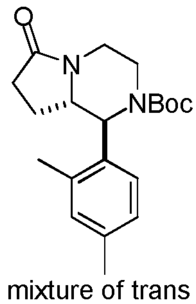

mixture of trans

To a solution of 1-(2,4-dimethylphenyl)hexahydropyrrolo[1,2-a]pyrazin-6(7H)-one (mixture of trans, 1.5 g, 6.1 mmol) was added $(Boc)_2O$ (2.65 g, 12.2mmol) at 0 °C, the reaction mixture was stirred overnight at 25 °C, then $N^1,N^1$-dimethylethane-1,2-diamine (5 ml) was added and the mixture was stirred at 25 °C for 8h. Saturated solution of $NH_4Cl$ was added, followed by EtOAc (2 x 20 mL). The combined organic layers were dried over sodium sulphate, filtered and concentrated in vacuo, to give the title compound (2 g, 95%) which was used in the next step without further purifications; m/z (ES+): 345 $[M+H]^+$.

Intermediate 5

tert-butyl 7,7-diallyl-1-(2,4-dimethylphenyl)-6-oxohexahydropyrrolo[1,2-a]pyrazine-2(1H)-carboxylate (mixture of trans)

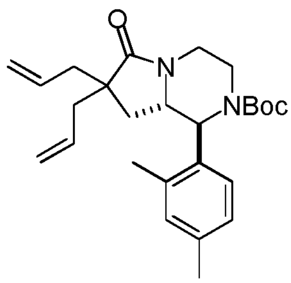

mixture of trans

To a solution of tert-butyl 1-(2,4-dimethylphenyl)-6-oxohexahydropyrrolo[1,2-a]pyrazine-2(1H)-carboxylate (mixture of trans, 2 g, 5.8 mmol) in anhydrous THF (60 mL) under $N_2$ atmosphere, was added LiHMDS (1 M in THF, 6.05 mL) at -78 °C. The reaction was stirred for 15 min, followed by addition of DMPU (1.8 mL, 15 mmol) and allylbromide (0.52 mL, 6 mmol). The reaction mixture was stirred at -78 °C for 30 min and allowed to warm to -30 °C, and then stirred at this temperature for another 2 h. After quenched with water at -78 °C and the mixture was then extracted with EtOAc (2 x 50 mL). The combined organic layers were washed with brine, dried over $Na_2SO_4$, filtered and concentrated in vacuo. The residue was purified by silica gel chromatography (PE/EtOAc=2/1 to 1/1) to give the title compound (2 g, yield: 81%); m/z (ES+): 425 $[M+H]^+$.

Intermediate 6

7,7-diallyl-1-(2,4-dimethylphenyl)hexahydropyrrolo[1,2-a]pyrazin-6(7H)-one (mixture of trans)

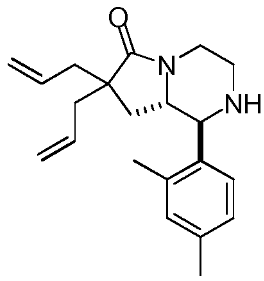

mix of trans

To a solution of tert-butyl 7,7-diallyl-1-(2,4-dimethylphenyl)-6-oxohexahydropyrrolo [1,2-a]pyrazine-2(1H)-carboxylate (mixture of trans, 2 g, 4.7 mmol) in DCM (40 mL) at 0 °C was added TFA (8 mL). The reaction was stirred at room temperature for 3 h. DCM was removed under reduced pressure, the residue was adjusted with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (1.2 g, yield: 79%); m/z (ES+): 325 $[M+H]^+$.

Intermediate 7 and 8

7,7-diallyl-1-(2,4-dimethylphenyl)-N-methyl-N-((R)-1-(3-methyl-5-(trifluoromethyl) phenyl)ethyl)-6-oxohexahydropyrrolo[1,2-a]pyrazine-2(1H)-carboxamide

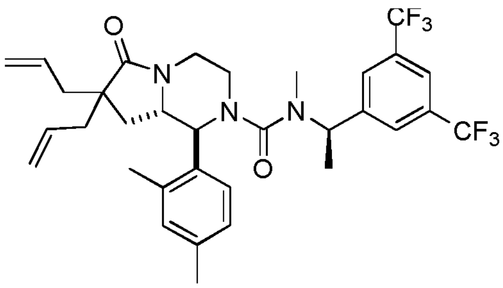

To a solution of triphosgene (205 mg, 0.70 mmol) in EtOAc (10 mL) at 0 °C was added solution of 7,7-diallyl-1-(2,4-dimethylphenyl)hexahydropyrrolo[1,2-a]pyrazin-6(7H)-one (mixture of trans, 500 mg, 1.54 mmol), DMAP (40 mg, 0.32 mmol) and TEA (480 mg, 4.8 mmol) in EtOAc (10 mL). The mixture was stirred for 1.5 h at room temperature, followed by addition of (R)-1-(3,5-bis(trifluoromethyl)phenyl)-N-methylethanamine (543 mg, 2 mmol) and TEA (480 mg, 4.8 mmol) in EtOAc (2 mL). The reaction was stirred at 45 °C for 48 h and quenched with $NH_4Cl$ (aq). The resulting mixture was extracted with EtOAc (2 x 100 mL); the combined organic layers were washed with brine, dried over $Na_2SO_4$ and then concentrated in high vacuum. The residue was purified by silica gel chromatography (PE/EtOAc=2/1 to 1/1) to give the title intermediate 7 (200 mg, yield: 20%) and 8 (180 mg, yield: 18%); m/z (ES+): 622 $[M+H]^+$.

Intermediate 9

7,7-diallyl-1-(2,4-dimethylphenyl)-N-methyl-N-((R)-1-(3-methyl-5-(trifluoromethyl) phenyl)ethyl)-6-oxo-hexahydropyrrolo[1,2-a]pyrazine-2(1H)-carboxamide (mixture of trans)

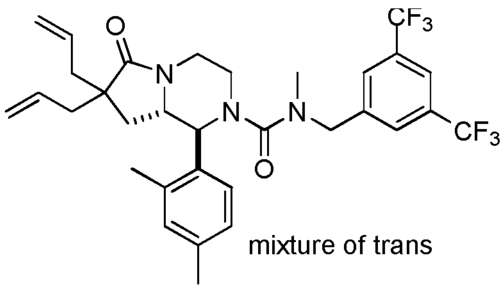

mixture of trans

To a solution of triphosgene (205 mg, 0.70 mmol) in EtOAc (10 mL) at 0 °C was added solution of 7,7-diallyl-1-(2,4-dimethylphenyl)hexahydropyrrolo[1,2-a]pyrazin-6(7H)-one (mixture of trans, 500 mg, 1.54 mmol), DMAP (40 mg, 0.32 mmol) and TEA (480 mg, 4.8 mmol) in EtOAc (10 mL). The mixture was stirred for 1.5 h at room temperature, followed by addition of (3,5-bis(trifluoromethyl)phenyl)-N-methylmethanamine (514 mg, 2 mmol) and TEA (480 mg, 4.8 mmol) in EtOAc (2 mL). The reaction was stirred at 45 °C for 48 h and quenched with $NH_4Cl$ (aq). The resulting mixture was extracted with EtOAc (2 x 100 mL); the combined organic layers were washed with brine, dried over $Na_2SO_4$ and then concentrated in high vacuum. The residue was purified by silica gel chromatography (PE/EtOAc=2/1 to 1/1) to give the title compound (160 mg, yield: 16%); m/z (ES+): 608 $[M+H]^+$.

Intermediate 10

(1S,8aS)-7,7-diallyl-1-(4-fluoro-2-methylphenyl)hexahydropyrrolo[1,2-a]pyrazin-6(7H)-one (mixture of trans)

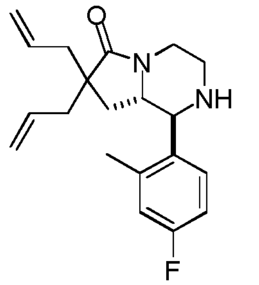

mix of trans

Intermediate 10 was synthesized using the same procedure for intermediate 6. m/z (ES+): 329 $[M+H]^+$.

Intermediate 11

(1S,8aS)-7,7-diallyl-N-((R)-1-(3-chloro-5-(trifluoromethyl)phenyl)ethyl)-1-(4-fluoro-2-methylphenyl)-N-methyl-6-oxo-hexahydropyrrolo[1,2-a]pyrazine-2(1H)-carboxamide (mix of trans)

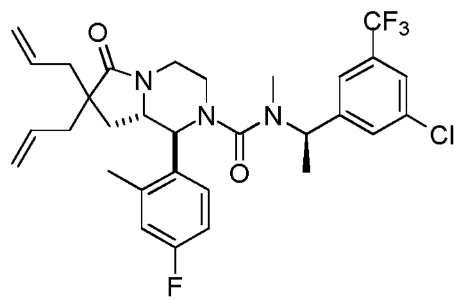

mix of trans

To a solution of triphosgene (47.2 mg, 0.16 mmol) in EtOAc (1 mL) at 0 °C was added solution of intermediate 10 (mix of trans, 100 mg, 0.32 mmol), DMAP (3.9 mg, 0.032 mmol) and TEA (97 mg, 0.96 mmol) in EtOAc (2 mL). The mixture was stirred for 2 h, followed by addition of (R)-1-(3-chloro-5-(trifluoromethyl)phenyl)-*N*-methylethanamine (100 mg, 0.44 mmol) and TEA (97 mg, 0.96 mmol) in EtOAc (2 mL). The reaction was stirred at 50 °C for 48 h and quenched with water. The resulting mixture was extracted with EtOAc (2 x 20 mL) and washed with brine. The combined organic layers were dried over anhydrous $Na_2SO_4$ and concentrated in high vacuum. The residue was purified by silica gel chromatography (DCM/MeOH=20/1 to 10/1) to give the title compound (66 mg, yield: 33%); m/z (ES+): 592 $[M+H]^+$.

Intermediate 12

(1S,8aS)-*7,7*-diallyl-1-(4-fluoro-2-methylphen)-*N*-methyl-*N*-(R)-1-(3-methyl-5-(trifluoromethyl)phenyl)ethyl)-6-oxohexahydropyrrolo[1,2-a]pyrazine-2(1*H*)-carboxamide (mix of trans)

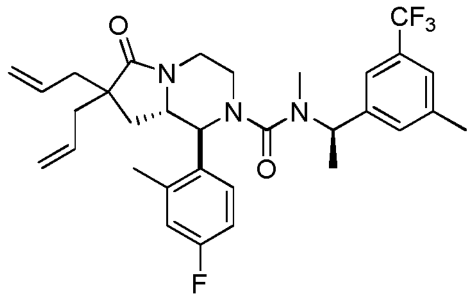

To a solution of triphosgene (47.2 mg, 0.16 mmol) in EtOAc (1 mL) at 0 °C was added solution of intermediate 10 (mix of trans, 100 mg, 0.32 mmol), DMAP (3.9 mg, 0.032 mmol) and TEA (97 mg, 0.96 mmol) in EtOAc (2 mL). The mixture was stirred for 2 h, followed by addition of (R)-*N*-methyl-1-(3-methyl-5-(trifluoromethyl)phenyl)ethanamine (100 mg, 0.44 mmol) and TEA (97 mg, 0.96 mmol) in EtOAc (2 mL). The reaction was stirred at 50 °C for 48 h and quenched with water. The resulting mixture was extracted with EtOAc (2 x 20 mL). The combined organic layers were dried over anhydrous $Na_2SO_4$ and concentrated in high vacuum. The residue was purified by silica gel chromatography (DCM/MeOH=20/1 to 10/1) to give the title compound (100 mg, yield: 51%); m/z (ES+): 572 $[M+H]^+$.

Intermediate 13

(1S,8aS)-*7,7*-diallyl-N-((R)-1-(3,5-bis(trifluoromethyl)phenyl)ethyl)-1-(4-fluoro-2-methylphenyl)-N-methyl-6-oxohexahydropyrrolo[1,2-a]pyrazine-2(1H)-carboxamide (mix of trans)

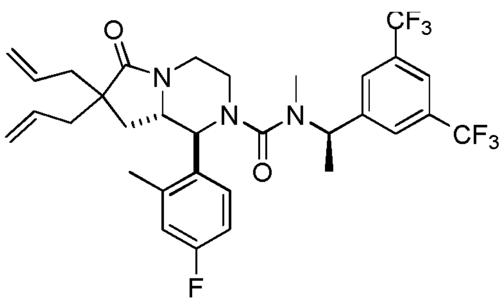

To a solution of triphosgene (149mg, 0.5 mmol) in EtOAc (5 mL) at 0 °C was added solution of intermediate 10 (mix of trans, 328 mg, 1 mmol), DMAP (12 mg, 0.1 mmol) and TEA (151 mg, 1.5mmol) in EtOAc (5 mL). The mixture was stirred for 2 h, followed by addition of (R)-1-(3,5-bis(trifluoromethyl)phenyl)-*N*-methylethanamine (407mg, 1.5 mmol) and TEA (151mg, 1.5 mmol) in EtOAc (5 mL). The reaction was stirred at 50 °C for 48 h and quenched with water. The resulting mixture was extracted with EtOAc (2 x 20 mL). The combined organic layers were washed with brine, dried over anhydrous $Na_2SO_4$ and then concentrated in high vacuum. The residue was purified by silica gel chromatography

(DCM/MeOH=20/1 to 10/1) to give the title compound (200 mg, yield: 32%); m/z (ES+): 626 $[M+H]^+$.

Intermediate 14

(1S,8aS)-1-(4-methoxy-2-methylphenyl)hexahydropyrrolo[1,2-a]pyrazin-6(7H)-one (mix of trans)

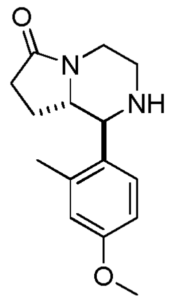

mix of trans

Intermediate 14 was prepared using the procedure for intermediate 3. m/z (ES+): 261 $[M+H]^+$.

Intermediate 15

tert-butyl *7,7*-diallyl-1-(4-methoxy-2-methylphenyl)-6-oxohexahydropyrrolo[1,2-a]pyrazine-2(1H)-carboxylate (mix of trans)

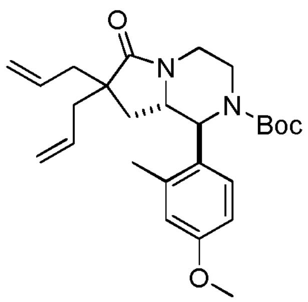

mix of trans

Intermediate 15 was prepared using the procedure for intermediate 3 to prepare. m/z (ES+): 341 $[M+H]^+$.

Intermediate 16

(1S,8aS)-*7,7*-diallyl-1-(4-methoxy-2-methylphenyl)hexahydropyrrolo[1,2-a]pyrazin-6(7H)-one(mix of trans)

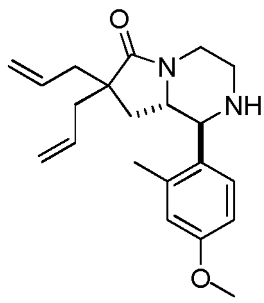

mix of trans

Intermediate 16 was prepared using the procedure for intermediate 6 to prepare. m/z (ES+): 341 $[M+H]^+$.

Intermediate 17

(1S,8aS)-*7,7*-diallyl-*N*-((R)-1-(3-ethyl-5-(trifluoromethyl)phenyl)ethyl)-1-(4-methoxy-2-methylphenyl)-*N*-methyl-6-oxo-hexahydropyrrolo[1,2-a]pyrazine-2(1*H*)-carboxamide (mix of trans)

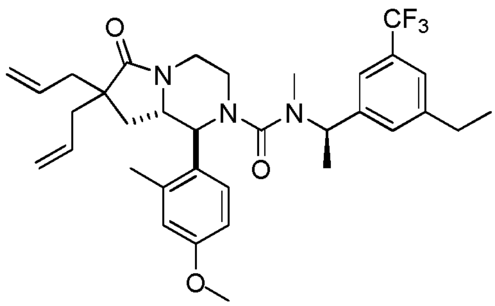

To a solution of triphosgene (47 mg, 0.16 mmol) in EtOAc (10 mL) at 0 °C was added solution of intermediate 15 (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-1-(3,5-bis(trifluoromethyl)phenyl)-N-methyl ethanamine (139 mg, 0.6 mmol) and TEA (107 mg, 1.05 mmol) in EtOAc (2 mL). The reaction was stirred at 45 °C for 48 h and quenched with saturated $NH_4Cl$ (aq). The resulting mixture was extracted with EtOAc (2 x 20 mL). The organic layers were washed with brine, dried over $Na_2SO_4$ and then concentrated in high vacuum. The residue was purified by silica gel chromatography (PE/EtOAc=2/1 to 1/1) to give the title compound (65 mg, yield: 31%); m/z (ES+): 598 $[M+H]^+$.

Intermediate 18

(S)-methyl 1-(cyanomethyl)-5-oxopyrrolidine-2-carboxylate

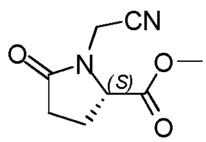

To a suspension of NaH (60%) (27.9 g, 0.7 mol) in THF (800 mL), was dropwise added (S)-methyl 5-oxopyrrolidine-2-carboxylate (100.0 g, 0.7 mol) in THF (200 mL) at 0 °C. The reaction was stirred at this temperature for 30 min followed by addition of bromoacetonitrile (48.4 ml, 0.7 mol) at 0 °C. The reaction mixture was allowed to warm to 15 °C and stired for 1 h, and then carefully poured into a mixture of ice-water (200 mL) and $H_2SO_4$ (6 mL). The resulting mixture was extracted with EtOAc (300 ml), washed with brine (2 x 100 mL). The organic layer was dried over anhydrous

$Na_2SO_4$ and then concentrated in high vacuum. The residue was purified by silica gel chromatography (PE/EtOAc =4/1 to 1/1) to give the title compound (65 g, yield: 51%, ee= 96.9%); m/z (ES+): 183 $[M+H]^+$.

Intermediate 19

(S)-tetrahydropyrrolo[1,2-a]pyrazine-1,6(*2H,7H*)-dione

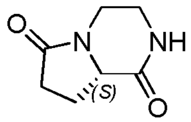

To a solution of Intermediate 1 (20 g, 0.11 mol) in MeOH (300 mL) under $N_2$ atmosphere, was added Raney Ni (5.0 g) and followed by NH4OH (0.2 mL). The reaction mixture was stirred under $H_2$ atmosphere (4 atm) for 24 h at room temperature. Raney Ni was filtered through a celite pad, and the filtrate was concentrated in high vacuum. The residue was purified by silica gel chromatography (DCM/MeOH=70/1 to 10/1) to give the title compound (8.3 g, yield: 52%, ee= 94.2%); m/z (ES+): 155 $[M+H]^+$.

Intermediate 20

(S)-tert-butyl 1,6-dioxohexahydropyrrolo[1,2-a]pyrazine-2(1*H*)-carboxylate

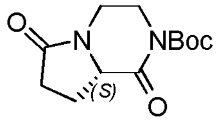

To a solution of Intermediate 2 (14.0 g, 90.3 mmol), Boc2O (39.4 g, 182.0 mmol) and DMAP (550 mg, 4.5 mmol) in THF (250 mL) was dropwise added Et3N (26.2 mL, 182.0 mmol). The reaction was stirred at room temperature for 3 h and quenched by addition of $H_2O$. The resulting mixture was extracted with EtOAc (2 x 100 mL). The combined organic layers were dried over anhydrous $Na_2SO_4$, and then concentrated in high vacuum. The residue was purified by silica gel chromatography (PE/EtOAc=5/1 to 1/3) to give the title compound (17.0 g, yield: 74%, ee= 92.9%); m/z (ES+): 255 $[M+H]^+$.

Intermediate 21

(S)-tert-butyl 2-(2-(4-fluoro-2-methylbenzoyl)-5-oxopyrrolidin-1-yl)ethylcarbamate

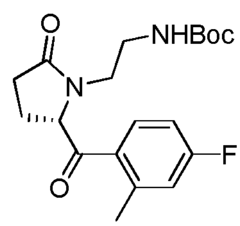

To a solution of 1-bromo-4-fluoro-2-methyl-benzene (2.34 g, 12.39 mmol) in THF (40 mL) at -70 °C was dropwise added n-BuLi (1.6 M in hexane, 7.52 mL, 12 mmol) over 5 min. After addition, the reaction was stirred at this temperature for 30 min. Then the resulting solution was added into a solution of (S)-tert-butyl 1, 6-dioxohexahydro pyrrolo [1,2-a]pyrazine-2(1*H)*-carboxylate (3 g, 11.8 mmol) in THF (40 mL). After stirred at -70 °C for 5 min, the reaction mixture was quenched with saturated $NH_4Cl$ and extracted with EtOAc (2 x 20 mL) and washed with brine. The combined organic layers were dried over anhydrous $Na_2SO_4$ and concentrated in high vacuum. The residue was purified by silica gel

chromatography (PE/EtOAc=5/1 to 1/1) to give the title compound (4.2 g, yield: 60%); m/z (ES+): 365 $[M+H]^+$.

Intermediate 22

tert-butyl 2-((S)-2-((R)-(4-fluoro-2-methylphenyl)(hydroxy)methyl)-5-oxopyrrolidin-1-yl)ethylcarbamate

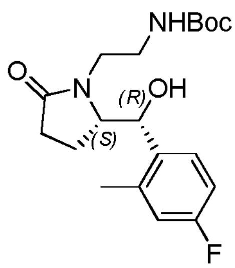

To a solution of (S)-tert-butyl 2-(2-(4-fluoro-2-methylbenzoyl)-5-oxopyrrolidin-1-yl)ethylcarbamate (2.3 g, 6.3 mmol) in THF (50 mL), was portionwise added $LiAl(OtBu_3)H$ (3.56 g, 14 mmol) at -20 °C. The mixture was stirred for 1.5 h at 20 °C and then quenched with water. The resulting mixture was filtered through a celite pad and the filtration was extracted with EtOAc (2 x 50 mL). The combined organic layers were dried over anhydrous $Na_2SO_4$ and then concentrated in high vacuum. The residue was purified by silica gel chromatography (PE/EtOAc=5/1 to 1/1) to give the title compound (2.1 g, yield: 90%); m/z (ES+): 379$[M+H]^+$.

Intermediate 23

(1S,8aS)-tert-butyl 1-(4-fluoro-2-methylphenyl)-6-oxohexahydropyrrolo[1,2-a]pyrazine-2(1*H*)-carboxylate

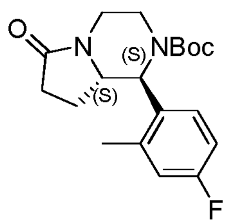

To a solution of tert-butyl 2-((S)-2-((R)-(4-fluoro-2-methylphenyl)(hydroxy)methyl)-5-oxopyrrolidin-1-yl)ethylcarbamate (3.6 g, 9.8 mmol) in DCM (20 mL) was added $Et_3N$ (3.54 g, 35 mmol) and DMAP (85.6 mg, 0.7 mmol). The resulting solution was cooled to 0 °C, and then MsCl (1.18 g, 10.3 mmol) was dropwise added into under $N_2$ atmosphere. The reaction was stirred for 20 h at room temperature and then quenched with saturated $NH_4Cl$ solution. The resulting mixture was extracted with DCM (2 x 20 mL) and washed with brine. The combined organic layers were dried over anhydrous $Na_2SO_4$ and concentrated in high vacuum. The residue was purified by silica gel chromatography (PE/EtOAc=5/1 to 1/1) to give the title compound (700 mg, yield: 20%); m/z (ES+): 349 $[M+H]^+$.

Intermediate 24

(1R,8aS)-tert-butyl 7,7-diallyl-1-(4-fluoro-2-methylphenyl)-6-oxohexahydropyrrolo[1,2-a]pyrazine-2(1*H*)-carboxylate

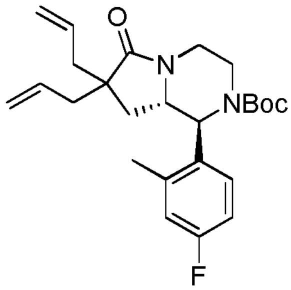

To a solution of (1S,8aS)-tert-butyl 1-(4-fluoro-2-methylphenyl)-6-oxohexahydropyrrolo [1,2-a]pyrazine-2(1*H*)-carboxylate (700 mg, 2.0 mmol) in anhydrous THF (100 mL) under $N_2$ atmosphere at -78 °C was added LiHMDS (1 M in THF, 3.4 mL). The reaction was stirred for 15 min at this temperature, followed by addition of DMPU (895 mg, 7 mmol) and allylbromide (316.5 mg, 2.62 mmol). The reaction mixture was stirred at -78 °C for another 30 min. allowed to warm to -30 °C, and then stirred at this temperature for another 2 h. After quenched with water at -78 °C, the mixture was then extracted with EtOAc (2 x 50 mL) and washed with brine. The combined organic layers were dried over anhydrous $Na_2SO_4$ and concentrated in high vacuum.. The residue was purified by silica gel chromatography (PE/EtOAc=2/1 to 1/1) to give the title compound (650 mg, yield: 81%); m/z (ES+): 429 $[M+H]^+$.

Intermediate 25

(1S,8aS)-1-(4-fluoro-2-methylphenyl)hexahydropyrrolo[1,2-a]pyrazin-6(7*H*)-one

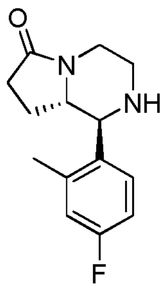

To a solution of (1S,8aS)-tert-butyl 1-(4-fluoro-2-methylphenyl)-6-oxohexahydro pyrrolo[1,2-a]pyrazine-2(1*H*)-carboxylate (220 mg, 0.59 mmol) in MeOH (20 mL) at 0 °C was added HCl/MeOH (7 M, 5 mL). The reaction was stirred at room temperature for 3 h and basified with 1 M aqueous NaOH solution to pH=10. The resulting mixture was extracted with EtOAc (2 x 20 mL) and washed with brine. The combined organic layers were dried over anhydrous $Na_2SO_4$ and concentrated in high vacuum. The residue was directly used in the next step without further purification (180 mg, yield: 95%); m/z (ES+): 249 $[M+H]^+$.

Intermediate 26

(1S,8aS)-7,7-diallyl-1-(4-fluoro-2-methylphenyl)hexahydropyrrolo[1,2-a]pyrazin-6(7H)-one

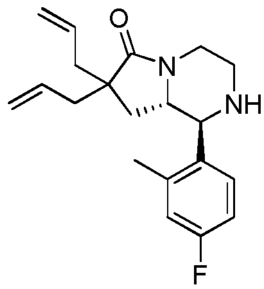

To a solution of 7,7-Diallyl-1-(4-fluoro-2-methyl-phenyl)-6-oxo-hexahydro-pyrrolo[1,2-a]pyrazine-2-carboxylic acid tert-butyl ester (850 mg, 1.98 mmol) in MeOH (20 mL) was added HCl/MeOH (7 M, 5 mL) at 0 °C. The reaction was stirred at room temperature for 3 h and basified with 1 M aqueous NaOH solution to pH=10. The resulting mixture was extracted with EtOAc (2 x 30 mL) and washed with brine. The combined organic layers were dried over anhydrous $Na_2SO_4$ and concentrated in high vacuum. The residue was directly used in the next step without further purification (650 mg, yield: 95%); m/z (ES+): 329 $[M+H]^+$.

Intermediate 27

(1S,8aS)-7,7-diallyl-*N*-((R)-1-(3-ethyl-5-(trifluoromethyl)phenyl)ethyl)-1-(4-fluoro-2-methylphenyl)-*N*-methyl-6-oxohexahydropyrrolo[1,2-a]pyrazine-2(1H)-carboxamide

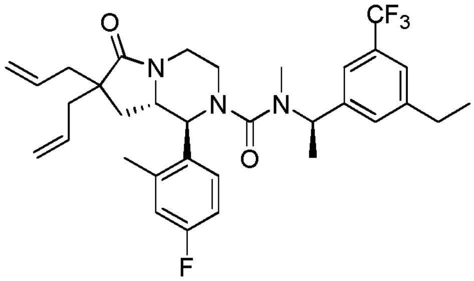

To a solution of triphosgene (73.45 mg, 0.25 mmol) in EtOAc (2 mL) at 0 °C was added a solution of (1S,8aS)-7,7-diallyl-1-(4-fluoro-2-methylphenyl)hexahydropyrrolo[1,2-a]pyrazin-6(7H)-one (164 mg, 0.5 mmol), DMAP (6.1 mg, 0.05 mmol) and TEA (151.68 mg, 1.5 mmol) in EtOAc (2 mL). The mixture was stirred for 1.5 h at room temperature, followed by addition of [1-(3-Ethyl-5-trifluoromethyl-phenyl)-ethyl]-methyl-amine (163.2 mg, 0.6 mmol) in EtOAc (2 mL) and TEA (151.68 mg, 1.5 mmol). The reaction was stirred at 45 °C for 48 h and quenched with saturated $NH_4Cl$ (aq). The resulting mixture was extracted with EtOAc (2 x 20 mL) and washed with brine. The combined organic layers were dried over anhydrous $Na_2SO_4$ and concentrated in high vacuum. The residue was purified by silica gel chromatography (PE/EtOAc=2/1 to 1/1) to give the title compound (120 mg, yield: 39%); m/z (ES+): 586 $[M+H]^+$.

Intermediate 28

(1S,8aS)-7,7-diallyl-1-(4-fluoro-2-methylphenyl)-N-((R)-1-(3-isopropyl-5-(trifluoro methyl)phenyl)ethyl)-*N*-methyl-6-oxohexahydropyrrolo[1,2-a]pyrazine-2(1H)-carboxamide

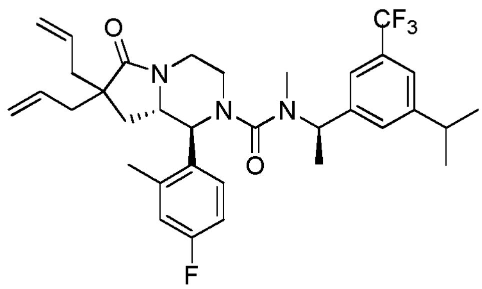

To a solution of triphosgene (73.45 mg, 0.25 mmol) in EtOAc (2 mL) at 0 °C was added a solution of (1S,8aS)-7,7-diallyl-1-(4-fluoro-2-methylphenyl)hexahydropyrrolo[1,2-a] pyrazin-6(7H)-one (164 mg, 0.5 mmol), DMAP (6.1 mg, 0.05 mmol) and TEA (151.7 mg, 1.5 mmol) in EtOAc (2 mL). The mixture was stirred for 1.5 h at room temperature, followed by addition of [1-(3-Isopropyl-5-trifluoromethyl-phenyl)-ethyl]-methyl-amine (163.2 mg, 0.6 mmol) and TEA (151.68 mg, 1.5 mmol) in EtOAc (2 mL). The reaction was stirred at 45 °C for 48 h and quenched with saturated $NH_4Cl$ (aq). The resulting mixture was extracted with EtOAc (2 x 20 mL) and washed with brine. The combined organic layers were dried over anhydrous $Na_2SO_4$ and concentrated in high vacuum. The residue was purified by silica gel chromatography (PE/EtOAc=2/1 to 1/1) to give the title compound (110 mg, yield: 37%); m/z (ES+): 600 $[M+H]^+$.

Intermediate 29

(S)-tert-butyl 2-(2-(4-methoxy-2-methylbenzoyl)-5-oxopyrrolidin-1-yl)ethylcarbamate

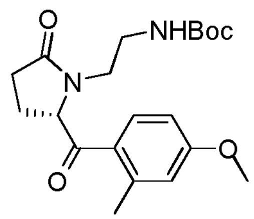

To a solution of 1-bromo-4-methoxy-2-methylbenzene (2.0 g, 10 mmol) in THF (20 mL) at -70 °C was dropwise added n-BuLi (1.6 M in hexane, 7.52 mL, 12 mmol) over 5 min. The reaction was stirred at this temperature for 30 min. The resulting solution was added into a solution of (S)-tert-butyl 1,6-dioxohexahydropyrrolo [1,2-a]pyrazine-2(1*H*)-carboxylate (2.5 g, 10 mmol) in THF (40 mL). After stirred at -70 °C for 5 min, the reaction mixture was quenched with saturated $NH_4Cl$ and extracted with EtOAc (2 x 20 mL). The organic layers were washed with brine, dried over $Na_2SO_4$, filtered and concentrated in high vacuum. The residue was purified by silica gel chromatography (PE/EtOAc=5/1 to 1/1) to give the title compound (2.3 g, yield: 60%); m/z (ES+): 377 [M+H]+.

Intermediate 30

tert-butyl 2-((S)-2-((R)-hydroxy(4-methoxy-2-methylphenyl)methyl)-5-oxopyrrolidin-1-yl)ethylcarbamate

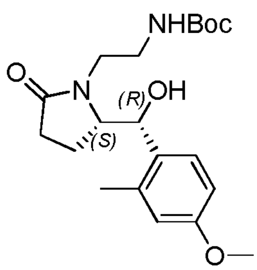

To a solution of (S)-tert-butyl 2-(2-(4-methoxy-2-methylbenzoyl)-5-oxopyrrolidin-1-yl) ethylcarbamate (2.3 g, 6.1 mmol) in THF (50 mL), was portionwise added LiAl(OtBu$_3$)H (3.56 g, 14 mmol) at -20 °C. The mixture was stirred for 1.5 h at 20 °C and then quenched with water. The resulting mixture was filtered through a celite pad and the filtration was extracted with EtOAc (2 x 50 mL). The combined organic layers were dried over anhydrous $Na_2SO_4$ and then concentrated in high vacuum. The residue was purified by silica gel chromatography (PE/EtOAc=5/1 to 1/1) to give the title compound (2.3 g, yield: 99.7%); m/z (ES+): 379[M+H] $^+$.

Intermediate 31

(1R,8aS)-tert-butyl1-(4-methoxy-2-methylphenyl)-6-oxohexahydropyrrolo[1,2-a]pyrazine-2(1*H*)-carboxylate

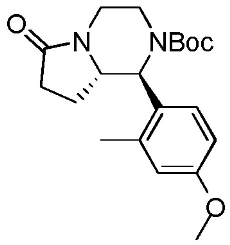

To a solution of tert-butyl 2-((S)-2-((R)-hydroxy(4-methoxy-2-methylphenyl)methyl)-5-oxopyrrolidin-1-yl)ethylcarbamate (2.3 g, 6.1 mmol) in DCM (20 mL) was added $Et_3N$ (3.54 g, 35 mmol), DMAP (85.6 mg, 0.7 mmol). The resulting solution was cooled to 0 °C, and then MsCl (1.18 g, 10.3 mmol) was added into the above solution dropwisely under $N_2$ atmosphere. The reaction was stirred for 20 h at room temperature and then quenched with saturated $NH_4Cl$ solution and extracted with DCM (2 x 20 mL). The combined organic layers were washed with brine, dried over $Na_2SO_4$, filtered and concentrated in vacuo. The residue was purified by silica gel chromatography (PE/EtOAc=5/1 to 1/1) to give the title compound (1.5 mg, yield: 67%); m/z (ES+): 361 [M+H]$^+$.

Intermediate 32

(1S,8aS)-1-(4-methoxy-2-methylphenyl)hexahydropyrrolo[1,2-a]pyrazin-6(7*H*)-one

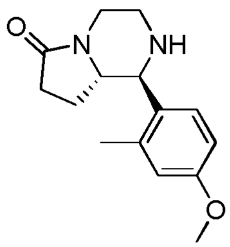

To a solution of (1R,8aS)-tert-butyl 1-(4-methoxy-2-methylphenyl)-6-oxohexahydro pyrrolo[1,2-a]pyrazine-2(1*H*)-carboxylate (0.5 g, 1.4 mmol) in DCM (10 mL) was added TFA (2.5 mL). The reaction was stirred at room temperature for 3 h and then basified with 1 M aqueous NaOH solution to pH=8. The resulting mixture was extracted with DCM (2 x 30 mL). The combined organic layers were dried over anhydrous $Na_2SO_4$, and then concentrated in high vacuum. The residue was purified by silica gel chromatography (DCM/MeOH=20/1 to 10/1) to give the title compound (0.4 g, yield: 100%); m/z (ES+): 261 [M+H]$^+$.

Intermediate 33

(1R,8aS)-tert-butyl 7,7-diallyl-1-(4-methoxy-2-methylphenyl)-6-oxohexahydropyrrolo [1,2-a]pyrazine-2(1*H*)-carboxylate

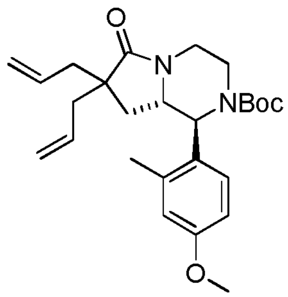

To a solution of (1R,8aS)-tert-butyl 1-(4-methoxy-2-methylphenyl)-6-oxohexahydro pyrrolo[1,2-a]pyrazine-2(1*H*)-carboxylate (1 g, 2.7 mmol) in anhydrous THF (15 mL) under $N_2$ atmosphere at -78 °C was added LiHMDS (1 M in THF, 13.5 mL). The reaction was stirred for 15 min, followed by addition of DMPU (1.3 g, 10.8 mmol) and allylbromide (1.3 g, 10.8 mmol). The reaction mixture was stirred at -78 °C for 30 min and allowed to warm to -30 °C. The mixture was stirred at this temperature for another 2 h. After quenched with water at -78 °C, the mixture was then extracted with EtOAc (2 x 50 mL). The combined organic layers were washed with brine, dried over $Na_2SO_4$, and concentrated in high vacuum. The residue was purified by silica gel chromatography (PE/EtOAc=2/1 to 1/1) to give the title compound (1.1 g, yield: 92.6%); m/z (ES+): 441 $[M+H]^+$.

Intermediate 34

(1S,8aS)-7,7-diallyl-1-(4-methoxy-2-methylphenyl)hexahydropyrrolo[1,2-a]pyrazin-6(7H)-one

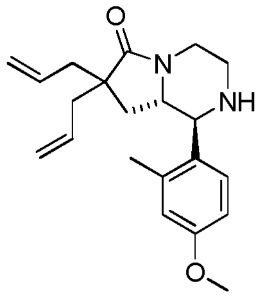

To a solution of (1R,8aS)-tert-butyl 7,7-diallyl-1-(4-methoxy-2-methylphenyl)-6-oxohexahydropyrrolo[1,2-a]pyrazine-2(1*H*)-carboxylate (1.1 g, 2.5mmol) in MeOH (20 mL) was added HCl/MeOH (7 M, 15 mL) at 0 °C. The reaction was stirred at room temperature for 3 h and then basified with 1 M aqueous NaOH solution to pH=8. The mixture was extracted with EtOAc (2 x 20 mL). The combined organic layers were washed with brine, dried over $Na_2SO_4$, and concentrated in high vacuum. The residue was directly used in the next step without further purification (811 mg, yield: 95%); m/z (ES+): 341 $[M+H]^+$.

Intermediate 35

(1S,8aS)-7,7-diallyl-N-((R)-1-(3,5-bis(trifluoromethyl)phenyl)ethyl)-1-(4-methoxy-2-methylphenyl)-N-methyl-6-oxohexahydropyrrolo[1,2-a]pyrazine-2(1H)-carboxamide

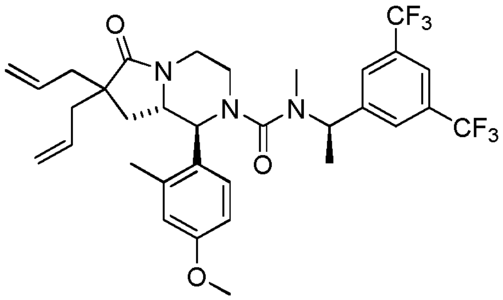

To a solution of triphosgene (47 mg, 0.16 mmol) in EtOAc (10 mL) at 0 °C was added solution of (1S,8aS)-7,7-diallyl-1-(4-methoxy-2-methylphenyl)hexahydropyrrolo[1,2-a]pyrazin-6(7H)-one (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-1-(3,5-bis(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with saturated $NH_4Cl$ (aq). The resulting mixture was extracted with EtOAc (2 x 20 mL). The combined organic layers were washed with brine, dried over $Na_2SO_4$ and then concentrated in high vacuum. The residue was purified by silica gel chromatography (PE/EtOAc=2/1 to 1/1) to give the title compound (70 mg, yield: 31%); m/z (ES+): 638 $[M+H]^+$.

Intermediate 36

(1S,8aS)-7,7-diallyl-1-(4-methoxy-2-methylphenyl)-N-methyl-N-((R)-1-(3-methyl-5-(trifluoromethyl)phenyl)ethyl)-6-oxohexahydropyrrolo[1,2-a]pyrazine-2(1H)-carboxamide

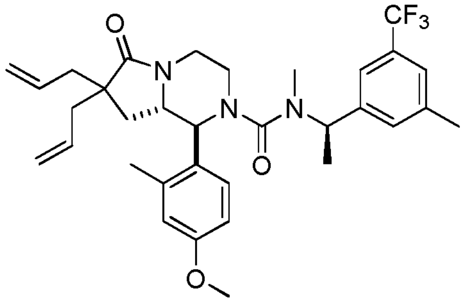

To a solution of triphosgene (47 mg, 0.16 mmol) in EtOAc (10 mL) at 0 °C was added solution of (1S,8aS)-7,7-diallyl-1-(4-methoxy-2-methylphenyl)hexahydropyrrolo[1,2-a]pyrazin-6(7H)-one (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-N-methyl-1-(3-methyl-5-(trifluoromethyl)phenyl)ethanamine (139 mg, 0.6 mmol) and TEA (107 mg, 1.05 mmol) in EtOAc (2 mL). The reaction was stirred at 45 °C for 48 h and quenched with saturated $NH_4Cl$ (aq). The resulting mixture was extracted with EtOAc (2 x 20 mL). The organic layers were washed with brine, dried over $Na_2SO_4$ and then concentrated in high vacuum. The residue was purified by silica gel chromatography (PE/EtOAc=2/1 to 1/1) to give the title compound (82 mg, yield: 40%); m/z (ES+): 584 $[M+H]^+$.

Intermediate 37

(S)-tert-butyl2-(2-(4-(benzyloxy)-2-methylbenzoyl)-5-oxopyrrolidin-1-yl)ethylcarbamate

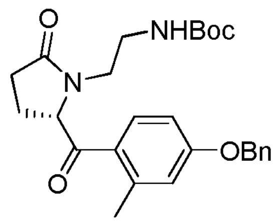

To a solution of 1-(benzyloxy)-4-bromo-2-methylbenzene (3.6 g, 12.9 mmol) in THF (40 mL) at -70 °C was added n-BuLi (1.6 M, 7.7 ml, 12.3 mmol). The reaction was stirred at the same temperature for 30 min, and added to (S)-tert-butyl 1,6-dioxohexahydro pyrrolo [1,2-a]pyrazine-2(1H)-carboxylate (3.0 g, 11.7 mmol) in 40 ml THF. After stirred for 5 min, the reaction mixture was quenched with saturated $NH_4Cl$ and extracted with EtOAc (2 x 20 mL). The combined organic layers were dried over anhydrous $Na_2SO_4$ and then concentrated in high vacuum. The residue was purified by silica gel chromatography (PE/EtOAc=10/1 to 1/1) to give the title compound (2.0 g, yield: 38%); m/z (ES+): 453 $[M+H]^+$.

Intermediate 38

tert-butyl 2-((S)-2-((R)-(4-(benzyloxy)-2-methylphenyl)(hydroxy)methyl)-5-oxopyrrolidin-1-yl)ethylcarbamate

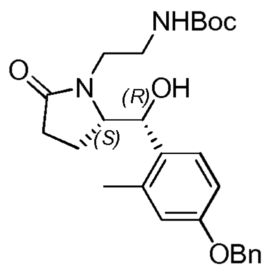

To a solution of (S)-tert-butyl 2-(2-(4-(benzyloxy)-2-methylbenzoyl)-5-oxopyrrolidin-1-yl)ethylcarbamate (2 g, 4.4 mmol) in THF (50 mL), was portionwise added $LiAl(OtBu_3)H$ (1.68 g, 6.6 mmol) at -20 °C. The mixture was stirred for 1.5 h at 20 °C and then quenched with water. The resulting mixture was filtered through a celite pad and the filtrate was extracted with EtOAc (2 x 50 mL). The combined organic layers were dried over anhydrous $Na_2SO_4$ and then concentrated in high vacuum. The residue was purified by silica gel chromatography (PE/EtOAc=5/1 to 1/1) to give the title compound (1.85 g, yield: 92.4%); m/z (ES+): 455$[M+H]^+$

Intermediate 39

(1S,8aS)-tert-butyl1-(4-(benzyloxy)-2-methylphenyl)-6-oxohexahydropyrrolo[1,2-a]pyrazine-2(1H)-carboxylate

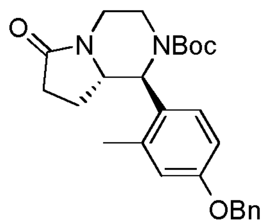

To a solution of tert-butyl 2-((S)-2-((R)-(4-(benzyloxy)-2-methylphenyl)(hydroxy) methyl) -5-oxopyrrolidin-1-yl)ethylcarbamate (1.85 g, 4.0 mmol) in DCM (20 mL) was added $Et_3N$ (2.02 g, 20 mmol), DMAP (85.6 mg, 0.7 mmol). The resulting solution was cooled to 0 °C, and then MsCl (860 mg, 6.0 mmol) was dropwise added into the above solution under $N_2$ atmosphere. The reaction was stirred for 20 h at room temperature and then quenched with saturated $NH_4Cl$ solution. The resulting mixture was extracted with DCM (2 x 20 mL). The combined organic layers were washed with

brine, dried over $Na_2SO_4$, and concentrated in high vacuum. The residue was purified by silica gel chromatography (PE/EtOAc =5/1 to 1/1) to give the title compound (1.2 g, yield: 69%); m/z (ES+): 437 $[M+H]^+$.

Intermediate 40

(1S,8aS)-1-(4-(benzyloxy)-2-methylphenyl)hexahydropyrrolo[1,2-a]pyrazin-6(7*H*)-one

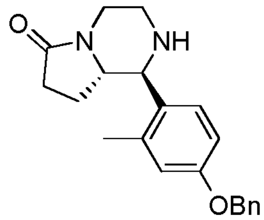

To a solution of (1S,8aS)-tert-butyl 1-(4-(benzyloxy)-2-methylphenyl)-6-oxohexahydro pyrrolo[1,2-a]pyrazine-2(1H)-carboxylate (200 mg, 0.46 mmol) in MeOH (5 mL) was added HCl/MeOH (7M, 2 mL) at 0 °C. The reaction was stirred at room temperature for 3 h and then basified with 1 M aqueous NaOH solution to pH=8. The mixture was extracted with EtOAc (2 x 20 mL). The combined organic layers were washed with brine, dried over $Na_2SO_4$, and concentrated in high vacuum. The residue was directly used in the next step without further purification (100 mg, yield: 65%); m/z (ES+): 337 $[M+H]^+$.

Intermediate 41

(1S,8aS)-1-(4-(benzyloxy)-2-methylphenyl)-N-((R)-1-(3-ethyl-5-(trifluoromethyl) phenyl)ethyl)-N-methyl-6-oxohexahydropyrrolo[1,2-a]pyrazine-2(1H)-carboxamide

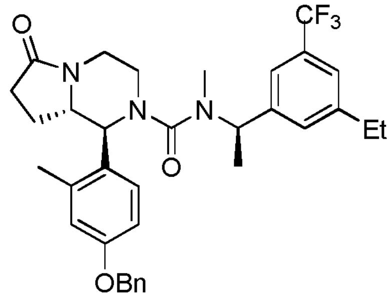

To a solution of triphosgene (73 mg, 0.25 mmol) in EtOAc (2 mL) at 0 °C was added solution of (1S,8aS)-1-(4-(benzyloxy)-2-methylphenyl)hexahydropyrrolo[1,2-a]pyrazin-6(7*H*)-one (162 mg, 0.5 mmol), DMAP (6.1 mg, 0.05 mmol) and TEA (151.7 mg, 1.5 mmol) in EtOAc (2 mL). The mixture was stirred for 1.5 h at room temperature, followed by addition of (R)-1-(3-ethyl-5-(trifluoromethyl)phenyl)-N-methylethanamine (163.2 mg, 0.6 mmol) and (151.7 mg, 1.5 mmol) in EtOAc (2 mL). The reaction was stirred at 45 °C for 48 h and quenched with saturated $NH_4Cl$ (aq). The resulting mixture was extracted with EtOAc (2 x 20 mL). The combined organic layers were washed with brine, dried over $Na_2SO_4$ and then concentrated in vacuum. The residue was purified by Prep-HPLC to give the title compound (120 mg, yield: 39%); m/z (ES+): 594 $[M+H]^+$.

Intermediate 42

(1R,8aS)-tert-butyl7,7-diallyl-1-(4-(benzyloxy)-2-methylphenyl)-6-oxohexahydro pyrrolo[1,2-a]pyrazine-2(1H)-carboxylate

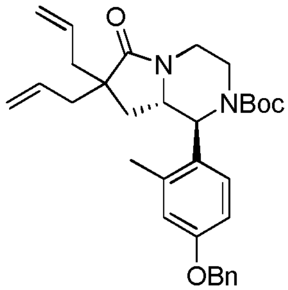

To a solution of (1S,8aS)-tert-butyl 1-(4-(benzyloxy)-2-methylphenyl)-6-oxohexahydro pyrrolo[1,2-a]pyrazine-2(1H)-carboxylate (600 mg, 1.37 mmol) in anhydrous THF (30 mL) under $N_2$ atmosphere at -78 °C was added LiHMDS (1 M in THF, 5.49 mL). The reaction was stirred for 15 min, followed by addition of DMPU (870 mg, 6.85 mmol) and allylbromide (400 mg, 3.42mmol). The reaction mixture was stirred at -78 °C for 30 min and allowed to warm to -30 °C. The reaction was stirred at this temperature for another 2 h. After quenched with water at -78 °C and the mixture was then extracted with EtOAc (2 x 50 mL). The combined organic layers were washed with brine, dried over $Na_2SO_4$, and concentrated in high vacuum. The residue was purified by silica gel chromatography (PE/EtOAc=2/1 to 1/1) to give the title compound (600 mg, yield: 85%); m/z (ES+): 425 $[M+H]^+$.

Intermediate 43

(1S,8aS)-7,7-diallyl-1-(4-(benzyloxy)-2-methylphenyl)hexahydropyrrolo[1,2-a]pyrazin-6(7H)-one

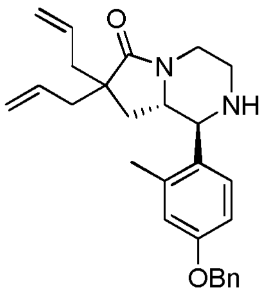

To a solution of (1R,8aS)-tert-butyl7,7-diallyl-1-(4-(benzyloxy)-2-methylphenyl)-6-oxohexahydro pyrrolo[1,2-a]pyrazine-2(1H)-carboxylate (600 mg, 1.16 mmol) in MeOH (20 mL) at 0 °C was added HCl/MeOH (7 M, 5 mL). The reaction was stirred at room temperature for 3 h and basified with 1 M aqueous NaOH solution to pH=10. The mixture was extracted with EtOAc (2 x 20 mL). The combined organic layers were washed with brine, dried over $Na_2SO_4$, and concentrated in high vacuum. The residue was directly used in the next step without further purification (400 mg, yield: 83%); m/z (ES+): 417 $[M+H]^+$.

Intermediate 44

(1S,8aS)-N-((R)-1-(3,5-bis(trifluoromethyl)phenyl)ethyl)-7,7-diallyl-1-(4-(benzyloxy)-2-methylphenyl)-N-methyl-6-oxo-hexahydropyrrolo[1,2-a]pyrazine-2(1H)-carboxamide

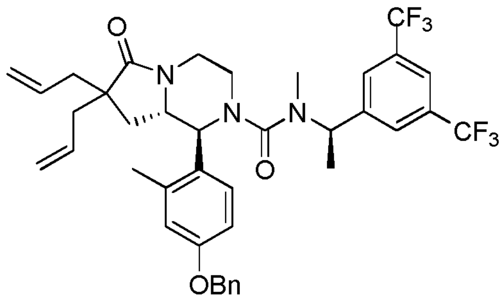

To a solution of triphosgene (53 mg, 0.18 mmol) in EtOAc (2 mL) at 0 °C was added solution of (1S,8aS)-7,7-diallyl-1-(4-(benzyloxy)-2-methylphenyl)hexahydropyrrolo[1,2-a]pyrazin-6(7H)-one (150 mg, 0.36 mmol), DMAP (4 mg, 0.05 mmol) and TEA (101 mg, 1 mmol) in EtOAc (2 mL). The mixture was stirred for 1.5 h at room temperature, followed by addition of (R)-1-(3,5-bis(trifluoromethyl)phenyl)-N-methylethanamine (147 mg, 0.54 mmol) in EtOAc (2 mL) and TEA (101 mg, 1 mmol). The reaction was stirred at 45 °C for 48 h and quenched with saturated $NH_4Cl$ (aq). The resulting mixture was extracted with EtOAc (2 x 50 mL). The combined organic layers were washed with brine, dried over $Na_2SO_4$ and then concentrated in high vacuum. The residue was purified by silica gel chromatography (PE/EtOAc=2/1 to 1/1) to give the title compound (110 mg, yield: 42%); m/z (ES+): 714 $[M+H]^+$.

Intermediate 45

(1S,8aS)-N-((R)-1-(3,5-bis(trifluoromethyl)phenl)ethyl)-1-(4-(benzyloxy)-2-methylphenyl)-7,7-bis(2-hydroxyethyl)-N-methyl-6-oxo-hexahydropyrrolo[1,2-a]pyrazine-2(1H)-carboxamide

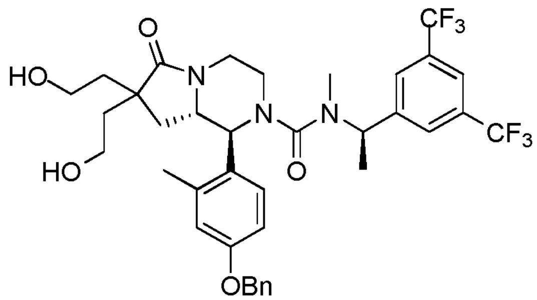

A slow stream of $O_3$ in $O_2$ was passed through a cooled solution of (1S,8aS)-N-((R)-1-(3,5-bis(trifluoromethyl)phenyl)ethyl)-7,7-diallyl-1-(4-(benzyloxy)-2-methylphenyl)-N-methyl-6-oxo-hexahydropyrrolo[1,2-a]pyrazine-2(1H)-carboxamide (110 mg, 0.14 mmol) in DCM (10 mL) at -78°C until a pale blue colour persisted. The reaction was purged with $N_2$ to remove the excess of $O_3$, followed by addition of $NaBH_4$ (106 mg, 2.8 mmol). The reaction mixture was allowed to warm to room temperature and stirred for 20 h. After quenched with saturated $NH_4Cl$ solution, the resulting mixture was extracted with DCM. The organic layer was washed with brine, dried over $Na_2SO_4$, and concentrated in high vacuum. The residue was purified by Prep-HPLC to give the title compound (50 mg, yield: 50%). $^1H$ NMR (400 MHz, $CDCl_3$) $\delta$ 7.78 (s, 1H), 7.55 (s, 2H), 7.44 (d, *J* = 6.8 Hz, 2H), 7.40 (t, *J* = 7.3 Hz, 2H), 7.34 (dd, *J* = 8.3, 5.6 Hz, 1H), 7.16 (d, *J* = 8.5 Hz, 1H), 6.82 (d, *J* = 2.5 Hz, 1H), 6.75 (dd, *J* = 8.5, 2.6 Hz, 1H), 5.56 (q, *J* = 6.8 Hz, 1H), 5.03 (s, 2H), 4.10 (d, *J* = 12.7 Hz, 1H), 3.98 (d, *J* = 9.8 Hz, 1H), 3.81 (dq, *J* = 11.6, 6.4 Hz, 3H), 3.71 (dt, *J* = 11.3, 5.5 Hz, 1H), 3.65 - 3.52 (m, 1H), 3.27 (d, *J* = 11.3 Hz, 1H), 3.06 (ddt, *J* = 20.7, 11.7, 6.0 Hz, 3H), 2.69 (s, 3H), 2.50 (s, 3H), 1.85 (ddd, *J* = 15.9, 11.9, 5.7 Hz, 4H), 1.78 - 1.65 (m, 3H), 1.44 - 1.37 (m, 3H); m/z (ES+): 722 $[M+H]^+$.

Intermediate 46

(1S,8aS)-7,7-diallyl-1-(4-(benzyloxy)-2-methylphenyl)-N-((R)-1-(3-ethyl-5-(trifluoromethyl)phenyl)ethyl)-N-methyl-6-oxohexahydropyrrolo[1,2-a]pyrazine-2(1H)-carboxamide

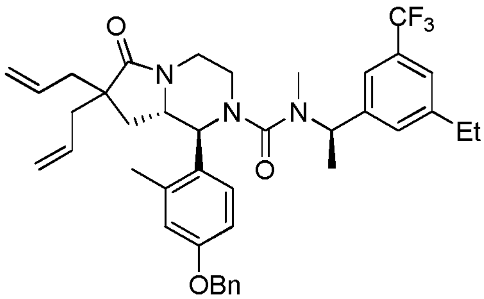

To a solution of triphosgene (53.4 mg, 0.18 mmol) in EtOAc (2 mL) at 0 °C was added solution of (1S,8aS)-7,7-diallyl-1-(4-(benzyloxy)-2-methylphenyl) hexahydropyrrolo [1,2-a]pyrazin-6(7H)-one (150 mg, 0.36 mmol), DMAP (4.6 mg, 0.036 mmol) and TEA (109.2 mg, 1.08 mmol) in EtOAc (2 mL). The mixture was stirred for 1.5 h at room temperature, followed by addition of (R)-1-(3-isopropyl-5-(trifluoromethyl) phenyl)-N-methylethanamine (100 mg, 0.432 mmol) and TEA (109.2 mg, 1.08 mmol) in EtOAc (2 mL). The reaction was stirred at 45 °C for 48 h and quenched with saturated $NH_4Cl$ (aq). The resulting mixture was extracted with EtOAc (2 x 20 mL). The combined organic layers were washed with brine, dried over $Na_2SO_4$ and then concentrated in high vacuum. The residue was purified by silica gel chromatography (PE/EtOAc=2/1 to 1/1) to give the title compound (100 mg, yield: 41%); m/z (ES+): 674 $[M+H]^+$.

Intermediate 47

(1S,8aS)-1-(4-(benzyloxy)-2-methylphenyl)-N-((R)-1-(3-ethyl-5-(trifluoromethyl)phenyl)ethyl)-7,7-bis(2-hydroxyethyl)-N-methyl-6-oxohexahydropyrrolo[1,2-a]pyrazine-2(1H)-carboxamide

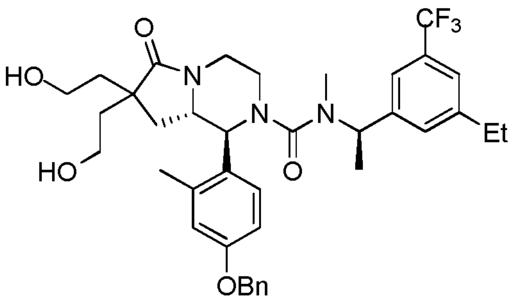

A slow stream of $O_3$ in $O_2$ was passed through a cooled solution of Intermediate 46 (100 mg , 0.15 mmol) in DCM (10 mL) at -78°C until a pale blue colour persisted. The reaction was purged with $N_2$ to remove the excess of $O_3$, followed by addition of $NaBH_4$ (57 mg, 1.5 mmol). The reaction mixture was allowed to warm to room temperature and stirred for 20 h. After quenched with saturated $NH_4Cl$ solution, the resulting mixture was extracted with DCM. The combined organic layers were washed with brine, dried over $Na_2SO_4$, and concentrated in high vacuum. The residue was purified by Prep-HPLC to give the title compound (50 mg, yield: 50%); m/z (ES+): 682 $[M+H]^+$.

Intermediate 48

2-methyl-6-(trifluoromethyl)pyridine

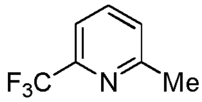

To a solution of 2-chloro-6-(trifluoromethyl)pyridine (25 g, 138 mmol) in THF (200 mL) was added $Me_3Al$ (1.0 M in toluene, 150 mL, 150 mmol) dropwise over 5 min by syringe at 0 °C. After addition, the reaction was stirred at room

temperature for 12 h. Then the reaction mixture was quenched with saturated $NH_4Cl$ solution and extracted with toluene (2 x 20 mL). The combined organic layers were washed with brine, dried over $Na_2SO_4$ and concentrated. The residue was used for next step directly; m/z (ES+): 162 [M+H]+.

Intermediate 49

2-methyl-6-(trifluoromethyl)pyridine 1-oxide

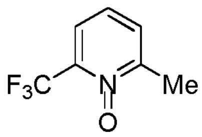

To a solution of 2-methyl-6-(trifluoromethyl)pyridine (22.2 g, 138 mmol) in toluene (200 mL) was added mCPBA (23 g, 150 mmol). The reaction mixture was refluxed for 24 h under $N_2$ atmosphere and quenched with saturated $NH_4Cl$ solution. The resulting mixture was extracted with EtOAc (2 x 150 mL). The combined organic layers were washed with brine, dried over $Na_2SO_4$, and concentrated in high vacuum. The residue was purified by silica gel chromatography (PE/EtOAc=5/1 to 1/1) to give the title compound (5.5 g, yield: 22%); m/z (ES+): 178 [M+H]

Intermediate 50

2-methyl-4-nitro-6-(trifluoromethyl)pyridine 1-oxide

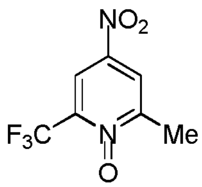

To a solution of 2-methyl-6-(trifluoromethyl)pyridine 1-oxide (6 g, 33.7 mmol) in $H_2SO_4$ (20 mL), was added nitric acid fuming (23 g, 36 mmol). The resulting solution was refluxed for 1 h under $N_2$ atmosphere. The reaction mixture was quenched with ice-water and extracted with EtOAc (2 x 50 mL). The combined organic layers were washed with brine, dried over $Na_2SO_4$, and concentrated in high vacuum. The residue was purified by silica gel chromatography (PE/EtOAc=5/1 to 2/1) to give the title compound (6 g, yield: 80%); m/z (ES+): 222 [M+H]

Intermediate 51

4-bromo-2-methyl-6-(trifluoromethyl)pyridine 1-oxide

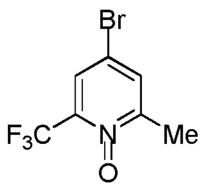

A solution of 2-methyl-4-nitro-6-(trifluoromethyl)pyridine 1-oxide (6 g, 27 mmol) in $CH_3COBr$ (20 mL) was stirred for 8 h and poured into ice-water. The resulting mixture was extracted with EtOAc (2 x 50 mL). The combined organic layers were washed with brine, dried over $Na_2SO_4$, and concentrated in vacuo. The residue was purified by silica gel chromatography (PE/EtOAc=5/1 to 2/1) to give the title compound (3.8 g, yield: 55%); m/z (ES+): 257 [M+H]

Intermediate 52

4-ethyl-2-methyl-6-(trifluoromethyl)pyridine 1-oxide

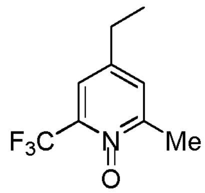

To a solution of 4-bromo-2-methyl-6-(trifluoromethyl)pyridine 1-oxide (2.3 g, 8.9 mmol) in THF (10 mL), was added $Pd(PPh_3)_4$ (115 mg, 1 mmol) and $ZnEt_2$ (1.0 M in toluene, 15 mmol). The resulting solution was stirred for 18 h at room temperature under $N_2$ atmosphere and quenched with saturated $NH_4Cl$ solution. The resulting mixture was extracted with DCM (2 x 20 mL). The combined organic layers were washed with brine, dried over $Na_2SO_4$, and concentrated in high vacuum. The residue was purified by silica gel chromatography (PE/EtOAc=5/1 to 2/1) to give the title compound (1 g, yield: 54%); m/z (ES+): 206$[M+H]^+$.

Intermediate 53

(4-ethyl-6-(trifluoromethyl)pyridin-2-yl)methyl acetate

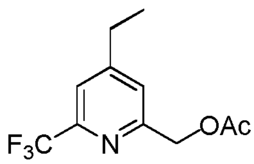

A solution of 4-ethyl-2-methyl-6-(trifluoromethyl)pyridine 1-oxide (1 g, 4.88 mmol) in $Ac_2O$ (10 mL) was stirred at 110 °C for 8 h. The resulting solution was poured into ice-water and extracted with EtOAc (2 x 50 mL). The combined organic layers were washed with brine, dried over $Na_2SO_4$, and concentrated in high vacuum. The residue was purified by silica gel chromatography (PE/EtOAc=5/1 to 2/1) to give the title compound (700 mg, yield: 57%); m/z (ES+): 248 [M+H]

Intermediate 54

(4-ethyl-6-(trifluoromethyl)pyridin-2-yl)methanol

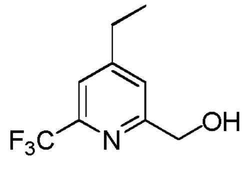

To a solution of (4-ethyl-6-(trifluoromethyl)pyridin-2-yl)methyl acetate (950 mg, 3.83 mmol) in MeOH (20 mL) was added 10% NaOH aq (10 mL). The reaction mixture was stirred for 4 h. The resulting solution was poured into ice-water and extracted with EtOAc (2 x 50 mL). The combined organic layers were washed with brine, dried over $Na_2SO_4$, and concentrated in high vacuum. The residue was purified by silica gel chromatography (PE/EtOAc=5/1 to 2/1) to give the title compound (800 mg, yield: 100%); m/z (ES+): 206 [M+H]

Intermediate 55

4-ethyl-6-(trifluoromethyl)picolinaldehyde

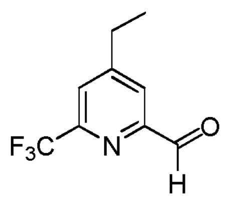

To a solution of (4-ethyl-6-(trifluoromethyl)pyridin-2-yl)methanol (780 g, 3.78 mmol) in DCM (10 mL) at 0 °C was added Dess-Martin reagent (1.1 g, 4.5 mmol). The reaction was stirred for 20 min. The resulting solution was poured into ice-water and extracted with DCM (2 x 50 mL). The combined organic layers were washed with brine, dried over $Na_2SO_4$, and concentrated in vacuo. The residue was purified by silica gel chromatography (PE/EtOAc = 5/1 to 2/1) to give the title compound (640 mg, yield: 83%); m/z (ES+): 204[M+H]$^+$.

Intermediate 56

(S)-N-((4-ethyl-6-(trifluoromethyl)pyridin-2-yl)methylene)-2-methylpropane-2-sulfinamide

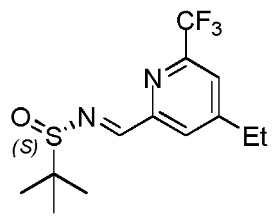

To a solution of 4-ethyl-6-(trifluoromethyl)picolinaldehyde (640 mg, 3.14 mmol) in THF (10 mL) was added (S)-2-methylpropane-2-sulfinamide (480 mg, 4 mmol) and Ti(O-iPr)$_4$ (1.3 g, 5 mmol) at 0 °C. After stirring for 6 h at room temperature, the reaction mixture was poured into a suspension of kieselguhr in water (20 mL) with vigorous stirring. The precipitate was filtered off and the filtrate was extracted with EtOAc (2 x 30 mL). The combined organic layers were washed with brine (10 mL), dried by anhydrous $MgSO_4$ and then concentrated in high vacuum. The residue was purified by silica gel chromatography (PE/EtOAc=10/1 to 1/1) to give the title compound (600 mg, yield: 62%); m/z (ES+): 307 [M+H]$^+$.

Intermediate 57

(S)-N-((R)-1-(4-ethyl-6-(trifluoromethyl)pyridin-2-yl)ethyl)-2-methylpropane-2-sulfinamide

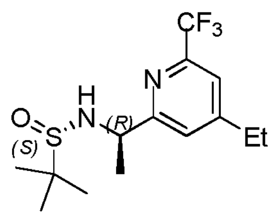

To a solution of (S)-N-((4-ethyl-6-(trifluoromethyl)pyridin-2-yl)methylene)-2-methylpropane-2-sulfinamide (600 mg, 1.94 mmol) in DCM (10 mL) at - 40 °C was dropwise added methyl magnesium bromide (2.1 mL, 1M in THF, 2.1

mmol). After addition, the reaction was warmed to room temperature and stirred for 2 h. The reaction mixture was then poured into saturated aqueous $NH_4Cl$ solution and extracted with DCM (2 x 10 mL). The combined organic layers were washed with brine (2 x 10 mL), dried over anhydrous $MgSO_4$ and then concentrated in high vacuum. The residue was purified by silica gel chromatography (PE/EtOAc=10/1 to 1/1) to give the title compound(550 mg, yield: 88%); m/z (ES+): 323 $[M+H]^+$.

Intermediate 58

(S)-N-((R)-1-(4-ethyl-6-(trifluoromethyl)pyridin-2-yl)ethyl)-N,2-dimethylpropane-2-sulfinamide

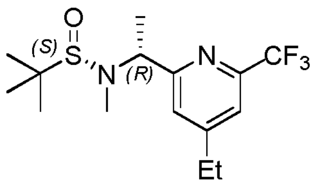

To a solution of (S)-N-((R)-1-(4-ethyl-6-(trifluoromethyl)pyridin-2-yl)ethyl)-2-methylpropane-2-sulfinamide (550 mg, 1.7 mmol) in THF (10 mL) under $N_2$ atmosphere was added LiHMDS (3.2 mL, 1 M in THF, 3.2 mmol) at -60 °C. The reaction was stirred for 15 min followed by addition of MeI (450 mg, 3.2 mmol). The reaction mixture was then stirred at room temperature for another 1 h and quenched with water at 0 °C. The resulting mixture was then extracted with EtOAc (2 x 10 mL). The combined organic layers were dried over anhydrous $Na_2SO_4$ and then concentrated in high vacuum. The residue was purified by silica gel chromatography (PE/EtOAc=10/1 to 1/1) to give the title compound (500 mg, yield: 87%); m/z (ES+): 337 $[M+H]^+$.

Intermediate 59

(R)-1-(4-ethyl-6-(trifluoromethyl)pyridin-2-yl)-N-methylethanamine

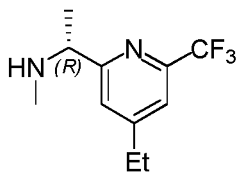

A solution of (S)-N-((R)-1-(4-ethyl-6-(trifluoromethyl)pyridin-2-yl)ethyl)-N,2-dimethylpropane-2-sulfinamide (500 mg, 1.48 mmol) in HCl/MeOH (10 mL) was stirred for 15 min under N2 atmosphere. The reaction was basified with 1 M NaOH to pH=10-11 and then extracted with EtOAc (2 x 20 mL). The combined organic layers were dried over anhydrous $Na_2SO_4$ and then concentrated to give the title compound (380 mg, yield: 110%); m/z (ES+): 233 $[M+H]^+$.

Intermediate 60

(1S,8aS)-7,7-diallyl-N-methyl-N-((4-methyl-6-(trifluoromethyl)pyridin-2-yl)methyl)-6-oxo-1-o-tolylhexahydropyrrolo[1,2-a]pyrazine-2(1H)-carboxamide

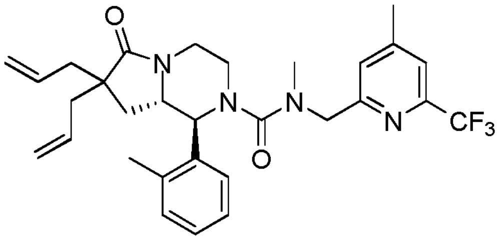

To a solution of triphosgene (47 mg, 0.16 mmol) in EtOAc (2 mL) at 0 °C was added solution of (1S,8aS)-7,7-diallyl-1-o-tolylhexahydropyrrolo[1,2-a]pyrazin-6(7H)-one (100 mg, 0.32 mmol), DMAP (4 mg, 0.032 mmol) and TEA (48 mg, 0.48 mmol) in EtOAc (2 mL). The mixture was stirred for 1.5 h at room temperature, followed by addition of N-methyl-1-(4-methyl-6-(trifluoromethyl)pyridin-2-yl)methanamine (97mg, 0.96 mmol) and TEA (48mg, 0.48 mmol) in EtOAc (2 mL). The reaction was stirred at 45 °C for 48 h and quenched with sat. $NH_4Cl$(aq). The resulting mixture was extracted with EtOAc (2 x 20 mL). The combined organic layers were washed with brine, dried over $Na_2SO_4$ and then concentrated in high vacuum. The residue was purified by silica gel chromatography (PE/EtOAc=2/1 to 1/1) to give the title compound (90 mg, yield: 52.1%); m/z (ES+): 541 $[M+H]^+$.

Intermediate 61

(S)-tert-butyl 2-(2-(2,4-dimethylbenzoyl)-5-oxopyrrolidin-1-yl)ethylcarbamate

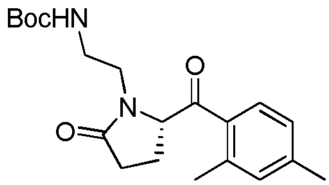

Intermediate 61 was prepared using the procedure for intermeidate 21. m/z (ES+): 395 $[M+H]^+$

Intermediate 62

tert-butyl 2-((S)-2-(R)-(2,4-dimethylphenyl)(hydroxy)methyl)-5-oxopyrrolidin-1-yl)ethylcarbamate

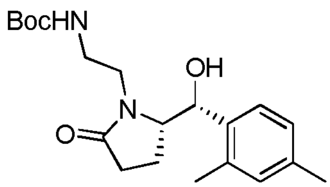

Intermediate 62 was prepared using the procedure for intermeidate 22. m/z (ES+): 363 $[M+H]^+$

Intermediate 63

(1S,8aS)-1-(2,4-dimethylphenyl)hexahydropyrrolo[1,2-a]pyrazin-6(7H)-one

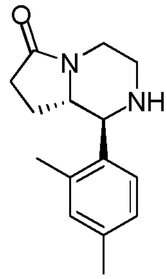

To a solution of (S)-benzyl 2-(2-(2,4-dimethylbenzoyl)-5-oxopyrrolidin-1-yl)ethylcarbamate (7 g, 17.8 mmol) in 80 mL of MeOH was added 1 M HCl (35 mL) and Pd/C 5% (1 g). The reaction was stirred under $H_2$ (5 atm) atmosphere for 12 h. The reaction mixture was filtered through a celite pad and concentrated in high vacuum. The residue was dissolved in MeOH (30ml) and $NaBH_3(CN)$ (1.3 g, 20.5 mmol) was added. The reaction was stirred for 5 h at room temperature and filtered. The filtrate was concentrated in high vacuum. The residue was purified by silica gel chromatography (PE/EtOAc=2/1 to 1/2) to give the title compound (2.3 g, 53%); m/z (ES+): 245 $[M+H]^+$

Intermediate 64

(1S,8aS)-tert-butyl 1-(2,4-dimethylphenyl)-6-oxohexahydropyrrolo[1,2-a]pyrazine-2(1H)-carboxylate

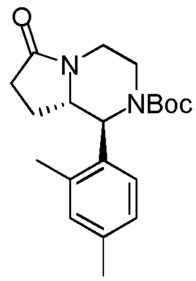

To a solution of (1S,8aS)-1-(2,4-dimethylphenyl)hexahydropyrrolo[1,2-a]pyrazin-6(7H)-one (1.5 g, 6.1 mmol) was added $(Boc)_2O$ (2.65 g, 12.2 mmol) at O°C, the reaction mixture was stirred overnight at 25 °C,followed by addition of $N^1,N^1$-dimethylethane-1,2-diamine (5 mL). The mixture was stirred at 25 °C for 8 h and quenched by sat. $NH_4Cl$ solution. The resulting mixture was extracted with EtOAc (2 x 20 mL). The combined organic layers were dried over anhydrous $Na_2SO_4$, and concentrated in high vacuum to give the title compound (2 g, 95%) which was used in the next step without further purification; m/z (ES+): 345 $[M+H]^+$.

Intermediate 65

(1S,8aS)-tert-butyl 7,7-diallyl-1-(2,4-dimethylphenyl)-6-oxohexahydropyrrolo[1,2-a]pyrazine-2(1H)-carboxylate

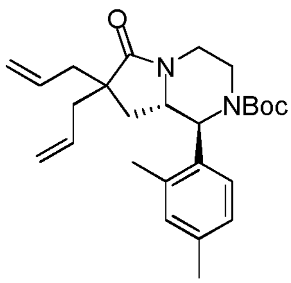

To a solution of (1S,8aS)-tert-butyl 1-(2,4-dimethylphenyl)-6-oxohexahydropyrrolo[1,2-a]pyrazine-2(1H)-carboxylate (2 g, 5.8 mmol) in anhydrous THF (60 mL) under $N_2$ atmosphere, was added LiHMDS (1 M in THF, 6.05 mL)

at -78 °C. The reaction was stirred for 15 min, followed by addition of DMPU (1.8 mL, 15 mmol) and allylbromide (0.52 mL, 6 mmol). The reaction mixture was stirred at -78 °C for 30 min and allowed to warm to -30 °C, and then stirred at this temperature for another 2 h. After quenched with water at -78 °C and the mixture was then extracted with EtOAc (2 x 50 mL). The combined organic layers were washed with brine, dried over $Na_2SO_4$, and concentrated in vacuo. The residue was purified by silica gel chromatography (PE/EtOAc=2/1 to 1/1) to give the title compound (2 g, yield: 81%); m/z (ES+): 425 $[M+H]^+$.

Intermediate 66

(1S,8aS)-7,7-diallyl-1-(2,4-dimethylphenyl)hexahydropyrrolo[1,2-a]pyrazin-6(7H)-one

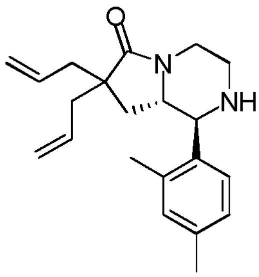

To a solution of (1S,8aS)-tert-butyl 7,7-diallyl-1-(2,4-dimethylphenyl)-6-oxohexahydropyrrolo [1,2-a]pyrazine-2(1H)-carboxylate (2 g, 4.7 mmol) in DCM (40 mL) at 0 °C was added TFA (8 mL). The reaction was stirred at room temperature for 3 h and basified with 1 M aqueous NaOH solution to pH=10. The mixture was extracted with EtOAc (2 x 20 mL). The combined organic layers were washed with brine, dried over anhydrous $Na_2SO_4$, and concentrated in vacuo. The residue was directly used in the next step without further purification (1.2 g, yield: 79%); m/z (ES+): 325 $[M+H]^+$.

Intermediate 67

(1S,8aS)-7,7-diallyl-N-((R)-1-(3-chloro-5-(trifluoromethyl)phenyl)ethyl)-1-(2,4-dimethylphenyl)-N-methyl-6-oxo-hexahydropyrrolo[1,2-a]pyrazine-2(1H)-carboxamide

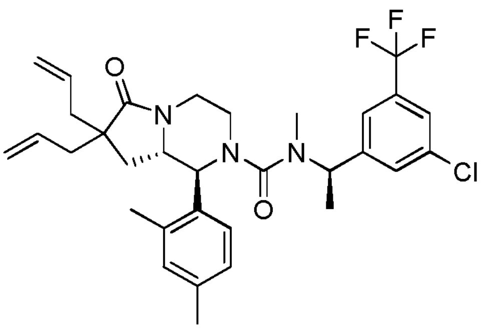

To a solution of triphosgene (205 mg, 0.70 mmol) in EtOAc (10 mL) at 0 °C was added solution of (1S,8aS)-7,7-diallyl-1-(2,4-dimethylphenyl)hexahydropyrrolo[1,2-a]pyrazin-6(7H)-one (500 mg, 1.54 mmol), DMAP (40 mg, 0.32 mmol) and TEA (480 mg, 4.8 mmol) in EtOAc (10 mL). The mixture was stirred for 1.5 h at room temperature, followed by addition of (3,5-bis(trifluoromethyl)phenyl)-N-methylmethan amine (514 mg, 2 mmol) and TEA (480 mg, 4.8 mmol) in EtOAc (2 mL). The reaction was stirred at 45 °C for 48 h and quenched with sat. $NH_4Cl$(aq) solution. The resulting mixture was extracted with EtOAc (2 x 100 mL). The combined organic layers were washed with brine, dried over anhydrous $Na_2SO_4$ and then concentrated in high vacuum. The residue was purified by silica gel chromatography (PE/EtOAc=2/1 to 1/1) to give the title compound (80 mg, yield: 9%); m/z (ES+): 588 $[M+H]^+$.

Intermediate 68

(1S,8aS)-7,7-diallyl-1-(2,4-dimethylphenyl)-N-((R)-1-(3-isopropyl-5-(trifluoromethyl)phenyl)ethyl)-N-methyl-6-oxohexahydropyrrolo[1,2-a]pyrazine-2(1H)-carboxamide

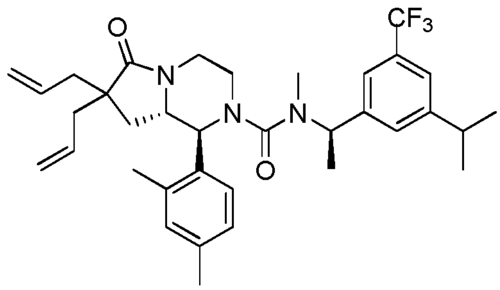

To a solution of triphosgene (65.3 mg, 0.22 mmol) in EtOAc (2 mL) at 0 °C was added solution of (1S,8aS)-7,7-diallyl-1-(2,4-dimethylphenyl)hexahydropyrrolo [1,2-a]pyrazin-6(7H)-one (140 mg, 0.43 mmol), DMAP (5.3 mg, 0.043 mmol) and TEA (130.5 mg, 1.29 mmol) in EtOAc (2 mL). The mixture was stirred for 1.5 h at room temperature, followed by addition of (R)-1-(3-isopropyl-5-(trifluoromethyl)phenyl)-N-methyl ethanamine (126.5 mg, 0.516 mmol) in EtOAc (2 mL) and TEA (130.5 mg, 1.29 mmol). The reaction was stirred at 45 °C for 48 h and quenched with sat. $NH_4Cl$ (aq) solution. The resulting mixture was extracted with EtOAc (2 x 100 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (120 mg, yield: 47%); m/z (ES+): 596 $[M+H]^+$.

Intermediate 69

(1S,8aS)-N-((R)-1-(3,5-bis(trifluoromethyl)phenyl)ethyl)-7,7-diallyl-1-(2-methoxyphenyl)-N-methyl-6-oxo-hexahydropyrrolo[1,2-a]pyrazine-2(1H)-carboxamide

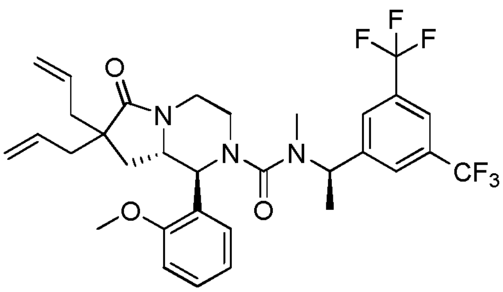

To a solution of triphosgene (65.3 mg, 0.22 mmol) in EtOAc (2 mL) at 0 °C was added solution of (1S,8aS)-7,7-diallyl-1-(2-methoxyphenyl)hexahydropyrrolo[1,2-a]pyrazin-6(7H)-one (140 mg, 0.43 mmol), DMAP (5.3 mg, 0.043 mmol) and TEA (130.5 mg, 1.29 mmol) in EtOAc (2 mL). The mixture was stirred for 1.5 h at room temperature, followed by addition of (R)-1-(3,5-bis(trifluoromethyl)phenyl)-N-methylethanamine (139 mg, 0.516 mmol) in EtOAc (2 mL) and TEA (130.5 mg, 1.29 mmol). The reaction was stirred at 45 °C for 48 h and quenched with sat. $NH_4Cl$ (aq) solution. The resulting mixture was extracted with EtOAc (2 x 100 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (45 mg, yield: 17%); m/z (ES+): 624 $[M+H]^+$.

Intermediate 70

(S)-tert-butyl 2-(2-(2,5-dimethylbenzoyl)-5-oxopyrrolidin-l-yl)ethylcarbamate

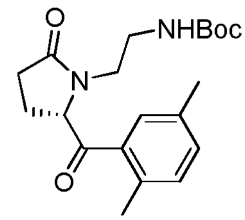

To a solution of 2-Bromo-1,4-dimethyl-benzene (1.7 g, 12.39 mmol) in THF (40 mL) was added n-BuLi (1.6 M in hexane) (7.52 mL, 12.036mmol) dropwise over 5 min at -70 °C. The reaction was stirred at this temperature for 30 min. Then the resulting solution was added into a solution of (S)-tert-butyl 1,6-dioxohexahydropyrrolo[1,2-a]pyrazine-2(1*H*)-carboxylate (3 g, 11.8 mmol) in THF (40 mL). After stirred at -70 °C for 5 min, the reaction mixture was quenched with saturated NH4Cl solution and extracted with EtOAc (2 x 20 mL). The combined organic layers were washed with brine, dried over anhydrous Na2SO4, and concentrated in vacuo. The residue was purified by silica gel chromatography (PE/EtOAc=2/1 to 1/1) to give the title compound (2.5 g, yield: 55.6%); m/z (ES+): 361 [M+H]+

Intermediate 71

tert-butyl 2-((S)-2-((R)-(2,5-dimethylphenyl)(hydroxy)methyl)-5-oxopyrrolidin-1-yl)ethylcarbamate

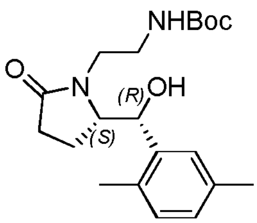

To a solution of (S)-tert-butyl 2-(2-(2,5-dimethylbenzoyl)-5-oxopyrrolidin-1-yl)ethyl carbamate (2.5 g, 6.9 mmol) in THF (50 mL) was dropwise added $LiAl(OtBu)_3H$ (3.56 g, 14 mmol) at -20 °C. Then the mixture was stirred at room temperature for 2 h and then poured into the saturated $NH_4Cl$ solution. The resulting suspension was filtered through a celite pad, washed by EtOAc. The filtrate was extracted with EtOAc (2 x 50 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (2.5 g, yield: 98%); m/z (ES+): $363[M+H]^+$.

Intermediate 72

(R-)-((S)-1-(2-(tert-butoxycarbonyl)ethyl)-5-oxopyrrolidin-2-yl)(2,5-dimethylphenyl) methyl methanesulfonate

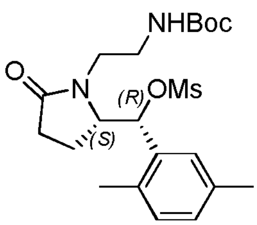

To a solution of tert-butyl 2-((S)-2-((R)-(2,5-dimethylphenyl)(hydroxy)methyl)-5-oxopyrrolidin-1-yl)ethylcarbamate (2.5 g, 6.9 mmol) in DCM (20 mL) was added $Et_3N$ (3.54 g, 35 mmol), DMAP (85.6 mg, 0.7 mmol). The resulting solution was cooled to 0 °C, and then MsCl (1.18 g, 10.3 mmol) was dropwisely added into the above solution under $N_2$ atmosphere. The reaction was stirred for 20 h at room temperature and then quenched with saturated $NH_4Cl$ solution and extracted with DCM (2 x 30 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (800 mg, yield: 27%); m/z (ES+): 441 $[M+H]^+$.

Intermediate 73

(1S,8aS)-tert-butyl 1-(2,5-dimethylphenyl)-6-oxo-hexahydropyrrolo[1,2-a]pyrazine-2(1H)-carboxylate

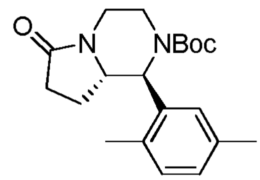

To a suspension of NaH (60%, 128 mg, 3.2 mmol) in THF (20 mL) was added a solution of (R)-((S)-1-(2-(tert-butoxycarbonyl)ethyl)-5-oxopyrrolidin-2-yl)(2,5-dimethylphenyl) methyl methanesulfonate (700 mg, 1.6 mmol) in THF (10 mL) at 0 °C under $N_2$ atmosphere. The mixture was stirred at room temperature for 20 h and then quenched with ice-water. The resulting mixture was extracted with EtOAc (2 x 30 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (500 mg, yield: 91%); m/z (ES+): 345 $[M+H]^+$.

Intermediate 74

(1R,8aS)-tert-butyl7,7-diallyl-1-(2,5-dimethylphenyl)-6-oxo-hexahydropyrrolo[1,2-a]pyrazine-2(1H)-carboxylate

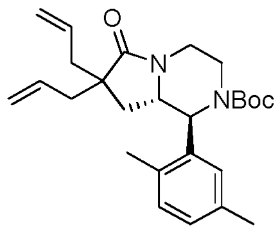

To a solution of (1S,8aS)-tert-butyl 1-(2,5-dimethylphenyl)-6-oxo-hexahydropyrrolo [1,2-a]pyrazine-2(1H)-carboxylate (700 mg, 2.03 mmol) in anhydrous THF (100 mL) under $N_2$ atmosphere, was added LiHMDS (1 M in THF, 3.4 mL) at -78 °C. The reaction was stirred for 15 min, followed by addition of DMPU (895 mg, 7 mmol) and allylbromide (316.5 mg, 2.62 mmol). The reaction mixture was stirred at -78 °C for 30 min and allowed to warm to -30 °C, and then stirred at this temperature for another 2 h. After quenched with water at -78 °C, the mixture was then extracted with EtOAc (2 x 50 mL). The combined organic layers were washed with brine, dried over anhydrous $Na_2SO_4$, and concentrated in vacuo. The residue was purified by silica gel chromatography (PE/EtOAc=2/1 to 1/1) to give the title compound (600mg, yield: 70%); m/z (ES+): 425 $[M+H]^+$

Intermediate 75

(1R,8aS)-tert-butyl7,7-diallyl-1-(2,5-dimethylphenyl)-6-oxo-hexahydropyrrolo[1,2-a]pyrazine-2(1H)-carboxylate

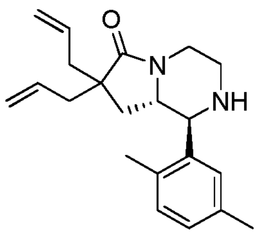

To a solution of (1R,8aS)-tert-butyl 7,7-diallyl-1-(2,5-dimethylphenyl)-6-oxo-hexahydropyrrolo[1,2-a]pyrazine-2(1H)-carboxylate (600 mg, 1.41 mmol) in MeOH (20 mL) was added HCl/MeOH (7 M, 5 mL) at 0 °C. The reaction was stirred at room temperature for 3 h and basified with 1 M aqueous NaOH solution to pH=10. The mixture was extracted with EtOAc (2 x 20 mL). The combined organic layers were washed with brine, dried over $Na_2SO_4$, and concentrated in vacuo. The residue was directly used in the next step without further purification (180 mg, yield: 95%); m/z (ES+): 325 $[M+H]^+$.

Intermediate 76

(1S,8aS)-N-((R)-1-(3,5-bis(trifluoromethyl)phenyl)ethyl)-7,7-diallyl-1-(2,5-dimethylphenyl)-N-methyl-6-oxo-hexahydropyrrolo[1,2-a]pyrazine-2(1H)-carboxamide

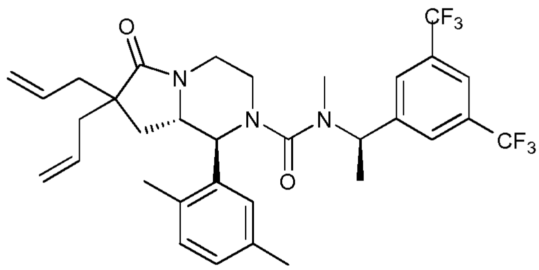

To a solution of triphosgene (73.45 mg, 0.25 mmol) in EtOAc (2 mL) at 0 °C was added solution of (1R,8aS)-tert-butyl 7,7-diallyl-1-(2,5-dimethylphenyl)-6-oxo-hexahydro pyrrolo[1,2-a]pyrazine-2(1H)-carboxylate (162 mg, 0.5 mmol), DMAP (6.1 mg, 0.05 mmol) and TEA (151.68 mg, 1.5 mmol) in EtOAc (2 mL). The mixture was stirred for 1.5 h at room temperature, followed by addition of (R)-1-(3,5-bis(trifluoromethyl)phenyl)-N-methylethanamine (163.2 mg, 0.6 mmol) and TEA (151.68 mg, 1.5 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed brine, dried over anhydrous $Na_2SO_4$ and then concentrated in high vacuum. The residue was purified by silica gel chromatography (PE/EtOAc=2/1 to 1/1) to give the title compound (120 mg, yield: 39%); m/z (ES+): 622 $[M+H]^+$.

Intermediate 77

(1S,8aS)-7,7-diallyl-1-(2,5-dimethylphenyl)-N-((R)-1-(3-isopropyl-5-(trifluoromethyl)phenyl)ethyl)-N-methyl-6-oxohexahydropyrrolo[1,2-a]pyrazine-2(1H)-carboxamide

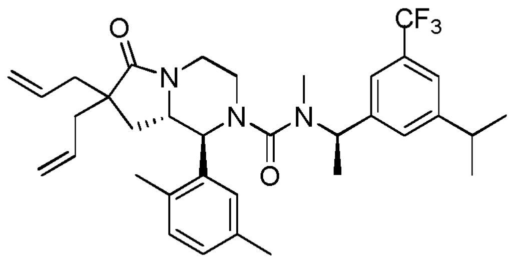

To a solution of triphosgene (44.5 mg, 0.15 mmol) in EtOAc (2 mL) at 0 °C was added solution of (1R,8aS)-tert-butyl 7,7-diallyl-1-(2,5-dimethylphenyl)-6-oxo-hexahydro pyrrolo[1,2-a]pyrazine-2(1H)-carboxylate (97.2 mg, 0.3 mmol), DMAP (3.7 mg, 0.03 mmol) and TEA (92 mg, 0.9 mmol) in EtOAc (2 mL). The mixture was stirred for 1.5 h at room temperature, followed by addition of (R)-1-(3-isopropyl-5-(trifluoromethyl) phenyl)-N-methyl ethanamine (91.5 mg, 0.36 mmol) in EtOAc (2 mL) and TEA (92 mg, 0.9 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (100 mg, yield: 56.1%); m/z (ES+): 596 $[M+H]^+$.

Intermediate 78

(S)-tert-butyl 2-(2-(2,3-dimethylbenzoyl)-5-oxopyrrolidin-1-yl)ethylcarbamate

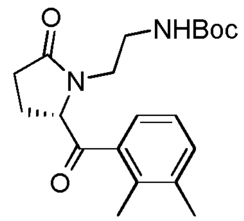

To a solution of 1-bromo-2, 3-dimethylbenzene (2.28 g, 12.39 mmol) in THF (40 mL) at -70 °C was dropwise added n-BuLi (1.6 M in hexane, 7.52 mL, 12.0 mmol) over 5 min. The reaction was stirred at this temperature for 30 min. Then the resulting solution was added into a solution of (S)-tert-butyl 1,6-dioxohexahydropyrrolo[1,2-a]pyrazine-2(1*H*)-carboxylate (3 g, 11.8 mmol) in THF (40 mL). After stirred at -70 °C for 5 min, the reaction mixture was quenched with saturated NH4Cl solution and extracted with EtOAc (2 x 20 mL). The combined organic layers were washed with brine, dried over anhydrous $Na_2SO_4$, and concentrated in vacuo. The residue was purified by silica gel chromatography (PE/EtOAc=5/1 to 1/1)to give the title compound (2.5 g, yield: 59%); m/z (ES+): 361 [M+H]+.

Intermediate 79

tert-butyl 2-((S)-2-(R)-(2,3-dimethylphenyl)(hydroxy)methyl)-5-oxopyrrolidin-1-yl)ethylcarbamate

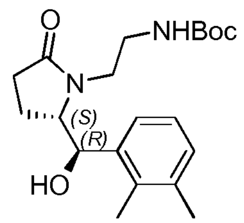

To a solution of (S)-tert-butyl 2-(2-(2,3-dimethylbenzoyl)-5-oxopyrrolidin-1-yl)ethyl carbamate (2.5 g, 6.9 mmol) in THF (50 mL) was dropwise added $LiAl(OtBu)_3H$ (3.56 g, 14 mmol) at -20 °C. Then the mixture was stirred at room temperature for 2 h and quenched with saturated $NH_4Cl$ solution. The resulting suspension was filtered through a celite pad, washed by EtOAc. The filtrate was extracted with EtOAc (2 x 50 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (2.5 g, yield: 99.7%); m/z (ES+): 363$[M+H]^+$.

Intermediate 80

(R)-((S)-1-(2-(tert-butoxycarbonylamino)ethyl)-5-oxopyrrolidin-2-yl)(2,3-dimethylphenyl)methyl methanesulfonate

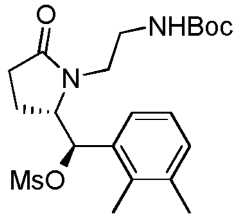

To a solution tert-butyl 2-((S)-2-((R)-(2,3-dimethylphenyl)(hydroxy)methyl)-5-oxopyrrolidin-1-yl)ethylcarbamate (2.5 g, 6.9 mmol), $Et_3N$ (3.54 g, 35 mmol) and DMAP (85.6 mg, 0.7 mmol) in DCM (20 mL), was dropwise added MsCl (1.18 g, 10.3 mmol) under $N_2$ atmosphere. The reaction was stirred for 20 h at room temperature and then quenched with saturated $NH_4Cl$ solution. The resulting mixture was extracted with DCM (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (800 mg, yield: 27%); m/z (ES+): 441 $[M+H]^+$.

Intermediate 81

(1S, 8aS)-tert-butyl1-(2,3-dimethylphenyl)-6-oxohexahydropyrrolo[1,2-a]pyrazine-2(1H)-carboxylate

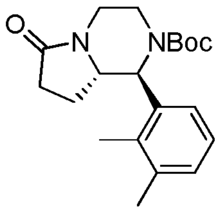

To a suspension of NaH (60%, 128 mg, 3.2 mmol) in THF (20 mL) was added a solution of (R)-((S)-1-(2-(tert-butoxycarbonylamino)ethyl)-5-oxopyrrolidin-2-yl)(2,3-dimethyl phenyl)methyl methanesulfonate (700 mg, 1.6 mmol) in THF (10 mL) at 0 °C under $N_2$ atmosphere. The mixture was stirred at room temperature for 20 h and then quenched with ice-water. The resulting mixture was extracted with EtOAc (2 x 30 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (500 mg, yield: 91%); m/z (ES+): 345 $[M+H]^+$.

Intermediate 82

(S)-tert-butyl 7,7-diallyl-1-(2,3-dimethylphenyl)-6-oxohexahydropyrrolo[1,2-a]pyrazine-2(1H)-carboxylate

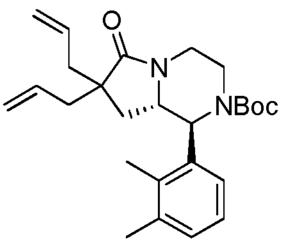

To a solution of (1S,8aS)-tert-butyl1-(2,3-dimethylphenyl)-6-oxohexahydropyrrolo[1,2-a]pyrazine-2(1H) -carboxylate (300 mg, 0.872 mmol) in anhydrous THF (100 mL) under $N_2$ atmosphere, was added LiHMDS (1 M in THF, 3.4 mL) at -78 °C. The reaction was stirred for 15 min, followed by addition of DMPU (895 mg, 7 mmol) and allylbromide (316.5 mg, 2.62 mmol). The reaction mixture was stirred at -78 °C for 30 min and allowed to warm to -30 °C, and then stirred at this temperature for another 2 h. After quenched with water at -78 °C, the mixture was then extracted with EtOAc (2 x 50 mL). The combined organic layers were washed with brine, dried over anhydrous $Na_2SO_4$, and concentrated in vacuo. The residue was purified by silica gel chromatography (PE/EtOAc=2/1 to 1/1) to give the title compound (250

mg, yield: 70%); m/z (ES+): 425 [M+H]$^+$.

Intermediate 83

(1S,8aS)-7,7-diallyl-1-(2,3-dimethylphenyl)hexahydropyrrolo[1,2-a]pyrazin-6(7H)-one

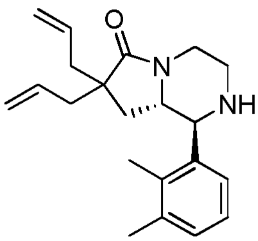

To a solution of (S)-tert-butyl 7,7-diallyl-1-(2,3-dimethylphenyl)-6-oxohexahydropyrrolo [1,2-a] pyrazine-2(1H)-carboxylate (250 mg, 0.59 mmol) in MeOH (20 mL) at 0 °C was added HCl/MeOH (7 M, 5 mL). The reaction was stirred at room temperature for 3 h and basified with 1 M aqueous NaOH solution to pH=10. The mixture was extracted with EtOAc (2 x 20 mL). The combined organic layers were washed with brine, dried over anhydrous $Na_2SO_4$, and concentrated in vacuo. The residue was directly used in the next step without further purification (180 mg, yield: 95%); m/z (ES+): 325 [M+H]$^+$.

Intermediate 84

(1S,8aS)-7,7-diallyl-N-((R)-1-(3,5-bis(trifluoromethyl)phenyl)ethyl)-1-(2,3-dimethylphenyl)-N-methyl-6-oxohexahydropyrrolo[1,2-a]pyrazine-2(1H)-carboxamide

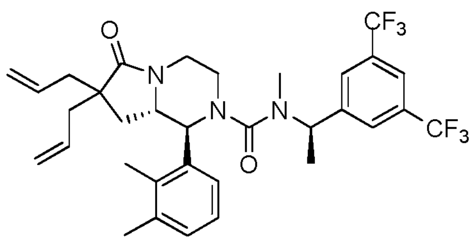

To a solution of triphosgene (73.5 mg, 0.25 mmol) in EtOAc (2 mL) at 0 °C was added solution of (1S,8aS)-7,7-diallyl-1-(2,3-dimethylphenyl)hexahydropyrrolo[1,2-a]pyrazin-6(7H)-one (162 mg, 0.5 mmol), DMAP (6.1 mg, 0.05 mmol) and TEA (151.7 mg, 1.5 mmol) in EtOAc (2 mL). The mixture was stirred for 1.5 h at room temperature, followed by addition of (R)-1-(3,5-bis(trifluoromethyl)phenyl)-N-methyl ethanamine (163.2 mg, 0.6 mmol) in EtOAc (2 mL) and TEA (151.68 mg, 1.5 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (120 mg, yield: 39%); m/z (ES+): 622 [M+H]$^+$.

Intermediate 85

(1S,8aS)-1-(2,3-dimethylphenyl)-hexahydropyrrolo[1,2-a]pyrazin-6(7H)-one

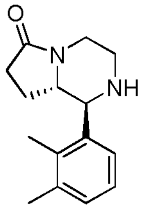

To a solution of (1S, 8aS)-tert-butyl1-(2,3-dimethylphenyl)-6-oxohexahydropyrrolo[1,2-a]pyrazine-2(1H) -carboxylate (500 mg, 1.45 mmol) in MeOH (20 mL) at 0 °C was added HCl/MeOH (7 M, 5 mL). The reaction was stirred at room temperature for 3 h and basified with 1 M aqueous NaOH solution to pH=10. The mixture was extracted with EtOAc (2 x 20 mL). The combined organic layers were washed with brine, dried over anhydrous $Na_2SO_4$, and concentrated in vacuo. The residue was directly used in the next step without further purification (300 mg, yield: 84%); m/z (ES+): 245 $[M+H]^+$.

Intermediate 86

(S)-tert-butyl 2-(2-(3-methylpicolinoyl)-5-oxopyrrolidin-1-yl)ethylcarbamate

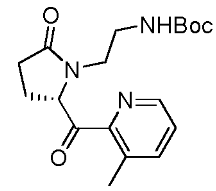

To a solution of 2-bromo-3-methylpyridine (1.7 g, 9.8 mmol) in THF (40 mL) at -70 °C was dropwise added n-BuLi (1.6 M in hexane, 7.52 mL, 12.0 mmol) over 5 min. The reaction was stirred at this temperature for 30 min. Then the resulting solution was added into a solution of (S)-tert-butyl 1,6-dioxohexahydropyrrolo[1,2-a]pyrazine-2(1*H*)-carboxylate (3 g, 11.8 mmol) in THF (40 mL). After stirred at -70 °C for 5 min, the reaction mixture was quenched with saturated NH4Cl solution and extracted with EtOAc (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (700 mg, yield: 28%); m/z (ES+): 348 $[M+H]^+$.

Intermediate 87

tert-butyl2-(2-((S)-hydroxy(3-methylpyridin-2-yl)methyl)-5-oxopyrrolidin-1-yl)ethylcarbamate (mixture)

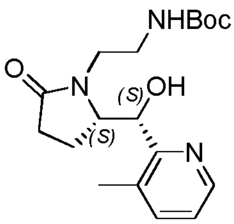

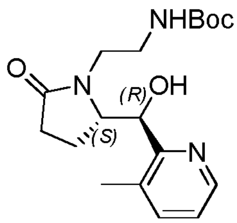

To a solution of (S)-tert-butyl 2-(2-(3-methylpicolinoyl)-5-oxopyrrolidin-1-yl)ethylcarbamate (2.5 g, 6.9 mmol) in THF (50 mL) was portionwise added $LiAl(OtBu)_3H$ (700 mg, 2.01 mmol) at -20 °C. Then the mixture was stirred at room temperature for 2 h and quenched with saturated $NH_4Cl$ solution. The resulting suspension was filtered through a celite pad, washed by EtOAc. The filtrate was extracted with EtOAc (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (400 mg, yield: 57.1%); m/z (ES+): 350$[M+H]^+$.

Intermediate 88

1-(2-(tert-butoxycarbonyl)ethyl)-5-oxopyrrolidin-2-yl)(3-methylpyridin-2-yl)methyl methanesulfonate (mixture)

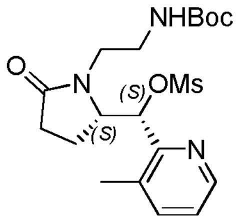
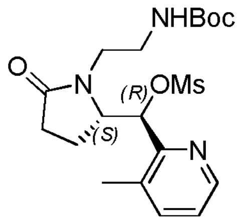

To a solution of tert-butyl 2-((S)-2-((S)-hydroxy(3-methylpyridin-2-yl)methyl)-5-oxopyrrolidin-1-yl)ethylcarbamate (400 mg, 1.14 mmol), $Et_3N$ (3.54 g, 35 mmol) and DMAP (85.6 mg, 0.7 mmol) in DCM (20 mL) was dropwise added MsCl (1.18 g, 10.3 mmol) under $N_2$ atmosphere. The reaction was stirred for 20 h at room temperature and then quenched with saturated $NH_4Cl$ solution. The resulting mixture was extracted with DCM (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (380 mg, yield: 95%); m/z (ES+): 428 $[M+H]^+$.

Intermediate 89

tert-butyl 1-(3-methylpyridin-2-yl)-6-oxo-hexahydropyrrolo[1,2-a]pyrazine-2(1H)-carboxylate (mixture)

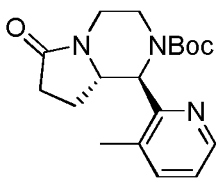
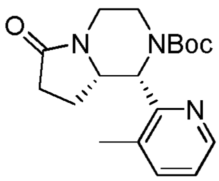

To a suspension of NaH (60%, 128 mg, 3.2 mmol) in THF (20 mL) was added a solution of (S)-((S)-1-(2-(tert-butoxycarbonyl)ethyl)-5-oxopyrrolidin-2-yl)(3-methylpyridin-2-yl)methyl methanesulfonate (380 mg, 0.89 mmol) in THF (10 mL) at 0 °C under $N_2$ atmosphere. The mixture was stirred at room temperature for 20 h and then quenched with ice-water. The resulting mixture was extracted with EtOAc (2 x 30 mL), washed with brine, dried over anhydrous $Na_2SO_4$, and concentrated in vacuo. The residue was purified by silica gel chromatography (PE/EtOAc=5/1 to 1/1) to give the title compound (200 mg, yield: 53%); m/z (ES+): 331 $[M+H]^+$.

Intermediate 90

(1R,8aS)-1-(3-methylpyridin-2-yl)-hexahydropyrrolo[1,2-a]pyrazin-6(7H)-one (mixture)

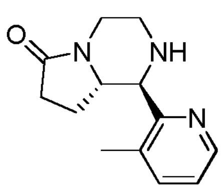
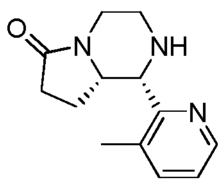

To a solution of (1R,8aS)-tert-butyl 1-(3-methylpyridin-2-yl)-6-oxo-hexahydropyrrolo [1,2-a]pyrazine-2(1H)-carboxylate (200 mg, 0.60 mmol) in MeOH (20 mL) at 0 °C was added HCl/MeOH (7 M, 5 mL). The reaction was stirred at

room temperature for 3 h and basified with 1 M aqueous NaOH solution to pH=10. The mixture was extracted with EtOAc (2 x 20 mL). The combined organic layers were washed with brine, dried over anhydrous $Na_2SO_4$, and concentrated in vacuo. The residue was directly used in the next step without further purification (180 mg, yield: 95%); m/z (ES+): 232 $[M+H]^+$.

Intermediate 91-93 were prepared using the procdure for intermediate 90.

Intermediate 91

(1S,8aS)-1-(4-methylpyridin-3-yl)hexahydropyrrolo[1,2-a]pyrazin-6(7H)-one

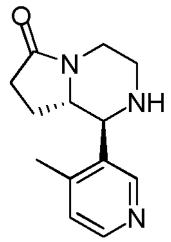

m/z (ES+): 232 $[M+H]^+$.

Intermediate 92

(1S,8aS)-1-(3-methylpyridin-4-yl)hexahydropyrrolo[1,2-a]pyrazin-6(7H)-one

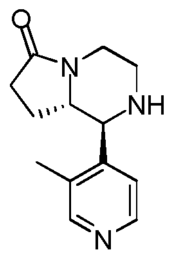

m/z (ES+): 232 $[M+H]^+$.

Intermediate 93

(1S,8aS)-1-(2-methylpyridin-3-yl)hexahydropyrrolo[1,2-a]pyrazin-6(7H)-one

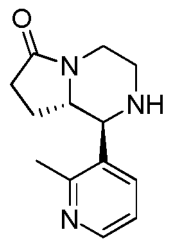

m/z (ES+): 232 $[M+H]^+$.

Intermediate 94

(S)-tert-butyl 2-(2-(4-methylthiophene-3-carbonyl)-5-oxopyrrolidin-1-yl)ethylcarbamate

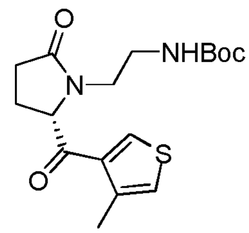

To a solution of 3-bromo-4-methylthiophene (1.25 g, 7.1 mmol) in THF (10 mL) was added n-BuLi(2.5 M) (2.8 ml, 7.1 mmol) at -35 °C. The reaction was stirred at the same temperature for 30 min, and added to (S)-tert-butyl 1,6-dioxohexahydropyrrolo[1,2-a]pyrazine-2(1H)-carboxylate (1.5 g, 5.9 mmol) in 10 ml THF. After stirred for 5 min, the reaction mixture was quenched with saturated $NH_4Cl$ solution and extracted with EtOAc (2 x 20 mL). The organic layers were dried over anhydrous $Na_2SO_4$ and concentrated in high vacuum. The residue was purified by silica gel chromatography (PE/EtOAc=10/1 to 1/1) to give the title compound (800 mg, yield: 38%); m/z (ES+): 353 $[M+H]^+$.

Intermediate 95

tert-butyl2-((S)-2-((R)-hydroxy(4-methylthiophen-3-yl)methyl)-5-oxopyrrrolidin-1-yl)ethylcarbamate

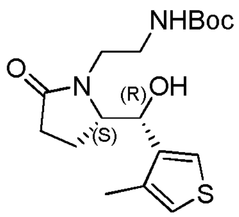

To a solution of (S)-tert-butyl 2-(2-(4-methylthiophene-3-carbonyl)-5-oxopyrrolidin-1-yl)ethylcarbamate (800 mg, 2.3 mmol) in THF (10 mL) was portion-wise added $LiAl(O^tBu_3)H$ (866 mg, 3.4 mmol) at -20 °C. Then the mixture was stirred at room temperature for 2 h and quenched with saturated $NH_4Cl$ solution. The resulting suspension was filtered through a celite pad, washed by EtOAc. The filtrate was extracted with EtOAc (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (730 mg, yield: 91%); m/z (ES+): 355$[M+H]^+$.

Intermediate 96

(R)-((S)-1-(2-(tert-butoxycarbonylamino)ethyl)-5-oxopyrrolidin-2-yl)(4-methylthiophen-3-yl)methyl methanesulfonate

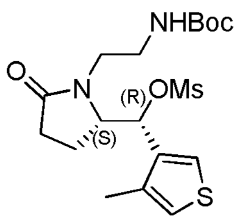

To a solution of tert-butyl 2-((S)-2-((R)-hydroxy(4-methylthiophen-3-yl)methyl)-5-oxopyrrolidin-1-yl)ethylcarbamate (700 mg, 1.98 mmol) and Et3N (2.57 ml, 17.8 mmol) in DCM (15 mL) at 0 °C was dropwise added MsCl (0.31 ml, 3.95 mmol) under $N_2$ atmosphere. The reaction was stirred for 20 h at room temperature and then quenched with saturated $NH_4Cl$ solution. The resulting mixture was extracted with DCM (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (750 mg, yield: 88%); m/z (ES+): 433 $[M+H]^+$.

Intermediate 97

(1R,8aS)-tert-butyl 1-(4-methylthiophen-3-yl)-6-oxohexahydropyrrolo[1,2-a]pyrazine-2(1H)-carboxylate

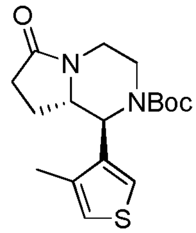

To a suspension of NaH (60%, 556 mg, 13.9 mmol) in THF (5 mL) at 0 °C was added solution of (R)-((S)-1-(2-(tert-butoxycarbonylamino)ethyl)-5-oxopyrrolidin-2-yl)(4-methylthiophen-3-yl)methyl methanesulfonate (750 mg, 1.74 mmol) in THF (5 mL). The mixture was stirred at 50 °C for 20 h and quenched with ice-water. The resulting mixture was extracted with EtOAc. The combined organic layers were dried over anhydrous $Na_2SO_4$ and then concentrated in high vacuum. The residue was purified by silica gel chromatography (PE/EtOAc=5/1 to 1/1) to give the title compound (300 mg, yield: 51%); m/z (ES+): 337 $[M+H]^+$.

Intermediate 98

(1S, 8aS)-1-(4-methylthiophen-3-yl)hexahydropyrrolo[1,2-a]pyrazin-6(7H)-one

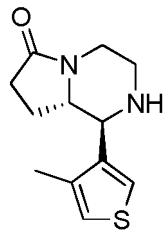

A solution of (1R, 8aS)-tert-butyl 1-(4-methylthiophen-3-yl)-6-oxohexahydropyrrolo[1,2 -a]pyrazine-2(1H)-carboxylate (300 mg, 0.89 mmol) in MeOH (20 mL) at 0 °C was added HCl/MeOH (7 M, 5 mL). The reaction was stirred at room temperature for 3 h and basified with 1 M aqueous NaOH solution to pH=10. The mixture was extracted with EtOAc (2 x 20 mL). The combined organic layers were washed with brine, dried over anhydrous $Na_2SO_4$, and concentrated in vacuo. The residue was purified by Prep-TLC (DCM/MeOH=10/1) to give the title compound (150 mg, yield: 72%); m/z (ES+): 237 $[M+H]^+$.

Intermediate 99-103 were prepared using the procdure for intermediate 98.

Intermediate 99

(1R, 8aS)-1-(3-methylthiophen-2-yl)hexahydropyrrolo[1,2-a]pyrazin-6(7H)-one

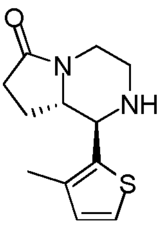

m/z (ES+): 237 [M+H]$^+$.

Intermediate 100

(1R,8aS)-1-(thiophen-2-yl)hexahydropyrrolo[1,2-a]pyrazin-6(7H)-one

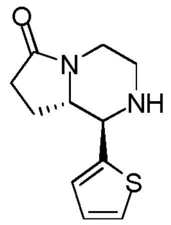

m/z (ES+): 223 [M+H]$^+$.

Intermediate 101

(1S,8aS)-1-(2-methylthiophen-3-yl)hexahydropyrrolo[1,2-a]pyrazin-6(7H)-one

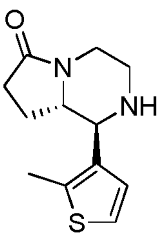

m/z (ES+): 237 [M+H]$^+$.

Intermediate 102

(1R,8aS)-1-(2-methylthiazol-5-yl)hexahydropyrrolo[1,2-a]pyrazin-6(7H)-one

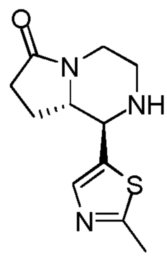

m/z (ES+): 238 $[M+H]^+$.

Intermediate 103

(1R,8aS)-1-(thiazol-5-yl)hexahydropyrrolo[1,2-a]pyrazin-6(7H)-one

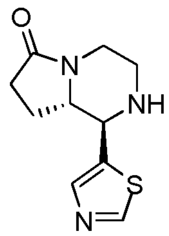

m/z (ES+): $224[M+H]^+$.

Intermediate 104

1-benzyl-4-bromo-3-methyl-1H-pyrazole

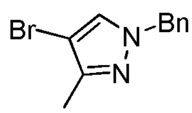

To a suspension of NaH (60%, 2.5 g, 62.9 mmol) in THF (800 mL), was dropwise added 4-bromo-3-methyl-1H-pyrazole (10 g, 62.9 mmol) in 100 ml THF at 0 °C. After stirred at the same temperature for 30 min, BnBr (4.4 ml, 62.9 mmol) was added at 0 °C. The reaction mixture was stirred at 15 °C for 1 h and carefully quenched with ice-water. The resulting mixture was extracted with EtOAc (2x150 mL). The combined organic layers were washed with brine (2x 100 mL), dried over anhydrous $Na_2SO_4$ and then concentrated. The residue was purified by silica gel chromatography (PE/EtOAc =4/1 to 1/1) to give the title compound (6 g, yield: 60%); m/z (ES+): 251 $[M+H]^+$.

Intermediate 105

(S)-tert-butyl 2-(2-(1-benzyl-3-methyl-1H-pyrazole-4-carbonyl)-5-oxopyrrolidin-1-yl)ethylcarbamate

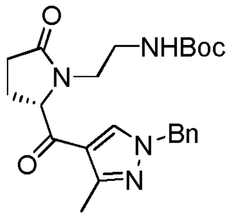

A solution of MeI (0.05 mmol), $I_2$ (10 mg) and Mg (2.8 g 120 mmol) in THF (10 mL) was dropwise added over 5 min to a stirred solution of the 1-benzyl-4-bromo-3-methyl-1H-pyrazole (3 g, 12 mmol) in THF(10 mL) at -40 °C under argon. The resulting solution was then stirred for 30 min, and (S)-tert-butyl 1,6-dioxohexahydropyrrolo[1,2-a]pyrazine-2(1H)-carboxylate (5 g, 19.7 mmol) was added. The reaction mixture was allowed to warm to room temperature and stirred till (S)-tert-butyl 1,6-dioxohexahydropyrrolo[1,2-a]pyrazine-2(1H)-carboxylate was consumed. The reaction mixture was quenched with saturated $NH_4Cl$ solution and extracted with EtOAc (2 x 20 mL). The combined organic layers were washed with brine, dried over anhydrous $Na_2SO_4$, and concentrated in vacuo.The crude residue was purified by column chromatography on silica gel (PE/EtOAc, 4:1) to give the alcohol compound (1.6 g, 30%); m/z (ES+): 427[M+H]$^+$.

Intermediate 106

tert-butyl 2-((S)-2-((R)-(1-benzyl-3-methyl-1H-pyrazol-4-yl)(hydroxy)methyl)pyrrolidin-1-yl)ethylcarbamate

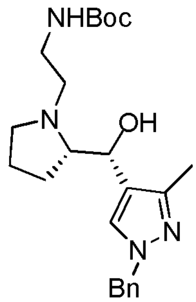

To a solution of (S)-tert-butyl 2-(2-(1-benzyl-3-methyl-1H-pyrazole-4-carbonyl)-5-oxopyrrolidin-1-yl)ethylcarbamate (1.5 g, 3.8 mmol) in THF (50 mL) was portion-wise added LiAl(O$^t$Bu$_3$)H (3.56 g, 14 mmol) at -20 °C. Then the mixture was stirred at room temperature for 2 h and quenched with saturated $NH_4Cl$ solution. The resulting suspension was filtered through a celite pad, washed by EtOAc. The filtrate was extracted with EtOAc (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (1.2 g, yield: 80%); m/z (ES+): 428[M+H]$^+$.

Intermediate 107

Methanesulfonicacid(1-benzyl-3-methyl-1H-pyrazol-4-yl)-[1-(2-tert-butoxycarbonyl amino ethyl)-5-oxo-pyrrolidin-2-yl]-methyl ester

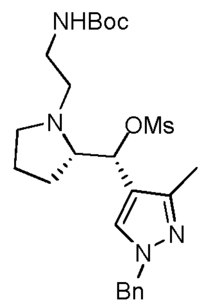

To a solution of tert-butyl 2-((S)-2-((R)-(1-benzyl-3-methyl-1H-pyrazol-4-yl)(hydroxy)methyl)pyrrolidin-1-yl)ethylcarbamate (1.2 g, 2.8 mmol) and $Et_3N$ (2.57 ml, 17.8 mmol) in DCM (20 mL), at 0 °C was dropwise added MsCl (1.18 g, 10.3 mmol) under $N_2$ atmosphere. The reaction was stirred for 20 h at room temperature and then quenched with saturated $NH_4Cl$ solution. The resulting mixture was extracted with DCM (2 x 20 mL). The combined organic layers were washed with brine, dried over anhydrous $Na_2SO_4$, and concentrated in vacuo. The residue was purified by silica gel chromatography (PE/EtOAc=5/1 to 1/1) to give the title compound (1.1 g, yield: 89%); m/z (ES+): 507 $[M+H]^+$.

Intermediate 108

(1S,8aS)-tert-butyl 1-(1-benzyl-3-methyl-1H-pyrazol-4-yl)-6-oxo-hexahydropyrrolo[1,2-a]pyrazine-2(1H)-carboxylate

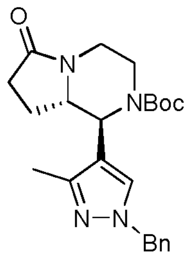

To a suspension of NaH (60%, 128 mg, 3.2 mmol) in THF (20 mL) was added a solution of Methanesulfonic acid (1-benzyl-3-methyl-1H-pyrazol-4-yl)-[1-(2-tert-butoxycarbonyl amino-ethyl)-5-oxo-pyrrolidin-2-yl]-methyl ester (1.2 g, 2.3 mmol) in THF (10 mL) at 0 °C under $N_2$ atmosphere. The mixture was stirred at 50 °C for 20 h and quenched with ice-water. The resulting mixture was extracted with EtOAc (2 x 30 mL). The combined organic layers were dried over anhydrous $Na_2SO_4$ and then concentrated in high vacuum. The residue was purified by silica gel (PE/EtOAc=5/1 to 1/1) to give the title compound (1.2 g, yield: 91%); m/z (ES+): 491 $[M+H]^+$.

Intermediate 109

(1S,8aS)-1-(1-benzyl-3-methyl-1H-pyrazol-4-yl)hexahydropyrrolo[1,2-a]pyrazin-6(7H)-one

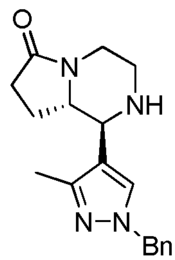

To a solution of (1S,8aS)-tert-butyl 1-(1-benzyl-3-methyl-1H-pyrazol-4-yl)-6-oxohexahydropyrrolo[1,2-a]pyrazine-2(1H)-carboxylate (402 g, 1 mmol) in MeOH (5 mL) at 0 °C was added HCl/MeOH (7M, 5 mL). The reaction was stirred at room temperature for 3 h until intermediate 6 was completely consumed. MeOH was removed under reduced pressure, the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (160 mg, yield: 51%); m/z (ES+): 311 $[M+H]^+$.

Intermediate 110

(1S,8aS)-1-(1-benzyl-3-methyl-1H-pyrazol-4-yl)-N-((R)-1-(3,5-bis(trifluoromethyl)phenyl)ethyl)-N-methyl-6-oxohexahydropyrrolo[1,2-a]pyrazine-2(1H)-carboxamide

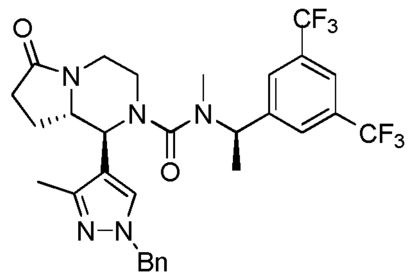

To a solution of triphosgene (47 mg, 0.16 mmol) in EtOAc (10 mL) at 0 °C was added solution of (1S,8aS)-1-(1-benzyl-3-methyl-1H-pyrazol-4-yl)hexahydropyrrolo[1,2-a]pyrazin-6(7H)-one (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-1-(3,5-bis(trifluoromethyl)phenyl)-N-methyl ethanamine (163.2 mg, 0.6 mmol) and TEA (107 mg, 1.05 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (70 mg, yield: 31%); m/z (ES+): 608 $[M+H]^+$.

Intermediate 111

(1S,8aS)-1-(1-benzyl-3-methyl-1H-pyrazol-4-yl)-N-((R)-1-(3-ethyl-5-(trifluoromethyl)phenyl)ethyl)-N-methyl-6-oxohexahydropyrrolo[1,2-a]pyrazine-2(1H)-carboxamide

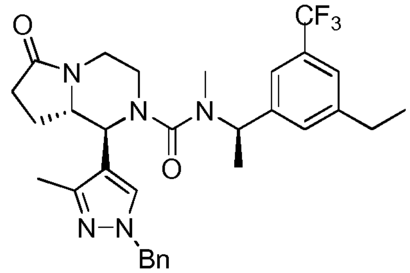

To a solution of triphosgene (47 mg, 0.16 mmol) in EtOAc (10 mL) at 0 °C was added solution of (1S,8aS)-1-(1-benzyl-3-methyl-1H-pyrazol-4-yl)hexahydropyrrolo[1,2-a]pyrazin-6(7H)-one (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-1-(3,5-bis(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (70 mg, yield: 31%); m/z (ES+): 568 $[M+H]^+$.

Intermediate 112

(1S,8aS)-tert-butyl7,7-diallyl-1-(1-benzyl-3-methyl-1H-pyrazol-4-yl)-6-oxohexahydropyrrolo[1,2-a]pyrazine-2(1H)-carboxylate

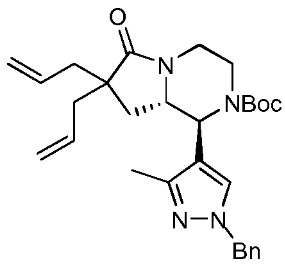

To a solution of (1S,8aS)-tert-butyl 1-(1-benzyl-3-methyl-1H-pyrazol-4-yl)-6-oxohexahydropyrrolo[1,2-a]pyrazine-2(1H)-carboxylate (1.2 g, 2.44 mmol) in anhydrous THF (100 mL) under $N_2$ atmosphere, was was added LiHMDS (1 M in THF, 3.4 mL at -78 °C. The reaction was stirred for 15 min, followed by addition of DMPU (895 mg, 7 mmol) and allylbromide (316.5 mg, 2.62 mmol). The reaction mixture was stirred at -78 °C for 30 min and allowed to warm to -30 °C, and then stirred at this temperature for another 2 h. After quenched with water at -78 °C, the mixture was then extracted with EtOAc (2 x 50 mL). The combined organic layers were washed with brine, dried over anhydrous $Na_2SO_4$, and concentrated in vacuo. The residue was purified by silica gel chromatography (PE/EtOAc=2/1 to 1/1) to give the title compound (550mg, yield: 46%); m/z (ES+): 491 $[M+H]^+$

Intermediate 113

(1S,8aS)-7,7-diallyl-1-(1-benzyl-3-methyl-1H-pyrazol-4-yl)hexahydropyrrolo[1,2-a]pyrazin-6(7H)-one

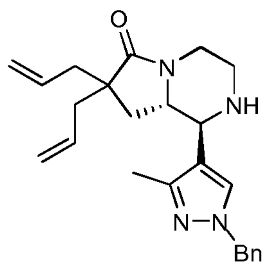

To a solution of (1S,8aS)-tert-butyl 7,7-diallyl-1-(1-benzyl-3-methyl-1H-pyrazol-4-yl)-6-oxohexahydropyrrolo[1,2-a]pyrazine-2(1H)-carboxylate (550 mg, 1.12 mmol) in MeOH (20 mL) at 0 °C was added HCl/MeOH (7M, 5 mL). The reaction was stirred at room temperature for 3 h and then basified with with 1 M aqueous NaOH solution to pH=10. The mixture was extracted with EtOAc (2 x 20 mL). The combined organic layers were washed with brine, dried over anhydrous $Na_2SO_4$, and concentrated in vacuo. The residue was directly used in the next step without further purification (360 mg, yield: 95%); m/z (ES+): 391 $[M+H]^+$.

Intermediate 114

(1S,8aS)-7,7-diallyl-1-(1-benzyl-3-methyl-1H-pyrazol-4-yl)-N-((R)-1-(3-ethyl-5-(trifluoromethyl)phenyl)ethyl)-N-methyl-6-oxohexahydropyrrolor[1,2-a]pyrazine-2(1H)-carboxamide

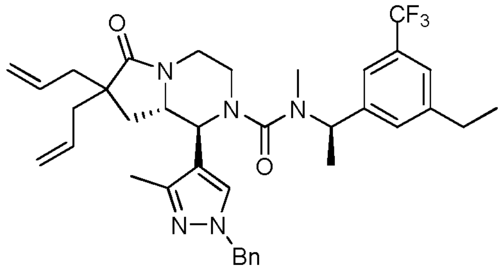

To a solution of triphosgene (53.4 mg, 0.18 mmol) in EtOAc (2 mL) at 0 °C was added solution of (1S,8aS)-7,7-diallyl-1-(1-benzyl-3-methyl-1H-pyrazol-4-yl)hexahydropyrrolo [1,2-a]pyrazin-6(7H)-one (83 mg, 0.36 mmol), DMAP (4.6 mg, 0.036 mmol) and TEA (109.2 mg, 1.08 mmol) in EtOAc (2 mL). The mixture was stirred for 1.5 h at room temperature, followed by addition of [1-(3-Ethyl-5-trifluoromethyl-phenyl)-ethyl]-methyl-amine (100 mg, 0.43 mmol) in EtOAc (2 mL) and TEA (109.2 mg, 1.08 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (80 mg, yield: 57%); m/z (ES+): 648 $[M+H]^+$.

Intermediate 115

(1S,8aS)-1-(1-benzyl-3-methyl-1H-pyrazol-4-yl)-N-((R)-1-(3-ethyl-5-(trifluoromethyl)phenyl)ethyl)-7,7-bis(2-hydroxyethyl)-N-methyl-6-oxohexahydropyrrolo[1,2-a]pyrazine-2(1H)-carboxamide

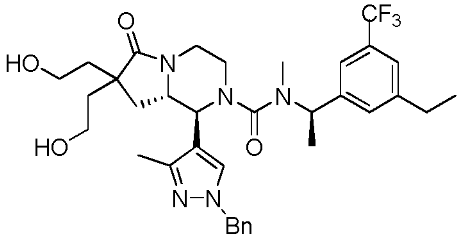

A slow stream of $O_3$ in $O_2$ was passed through a cooled solution of (1S,8aS)-7,7-diallyl-1-(1-benzyl-3-methyl-1H-pyrazol-4-yl)-N-((R)-1-(3-ethyl-5-(trifluoromethyl)phenyl) ethyl)-N-methyl-6-oxohexahydropyrrolo[1,2-a]pyrazine-2(1H)-carboxamide (80 mg , 0.12 mmol) in DCM (10 mL) at -78°C until a pale blue colour persisted. The reaction was purged with $N_2$ to remove the excess of $O_3$ followed by addition of $NaBH_4$ (57 mg, 1.5 mmol). The reaction mixture was allowed to warm to room temperature and stirred for 20 h. After quenched with $NH_4Cl$ solution, the resulting mixture was extracted with DCM. The combined organic layers were washed with brine, dried over anhydrous $Na_2SO_4$, and concentrated in vacuo. The residue was purified by Prep-HPLC to give the title compound (40 mg, yield: 50%); m/z (ES+): 696 $[M+H]^+$.

Intermediate 116

(1S,8aS)-7,7-diallyl-1-(1-benzyl-3-methyl-1H-pyrazol-4-yl)-N-methyl-N-((R)-1-(3-methyl-5-(trifluoromethyl)phenyl)ethyl)-6-oxohexahydropyrrolo[1,2-a]pyrazine-2(1H)-carboxamide

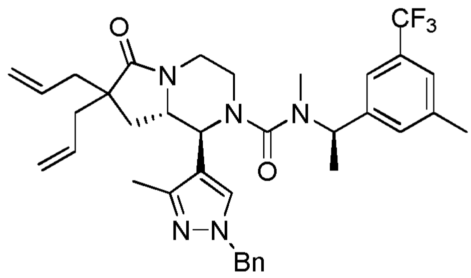

To a solution of triphosgene (44 mg, 0.15 mmol) in EtOAc (2 mL) at 0 °C was added solution of (1S,8aS)-7,7-diallyl-1-(1-benzyl-3-methyl-1H-pyrazol-4-yl)hexahydropyrrolo [1,2-a]pyrazin-6(7H)-one (120 mg, 0.31 mmol), DMAP (4 mg, 0.03 mmol) and TEA (90 mg, 0.9 mmol) in EtOAc (2 mL). The mixture was stirred for 1.5 h at room temperature, followed by addition of (R)-N-methyl-1-(3-methyl-5-(trifluoromethyl)phenyl)ethanamine (100 mg, 0.46 mmol) in EtOAc (2 mL) and TEA (90 mg, 1 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (90 mg, yield: 46%); m/z (ES+): 634 $[M+H]^+$.

Intermediate 117

(1S,8aS)-1-(1-benzyl-3-methyl-1H-pyrazol-4-yl)-7,7-bis(2-hydroxyethyl)-N-methyl-N-((R)-1-(3-methyl-5-(trifluoromethyl)phenyl)ethyl)-6-oxo-hexahydropyrrolo[1,2-a]pyrazine-2(1H)-carboxamide

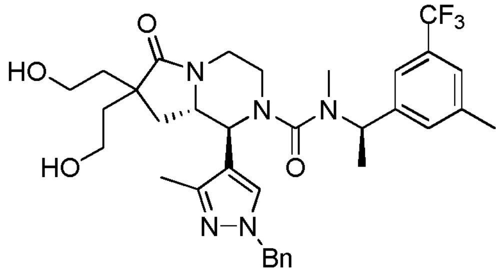

A slow stream of $O_3$ in $O_2$ was passed through a cooled solution of (1S,8aS)-7,7-diallyl-1-(1-benzyl-3-methyl-1H-pyrazol-4-yl)-N-methyl-N-((R)-1-(3-methyl-5-(trifluoromethyl) phenyl)ethyl)-6-oxohexahydropyrrolo[1,2-a]pyrazine-2(1H)-carboxamide (90 mg , 0.14 mmol) in DCM (10 mL) at -78°C until a pale blue colour persisted. The reaction was purged with $N_2$ to remove the excess of $O_3$ followed by addition of $NaBH_4$ (106 mg, 2.8 mmol). The reaction mixture was allowed to warm to room temperature and stirred for 20 h. After quenched with $NH_4Cl$ solution, the resulting mixture was extracted with DCM. The organic layer was washed with brine, dried over anhydrous $Na_2SO_4$, and concentrated in vacuo. The residue was purified by Prep-HPLC to give the title compound (40 mg, yield: 45%); m/z (ES+): 642 $[M+H]^+$.

Intermediate 118

(S)-tert-butyl(2-(-2-(3-chloro-2-methylbenzoyl)-5-oxopyrrolidin-1-yl)ethyl)carbamate

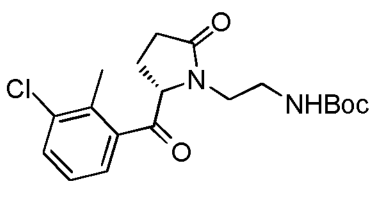

To a solution of 1-bromo-3-chloro-2-methylbenzene (2.74 g, 12.39 mmol) in THF (40 mL) at -70 °C was added

n-BuLi (1.6 M in hexane) (7.75 mL, 12.39 mmol) drop wise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2 (1H) -carboxylate (3 g, 11.8 mmol) in THF (40 mL). After stirred at -70°C for 5 min, the reaction mixture was quenched with saturated $NH_4Cl$ and extracted with EtOAc (2 x 20 mL), washed with brine, dried over $Na_2SO_4$, filtered and concentrated in vacuo. The residue was purified by silica gel chromatography to give the title compound (3.6 g, yield: 80%); m/z ($ES^+$): 381 $[M+H]^+$.

Intermediate 119

tert-butyl (2-((2S)-2-((3-chloro-2-methylphenyl)(hydroxy)methyl)-5- oxopyrrolidi -1-yl)ethyl)carbamate

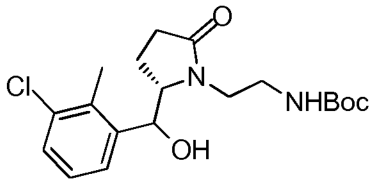

LiAl(OtBu) $_3$H (3.56 g, 14 mmol) was added portion wisely into intermediate 118 (3.6 g, 9.45 mmol) in THF (50 mL) at -20 °C. Then the mixture was stirred at room temperature. 2 h later, intermediate 2 was completely consumed. The whole solution was poured into the saturated solution of potassium sodium tartrate. 30 min later, the suspension was filtered through a celite pad, washed by EtOAc for several times. The filtration was extracted with EtOAc (2 x 20 mL) washed with brine, dried over $Na_2SO_4$, filtered and concentrated in vacuo. The residue was purified by silica gel chromatography to give the title compound (2.9 g, yield: 80%); m/z (ES+): 383$[M+H]^+$.

Intermediate 120

((S)-1-(2-((tert-butoxycarbonyl)amino)ethyl)-5-oxopyrrolidin-2-yl)(3-chloro-2-methylphenyl)methyl methanesulfonate

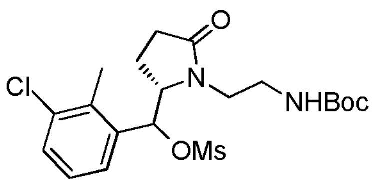

To a solution of intermediate 119 (2.9 g, 7.6 mmol) and $Et_3N$ (3.54 g, 35 mmol) in DCM at 0 °C was added MsCl (1.18 g, 10.3 mmol) dropwisely under $N_2$ atmosphere. The reaction was stirred for 20 h at room temperature and then quenched with saturated $NH_4Cl$ and extracted with DCM (2 x 20 mL), washed with brine, dried over $Na_2SO_4$, filtered and concentrated in vacuo. The residue was purified by silica gel chromatography to give the title compound (2.7 g, yield: 77%); m/z (ES+): 461 $[M+H]^+$.

Intermediate 121

(1S,8aS)-tert-butyl 1-(3-chloro-2-methylphenyl)-6-oxohexahydropyrrolo[1,2-a] pyrazine-2(1H)-carboxylate

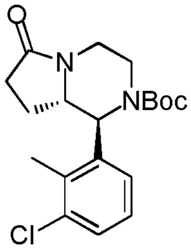

Intermediate 120 (2.7 g, 5.8 mmol) in THF (10 mL) was added into a suspension of NaH (60%, 468 mg, 11.7 mmol) in THF (20 mL) at 0 °C under $N_2$ atmosphere. The mixture was stirred at RT for 20 h and then quenched with ice-water. The resulting mixture was extracted with EtOAc (2 x 30 mL), the combined organic layers were washed with brine, dried over $Na_2SO_4$, filtered and concentrated in vacuo. The residue was purified by silica gel chromatography to give the title compound (600 mg, yield: 29%); m/z (ES+): 365 $[M+H]^+$.

Intermediate 122

(1S,8aS)-1-(3-chloro-2-methylphenyl)hexahydropyrrolo[1,2-a]pyrazin-6(2H)-one

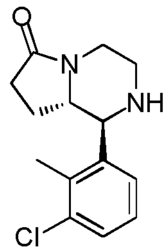

To a solution of intermediate 121 (600 mg, 1.64 mmol) in MeOH (20 mL) at 0 °C was added HCl/MeOH (7M, 5 mL). The reaction was stirred at room temperature until the starting materials was completely consumed. MeOH was removed under reduced pressure, the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (305 mg, yield: 70%); m/z (ES+): 265 $[M+H]^+$.

Intermediate 123

(S)-tert-butyl 2-(2-(2-ethylbenzoyl)-5-oxopyrrolidin-1-yl)ethylcarbamate

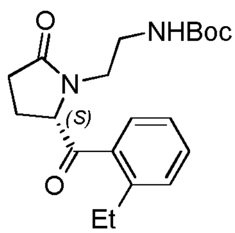

To a solution of 1-bromo-2-ethylbenzene (2.29 g, 12.39 mmol) in THF (40 mL) at -70 °C was added n-BuLi (1.6 M in hexane) (7.52 mL, 12.036mmol) drop wise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2 (1H) -carboxylate (3 g, 11.8 mmol) in THF (40 mL). After stirred at -70°C for 10 min, the reaction mixture was quenched with saturated $NH_4Cl$ and extracted with EtOAc (2 x 20 mL), washed with brine, dried over $Na_2SO_4$, filtered and concentrated in vacuo. The residue was purified by silica gel chromatography to give the title compound (1.3 g, yield: 30%); m/z $(ES^+)$: 361 $[M+H]^+$.

Intermediate 124

(S)-1-(2-aminoethyl)-5-(2-ethylbenzoyl)pyrrolidin-2-one

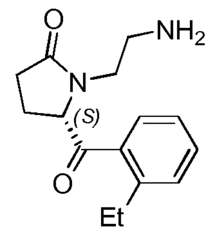

To a stirred solution of intermediate 123 (500 mg, 1.39 mmol) in dioxane (5 mL) was treated with saturated solution of HCl in dioxane (10 mL) drop wise for 10 min and later stirred at room temperature for 24 h. Solvent was evaporated under reduced pressure and the residue was washed with $Et_2O$ used for next step without any further purification (361 mg, yield: 100%); m/z (ES+): 261 $[M+H]^+$.

Intermediate 125

1-(2-ethylphenyl)hexahydropyrrolo[1,2-a]pyrazin-6(7H)-one (mixture of trans-isomers)

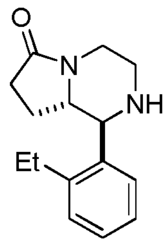

To a stirred solution of intermediate 124 (361 mg, 1.39 mmol) in MeOH (1.2 L) was treated with cat. AcOH (0.5 mL) and $NaCNBH_3$ (627 mg, 5.56 mmol) at 0 °C and allowed to RT and maintained for 16 h. On completion, the solvent was removed under reduced pressure; sat.$NaHCO_3$ solution was added to the residue and extracted with DCM, washed with brine, dried over $Na_2SO_4$, filtered and concentrated in vacuo. The residue was purified by silica gel chromatography to give the title compound (200 mg, yield: 59%); m/z (ES+): 245 $[M+H]^+$.

Intermediate 126

(S)-tert-butyl 2-(2-(2-chlorobenzoyl)-5-oxopyrrolidin-1-yl)ethylcarbamate

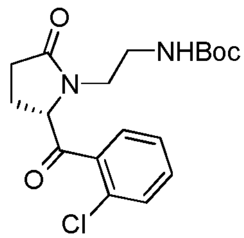

To a solution of 1-bromo-2-chlorobenzene (2.38 g, 12.39 mmol) in toluene (40 mL) at - 70 °C was added n-BuLi (1.6 M in hexane) (7.52 mL, 12.04 mmol) drop wise over 5 min by syringe along the wall of the flask. After adding,

the reaction was stirred at this temperature for 20 min. Then the resulting solution was added into a solution of (S)-tert-butyl 1,6-dioxohexahydropyrrolo[1,2-a]pyrazine-2 (1H) -carboxylate (3 g, 11.8 mmol) in toluene (40 mL). After stirred at -70°C for 10 min, the reaction mixture was quenched with saturated $NH_4Cl$ and extracted with EtOAc (2 x 20 mL), washed with brine, dried over $Na_2SO_4$, filtered and concentrated in vacuo. The residue was purified by silica gel chromatography to give the title compound (1 g, yield: 23%); m/z (ES+): 367 $[M+H]^+$.

Intermediate 127

(S)-1-(2-aminoethyl)-5-(2-chlorobenzoyl)pyrrolidin-2-one

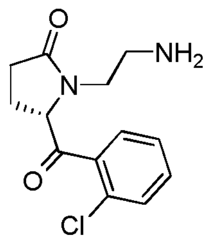

To a stirred solution of intermediate 126 (728 mg, 2.73 mmol) in dioxane (5 mL) was treated with saturated solution of HCl in dioxane drop wise for 10 min and later stirred at room temperature for 24 h. Solvent was evaporated under reduced pressure and the residue was washed with $Et_2O$ used for next step without any further purification; m/z (ES+): 267 $[M+H]^+$.

Intermediate 128 (mixture of trans-isomers)

1-(2-chlorophenyl)hexahydropyrrolo[1,2-a]pyrazin-6(7H)-one

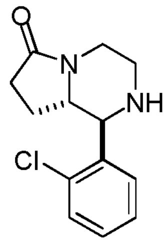

To a stirred solution of intermediate 127 (728 mg, 2.73 mmol) in MeOH (1.2 L) was treated with cat. AcOH (0.5 mL) and $NaCNBH_3$ (860 mg, 13.65 mmol) at 0 °C and allowed to RT and maintained for 16 h. On completion, the solvent was removed under reduced pressure; sat.$NaHCO_3$ solution was added to the residue and extracted with DCM, washed with brine, dried over $Na_2SO_4$, filtered and concentrated in vacuo. The residue was purified by silica gel chromatography to give the title compound (125 mg, yield: 18%); m/z ($ES^+$): 251 $[M+H]^+$.

Intermediate 129

(S)-tert-butyl 2-(2-oxo-5-(2-vinylbenzoyl)pyrrolidin-l-yl)ethylcarbamate

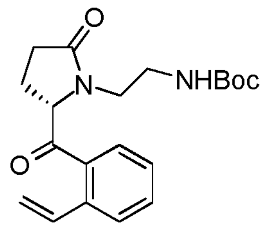

To a solution of 1-bromo-2-vinylbenzene (4.03 g, 22 mmol) in THF (15 mL) at -70 °C was added n-BuLi (1.6 M in hexane) (14.45 mL, 23.1 mmol) dropwisely by syringe along the wall of the flask. After adding, the reaction was stirred for 30 min at this temperature. Then (S)-tert-butyl 1,6-dioxohexahydropyrrolo[1,2-a]pyrazine -2(1H)-carboxylate (7.0 g, 27 mmol) was added into the above solution in one portion. After stirred at -40 °C for 15 min, the reaction mixture was quenched with saturated $NH_4Cl$ and extracted with EtOAc (2 x 20 mL), washed with brine, dried over $Na_2SO_4$, filtered and concentrated in vacuo. The residue was purified by silica gel chromatography to give the title compound (5.6 g, yield: 71%); m/z (ES+): 359 $[M+H]^+$.

Intermediate 130

tert-butyl2-((2S)-2-(hydroxy(2-vinylphenyl)methyl)-5-oxopyrrolidin-1-yl)ethylcarbamate

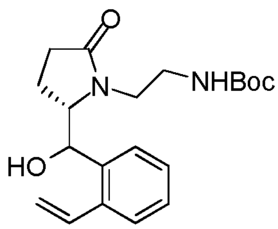

LiAl (OtBu) $_3$H (5.97 g, 23.45 mmol) was added portion wisely into intermediate 129 (5.6 g, 15.6 mmol) in THF (50 mL) at -20 °C. Then the solution was slowly warmed to room temperature. 2 h later, the starting material was completely consumed; the whole solution was poured into the saturated $NH_4Cl$, the suspension was filtered through a celite pad, washed by EtOAc. The filtration was extracted with EtOAc (2 x 100 mL) washed with brine, dried over $Na_2SO_4$, filtered and concentrated in vacuo. The residue was purified by silica gel chromatography to give the title compound (3.86 g, yield: 68.5%); m/z (ES+): 361 $[M+H]^+$.

Intermediate 131

((S)-1-(2-(tert-butoxycarbonylamino)ethyl)-5-oxopyrrolidin-2-yl)(2-vinylphenyl)methyl methanesulfonate

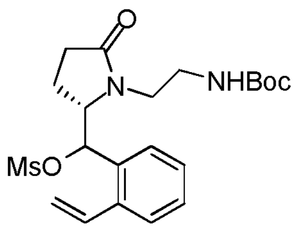

Intermediate 130 (3.86 g, 10.72 mmol), $Et_3N$ (5.41 g, 53.6 mmol) in DCM (20 mL), then the mixture was cooled to 0 °C. MsCl (1.842 g, 16.08 mmol) was added into the above solution drop wisely under $N_2$ atmosphere. The reaction was stirred overnight at 30°C. After most of starting material was consumed, the reaction was quenched with saturated $NH_4Cl$ and extracted with DCM (2 x 20 mL), washed with brine, dried over $Na_2SO_4$, filtered and concentrated in vacuo.

The residue was purified by silica gel chromatography to give the title compound (3.1 g, yield: 66%); m/z (ES+): 439 $[M+H]^+$.

Intermediate 132

(1S,8aS)-tert-butyl6-oxo-1-(2-vinylphenyl)hexahydropyrrolo[1,2-a]pyrazine-2(1H)-carboxylate

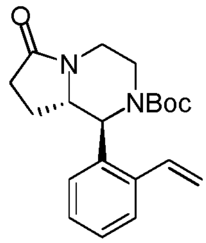

Intermediate 131 (3.1 g, 7.1 mmol) in THF (10 mL) was added into a suspension of NaH (60%, 128 mg, 14.15 mmol) in THF (20 mL) at 0 °C under $N_2$ atmosphere. The mixture was stirred at 40'C overnight and then quenched with ice-water. The resulting mixture was extracted with EtOAc (2 x 30 mL), washed with brine, dried over $Na_2SO_4$, filtered and concentrated in vacuo. The residue was purified by silica gel chromatography to give the title compound (617 mg, yield: 26%); m/z (ES+): 343 $[M+H]^+$.

Intermediate 133

(1S, 8aS)-1-(2-vinylphenyl)hexahydropyrrolo[1,2-a]pyrazin-6(7H)-one

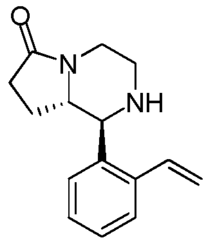

To a solution of Intermediate 132 (617 mg, 1.8 mmol) in MeOH (15 mL) at 0 °C was added HCl/MeOH (7M, 15 mL). The reaction was stirred at room temperature for 3 h until the strating material was completely consumed. MeOH was removed under reduced pressure, the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was purified by silica gel chromatography to give the title compound (500 mg, yield: 90%); m/z (ES+): 243 $[M+H]^+$.

Intermediate 134

(1S,8aS)-N-((R)-1-(3,5-bis(trifluoromethyl)phenyl)ethyl)-N-methyl-6-oxo-1-(2-vinylphenyl)hexahydropyrrolo[1,2-a]pyrazine-2(1H)-carboxamide

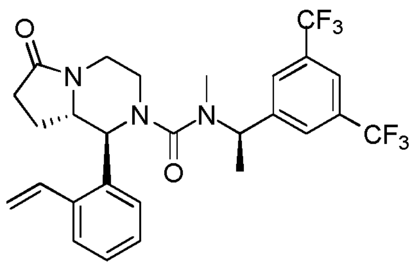

To a solution of triphosgene (489 mg, 1.662 mmol) in EtOAc (10 mL) at 0 °C was added (R)-1-(3,5-bis(trifluoromethyl)phenyl)-N-methyl ethanamine (904 mg, 3.324 mmol), DMAP (41 mg, 0.03324 mmol) and TEA (840 mg, 8.3 mmol). The mixture was stirred for 2 h at RT, followed by addition of intermediate 133 (670 mg, 2.77 mmol) in EtOAc (2 mL) and TEA (840 mg, 8.3 mmol). The reaction was stirred at 45 °C for 48 h and quenched with $NH_4Cl$ (aq). The resulting mixture was extracted with EtOAc; the combined organic layer was washed with 1 M aqueous HCl solution, dried over $Na_2SO_4$ and then concentrated in high vacuum. The residue was purified by silica gel chromatography to give the title compound (450 mg, yield: 31%); m/z (ES+): 540[M+H]$^+$.

Intermediate 135

(S)-tert-butyl 2-(2-(3,4-dimethylthiophene-2-carbonyl)-5-oxopyrrolidin-1-yl) ethylcarbamate

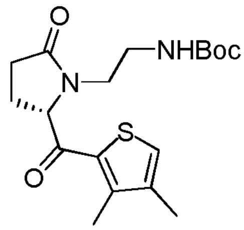

To a solution of 3,4-dimethylthiophene (2.5 g, 10 mmol) in THF (20 mL) at -70 °C was added LDA (1.6 M in hexane) (7.52 mL, 12.036mmol) dropwise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2(1H)-carboxylate (1.8 g, 10 mmol) in THF (40 mL). After stirred at -70°C for 5 min, the reaction mixture was quenched with saturated $NH_4Cl$ and extracted with EtOAc (2 x 20 mL), washed with brine, dried over $Na_2SO_4$, filtered and concentrated in vacuo. The residue was purified by silica gel chromatography to give the title compound (1.5 g, yield: 20%); m/z (ES+): 367 [M+H]$^+$.

Intermediate 136

tert-butyl 2-((2S)-2-((3,4-dimethylthiophen-2-yl)(hydroxy)methyl)-5-oxopyrrolidin-1-yl)ethylcarbamate

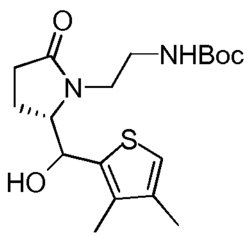

LiAl(OtBu)$_3$H (1 g, 2.8 mmol) was added portion wisely into intermediate 135 (1.3 g, 5 mmol) in THF (20 mL) at -20 °C. Then the mixture was stirred at room temperature. 2 h later, the starting material was completely consumed;

the whole solution was poured into the saturated $NH_4Cl$, the suspension was filtered through a celite pad, washed by EtOAc. The filtration was extracted with EtOAc (2 x 20 mL) washed with brine, dried over $Na_2SO_4$, filtered and concentrated in vacuo. The residue was purified by silica gel chromatography to give the title compound (1 g, yield: 100%); m/z (ES+): 369 $[M+H]^+$.

Intermediate 137

(8aS)-tert-butyl 1-(3,4-dimethylthiophen-2-yl)-6-oxohexahydropyrrolo [1,2-a] pyrazine -2(1H)-carboxylate

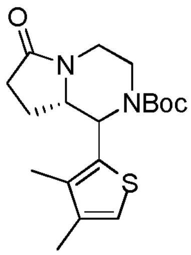

Intermediate 136 (1 g, 2.8 mmol) in DCM (10 mL), then $Et_3N$ (707 mg, 7 mmol), DMAP (18 mg, 0.15 mmol) were added. The resulting solution was cooled to 0 °C, and then MsCl (400 mg, 3.5 mmol) was added into the above solution dropwisely under $N_2$ atmosphere. The reaction was stirred for 20 h at room temperature and then quenched with saturated $NH_4Cl$ and extracted with DCM (2 x 20 mL), washed with brine, dried over $Na_2SO_4$, filtered and concentrated in vacuo. The residue was purified by silica gel chromatography to give the title compound (980 mg, yield: 103%); m/z (ES+): 350 $[M+H]^+$.

Intermediate 138

(8aS)-1-(3,4-dimethylthiophen-2-yl)hexahydropyrrolo[1,2-a]pyrazin-6(7H)-one

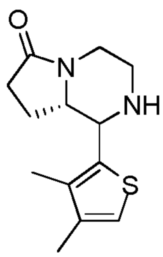

To a solution of Intermediate 137 (980 g, 2.8 mmol) in MeOH (15 mL) at 0 °C was added HCl/MeOH (7M, 15 mL). The reaction was stirred at room temperature for 3 h until intermediate 6 was completely consumed. MeOH was removed under reduced pressure, the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (600 mg, yield: 90%); m/z (ES+): 251$[M+H]^+$.

Intermediate 139

bromo-3-methoxy-2-methylbenzene

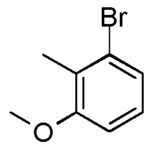

To 3-methoxy-2-methyl aniline(2.74 g, 20 mmol), water (6 mL) and 48% aqueous solution of hydrobromic acid (2.4 mL) were added. To this sodium nitrite (2.76 g, 40 mmol) in water (1.20 mL) was added dropwise at 0°C for 10 minutes. After adding acetone (24 mL) to the reaction mixture, copper bromide (5.64 g, 40 mmol) was added at 0°C and stirred for 3hours. The reaction mixture was warmed to room temperature and concentrated. The residue was diluted with dichloromethane and washed with a mixture of saturated aqueous sodium hydrogen carbonate and aqueous ammonia. The organic layer was dried over anhydrous sodium sulfate and the filtrate was concentrated to obtain the title compound (2.5 g, yield: 62%); m/z (ES+): 201$[M+H]^+$.

Intermediate 140

(S)-tert-butyl2-(2-(3-methoxy-2-methylbenzoyl)-5-oxopyrrolidin-1-yl)ethylcarbamate

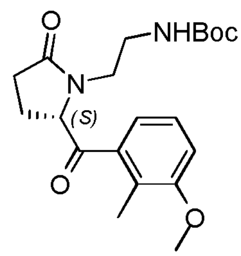

To a solution of intermediate 139 (2.5 g, 12.4 mmol) in THF (30 mL) at -70 °C was added n-BuLi (1.6 M in hexane) (7.8 mL, 12.4mmol) dropwise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2(1H)-carboxylate (2.0 g, 12 mmol) in THF (30 mL). After stirred at -70°C for 5 min, the reaction mixture was quenched with saturated NH4Cl and extracted with EtOAc (2 x 20 mL), washed with brine, dried over Na2SO4, filtered and concentrated in vacuo. The residue was purified by silica gel chromatography to give the title compound (2 g, yield: 45 %); m/z (ES+): 377 $[M+H]^+$.

Intermediate 141

tert-butyl2-((2S)-2-(hydroxy(3-methoxy-2-methylphenyl)methyl)-5-oxopyrrolidin-1-yl) ethylcarbamate

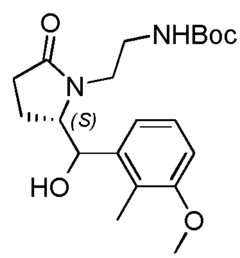

LiAl(OtBu) $_3$H (2 g, 8 mmol) was added portion wisely into intermediate 140 (2 g, 5.3 mmol) in THF (30 mL) at -20 °C. Then the mixture was stirred at room temperature. 2 h later, the starting material was completely consumed; the whole solution was poured into the saturated $NH_4Cl$, the suspension was filtered through a celite pad, washed by EtOAc. The filtration was extracted with EtOAc (2 x 50 mL) washed with brine, dried over $Na_2SO_4$, filtered and concentrated in vacuo. The residue was purified by silica gel chromatography to give the title compound (2 g, yield: 100%); m/z (ES+):

379 [M+H] +.

Intermediate 142

(1S,8aS)-tert-butyl1-(3-methoxy-2-methylphenyl)-6-oxohexahydropyrrolo[1,2-a] pyrazine-2(1H)-carboxylate

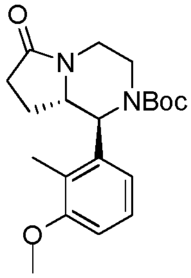

Intermediate 141 (2.3 g, 6.1 mmol) in DCM (20 mL), then $Et_3N$ (3.54 g, 35 mmol), DMAP (85.6 mg, 0.7 mmol) were added. The resulting solution was cooled to 0 °C, and then MsCl (1.18 g, 10.3 mmol) was added into the above solution dropwisely under $N_2$ atmosphere. The reaction was stirred for 20 h at room temperature and then quenched with saturated $NH_4Cl$ and extracted with DCM (2 x 20 mL), washed with brine, dried over $Na_2SO_4$, filtered and concentrated in vacuo. The residue was purified by silica gel chromatography to give the title compound (1.5 g, yield: 67%); m/z (ES+): 361 $[M+H]^+$.

Intermediate 143

(1S,8aS)-1-(3-methoxy-2-methylphenyl)hexahydropyrrolo[1,2-a]pyrazin-6(7H)-one

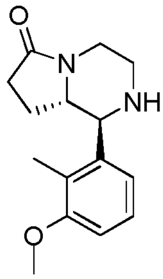

To a solution of intermediate 142 (1.1 g, 3 mmol) in MeOH (20 mL) at 0 °C was added HCl/MeOH (7M, 15 mL). The reaction was stirred at room temperature for 3 h until intermediate 6 was completely consumed. MeOH was removed under reduced pressure, the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (811 mg, yield: 95%); m/z (ES+): 261 $[M+H]^+$.

Intermediate 144

4-bromo-2,3-dihydro-1H-indene

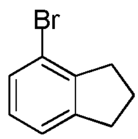

To a solution of 4-bromo-2,3-dihydro-1H-inden-1-one (2 g, 10 mmol) in DCM (10mL) was added TFA, then cooled to - 20°C. $Et_3SiH$ (15 mL, 1N in THF, 15 mmol) was added drop wise. After stirring for 2h at RT the reaction mixture was poured into $NH_4Cl$ aqueous and extracted with DCM (10 mL*2), washed with brine (10 mL*2), dried by $MgSO_4$, concentrated, purified by FC to afford compound (1.7 g, yield: 85%); m/z (ES+): 197 $[M+H]^+$.

Intermediate 145

(S)-tert-butyl 2-(2-(2,3-dihydro-1H-indene-4-carbonyl)-5-oxopyrrolidin-1-yl)ethylcarbamate

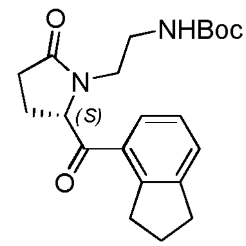

To a solution of intermediate 144 (1.7 g, 12.39 mmol) in THF (40 mL) at -70 °C was added n-BuLi (1.6 M in hexane) (7.52 mL, 12.036mmol) dropwise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2(1H)-carboxylate (3 g, 11.8 mmol) in THF (40 mL). After stirred at -70°C for 5 min, the reaction mixture was quenched with saturated $NH_4Cl$ and extracted with EtOAc (2 x 20 mL), washed with brine, dried over $Na_2SO_4$, filtered and concentrated in vacuo. The residue was purified by silica gel chromatography to give the title compound (1.6 g, yield: 41%); m/z (ES+): 373 $[M+H]^+$.

Intermediate 146

tert-butyl 2-((S)-2-((R)-(2,3-dihydro-1H-inden-4-yl)(hydroxy)methyl)-5-oxopyrrolidin-1-yl)ethylcarbamate

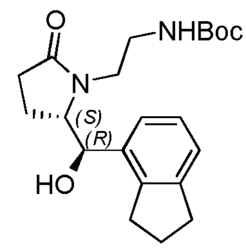

$LiAl(OtBu)_3H$ (1.7 g, 6.5 mmol) was added portion wisely into intermediate 145 (1.6 g, 4.3 mmol) in THF (50 mL) at -20 °C. Then the mixture was stirred at room temperature. 2 h later, the starting material was completely consumed.The whole solution was poured into the saturated $NH_4Cl$, the suspension was filtered through a celite pad, washed by EtOAc. The filtration was extracted with EtOAc (2 x 20 mL) washed with brine, dried over $Na_2SO_4$, filtered and concentrated in vacuo. The residue was purified by silica gel chromatography to give the title compound (1.5 g, yield: 95%); m/z (ES+): 375 $[M+H]^+$.

Intermediate 147

(R)-((S)-1-(2-(tert-butoxycarbonylamino)ethyl)-5-oxopyrrolidin-2-yl)(2,3-dihydro-1H-inden-4-yl)methyl methanesulfonate

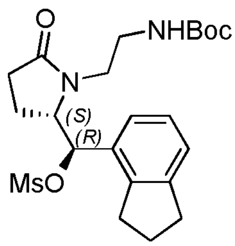

Intermediate 146 (1.5 g, 4 mmol) in DCM (20 mL), then $Et_3N$ (0.7 g, 4 mmol), DMAP (85.6 mg, 0.7 mmol) were added. The resulting solution was cooled to 0 °C, and then MsCl (0.94 g, 8 mmol) was added into the above solution dropwisely under $N_2$ atmosphere. The reaction was stirred for 20 h at room temperature and then quenched with saturated $NH_4Cl$ and extracted with DCM (2 x 20 mL), washed with brine, dried over $Na_2SO_4$, filtered and concentrated in vacuo. The residue was purified by silica gel chromatography to give the title compound (600 mg, yield: 40%); m/z (ES+): 453 $[M+H]^+$.

Intermediate 148

(1S,8aS)-tert-butyl 1-(2,3-dihydro-1H-inden-4-yl)-6-oxohexahydropyrrolo[1,2-a]pyrazine-2(1H)-carboxylate

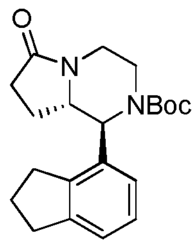

Intermediate 147 (600 mg, 1.1 mmol) in THF (10 mL) was added into a suspension of NaH (60%, 128 mg, 3.2 mmol) in THF (20 mL) at 0 °C under $N_2$ atmosphere. The mixture was stirred at RT for 20 h and then quenched with ice-water. The resulting mixture was extracted with EtOAc (2 x 30 mL), washed with brine, dried over $Na_2SO_4$, filtered and concentrated in vacuo. The residue was purified by silica gel chromatography to give the title compound (400 mg, yield: 66.7%); m/z (ES+): 357 $[M+H]^+$.

Intermediate 149

(1S,8aS)-1-(2,3-dihydro-1H-inden-4-yl)hexahydropyrrolo[1,2-a]pyrazin-6(7H)-one

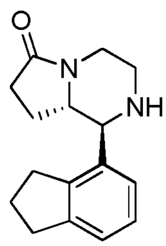

To a solution of intermediate 148 (400 mg, 1.12 mmol) in MeOH (20 mL) at 0 °C was added HCl/MeOH (7M, 5 mL). The reaction was stirred at room temperature for 3 h until intermediate 6 was completely consumed. MeOH was removed under reduced pressure, the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (250 mg, yield: 95%); m/z (ES+): 257 $[M+H]^+$.

Intermediate 150

(S)-tert-butyl 2-(2-(2-fluoro-6-methylbenzoyl)-5-oxopyrrolidin-1-yl)ethylcarbamate

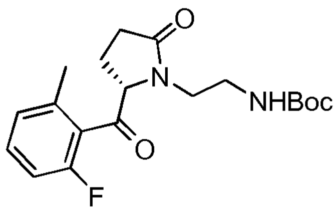

To a solution of 2-bromo-1-fluoro-3-methylbenzene (2.5 g, 10 mmol) in THF (20 mL) at -70 °C was added n-BuLi (1.6 M in hexane) (6.9 mL, 11.0mmol) dropwise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2(1H)-carboxylate (2.07 g, 11.0 mmol) in THF (10 mL). After stirred at -70°C for 5 min, the reaction mixture was quenched with saturated $NH_4Cl$ and extracted with EtOAc (2 x 20 mL), washed with brine, dried over $Na_2SO_4$, filtered and concentrated in vacuo. The residue was purified by silica gel chromatography to give the title compound (2.2 g, yield: 40%); m/z (ES+): 365 $[M+H]^+$.

Intermediate 151

tert-butyl2-((2S)-2-((2-fluoro-6-methylphenyl)(hydroxy)methyl)-5-oxopyrrolidin-1-yl) ethylcarbamate

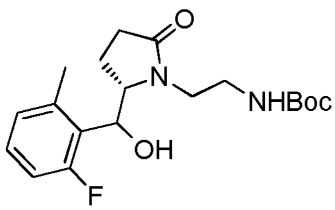

$LiAl(OtBu)_3H$ (4.2 g, 16.5 mmol) was added portion wisely into intermediate 150 (2 g, 5.5 mmol) in THF (15 mL) at -20 °C. Then the mixture was stirred at room temperature. 2 h later, (S)-tert-butyl2-(2-(2-fluoro-6-methylbenzoyl)-5-oxopyrro lidin- 1-yl) ethylcarbamate was completely consumed; the whole solution was poured into the saturated $NH_4Cl$, the suspension was filtered through a celite pad, washed by EtOAc. The filtration was extracted with EtOAc (2 x 20 mL) washed with brine, dried over $Na_2SO_4$, filtered and concentrated in vacuo. The residue was purified by silica gel chromatography to give the title compound (0.9 g, yield: 45%); m/z (ES+): 367$[M+H]^+$.

Intermediate 152

((S)-1-(2-(tert-butoxycarbonylamino)ethyl)-5-oxopyrrolidin-2-yl)(2-fluoro-6-methylphenyl)methyl methanesulfonate

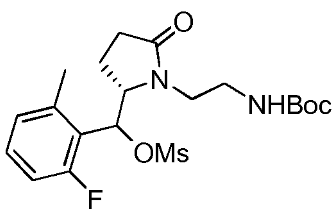

Intermediate 151 (0.9 g, 2.46 mmol) in DCM (20 mL), then $Et_3N$ (746 g, 7.38 mmol) was added. The resulting

solution was cooled to 0 °C, and then MsCl (421 mg, 3.69 mmol) was added into the above solution dropwisely under $N_2$ atmosphere. The reaction was stirred for 20 h at room temperature and then quenched with saturated $NH_4Cl$ and extracted with DCM (2 x 20 mL), washed with brine, dried over $Na_2SO_4$, filtered and concentrated in vacuo. The residue was purified by silica gel chromatography to give the title compound (300 mg, yield: 27.4%); m/z (ES+): 445 $[M+H]^+$.

Intermediate 153

(1S,8aS)-tert-butyl1-(2-fluoro-6-methylphenyl)-6-oxohexahydropyrrolo[1,2-a]pyrazine-2(1H)-carboxylate

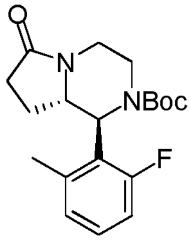

Intermediate 152 (300 mg, 0.68 mmol) in THF (5 mL) was added into a suspension of NaH (60%, 49 mg, 2.04 mmol) in THF (2 mL) at 0 °C under $N_2$ atmosphere. The mixture was stirred at RT for 20 h and then quenched with ice-water. The resulting mixture was extracted with EtOAc (2 x 30 mL), washed with brine, dried over $Na_2SO_4$, filtered and concentrated in vacuo. The residue was purified by silica gel chromatography to give the title compound (150 mg, yield: 63.5%); m/z (ES+): 349 $[M+H]^+$.

Intermediate 154

(1S,8aS)-7,7-diallyl-1-(2,3-dimethylphenyl)hexahydropyrrolo[1,2-a]pyrazin-6(7H)-one

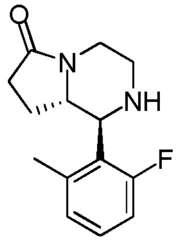

To a solution of intermediate 153 (70 mg, 0.20 mmol) in MeOH (2 mL) at 0 °C was added HCl/MeOH (7M, 5 mL). The reaction was stirred at room temperature for 3 h until intermediate 5 was completely consumed. MeOH was removed under reduced pressure, the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (45 mg, yield: 90%); m/z (ES+): 249 $[M+H]^+$.

Intermediate 155

(S)tert-butyl 2-(2-(2-chloro-4-fluorobenzoyl)-5-oxopyrrolidin-1-vl)ethylcarbamate

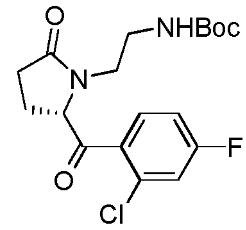

To a solution of 1-bromo-2-chloro-4-fluorobenzene (2.5 g, 12.0 mmol) in toluene (10 mL) at -70 °C was added n-BuLi (1.6 M in hexane) (7.5 mL, 12.0 mmol) dropwise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2(1H) -carboxylate (2.8 g, 10.9 mmol) in toulene (10 mL). After stirred at -70 °C for 5 min, the reaction mixture was quenched with saturated $NH_4Cl$ and extracted with EtOAc (2 x 20 mL). The organic layer was washed with brine, dried over $Na_2SO_4$, filtered and concentrated in vacuo. The residue was purified by silica gel chromatography to give the title compound (850.0 mg, yield: 20%); m/z ($ES^+$): 385 $[M+H]^+$.

Intermediate 156

(S)-1-(2-aminoethyl)-5-(2-chloro-4-fluorobenzoyl)pyrrolidin-2-one

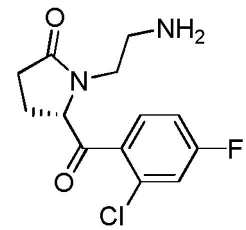

To a solution of intermediate 155 (900.0 mg, 2.3 mmol) in dioxane (20 mL) at 0 °C was added HCl/dioxane (6 M, 10 mL). The reaction was stirred at room temperature for 3 h until intermediate 2 was completely consumed. Dioxane was removed under reduced pressure, the residue was directly used in the next step without further purification (621.0 mg, yield: 95%); m/z ($ES^+$): 285 $[M+H]^+$.

Intermediate 157

1-(2-chloro-4-fluorophenyl)hexahydropyrrolo[1,2-a]pyrazin-6(7H)-one (mixture of trans-isomers)

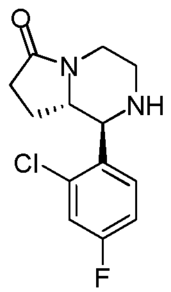

Intermediate 156 (621.0 mg, 2.2 mmol) was dissloved in MeOH (10 mL) and then $NaBH_3CN$ (277.0 mg, 4.4 mmol) and HOAc (1 d) were added to above solution at 0 °C under $N_2$ atmosphere. The mixture was stirred at RT for 20 h and then quenched with saturated $NH_4Cl$. The resulting mixture was extracted with EtOAc (2 x 30 mL), washed with brine, dried over $Na_2SO_4$, filtered and concentrated in vacuo. The residue was purified by silica gel chromatography

to give the title compound (84 mg, yield: 14%); m/z ($ES^+$): 269 $[M+H]^+$.

Intermediate 158

(S)-tert-butyl 2-(2-(4-fluorobenzoyl)-5-oxopyrrolidin-1-yl)ethylcarbamate

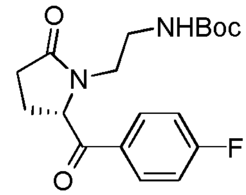

To a solution of 1-bromo-4-fluorobenzene (1.9 g, 11.0 mmol) in THF (10 mL) at -70 °C was added n-BuLi (1.6 M in hexane) (6.9 mL, 11.0 mmol) dropwise over 5 min by syringe along the wall of the flask. After adding, the reaction was stirred at this temperature for 30 min. Then the resulting solution was added into a solution of (S)-tert-butyl 1,6-dioxohexahydropyrrolo[1,2-a]pyrazine-2(1H)-carboxylate (2.5 g, 10.0 mmol) in THF (10 mL). After stirred at -70 °C for 5 min, the reaction mixture was quenched with saturated $NH_4Cl$ and extracted with EtOAc (2 x 20 mL). The organic layer was washed with brine, dried over $Na_2SO_4$, filtered and concentrated in vacuo. The residue was purified by silica gel chromatography to give the title compound (960.0 mg, yield: 27%); m/z ($ES^+$): 351 $[M+H]^+$.

Intermediate 159

tert-butyl 2-((2S)-2-((4-fluorophenyl)(hydroxy)methyl)-5-oxopyrrolidin-1-yl)ethyl carbamate

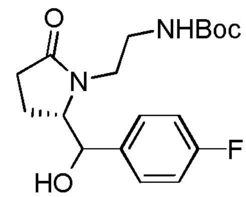

$LiAl(O^tBu)_3H$ (1.4 g, 5.6 mmol) was added portion wisely into intermediate 158 (960.0 mg, 2.8 mmol) in THF (10 mL) at -20 °C. Then the mixture was stirred at room temperature. After two hours, intermediate 2 was completely consumed; the whole solution was poured into the saturated $NH_4Cl$. The suspension was filtered through a celite pad, and washed by EtOAc. The filtration was extracted with EtOAc (2 x 20 mL) and the organic layer was washed with brine, dried over $Na_2SO_4$, filtered and concentrated in vacuo. The residue was purified by silica gel chromatography to give the title compound (770.0 mg, yield: 78.0%); m/z (ES+): 353 $[M+H]^+$.

Intermediate 160

((S)-1-(2-(tert-butoxycarbonylamino)ethyl)-5-oxopyrrolidin-2-yl)(4-fluorophenyl)methyl methanesulfonate

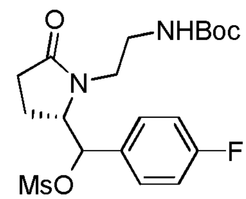

Intermediate 159 (770.0 mg, 2.2 mmol) was dissloved in DCM (15 mL), and then $Et_3N$ (2.2 g, 22.0 mmol) was added. The resulting solution was cooled to 0 °C, and then MsCl (374.0 g, 3.3 mmol) was added into the above solution drop wisely under $N_2$ atmosphere. The reaction was stirred for 20 h at room temperature and quenched with saturated $NH_4Cl$ and extracted with DCM (2 x 20 mL). The organic layer was washed with brine, dried over $Na_2SO_4$, filtered and concentrated in vacuo. The residue was directly used in the next step without further purification (860.0 mg, yield: 91%); m/z (ES+): 431 $[M+H]^+$.

Intermediate 161

(1S,8aS)-tert-butyl 1-(4-fluorophenyl)-6-oxohexahydropyrrolo[1,2-a]pyrazine-2(1H)-carboxylate

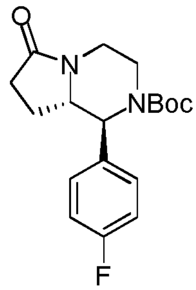

Intermediate 160 (860.0 mg, 2.0 mmol) in THF (10 mL) was added into a suspension of NaH (60%, 160.0 mg, 4.0 mmol) in THF (20 mL) at 0 °C under $N_2$ atmosphere. The mixture was stirred at RT for 20 h and then quenched with ice-water. The resulting mixture was extracted with EtOAc (2 x 30 mL), washed with brine, dried over $Na_2SO_4$, filtered and concentrated in vacuo. The residue was purified by silica gel chromatography to give the title compound (100.0 mg, yield: 15%); m/z (ES+): 335 $[M+H]^+$.

Intermediate 162

1S,8aS)-1-(4-fluorophenyl)hexahydropyrrolo[1,2-a]pyrazin-6(7H)-one

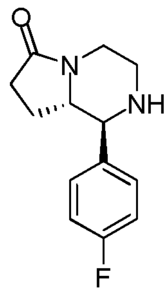

To a solution of intermediate 161 (100.0 mg, 0.3 mmol) in MeOH (20 mL) at 0 °C was added HCl/MeOH (7 M, 5 mL). The reaction was stirred at room temperature for 3 h until intermediate 5 was completely consumed. MeOH was removed under reduced pressure, and the residue was neutralized with 1 M aqueous NaOH solution to pH=10. The mixture was extracted with EtOAc (2 x 20 mL), washed with brine, dried over $Na_2SO_4$, filtered and concentrated in vacuo. The residue was directly used in the next step without further purification (50.0 mg, yield: 71%); m/z ($ES^+$): 235 $[M+H]^+$.

Example 1 & 2: Compound 1 & 2

N-((R)-1-(3,5-bis(trifluoromethyl)phenyl)ethyl)-1-(2,4-dimethylphenyl)-N-methyl-6-oxohexahydropyrrolo[1,2-a]pyrazine-2(1H)-carboxamide (single trans isomers with unknown configuration)

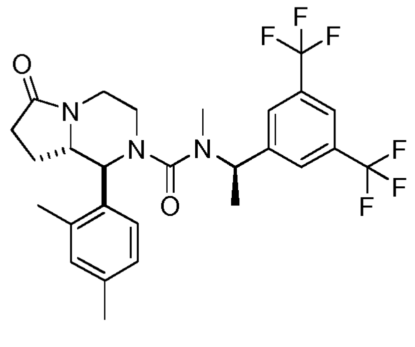

To a solution of triphosgene (65 mg, 0.22 mmol) in EtOAc (10 mL) at 0 °C was added solution of intermediate 3 (mixture of trans, 120 mg, 0.49 mmol), DMAP (4 mg, 0.035 mmol) and TEA (150 mg, 1.5 mmol) in EtOAc (2 mL). The mixture was stirred for 1.5 h at room temperature, followed by addition of (R)-1-(3,5-bis(trifluoromethyl)phenyl)-N-methylethanamine (266 mg, 0.98 mmol) and TEA (107 mg, 1.05 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound 1 (10 mg, 4%) and compound 2 (10 mg, 4%).

compound 1: $^1H$ NMR (400 MHz, $CDCl_3$) δ 7.77 (s, 1H), 7.53 (s, 2H), 7.26 (s, 1H), 7.10 (d, *J* = 7.8 Hz, 1H), 7.00 (s, 1H), 6.92 (d, *J* = 7.6 Hz, 1H), 5.57 (d, *J* = 6.8 Hz, 1H), 4.14 (d, *J* = 12.7 Hz, 1H), 4.02 (d, *J* = 9.8 Hz, 1H), 3.72 (dd, *J* = 16.0, 7.5 Hz, 1H), 3.26 (d, *J* = 11.7 Hz, 1H), 3.10 (t, *J* = 12.3 Hz, 1H), 2.96 (dd, *J* = 11.8, 9.4 Hz, 1H), 2.68 (s, 3H), 2.48 (s, 3H), 2.43 - 2.36 (m, 1H), 2.33 (d, *J* = 8.9 Hz, 1H), 2.28 (s, 3H), 1.91 - 1.78 (m, 1H), 1.70 - 1.59 (m, 1H), 1.44 (d, *J* = 6.8 Hz, 3H); m/z (ES+): 542 $[M+H]^+$

compound 2: $^1H$ NMR (400 MHz, $CDCl_3$) δ 7.71 (s, 1H), 7.36 (s, 2H), 7.11 (d, *J* = 7.8 Hz, 1H), 6.99 (s, 1H), 6.96 (d, *J* = 8.0 Hz, 1H), 5.59 (q, *J* = 7.0 Hz, 1H), 4.16 (d, *J* = 12.8 Hz, 1H), 4.02 (d, *J* = 9.8 Hz, 1H), 3.78 - 3.58 (m, 1H), 3.31 (d, *J* = 12.2 Hz, 1H), 3.12 (t, *J* = 12.4 Hz, 1H), 2.90 (td, *J* = 12.0, 3.0 Hz, 1H), 2.81 (s, 3H), 2.46 (d, *J* = 8.3 Hz, 3H), 2.40 (dd, *J* = 10.0, 3.8 Hz, 1H), 2.35 - 2.30 (m, 1H), 2.28 (s, 3H), 1.85 (ddd, *J* = 16.7, 13.1, 3.9 Hz, 2H), 1.69 (ddd, *J* = 22.5, 13.2, 9.3 Hz, 3H), 1.51 (d, *J* = 7.1 Hz, 3H), 1.24 (t, *J* = 7.0 Hz, 1H).m/z (ES+): 542 $[M+H]^+$.

Example 3: Compound 3

N-(3,5-bis(trifluoromethyl)benzyl)-1-(2,4-dimethylphenyl)-N-methyl-6-oxohexahydropyrrolo[1,2-a]pyrazine-2(1H)-carboxamide (mixture of trans isomers)

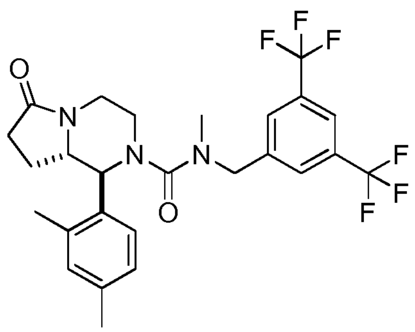

To a solution of triphosgene (65 mg, 0.22 mmol) in EtOAc (10 mL) at 0 °C was added solution of intermediate

3 (mixture of trans, 120 mg, 0.49 mmol), DMAP (4 mg, 0.035 mmol) and TEA (209 μL, 1.5 mmol) in EtOAc (2 mL). The mixture was stirred for 1.5 h at room temperature, followed by addition of 1-(3,5-bis(trifluoromethyl)phenyl)-N-methylmethanamine (251 mg, 0.98 mmol) and TEA (107 mg, 1.05 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound 3 (10 mg, 4%). $^1H$ NMR (600 MHz, $CDCl_3$) δ 7.74 (s, 1H), 7.38 (s, 2H), 7.14 (d, *J* = 7.8 Hz, 1H), 6.99 (s, 1H), 6.95 (d, *J* = 7.8 Hz, 1H), 4.69 (d, *J* = 15.5 Hz, 1H), 4.37 (d, *J* = 15.4 Hz, 1H), 4.16 (d, *J* = 12.9 Hz, 1H), 4.03 (d, *J* = 9.9 Hz, 1H), 3.70 (dd, *J* = 16.9, 7.3 Hz, 1H), 3.36 (d, *J* = 11.7 Hz, 1H), 3.18 - 3.08 (m, 1H), 2.99 - 2.90 (m, 4H), 2.47 (s, 3H), 2.42 (ddd, *J* = 17.0, 10.0, 3.7 Hz, 1H), 2.34 (dd, *J* = 17.7, 8.9 Hz, 1H), 2.28 (s, 3H), 1.86 (ddd, *J* = 13.2, 12.1, 3.8 Hz, 1H), 1.67 (ddd, *J* = 17.3, 13.2, 9.3 Hz, 1H); m/z (ES+): 528 $[M+H]^+$

Example 4 & 5: Compound 4 & 5

1-(2,4-dimethylphenyl)-N-methyl-N-((R)-1-(3-methyl-5-(trifluoromethyl)phenyl)ethyl)-6-oxohexahydropyrrolo[1,2-a]pyrazine-2(1H)-carboxamide

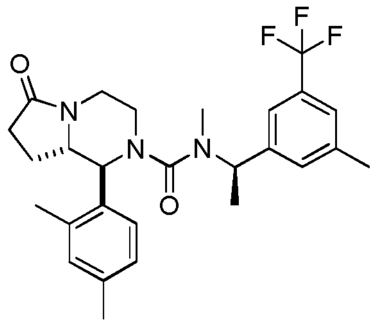

To a solution of triphosgene (47.2 mg, 0.16 mmol) in EtOAc (1 mL) at 0 °C was added solution of intermediate 3 (mixture of trans, 100 mg, 0.32 mmol), DMAP (4 mg, 0.035 mmol) and TEA (97 mg, 0.96 mmol) in EtOAc (2 mL). The mixture was stirred for 1.5 h at room temperature, followed by addition of 1-(3,5-bis(trifluoromethyl)phenyl)-N-methylmethanamine (251 mg, 0.98 mmol) and TEA (107 mg, 1.05 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound 4 (76 mg, yield: 38%) and compound 5 (62 mg, yield: 28%).

Compound 4: $^1H$ NMR (400 MHz, $CDCl_3$) δ 7.25 (s, 1H), 7.16 (d, J = 7.8 Hz, 1H), 7.00 (dd, J = 19.0, 7.2 Hz, 3H), 6.74 (s, 1H), 5.53 (q, J = 6.9 Hz, 1H), 4.15 (d, J = 12.8 Hz, 1H), 4.02 (d, J = 9.8 Hz, 1H), 3.66 (dd, J = 16.3, 7.4 Hz, 1H), 3.29 (d, J = 11.9 Hz, 1H), 3.10 (t, J = 12.4 Hz, 1H), 2.90 (dt, J = 11.1, 5.5 Hz, 1H), 2.77 (s, 3H), 2.48 (d, J = 13.1 Hz, 3H), 2.44 - 2.31 (m, 2H), 2.29 (s, 3H), 2.24 (s, 3H), 1.85 (dd, J = 12.2, 8.3 Hz, 1H), 1.64 - 1.61 (m, 1H), 1.45 (d, J = 7.1 Hz, 3H); m/z (ES+): 488 $[M+H]^+$

Compound 5 $^1H$ NMR (400 MHz, $CDCl_3$) δ 7.31 (s, 1H), 7.27 (s, 1H), 7.12 (d, J = 7.8 Hz, 1H), 7.01 (s, 2H), 6.92 (d, J = 7.7 Hz, 1H), 5.50 (q, J = 6.8 Hz, 1H), 4.12 (d, J = 12.8 Hz, 1H), 4.03 (d, J = 9.9 Hz, 1H), 3.69 (dd, J = 16.6, 7.2 Hz, 1H), 3.28 (d, J = 11.9 Hz, 1H), 3.10 (t, J = 12.3 Hz, 1H), 2.94 (td, J = 11.8, 3.0 Hz, 1H), 2.62 (s, 3H), 2.50 (s, 3H), 2.43 - 2.35 (m, 1H), 2.33 (s, 3H), 2.29 (s, 3H), 1.84 (ddd, J = 16.8, 13.0, 3.9 Hz, 1H), 1.70 - 1.60 (m, 2H), 1.40 (d, J = 6.6 Hz, 3H); m/z (ES+): 488 $[M+H]^+$.

Example 6 & 7: Compound 6 & 7

N-((R)-1-(3,5-bis(trifluoromethyl)phenyl)ethyl)-1-(2,4-dimethylphenyl)-7,7-bis(2-hydroxyethyl)-N-methyl-6-oxohexahydropyrrolo[1,2-a]pyrazine-2(1H)-carboxamide

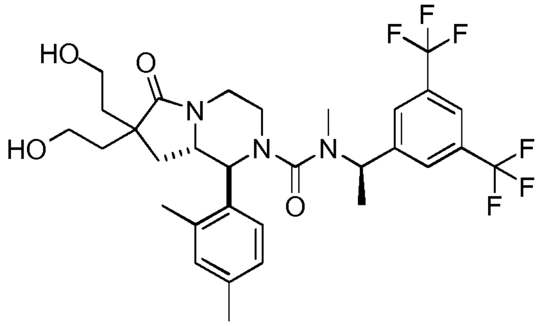

A slow stream of $O_3$ in $O_2$ was passed through a -78 °C cooled solution of intermediate 7 (200 mg, 0.32 mmol) in DCM (30 mL) until a pale blue colour persisted. The reaction was purged with $N_2$ to remove the excess of $O_3$ followed by addition of $NaBH_4$ (1.2 g, 32 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the title compound 6 (15 mg, 7%) and compound 7 (12 mg, 6%).

Compound 6 $^1$H NMR (400 MHz, $CDCl_3$) δ 7.71 (s, 1H), 7.35 (s, 2H), 7.11 (d, *J* = 7.8 Hz, 1H), 7.00 - 6.89 (m, 2H), 5.57 (q, *J* = 6.8 Hz, 1H), 4.11 (d, *J* = 12.6 Hz, 1H), 4.00 (d, *J* = 9.7 Hz, 1H), 3.76 (ddt, *J* = 29.0, 23.0, 8.4 Hz, 4H), 3.55 (dt, *J* = 39.9, 17.1 Hz, 1H), 3.33 (d, *J* = 12.0 Hz, 2H), 3.13 (td, *J* = 12.5, 3.2 Hz, 1H), 2.93 (dd, *J* = 12.1, 9.0 Hz, 1H), 2.80 (s, 3H), 2.48 (s, 3H), 2.27 (s, 3H), 1.89 (t, *J* = 5.6 Hz, 2H), 1.82 (dd, *J* = 13.5, 7.0 Hz, 2H), 1.74 - 1.66 (m, 2H), 1.50 (d, *J* = 7.1 Hz, 3H); m/z (ES+): 630 $[M+H]^+$

Compound 7 $^1$H NMR (400 MHz, $CDCl_3$) δ 7.77 (s, 1H), 7.52 (s, 2H), 7.11 (d, *J* = 7.8 Hz, 1H), 7.00 (s, 1H), 6.93 (d, *J* = 7.7 Hz, 1H), 5.57 (d, *J* = 6.9 Hz, 1H), 4.10 (d, *J* = 12.7 Hz, 1H), 4.01 (d, *J* = 9.8 Hz, 1H), 3.88 - 3.65 (m, 5H), 3.64 - 3.54 (m, 1H), 3.29 (d, J = 11.5 Hz, 1H), 3.12 (t, *J* = 12.3 Hz, 1H), 3.00 (t, *J* = 10.7 Hz, 1H), 2.67 (s, 3H), 2.49 (s, 4H), 2.31 (d, *J* = 17.3 Hz, 4H), 1.85 (ddd, *J* = 19.1, 11.7, 6.0 Hz, 5H), 1.70 (dd, *J* = 12.6, 7.9 Hz, 6H), 1.44 (d, *J* = 6.7 Hz, 3H); m/z (ES+): 630 $[M+H]^+$.

Example 8: Compound 8

1-(2,4-dimethylphenyl)-7,7-bis(2-hydroxyethyl)-N-methyl-N-((R)-1-(3-methyl-5-(trifluoromethyl)phenyl)ethyl)-6-oxo-hexahydropyrrolo[1,2-a]pyrazine-2(1H)-carboxamide (mixture of trans isomers)

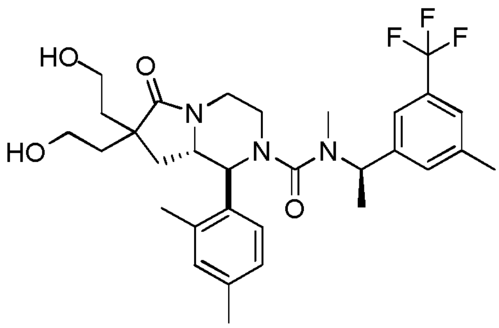

A slow stream of $O_3$ in $O_2$ was passed through a -78°C cooled solution of intermediate 8 (mixture of trans, 180 mg, 0.32 mmol) in DCM (30mL) until a pale blue colour persisted. The reaction was purged with $N_2$ to remove the excess of $O_3$ followed by addition of $NaBH_4$ (1.2 g, 32 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the title compound (22 mg, 12%). $^1$H NMR (400 MHz, $CDCl_3$) δ 7.33 - 7.23 (m, 1H), 7.20 - 7.09 (m, 1H), 6.97 (dt, *J* = 22.5, 8.9 Hz, 1H), 6.73 (s, 1H), 5.51 (dd, *J* = 14.2, 7.1 Hz, 1H), 4.14 - 3.95 (m, 1H), 3.85 - 3.63 (m, 2H), 3.61 - 3.50 (m, 1H), 3.31 (d, *J* = 11.7 Hz, 1H), 3.12 (t, *J* = 12.3 Hz, 1H), 3.07 - 2.82 (m, 1H), 2.76 (s, 1H), 2.61 (s, 1H), 2.47 (d, *J* = 21.7 Hz, 1H), 2.28 (t, *J* = 17.6 Hz, 2H), 1.91 - 1.77 (m, 1H), 1.76 - 1.63 (m, 1H), 1.42

(dd, $J$ = 19.8, 6.4 Hz, 1H); m/z (ES+): 576 $[M+H]^+$.

Example 9: Compound 9

N-(3,5-bis(trifluoromethyl)benzyl)-1-(2,4-dimethylphenyl)-7,7-bis(2-hydroxyethyl)-N-methyl-6-oxohexahydropyrrolo[1,2-a]pyrazine-2(1H)-carboxamide (mixture of trans isomers)

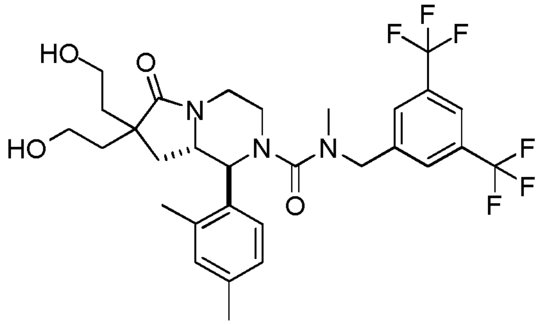

A slow stream of $O_3$ in $O_2$ was passed through a -78 °C cooled solution of intermediate 9 (mixture of trans, 180 mg, 0.32 mmol) in DCM (30mL) until a pale blue colour persisted. The reaction was purged with $N_2$ to remove the excess of $O_3$ followed by addition of $NaBH_4$ (1.2 g, 32 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the title compound (60 mg, 30%). $^1H$ NMR (400 MHz, CDCl3) δ 7.74 (s, 1H), 7.37 (s, 2H), 7.15 (d, J = 7.8 Hz, 1H), 7.02 - 6.92 (m, 2H), 4.69 (d, J = 15.5 Hz, 1H), 4.37 (d, J = 15.3 Hz, 1H), 4.10 (d, J = 12.7 Hz, 1H), 4.02 (d, J = 9.8 Hz, 1H), 3.82 - 3.67 (m, 5H), 3.60 - 3.49 (m, 1H), 3.39 (d, J = 11.9 Hz, 1H), 3.15 (td, J = 12.6, 2.9 Hz, 2H), 3.02 - 2.87 (m, 5H), 2.48 (s, 4H), 2.32 (d, J = 33.5 Hz, 4H), 1.93 - 1.78 (m, 4H), 1.69 (dt, J = 12.0, 6.9 Hz, 2H). m/z (ES+): 616 [M+H]+.

Example 10 and 11: Compound 10 and 11

*N*-((R)-1-(3-chloro-5-(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide

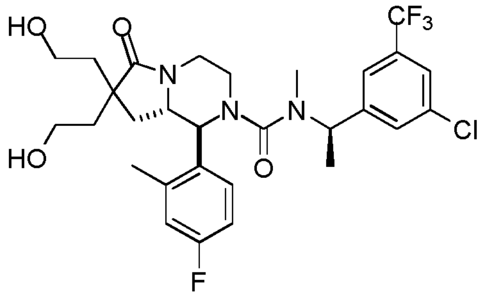

A slow stream of $O_3$ in $O_2$ was passed through a cooled solution of Intermediate 11 (trans mixture, 66 mg, 0.11 mmol) in DCM (10 mL) at -78°C until a pale blue colour persisted. The reaction was purged with $N_2$ to remove the excess of $O_3$ followed by addition of $NaBH_4$ (44 mg, 1.1 mmol). The reaction mixture was allowed to warm to room temperature and stirred for 20 h. The reaction was quenched with water and the resulting mixture was extracted with DCM (2 x 20 mL). The combined organic layers were washed with brine, dried over anhydrous $Na_2SO_4$ and then concentrated in vacuum. The residue was purified by silica gel chromatography (DCM/MeOH=20/1 to 10/1) to give the title compound 10 (20 mg, yield: 30%) and compound 11 (17 mg, yield: 23%).

Compound 10). $^1H$ NMR (400 MHz, $CDCl_3$) δ 7.51 (s, 1H), 7.21 (dd, J = 9.3, 7.1 Hz, 3H), 6.93 - 6.81 (m, 2H), 5.43 (t, J = 24.7 Hz, 1H), 4.10 (d, J = 12.7 Hz, 1H), 4.01 (d, J = 9.8 Hz, 1H), 3.84 - 3.68 (m, 4H), 3.59 (s, 1H), 3.40 (s, 1H), 3.29 (d, J = 11.7 Hz, 1H), 3.18 - 3.08 (m, 1H), 2.98 (dd, J = 11.8, 9.6 Hz, 1H), 2.66 (s, 3H), 2.53 (s, 3H), 1.93 - 1.81 (m, 5H), 1.74 - 1.67 (m, 2H), 1.40 (d, J = 6.3 Hz, 3H); m/z (ES+): 600 $[M+H]^+$.

Compound 11 $^1H$ NMR (400 MHz, $CDCl_3$) δ 7.45 (s, 1H), 7.21 (d, *J* = 6.3 Hz, 1H), 7.09 (s, 1H), 6.94 - 6.85 (m, 3H), 5.50 (d, *J* = 6.8 Hz, 1H), 4.12 (d, *J* = 12.3 Hz, 1H), 4.00 (d, *J* = 9.6 Hz, 1H), 3.79 - 3.66 (m, 4H), 3.61 - 3.55 (m, 1H), 3.31 (d, *J* = 11.2 Hz, 1H), 3.12 (t, *J* = 11.2 Hz, 1H), 2.94 (d, *J* = 10.5 Hz, 1H), 2.80 (s, 3H), 2.64 (d, *J* = 16.5 Hz, 1H), 2.53 (s, 3H), 1.87 (dd, *J* = 16.8, 6.1 Hz, 4H), 1.71 (dd, *J* = 13.3, 5.5 Hz, 2H), 1.47 (d, *J* = 6.9 Hz, 3H); m/z (ES+): 600 $[M+H]^+$.

Example 12 and 13: Compound 12 and 13

1-(4-fluoro-2-methylphenyl)-7,7-bis(2-hydroxyethyl)-*N*-methyl-*N*-((R)-1-(3-methyl-5-(trifluoromethyl)phenyl)ethyl)-6-oxohexahydropyrrolo[1,2-a]pyrazine-2(1*H*)-carboxamide

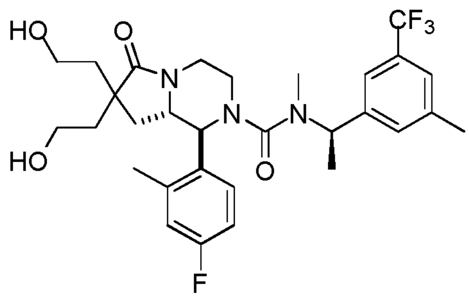

A slow stream of $O_3$ in $O_2$ was passed through a cooled solution of Intermediate 12 (mixture of trans, 100 mg, 0.17 mmol) in DCM (10 mL) at -78 °C until a pale blue colour persisted. The reaction was purged with $N_2$ to remove the excess of $O_3$ followed by addition of $NaBH_4$ (44 mg, 1.1 mmol). The reaction mixture was allowed to warm to room temperature and stirred for 20 h. The raction was quenched with water and the resulting mixture was extracted with DCM (2 x 20 mL). The combined organic layers were washed with brine, dried over anhydrous $Na_2SO_4$ and then concentrated in vacuum. The residue was purified by silica gel chromatography (DCM/MeOH=20/1 to 10/1) to give the title compound 12 (16 mg, yield: 16%) and compound 13 (14 mg, yield: 15%).

Compound 12 $^1H$ NMR (400 MHz, $CDCl_3$) δ 7.24 (d, *J* = 12.8 Hz, 2H), 7.02 (s, 1H), 6.93 - 6.78 (m, 2H), 6.71 (s, 1H), 5.50 (d, *J* = 6.9 Hz, 1H), 4.10 (d, *J* = 12.7 Hz, 1H), 3.99 (d, *J* = 9.7 Hz, 1H), 3.84 - 3.66 (m, 4H), 3.58 (d, *J* = 5.7 Hz, 1H), 3.29 (d, *J* = 12.2 Hz, 1H), 3.10 (t, *J* = 11.4 Hz, 1H), 2.91 (t, *J* = 10.8 Hz, 1H), 2.74 (s, 3H), 2.53 (s, 3H), 2.24 (s, 3H), 1.94 - 1.78 (m, 6H), 1.73 (d, *J* = 9.4 Hz, 3H), 1.44 (d, *J* = 6.8 Hz, 3H); m/z (ES+): 600 $[M+H]^+$.
compound 13. $^1H$ NMR (400 MHz, $CDCl_3$) δ 7.31 (s, 1H), 7.27 - 7.21 (m, 1H), 7.15 (s, 1H), 6.97 (s, 1H), 6.88 (d, *J* = 9.7 Hz, 1H), 6.80 (t, *J* = 7.8 Hz, 1H), 5.45 (d, *J* = 6.9 Hz, 1H), 4.04 (dd, *J* = 26.8, 11.4 Hz, 2H), 3.73 (ddd, *J* = 20.2, 13.2, 6.0 Hz, 4H), 3.61 - 3.53 (m, 1H), 3.28 (d, *J* = 11.7 Hz, 1H), 3.10 (t, *J* = 12.4 Hz, 1H), 2.96 (t, *J* = 10.7 Hz, 1H), 2.62 (s, 3H), 2.53 (s, 3H), 2.33 (s, 3H), 1.90 - 1.80 (m, 4H), 1.71 - 1.64 (m, 2H), 1.37 (d, *J* = 5.7 Hz, 3H); m/z (ES+): 580 $[M+H]^+$.

Example 14 and 15: Compound 14 and 15

*N*-((R)-1-(3,5-bis(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide

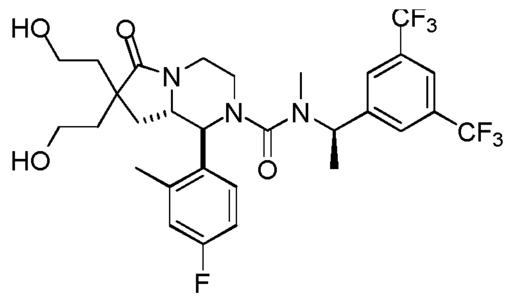

A slow stream of $O_3$ in $O_2$ was passed through a cooled solution of Intermediate 13 (mixture of trans, 200 mg, 0.32 mmol) in DCM (10 mL) at -78 °C until a pale blue colour persisted. The reaction was purged with $N_2$ to remove the excess of $O_3$ followed by addition of $NaBH_4$ (243 mg, 6.4 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the title compound 14 (10 mg, yield: 4.9%) and compound 15 (10 mg, yield: 4.9%).

Compound 14. $^1$H NMR (400 MHz, $CDCl_3$) δ 7.73 (s, 1H), 7.38 - 7.18 (m, 3H), 6.93 - 6.76 (m, 2H), 5.57 (d, *J* = 6.6 Hz, 1H), 4.07 (dd, *J* = 44.3, 11.2 Hz, 2H), 3.91 - 3.48 (m, 5H), 3.34 (d, *J* = 11.8 Hz, 1H), 3.13 (t, *J* = 11.2 Hz, 1H), 3.02 - 2.70 (m, 4H), 2.53 (s, 3H), 1.83 (dd, *J* = 41.1, 27.5 Hz, 7H), 1.52 (d, *J* = 6.6 Hz, 3H); m/z (ES+): 634 $[M+H]^+$.

Compound 15. $^1$H NMR (400 MHz, $CDCl_3$) δ 7.78 (s, 1H), 7.53 (s, 2H), 7.24 (d, *J* = 19.4 Hz, 2H), 7.02 - 6.67 (m, 2H), 5.54 (s, 1H), 4.06 (dd, *J* = 37.9, 10.7 Hz, 2H), 3.89 - 3.50 (m, 5H), 3.28 (d, *J* = 10.0 Hz, 1H), 3.05 (dd, *J* = 37.8, 10.5 Hz, 2H), 2.61 (d, *J* = 66.8 Hz, 7H), 1.79 (dd, *J* = 60.9, 12.4 Hz, 7H), 1.43 (s, 3H); m/z (ES+): 634 $[M+H]^+$.

Example 16: Compound 16

N-((R)-1-(3,5-bis(trifluoromethyl)phenyl)ethyl)-7,7-bis(2-hydroxyethyl)-1-(4-methoxy-2-methylphenyl)-N-methyl-6-oxo-hexahydropyrrolo[1,2-a]pyrazine-2(1H)-carboxamide

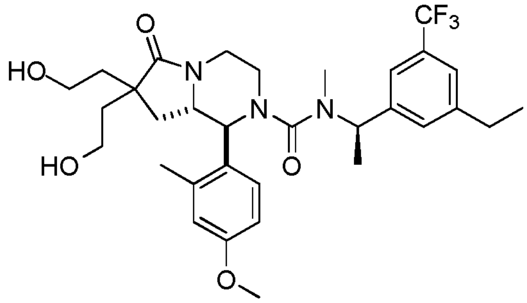

A slow stream of $O_3$ in $O_2$ was passed through a -78 °C cooled solution of intermediate 16 (mixture of trans, 65 mg, 0.1 mmol) in DCM (10 mL) until a pale blue colour persisted. The reaction was purged with $N_2$ to remove the excess of $O_3$ followed by addition of $NaBH_4$ (60 mg, 1.5 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by prep-HPLC to give the title compound (12 mg, 20%). $^1$H NMR (400 MHz, $CDCl_3$) δ 7.33 (s, 1H), 7.17 (d, *J* = 8.8 Hz, 2H), 7.04 (s, 1H), 6.72 (d, *J* = 2.5 Hz, 1H), 6.67 (dd, *J* = 8.5, 2.3 Hz, 1H), 5.50 (q, *J* = 6.8 Hz, 1H), 4.08 (d, *J* = 12.8 Hz, 1H), 3.98 (d, *J* = 9.8 Hz, 1H), 3.88 - 3.65 (m, 7H), 3.59 (dt, *J* = 11.7, 6.0 Hz, 1H), 3.28 (d, *J* = 11.5 Hz, 1H), 3.11 (td, *J* = 12.4, 3.2 Hz, 1H), 2.97 (dd, *J* = 11.9, 9.1 Hz, 1H), 2.65 - 2.57 (m, 5H), 2.52 (s, 3H), 1.88 (dd, *J* = 10.4, 4.6 Hz, 3H), 1.85 - 1.80 (m, 2H), 1.76 - 1.61 (m, 4H), 1.38 (d, *J* = 6.1 Hz, 3H), 1.20 (t, *J* = 7.6 Hz, 3H); m/z (ES+): 606 $[M+H]^+$.

Example 17: Compound 17

(1S,8aS)-7,7-bis(2-hydroxyethyl)-1-(4-methoxy-2-methylphenyl)-N-methyl-N-((R)-1-(3-methyl-5-(trifluoromethyl)phenyl)ethyl)-6-oxohexahydropyrrolo[1,2-a]pyrazine-2(1H)-carboxamide

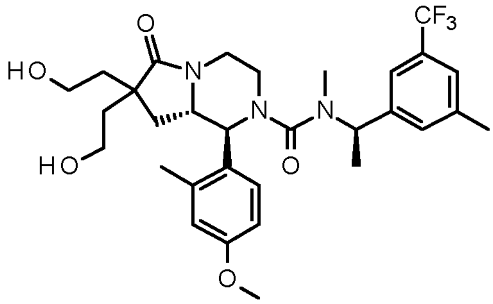

A slow stream of $O_3$ in $O_2$ was passed through a -78 °C cooled solution of Intermediate 16 (82 mg, 0.15 mmol) in DCM (10 mL) until a pale blue colour persisted. The reaction was purged with $N_2$ to remove the excess of $O_3$ followed by addition of $NaBH_4$ (60 mg, 1.5 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by prep-HPLC to give the title compound (33.0 mg, yield: 40%). $^1H$ NMR (400 MHz, $CDCl_3$) $\delta$ 7.31 (s, 1H), 7.27 (s, 1H), 7.16 (d, *J* = 8.5 Hz, 2H), 6.99 (s, 1H), 6.72 (d, *J* = 2.5 Hz, 1H), 6.68 - 6.61 (m, 1H), 5.49 (q, *J* = 6.7 Hz, 1H), 4.08 (d, *J* = 12.7 Hz, 1H), 3.98 (d, *J* = 9.8 Hz, 1H), 3.84 (dd, *J* = 11.7, 5.9 Hz, 1H), 3.78 (s, 3H), 3.71 (d, *J* = 5.7 Hz, 1H), 3.60 (dd, *J* = 11.6, 5.8 Hz, 1H), 3.29 (d, *J* = 12.1 Hz, 1H), 3.11 (td, *J* = 12.3, 3.3 Hz, 1H), 2.97 (dd, *J* = 11.9, 9.0 Hz, 1H), 2.62 (s, 3H), 2.52 (s, 3H), 2.33 (s, 3H), 1.89 (t, *J* = 5.9 Hz, 2H), 1.83 (dd, *J* = 8.2, 5.6 Hz, 2H), 1.81 - 1.62 (m, 5H), 1.39 (d, *J* = 6.3 Hz, 3H) ; m/z (ES+): 592 $[M+H]^+$.

Example 18 & 19: Compound 18 & 19

*N*-((R)-1-(3,5-bis(trifluoromethyl)phenyl)ethyl)-1-(4-methoxy-2-methylphenyl)-*N*-methyl-6-oxohexahydropyrrolo[1,2-a]pyrazine-2(1*H*)-carboxamide

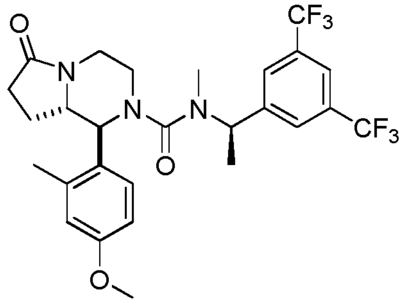

To a solution of triphosgene (47.2 mg, 0.16 mmol) in EtOAc (1 mL) at 0 °C was added solution of intermediate 17 (mixture of trans, 100 mg, 0.32 mmol), DMAP (3.9 mg, 0.032 mmol) and TEA (97 mg, 0.96 mmol) in EtOAc (2 mL). The mixture was stirred for 2 h, followed by addition of (R)-1-(3, 5-bis(trifluoromethyl)phenyl)-N-methylethanamine (100 mg, 0.44 mmol) and TEA (97 mg, 0.96 mmol) in EtOAc (2 mL). The reaction was stirred at 50 °C for 48 h and quenched with water. The resulting mixture was extracted with EtOAc. The organic layers were washed with brine, dried over anhydrous $Na_2SO_4$ and then concentrated in high vacuum. The residue was purified by silica gel chromatography (DCM/MeOH=20/1 to 10/1) to give give the compound 18 (34 mg, yield: 20%) and compound 19 (74 mg, yield: 37%);

Compound 18. $^1H$ NMR (400 MHz, $CDCl_3$) $\delta$ 7.68 (s, 1H), 7.45 (s, 2H), 7.05 (d, *J* = 8.5 Hz, 1H), 6.61 (dd, *J* = 21.6, 5.5 Hz, 2H), 5.50 - 5.43 (m, 1H), 4.04 (d, *J* = 12.9 Hz, 1H), 3.89 (d, *J* = 9.9 Hz, 1H), 3.69 (s, 3H), 3.62 (d, *J* = 8.7

Hz, 1H), 3.15 (d, J = 11.7 Hz, 1H), 2.98 (d, *J* = 12.2 Hz, 1H), 2.86 (dd, *J* = 11.8, 9.1 Hz, 1H), 2.59 (s, 3H), 2.41 (s, 3H), 2.28 (ddd, *J* = 31.9, 17.1, 10.8 Hz, 2H), 1.83 - 1.71 (m, 1H), 1.33 (d, *J* = 7.0 Hz, 3H); m/z (ES+): 558 $[M+H]^+$.
Compound 19: $^1H$ NMR (400 MHz, $CDCl_3$) δ 7.62 (s, 1H), 7.29 (s, 2H), 7.05 (d, *J* = 8.4 Hz, 1H), 6.64 - 6.57 (m, 2H), 5.51 (d, *J* = 7.4 Hz, 1H), 4.06 (d, *J* = 13.2 Hz, 1H), 3.89 (d, *J* = 9.9 Hz, 1H), 3.69 (s, 3H), 3.56 (d, *J* = 10.1 Hz, 1H), 3.22 (d, *J* = 12.0 Hz, 1H), 3.00 (d, *J* = 12.7 Hz, 1H), 2.81 (dd, *J* = 11.9, 8.6 Hz, 1H), 2.72 (s, 3H), 2.40 (s, 3H), 2.27 (dd, *J* = 19.9, 6.0 Hz, 2H), 1.94 - 1.86 (m, 1H), 1.76 (s, 1H), 1.42 (d, *J* = 7.2 Hz, 3H); m/z (ES+): 558 $[M+H]^+$.

Example 20: Compound 20

(1S,8aS)-N-((R)-1-(3,5-bis(trifluoromethyl)phenyl)ethyl)-1-(4-fluoro-2-methylphenyl)-N-methyl-6-oxohexahydropyrrolo[1,2-a]pyrazine-2(1H)-carboxamide

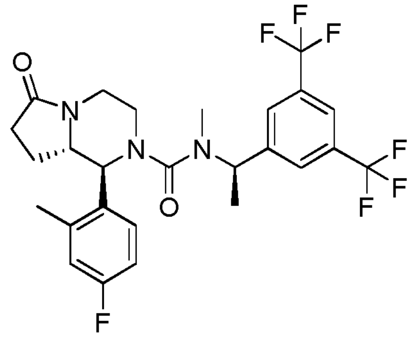

To a solution of triphosgene (36 mg, 0.1 mmol) in EtOAc (2 mL) at 0 °C was added solution of Intermediate 25 (60 mg, 0.24 mmol), DMAP (3 mg, 0.02 mmol) and TEA (73 mg, 0.72 mmol) in EtOAc (2 mL). The mixture was stirred for 1.5 h at room temperature, followed by addition of (R)-1-(3,5-bis(trifluoromethyl)phenyl)-N-methylethanamine (78 mg, 0.28 mmol) and TEA (73 mg, 0.72 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (30 mg, yield: 23%). $^1H$ NMR (400 MHz, CDCl3) δ 7.78 (s, 1H), 7.54 (s, 2H), 7.24 - 7.17 (m, 1H), 6.90 (d, J = 9.9 Hz, 1H), 6.83 (s, 1H), 5.53 (d, J = 7.0 Hz, 1H), 4.15 (d, J = 12.6 Hz, 1H), 4.01 (d, J = 9.8 Hz, 1H), 3.71 (s, 1H), 3.25 (d, J = 11.4 Hz, 1H), 3.10 (s, 1H), 2.96 (d, J = 12.1 Hz, 1H), 2.70 (s, 3H), 2.52 (s, 3H), 2.46 - 2.29 (m, 2H), 1.86 (s, 1H), 1.60 (s, 1H), 1.42 (d, J = 7.1 Hz, 3H); m/z (ES+): 546 [M+H]+.

Example 21: Compound 21

(1S,8aS)-1-(4-fluoro-2-methylphenyl)-N-methyl-N-((R)-1-(3-methyl-5-(trifluoromethyl) phenyl)ethyl)-6-oxohexahydropyrrolo[1,2-a]pyrazine-2(1H)-carboxamide

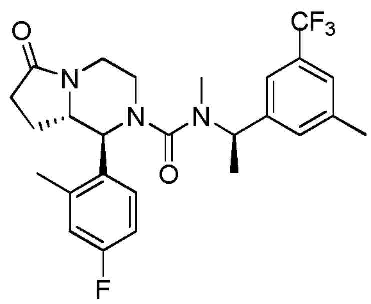

To a solution of triphosgene (23 mg, 0.08 mmol) in EtOAc (2 mL) at 0 °C was added a solution of Intermediate 25 (40 mg, 0.161 mmol), DMAP (2 mg, 0.016 mmol) and TEA (49 mg, 0.483 mmol) in EtOAc (2 mL). The mixture was stirred for 2 h, followed by addition of (R)-N-methyl-1-(3-methyl-5-(trifluoromethyl)phenyl)ethanamine (44 mg, 0.24 mmol)

in EtOAc (2 mL) and TEA (49 mg, 0.483 mmol). The reaction was stirred at 50 °C for 48 h and quenched with water. The resulting mixture was extracted with EtOAc (2 x 20 mL). The organic layers were washed with brine, dried over anhydrous $Na_2SO_4$ and then concentrated in high vacuum. The residue was purified by silica gel chromatography (DCM/MeOH=100/1 to 75/1) to give the title compound (10 mg, yield: 13%). $^1H$ NMR (400 MHz, $CDCl_3$) $\delta$ 7.32 (s, 1H), 7.20 (dd, $J$ = 14.3, 7.7 Hz, 2H), 6.99 (s, 1H), 6.90 (d, $J$ = 8.4 Hz, 1H), 6.81 (t, $J$ = 7.5 Hz, 1H), 5.47 (d, $J$ = 6.7 Hz, 1H), 4.14 (d, $J$ = 12.8 Hz, 1H), 4.02 (d, $J$ = 9.9 Hz, 1H), 3.67 (d, $J$ = 8.2 Hz, 1H), 3.27 (d, $J$ = 11.7 Hz, 1H), 3.09 (t, $J$ = 12.0 Hz, 1H), 2.93 (t, $J$ = 10.8 Hz, 1H), 2.64 (s, 3H), 2.54 (s, 3H), 2.40 (dd, $J$ = 10.3, 3.9 Hz, 1H), 2.34 (s, 3H), 2.29 (s, 1H), 1.92 - 1.77 (m, 1H), 1.66 (d, $J$ = 17.5 Hz, 1H), 1.39 (d, $J$ = 5.9 Hz, 3H); m/z (ES+): 492 $[M+H]^+$.

Example 22: Compound 22

(1S,8aS)-1-(4-fluoro-2-methylphenyl)-N-(1-(3-isopropyl-5-(trifluoromethyl)phenyl)ethyl) -N-methyl-6-oxohexahydropyrrolo[1,2-a]pyrazine-2(1H)-carboxamide

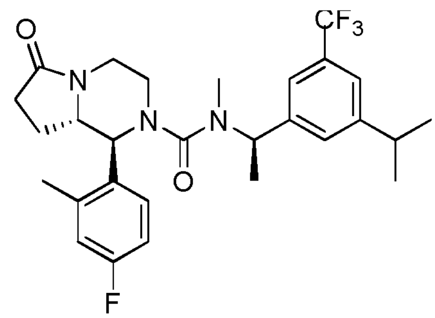

To a solution of triphosgene (73.45 mg, 0.25 mmol) in EtOAc (2 mL) at 0 °C was added solution of Intermediate 25 (162 mg, 0.5 mmol), DMAP (6.1 mg, 0.05 mmol) and TEA (151.68 mg, 1.5 mmol) in EtOAc (2 mL). The mixture was stirred for 1.5 h at room temperature, followed by addition of [1-(3-Isopropyl-5-trifluoromethyl-phenyl)-ethyl]-methyl-amine (163.2 mg, 0.6 mmol) and TEA (151.68 mg, 1.5 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (DCM/MeOH=100/1 to 75/1) to give the title compound (120 mg, yield: 39%). $^1H$ NMR (400 MHz, $CDCl_3$) $\delta$ 7.36 (s, 1H), 7.24 - 7.17 (m, 2H), 7.10 (s, 1H), 6.90 (dd, $J$ = 9.8, 2.4 Hz, 1H), 6.83 (dd, $J$ = 11.3, 5.3 Hz, 1H), 5.48 (q, $J$ = 7.0 Hz, 1H), 4.07 (dd, $J$ = 45.2, 11.3 Hz, 2H), 3.67 (dd, $J$ = 16.8, 7.6 Hz, 1H), 3.25 (d, $J$ = 11.8 Hz, 1H), 3.09 (t, $J$ = 12.4 Hz, 1H), 2.91 (ddd, $J$ = 20.5, 12.9, 4.9 Hz, 2H), 2.65 (s, 3H), 2.54 (s, 3H), 2.47 - 2.28 (m, 2H), 1.84 (dt, $J$ = 16.7, 6.5 Hz, 1H), 1.69 - 1.64 (m, 1H), 1.37 (d, $J$ = 6.7 Hz, 3H), 1.22 (t, $J$ = 6.8 Hz, 6H); m/z (ES+): 520 $[M+H]^+$.

Example 23: Compound 23

(1S,8aS)-N-((R)-1-(3-ethvl-5-(trifluoromethyl)phenyl)ethyl)-1-(4-fluoro-2-methylphenyl)-N-methyl-6-oxohexahydropyrrolo[1,2-a]pyrazine-2(1H)-carboxamide

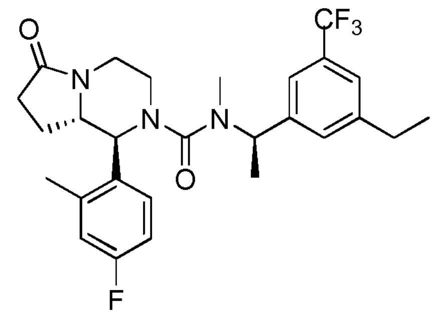

To a solution of triphosgene (29.6 mg, 0.1 mmol) in EtOAc (2 mL) at 0 °C was added solution of Intermediate 25 (49.6 mg, 0.2 mmol), DMAP (2.4 mg, 0.02 mmol) and TEA (61 mg, 0.6 mmol) in EtOAc (2 mL). The mixture was stirred for 1.5 h at room temperature, followed by addition of (R)-1-(3-ethyl-5-(trifluoromethyl)phenyl)-N-methyl ethanamine (55.5 mg, 0.24 mmol) and TEA (61 mg, 0.6 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (10 mg, yield: 10%); $^1$H-NMR (400 MHz, $CDCl_3$) $\delta$ ppm 7.34 (s, 1H), 7.04 - 6.91 (m, 2H), 6.90 - 6.81 (m, 3H), 5.49-5.47 (q, 1H), 4.15 - 4.00 (dd, 2H), 3.68 (m, 1H), 3.27 - 2.92 (m, 3H), 2.54 (m, 5H), 2.39 (s, 3H), 2.33 (m, 2H), 2.4 (s, 3H), 1.98 (m, 1H), 1.7 (m, 1H), 1.40 (d, 3H), 1.24 (d, 3H); m/z (ES+): 506 $[M+H]^+$.

Example 24: Compound 24

(1S,8aS)-N-((R)-1-(3-ethyl-5-(trifluoromethyl)phenyl)ethyl)-1-(4-fluoro-2-methylphenyl)-7,7-bis(2-hydroxyethyl)-N-methyl-6-oxohexahydropyrrolo[1,2-a]pyrazine-2(1H)-carboxamide

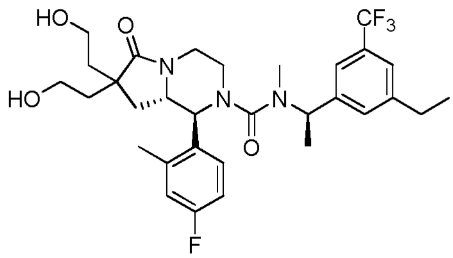

A slow stream of $O_3$ in $O_2$ was passed through a cooled solution of Intermediate 27 (120 mg , 0.193 mmol) in DCM (10 mL) at -78 °C until a pale blue colour persisted. The reaction mixture was purged with $N_2$ to remove the excess of $O_3$ followed by addition of $NaBH_4$ (74 mg, 1.93 mmol). The reaction mixture was allowed to warm to room temperature and stirred for 20 h. After quenched with $NH_4Cl$ solution, the resulting mixture was extracted with DCM (2x 20 mL). The combined organic layers were washed with brine, dried over anhydrous $Na_2SO_4$, and concentrated in vacuo. The residue was purified by Prep-HPLC to give the title compound (60 mg, yield: 50%). $^1$H NMR (400 MHz, $CDCl_3$) $\delta$ 7.34 (s, 1H), 7.26 - 7.15 (m, 2H), 7.04 (s, 1H), 6.91 - 6.78 (m, 2H), 5.48 (q, *J* = 6.7 Hz, 1H), 4.05 (dd, *J* = 31.0, 11.3 Hz, 2H), 3.77 (dtd, *J* = 23.6, 11.7, 5.9 Hz, 4H), 3.63 - 3.54 (m, 1H), 3.28 (d, *J* = 11.8 Hz, 1H), 3.16 - 3.07 (m, 1H), 2.97 (dt, *J* = 11.8, 5.8 Hz, 1H), 2.69 - 2.60 (m, 5H), 2.54 (s, 3H), 1.89 (t, *J* = 5.9 Hz, 2H), 1.86 - 1.79 (m, 3H), 1.72 (dd, *J* = 13.1, 5.6 Hz, 2H), 1.68 (d, *J* = 7.7 Hz, 1H), 1.40 (t, *J* = 11.2 Hz, 3H), 1.21 (t, *J* = 7.6 Hz, 3H); m/z (ES+): 594 $[M+H]^+$.

Example 25: Compound 25

(1S,8aS)-1-(4-fluoro-2-methylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide

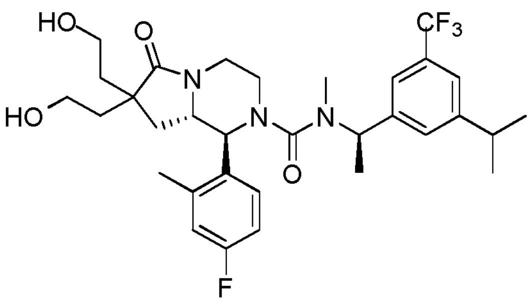

A slow stream of $O_3$ in $O_2$ was passed through a cooled solution of Intermediate 28 (110 mg, 0.193 mmol) in

DCM (10 mL) at -78°C until a pale blue colour persisted. The reaction was purged with $N_2$ to remove the excess of $O_3$ followed by addition of $NaBH_4$ (74 mg, 1.93 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the title compound (60 mg, yield: 50%). $^1H$ NMR (400 MHz, $CDCl_3$) δ 7.36 (s, 1H), 7.25 (dd, *J* = 11.2, 5.3 Hz, 1H), 7.17 (s, 1H), 7.09 (s, 1H), 6.89 (dd, *J* = 9.8, 2.5 Hz, 1H), 6.86 - 6.80 (m, 1H), 5.48 (q, *J* = 6.8 Hz, 1H), 4.08 (d, *J* = 12.7 Hz, 1H), 4.01 (d, *J* = 9.8 Hz, 1H), 3.82 (dt, *J* = 9.9, 5.1 Hz, 1H), 3.74 (ddd, *J* = 17.0, 12.0, 6.4 Hz, 3H), 3.63 - 3.55 (m, 1H), 3.27 (d, *J* = 11.6 Hz, 1H), 3.10 (td, *J* = 12.5, 3.3 Hz, 1H), 3.03 - 2.85 (m, 2H), 2.64 (s, 3H), 2.54 (s, 3H), 1.85 (ddd, *J* = 21.0, 11.4, 5.5 Hz, 5H), 1.75 - 1.66 (m, 2H), 1.37 (d, *J* = 6.4 Hz, 3H), 1.26 - 1.17 (m, 7H); m/z (ES+): 608 $[M+H]^+$.

Example 26: Compound 26

(1S,8aS)-1-(4-(benzyloxy)-2-methylphenyl)-N-((R)-1-(3,5-bis(trifluoromethyl)phenyl)

ethyl)-N-methyl-6-oxohexahydropyrrolo[1,2-a]pyrazine-2(1H)-carboxamide

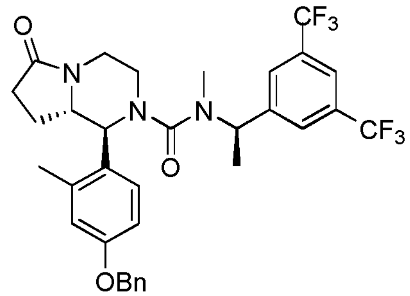

To a solution of triphosgene (44 mg, 0.15 mmol) in EtOAc (2 mL) at 0 °C was added solution of Intermediate 40 (100 mg, 0.3 mmol), DMAP (4 mg, 0.05 mmol) and TEA (91 mg, 0.9 mmol) in EtOAc (2 mL). The mixture was stirred for 1.5 h at room temperature, followed by addition of (R)-1-(3,5-bis(trifluoromethyl)phenyl)-N-methylethanamine (122 mg, 0.45 mmol) in EtOAc (2 mL) and TEA (91 mg, 0.9 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (DCM/MeOH=20/1 to 10/1) to give the title compound (90 mg, yield: 47%); m/z (ES+): 634 $[M+H]^+$.

Example 27: Compound 27

(1S,8aS)-N-((R)-1-(3,5-bis(trifluoromethyl)phenyl)ethyl)-1-(4-hydroxy-2-methylphenyl)-N-methyl-6-oxohexahydropyrrolo[1,2-a]pyrazine-2(1H)-carboxamide

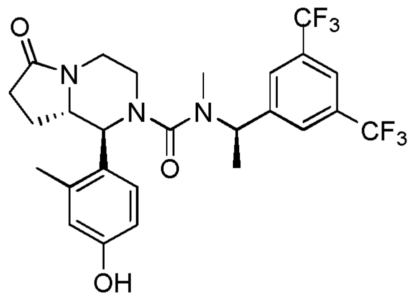

To a solution of compound 26 (90 mg, 0.142 mmol) in MeOH (20 mL) was added Pd/C (5%, 100 mg). The

reaction was stirred under $H_2$ (4 atm) for 12 h. The catalyst was filtered through a celite pad and the solvent was removed in vacuo. The residue was purified by silica gel chromatography (DCM/MeOH=50/1 to 20/1) to give the title compound (20 mg, yield: 26%). $^1H$ NMR (400 MHz, $CDCl_3$) δ 7.78 (s, 1H), 7.54 (s, 2H), 7.09 (d, *J* = 8.4 Hz, 1H), 6.63 (d, *J* = 2.4 Hz, 1H), 6.55 (d, *J* = 8.3 Hz, 1H), 6.01 (s, 1H), 5.56 (q, *J* = 6.9 Hz, 1H), 4.14 (d, *J* = 12.7 Hz, 1H), 3.98 (d, *J* = 9.9 Hz, 1H), 3.75 (dd, *J* = 16.6, 7.5 Hz, 1H), 3.26 (d, *J* = 11.9 Hz, 1H), 3.17 - 2.88 (m, 2H), 2.70 (s, 3H), 2.43 (d, *J* = 7.6 Hz, 3H), 2.35 (ddd, *J* = 29.1, 15.3, 9.0 Hz, 2H), 1.89 (ddd, *J* = 16.8, 13.0, 4.0 Hz, 1H), 1.66 - 1.54 (m, 1H), 1.43 (d, *J* = 6.9 Hz, 3H) ; m/z (ES+): 544 $[M+H]^+$.

Example 28: Compound 28

(1S,8aS)-N-((R)-1-(3-ethyl-5-(trifluoromethyl)phenyl)ethyl)-1-(4-hydroxy-2-methylphenyl)-N-methyl-6-oxohexahydropyrrolo[1,2-a]pyrazine-2(1H)-carboxamide

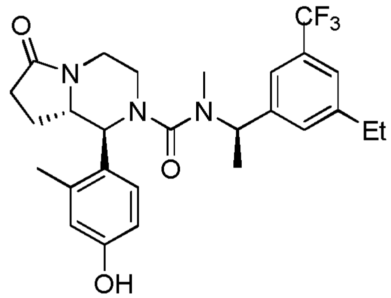

To a solution of Intermediate 41 (50 mg, 0.073 mol) in MeOH (5 mL) was added Pd/C (5%, 30 mg). The reaction was stirred under $H_2$ (4 atm) for 12 h. The catalyst was filtered through a celite pad and the solvent was removed in vacuo. The residue was purified by pre_HPLC to give the title compound (15 mg, yield: 30%); $^1H$ NMR (400 MHz, $CDCl_3$) δ 7.34 (s, 1H), 7.19 (s, 1H), 7.10 (d, *J* = 8.4 Hz, 1H), 7.04 (s, 1H), 6.60 (d, *J* = 2.4 Hz, 1H), 6.51 (d, *J* = 7.6 Hz, 1H), 6.15 (s, 1H), 5.50 (q, *J* = 6.8 Hz, 1H), 4.11 (d, *J* = 12.7 Hz, 1H), 3.98 (d, *J* = 9.9 Hz, 1H), 3.75 (d, *J* = 8.3 Hz, 1H), 3.28 (d, *J* = 11.7 Hz, 1H), 3.10 (t, *J* = 12.2 Hz, 1H), 2.97 (dd, *J* = 11.7, 9.1 Hz, 1H), 2.64 (d, *J* = 4.8 Hz, 4H), 2.46 - 2.42 (m, 3H), 2.37 (dd, *J* = 14.1, 5.9 Hz, 1H), 1.92 - 1.85 (m, 1H), 1.70 - 1.61 (m, 3H), 1.40 (d, *J* = 6.8 Hz, 3H), 1.21 (t, *J* = 7.6 Hz, 3H); m/z (ES+): 504 $[M+H]^+$.

Example 29: Compound 29

(1S,8aS)-N-((R)-1-(3,5-bis(trifluoromethyl)phenyl)ethyl)-7,7-bis(2-hydroxyethyl)-1-(4-methoxy-2-methylphenyl)-N-methyl-6-oxohexahydropyrrolo[1,2-a]pyrazine-2(1H)-carboxamide

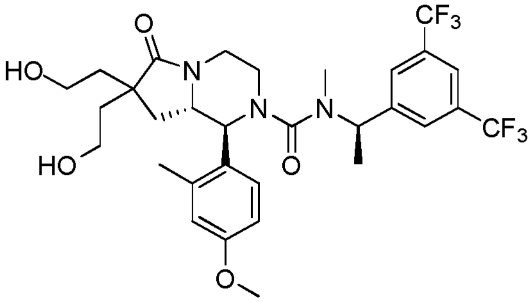

A slow stream of $O_3$ in $O_2$ was passed through a -78 °C cooled solution of intermediate 35 (200 mg, 0.32 mmol) in DCM (20 mL) until a pale blue colour persisted. The reaction was purged with $N_2$ to remove the excess of $O_3$ followed by addition of $NaBH_4$ (120 mg, 3.2 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was

purified by prep-HPLC to give the title compound (15 mg, 7%). $^1H$ NMR (400 MHz, $CDCl_3$) δ 7.78 (s, 1H), 7.54 (s, 2H), 7.27 (s, 1H), 6.82 - 6.61 (m, 2H), 5.56 (q, *J* = 6.8 Hz, 1H), 4.10 (d, *J* = 12.7 Hz, 1H), 3.97 (d, *J* = 9.8 Hz, 1H), 3.88 - 3.76 (m, 6H), 3.72 - 3.51 (m, 2H), 3.27 (d, *J* = 11.6 Hz, 2H), 3.17 - 2.90 (m, 2H), 2.75 - 2.57 (m, 3H), 2.50 (s, 3H), 1.94 - 1.78 (m, 4H), 1.69 (dd, *J* = 14.1, 10.8 Hz, 6H), 1.42 (d, *J* = 6.6 Hz, 4H); m/z (ES+): 646 $[M+H]^+$.

Example 30: Compound 30

(1S,8aS)-N-((R)-1-(3,5-bis(trifluoromethyl)phenyl)ethyl)-1-(4-hydroxy-2-methylphenyl) -7,7-bis(2-hydroxyethyl)-N-methyl-6-oxo-hexahydropyrrolo[1,2-a]pyrazine-2(1H)-carboxamide

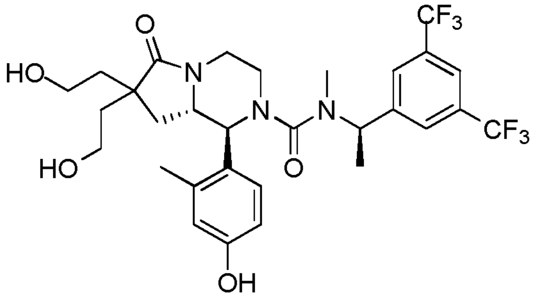

To a solution of intermediate 45 (40 mg, 0.055 mmol) in MeOH (20 mL) was added Pd/C 5% (100 mg). The reaction was stirred under $H_2$ (4 atm) for 12 h. The catalyst was filtered through a celite pad and the solvent was removed in vacuo. The residue was purified by silica gel chromatography (DCM/MeOH=50/1 to 20/1) to give the title compound (20 mg, yield: 58%). $^1H$ NMR (400 MHz, $CDCl_3$) δ 7.78 (s, 1H), 7.54 (s, 2H), 7.10 (d, *J* = 8.4 Hz, 1H), 6.63 (d, *J* = 2.5 Hz, 1H), 6.56 (d, *J* = 8.4 Hz, 1H), 5.64 - 5.49 (m, 1H), 5.42 (s, 1H), 4.10 (d, *J* = 12.6 Hz, 1H), 3.96 (d, *J* = 9.8 Hz, 1H), 3.81 (td, *J* = 11.6, 5.5 Hz, 3H), 3.76 - 3.52 (m, 2H), 3.28 (d, *J* = 11.6 Hz, 1H), 3.19 - 3.06 (m, 2H), 3.00 (t, *J* = 10.7 Hz, 1H), 2.69 (s, 3H), 2.45 (s, 3H), 2.25 (s, 1H), 1.89 (t, *J* = 5.8 Hz, 3H), 1.86 - 1.63 (m, 4H), 1.45 - 1.40 (m, 3H); m/z (ES+): 632 $[M+H]^+$.

Example 31: Compound 31

(1S,8aS)-N-((R)-1-(3-ethyl-5-(trifluoromethyl)phenyl)ethyl)-1-(4-hydroxy-2-methylphenyl)-7,7-bis(2-hydroxyethyl)-N-methyl-6-oxohexahydropyrrolo[1,2-a]pyrazine-2(1H)-carboxamide

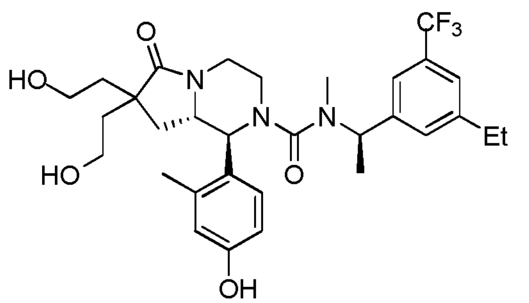

To a solution of Intermediate 47 (50 mg, 0.073 mol) in MeOH (20 mL) under $N_2$ atmosphere, was added Pd/C (30 mg). The reaction was stirred under $H_2$ (4 atm) for 12 h. The catalyst was filtered through a celite pad and the solvent was removed in vacuo. The residue was purified by pre_HPLC to give the title compound (15 mg, yield: 30%); $^1H$-NMR (400 MHz, $CDCl_3$) *δ* ppm 7.34 (s, 1H), 7.18 - 7.04 (m, 3H), 6.62 - 6.54 (m, 2H), 5.51 (q, 1H), 5.36 (s, 1H), 4.10 - 4.07 (d, 1H), 3.97 - 3.95 (d, 1H), 3.78- 3.6 (m, 5H), 3.26- - 2.9 (m, 3H), 2.64 (m, 5H), 2.46 (s, 3H), 2.1 (m, 1H), 1.9 - 1.6 (m, 6H), 1.37 (d, 3H), 1.2 (d, 3H); m/z (ES+): 592 $[M+H]^+$.

Example 32: Compound 32

(1S,8aS)-N-((R)-1-(4-ethyl-6-(trifluoromethyl)pyridin-2-yl)ethyl)-N-methyl-6-oxo-1-o-tolylhexahydropyrrolo[1,2-a]pyrazine-2(1H)-carboxamide

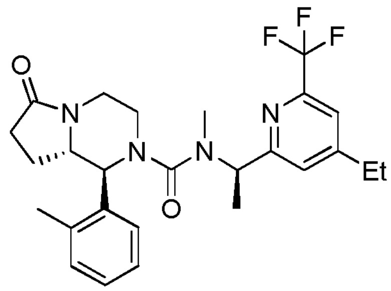

To a solution of triphosgene (47 mg, 0.16 mmol) in EtOAc (10 mL) at 0 °C was added solution of (1S,8aS)-1-o-tolylhexahydropyrrolo[1,2-a]pyrazin-6(7H)-one (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-1-(4-ethyl-6-(trifluoromethyl)pyridin-2-yl)-N-methylethanamine (163.2 mg, 0.6 mmol) and TEA (107 mg, 1.05 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (70 mg, yield: 31%); $^1H$ NMR (400 MHz, $CDCl_3$) δ 7.31 (s, 1H), 7.14 (d, $J$ = 11.3 Hz, 2H), 6.68 (s, 1H), 5.53 (s, 1H), 4.14 (d, $J$ = 12.4 Hz, 1H), 4.06 (d, $J$ = 9.9 Hz, 1H), 3.71 (s, 1H), 3.45 (s, 1H), 3.11 (s, 1H), 2.90 (s, 1H), 2.83 (s, 2H), 2.53 (s, 3H), 2.38 (m, 2H), 1.83 (s, 1H), 1.51 (d, $J$ = 6.3 Hz, 3H), 1.15 (t, $J$ = 7.1 Hz, 3H); m/z (ES+): 489 $[M+H]^+$.

Example 33: Compound 33

(1S,8aS)-7,7-bis(2-hydroxyethyl)-N-methyl-N-((4-methyl-6-(trifluoromethyl)pyridin-2-yl)methyl)-6-oxo-1-o-tolylhexahydropyrrolo[1,2-a]pyrazine-2(1H)-carboxamide

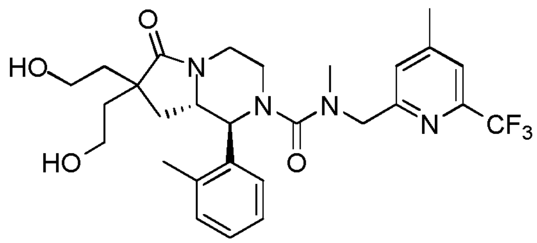

A slow stream of $O_3$ in $O_2$ was passed through a -78°C cooled solution of intermediate 60 (90 mg, 0.167 mmol) in DCM (10 mL) until a pale blue colour persisted. The reaction was purged with $N_2$ to remove the excess of $O_3$ followed by addition of $NaBH_4$ (127 mg, 3.34 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by prep-HPLC to give the title compound (20 mg, yield: 21.8%). $^1H$ NMR (400 MHz, $CDCl_3$) δ 7.33 (d, $J$ = 9.1 Hz, 2H), 7.24 - 7.12 (m, 3H), 6.59 (s, 1H), 4.68 (d, $J$ = 16.1 Hz, 1H), 4.44 (d, $J$ = 16.0 Hz, 1H), 4.11 (dd, $J$ = 23.5, 11.4 Hz, 2H), 3.90 - 3.66 (m, 4H), 3.65 - 3.50 (m, 2H), 3.33 - 3.12 (m, 2H), 3.10 - 2.94 (m, 4H), 2.65 - 2.38 (m, 4H), 2.27 (s, 3H), 1.91 (t, $J$ = 5.9 Hz, 2H), 1.84 (dd, $J$ = 12.7, 5.7 Hz, 2H), 1.77 - 1.74 (m, 2H); m/z (ES+): 549 $[M+H]^+$.

Example 34: Compound 34

(1S,8aS)-N-((R)-1-(3-chloro-5-(trifluoromethyl)phenyl)ethyl)-1-(2,4-dimethylphenyl)-7,7-bis(2-hydroxyethyl)-N-methyl-6-oxo-hexahydropyrrolo[1,2-a]pyrazine-2(1H)-carboxamide

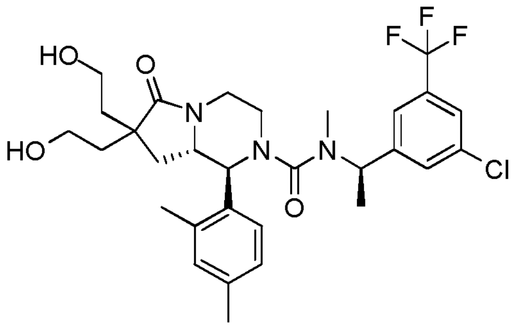

A slow stream of $O_3$ in $O_2$ was passed through a -78 °C cooled solution of intermediate 67 (80 mg, 0.13 mmol) in DCM (10 mL) until a pale blue colour persisted. The reaction was purged with $N_2$ to remove the excess of $O_3$ followed by addition of $NaBH_4$ (0.6 g, 16 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the title compound (15 mg, 19%). $^1$H NMR (400 MHz, $CDCl_3$) δ 7.50 (s, 1H), 7.24 (s, 1H), 7.19 (s, 1H), 7.11 (d, *J* = 7.9 Hz, 1H), 7.00 (s, 1H), 6.94 (d, *J* = 7.8 Hz, 1H), 5.51 (q, *J* = 6.7 Hz, 1H), 4.09 (d, *J* = 12.7 Hz, 1H), 3.99 (s, 1H), 3.91 - 3.62 (m, 4H), 3.64 - 3.50 (m, 1H), 3.30 (d, *J* = 11.9 Hz, 1H), 3.12 (td, *J* = 12.4, 3.3 Hz, 1H), 2.99 (td, *J* = 11.9, 2.9 Hz, 1H), 2.64 (s, 3H), 2.60 - 2.55 (m, 1H), 2.49 (s, 4H), 2.31 (d, *J* = 18.3 Hz, 3H), 1.90 - 1.74 (m, 5H), 1.76 - 1.57 (m, 3H), 1.47 - 1.32 (m, 4H), 1.25 (s, 1H); m/z (ES+): $[M+H]^+$597.

Example 35: Compound 35

(15,8aS)-1-(2,4-dimethylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide

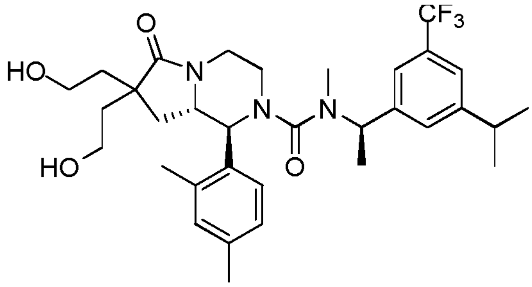

A slow stream of $O_3$ in $O_2$ was passed through a cooled solution of Intermediate 68 (120 mg , 0.2 mmol) in DCM (10 mL) at -78 °C until a pale blue colour persisted. The reaction was purged with $N_2$ to remove the excess of $O_3$ followed by addition of $NaBH_4$ (72 mg, 2 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue The residue was purified by Prep-HPLC to give the compound 1 (20 mg, yield: 17%). $^1$H-NMR (400 MHz, $CDCl_3$) δ ppm 7.35 (s, 1H), 7.17-6.92 (m, 5H), 5.50-5.49 (q, 1H), 4.09 - 4.06 (d, 1H), 4.02- 3.99 (d, 1H), 3.82 - 3.70 (m, 5H), 3.59 - 2.87 (m, 4H), 2.63 (s, 3H), 2.50 (s, 3H), 2.28 (s, 3H), 1.89-1.6 (m, 6H), 1.38 - 1.3 (m, 9H). m/z (ES+): 604 $[M+H]^+$.

Example 36: Compound 36

(1S,8aS)-N-((R)-1-(3,5-bis(trifluoromethyl)pheny)ethyl)-7,7-bis(2-hydroxyethyl)-1-(2-methoxyphenyl)-N-methyl-6-oxo-hexahydropyrrolo[1,2-a]pyrazine-2(1H)-carboxamide

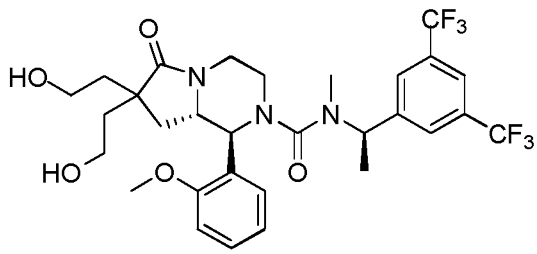

A slow stream of $O_3$ in $O_2$ was passed through a cooled solution of Intermediate 69 (45 mg, 0.07 mmol) in DCM (10 mL) at -78°C until a pale blue colour persisted. The reaction was purged with $N_2$ to remove the excess of $O_3$ followed by addition of $NaBH_4$ (72 mg, 2 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the compound (15 mg, yield: 34%).$^1$H-NMR (400 MHz, $CDCl_3$) $\delta$ ppm 7.75 (s, 1H), 7.46 (s, 2H), 7.28-7.24 (m, 1H), 7.20-7.18 (m, 1H), 6.95-6.90 (m, 2H), 5.61-5.57 (q, 1H), 4.47-4.44 (d, *J*= 10 Hz, 1H), 4.15-4.11 (m, 1H), 3.90 (s, 3H), 3.88 - 3.80 (m, 3H), 3.78 - 3.60 (m, 2H), 3.41 - 3.37 (m, 1H), 3.20 - 3.13 (m, 1H), 3.04 - 2.97 (m, 2H), 2.84 (s, 3H), 2.32 (s, 1H), 1.99-1.92 (m, 3H), 1.85 - 1.78 (m, 3H), 1.50 (d, 2H); m/z (ES+): 632 $[M+H]^+$.

Example 37: Compound 37

(1S,8aS)-N-((R)-1-(3,5-bis(trifluoromethyl)phenyl)ethyl)-1-(2,5-dimethyphenyl)-7,7-bis(2-hydroxyethyl)-N-methyl-6-oxo-hexahydropyrrolo[1,2-a]pyrazine-2(1H)-carboxamide

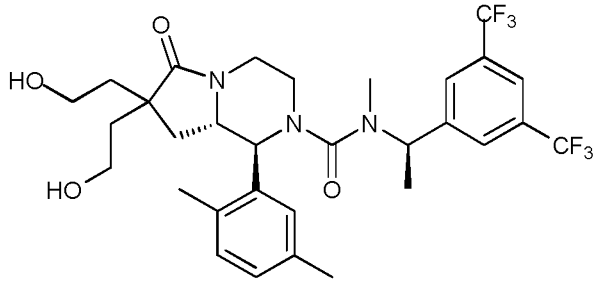

A slow stream of $O_3$ in $O_2$ was passed through a cooled solution of Intermediate 76 (120 mg , 0.193 mmol) in DCM (10 mL) at -78°C until a pale blue colour persisted. The reaction was purged with $N_2$ to remove the excess of $O_3$ followed by addition of $NaBH_4$ (74 mg, 1.93 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the compound (60 mg, yield: 50%). $^1$H NMR (400 MHz, $CDCl_3$) $\delta$ 7.77 (s, 1H), 7.53 (s, 2H), 7.05 (s, 2H), 6.98 (s, 1H), 5.54 (d, *J* = 5.6 Hz, 1H), 4.06 (dd, *J* = 27.0, 10.7 Hz, 2H), 3.76 (d, *J* = 28.8 Hz, 4H), 3.60 (s, 1H), 3.26 (d, *J* = 9.3 Hz, 1H), 3.12 (d, *J* = 11.3 Hz, 1H), 3.01 (d, *J* = 10.4 Hz, 1H), 2.72 (s, 3H), 2.49 (s, 1H), 2.47 (s, 3H), 2.22 (s, 3H), 1.98 - 1.78 (m, 5H), 1.73 (s, 2H), 1.36 (d, *J* = 4.9 Hz, 3H); m/z (ES+): 630 $[M+H]^+$.

Example 38: Compound 38

(15,8aS)-1-(2,5-dimethylphenyl)-7,7-bis(2-hydroxyethyl)-N-((R)-1-(3-isopropyl 5-(trifluoromethyl)phenyl)ethyl)-N-methyl-6-oxohexahydropyrrolo[1,2-a]pyrazine-2(1H)-carboxamide

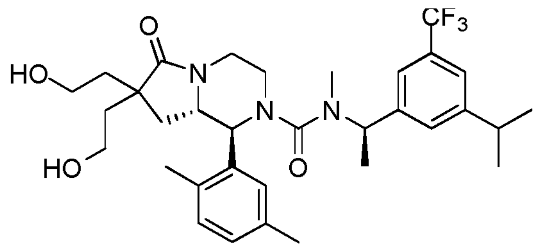

A slow stream of $O_3$ in $O_2$ was passed through a cooled solution of Intermediate 77 (100 mg , 0.17 mmol) in DCM (10 mL) at -78 °C until a pale blue colour persisted. The reaction was purged with $N_2$ to remove the excess of $O_3$ followed by addition of $NaBH_4$ (64.31 mg, 1.7 mmol). The reaction mixture was allowed to warm to room temperature and stirred for 20 h. After quenched with $NH_4Cl$ solution, the resulting mixture was extracted with DCM, washed with brine, dried over $Na_2SO_4$, filtered and concentrated in vacuo. The residue was purified by Prep-HPLC to give the compound (20 mg, yield: 20%). $^1$H-NMR (400 MHz, $CDCl_3$) $\delta$ppm 7.35 (s, 1H), 7.26 (s, 1H), 7.17-6.97 (m, 4H), 5.50-5.48 (q, 1H), 4.11 - 4.08 (q, 2H), 3.85 - 3.70 (m, 4H), 3.61 (m, 1H), 3.12 - 2.97 (m, 4H), 2.68 (s, 3H), 2.48 (s, 3H), 2.23 (s, 3H), 1.9-1.6 (m, 6H), 1.5 - 1.40(m, 9H); m/z (ES+): 604 $[M+H]^+$.

Example 39: Compound 39

(1S,8aS)-N-((R)-1-(3,5-bis(trifluoromethyl)phenyl)ethyl)-1-(2,3-dimethylphenyl)-7,7-bis(2-hydroxyethyl)-N-methyl-6-oxohexahydropyrrolo[1,2-a]pyrazine-2(1H)-carboxamide

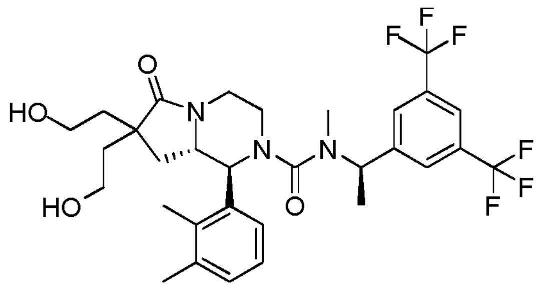

A slow stream of $O_3$ in $O_2$ was passed through a cooled solution of Intermediate 84 (120 mg, 0.193 mmol) in DCM (10 mL) at -78 °C until a pale blue colour persisted. The reaction was purged with $N_2$ to remove the excess of $O_3$ followed by addition of $NaBH_4$ (74 mg, 1.93 mmol). The reaction mixture was allowed to warm to room temperature and stirred for 20 h. After quenched with $NH_4Cl$ solution, the resulting mixture was extracted with DCM (2x20 mL). The combined organic layers were washed with brine, dried over anhydrous $Na_2SO_4$, and concentrated in vacuo. The residue was purified by Prep-HPLC to give the compound (60 mg, yield: 50%). $^1$H NMR (400 MHz, CDCl3) $\delta$ ppm 7.77 (s, 1H), 7.53 (s, 2H), 7.10-7.04 (m, 3H), 5.56-5.54 (q, 1H), 4.13 (t, 3H), 3.82 - 3.78 (m, 4H), 3.77 (m, 1H), 3.28 (d, 1H), 3.13 - 3.10 (m, 1H), 2.7 (s, 3H), 2.4 (s, 3H), 2.31 (s, 3H), 1.9-1.6 (m, 6H), 1.40 (d, 3H); m/z (ES+): 630 $[M+H]^+$.

Example 40: Compound 40

(1S,8aS)-N-((R)-1-(3,5-bis(trifluoromethyl)phenyl)ethyl)-1-(2,3-dimethylphenyl)-N-methyl-6-oxo-hexahydropyrrolo[1,2-a]pyrazine-2(1H)-carboxamide

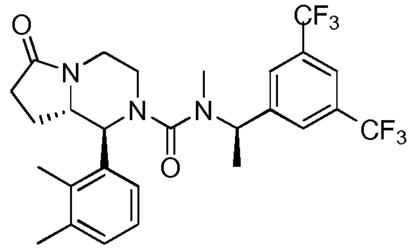

To a solution of triphosgene (30 mg, 0.102 mmol) in EtOAc (2 mL) at 0 °C was added solution of Intermediate 85 (50 mg, 0.204 mmol), DMAP (3 mg, 0.022 mmol) and TEA (62 mg, 0.612 mmol) in EtOAc (2 mL). The mixture was stirred for 1.5 h at room temperature, followed by addition of (R)-1-(3,5-bis(trifluoromethyl)phenyl)-N-methylethanamine (83 mg, 0.306 mmol) in EtOAc (2 mL) and TEA (62 mg, 0.612 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (10 mg, yield: 9%). $^1$H NMR (400 MHz, $CDCl_3$) $\delta$ 7.77 (s, 1H), 7.54 (s, 2H), 7.11 (d, $J$ = 7.4 Hz, 1H), 7.04 (dd, $J$ = 16.2, 7.2 Hz, 2H), 5.55 (d, $J$ = 6.8 Hz, 1H), 4.15 (t, $J$ = 10.8 Hz, 2H), 3.72 (d, $J$ = 8.6 Hz, 1H), 3.27 (d, $J$ = 11.2 Hz, 1H), 3.11 (t, $J$ = 12.4 Hz, 1H), 2.98 (t, $J$ = 10.4 Hz, 1H), 2.70 (s, 3H), 2.41 (s, 3H), 2.33 (d, $J$ = 7.0 Hz, 2H), 2.31 (s, 3H), 1.82 (s, 1H), 1.65 (s, 1H), 1.42 (d, $J$ = 7.0 Hz, 3H); m/z (ES+): 542 $[M+H]^+$.

Example 41: Compound 41

(1S,8aS)-1-(2,3-dimethylphenyl)-N-((R)-1-(3-ethyl-5-(trifluoromethyl)phenyl)ethyl)-N-methyl-6-oxo-hexahydropyrrolo[1,2-a]pyrazine-2(1H)-carboxamide

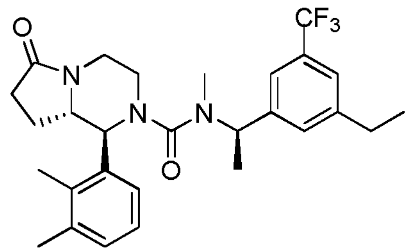

To a solution of triphosgene (30 mg, 0.102 mmol) in EtOAc (2 mL) at 0 °C was added solution of Intermediate 85 (50 mg, 0.204 mmol), DMAP (3 mg, 0.022 mmol) and TEA (62 mg, 0.612 mmol) in EtOAc (2 mL). The mixture was stirred for 1.5 h at room temperature, followed by addition of (R)-1-(3,5-bis(trifluoromethyl)phenyl)-N-methylethanamine (83 mg, 0.306 mmol) and TEA (62 mg, 0.612 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (10 mg, yield: 10%). $^1$H NMR (400 MHz, $CDCl_3$) $\delta$ 7.33 (s, 1H), 7.20 (s, 1H), 7.13 (d, $J$ = 7.4 Hz, 1H), 7.03 (d, $J$ = 14.4 Hz, 3H), 5.49 (d, $J$ = 6.8 Hz, 1H), 4.15 (dd, $J$ = 20.9, 11.3 Hz, 2H), 3.69 (d, $J$ = 7.7 Hz, 1H), 3.28 (d, $J$ = 11.5 Hz, 1H), 3.09 (d, $J$ = 12.1 Hz, 1H), 2.98 (d, $J$ = 11.3 Hz, 1H), 2.65 (s, 3H), 2.61 (d, $J$ = 7.3 Hz, 2H), 2.42 (s, 3H), 2.41 - 2.33 (m, 2H), 2.32 (s, 3H), 1.80 (s, 1H), 1.64 (s, 1H), 1.37 (d, $J$ = 6.3 Hz, 3H), 1.20 (t, $J$ = 7.6 Hz, 3H); m/z (ES+): 502 $[M+H]^+$

Example 42: Compound 42

(1S,8aS)-1-(2,3-dimethylphenyl)-N-((R)-1-(3-isopropyl-5-trifluoromethyl)phenyl)ethyl)-N-methyl-6-oxo-hexahydropyrrolo[1,2-a]pyrazine-2(1H)-carboxamide

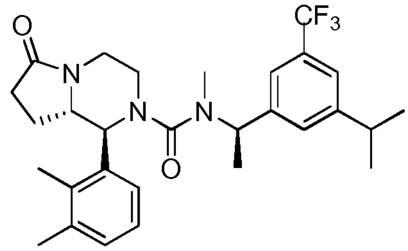

To a solution of triphosgene (30 mg, 0.102 mmol) in EtOAc (2 mL) at 0 °C was added solution of Intermediate 85 (50 mg, 0.204 mmol), DMAP (3 mg, 0.022 mmol) and TEA (62 mg, 0.612 mmol) in EtOAc (2 mL). The mixture was stirred for 1.5 h at room temperature, followed by addition of (R)-1-(3-isopropyl-5-(trifluoromethyl)phenyl)-N-methylethanamine (83 mg, 0.306 mmol) and TEA (62 mg, 0.612 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (10 mg, yield: 9%). $^1H$ NMR (400 MHz, $CDCl_3$) $\delta$ 7.36 (s, 1H), 7.20 (s, 1H), 7.17 - 7.09 (m, 2H), 7.03 (d, *J* = 8.2 Hz, 2H), 5.49 (d, *J* = 7.0 Hz, 1H), 4.15 (dd, *J* = 19.5, 11.4 Hz, 2H), 3.70 (s, 1H), 3.27 (d, *J* = 11.7 Hz, 1H), 3.08 (d, *J* = 12.4 Hz, 1H), 3.00 - 2.84 (m, 2H), 2.66 (s, 3H), 2.42 (s, 3H), 2.33 (s, 2H), 2.32 (s, 3H), 1.80 (s, 1H), 1.62 (s, 1H), 1.36 (d, *J* = 7.0 Hz, 3H), 1.22 (t, *J* = 7.0 Hz, 6H); m/z (ES+): 516 $[M+H]^+$.

Example 43 & 44: Compound 43 & 44

(1R,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide (example 43).

(1S,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide (example 44)

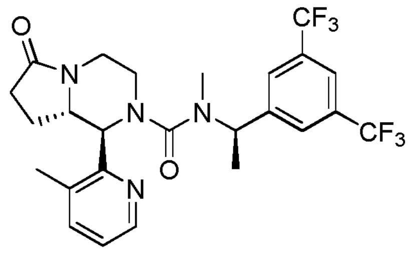

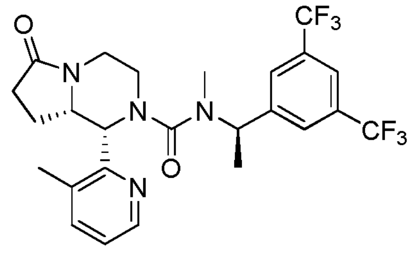

To a solution of triphosgene (73.45 mg, 0.25 mmol) in EtOAc (2 mL) at 0 °C was added solution of [1-(3,5-Bis-trifluoromethyl-phenyl)-ethyl]-methyl-amine (163.2 mg, 0.6 mmol), DMAP (6.1 mg, 0.05 mmol) and TEA (151.68 mg, 1.5 mmol) in EtOAc (2 mL). The mixture was stirred for 1.5 h at room temperature, followed by addition of Intermediate 82 (115.5 mg, 0.5 mmol) in EtOAc (2 mL) and TEA (151.68 mg, 1.5 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound 43 (120 mg, yield: 39%) and compound 4 4 (25 mg, yield: 8%).

compound 43: $^1H$ NMR (400 MHz, $CDCl_3$) $\delta$ 8.36 (d, *J* = 4.0 Hz, 1H), 7.77 (s, 1H), 7.54 - 7.48 (m, 3H), 7.11 (m, 1H), 5.49 (q, 1H), 4.24 - 4.09 (m, 3H), 3.48 (m, 1H), 3.24 - 3.05 (m, 2H), 2.60 (s, 1H), 2.55 (s, 1H), 2.46 - 2.31 (m, 2H), 1.87 - 1.80 (m, 1H), 1.50 (d, *J*= 6.4 Hz, 4H); m/z (ES+): 529 $[M+H]^+$.

compound 44: $^1H$ NMR (400 MHz, $CDCl_3$) $\delta$ 8.37 (d, *J* = 4.0 Hz, 1H), 7.87 (s, 2H), 7.79 (s, 1H), 7.45 (d, *J* = 7.3 Hz, 1H), 7.10 (dd, *J* = 7.5, 4.8 Hz, 1H), 5.54 - 5.39 (m, 2H), 4.37 - 4.30 (m, 1H), 4.18 (d, *J* = 10.6 Hz, 1H), 3.53 (dd, *J* = 12.8, 9.4 Hz, 1H), 3.24 (d, *J* = 13.1 Hz, 1H), 2.98 (dd, *J* = 12.8, 8.7 Hz, 1H), 2.50 (s, 3H), 2.35 (t, *J* = 8.2 Hz, 2H), 2.26 (s, 3H), 2.18 - 2.04 (m, 1H), 1.61 (d, *J* = 7.1 Hz, 4H) ; m/z (ES+): 529 $[M+H]^+$.

Example 45: Compound 45

(1S,8aS)-N-((R)-1-(3,5-bis(trifluoromethyl)phenyl)ethyl)-N-methyl-1-(4-methylpyridin-3-yl)-6-oxo-hexahydropyrrolo[1,2-a]pyrazine-2(1H)-carboxamide

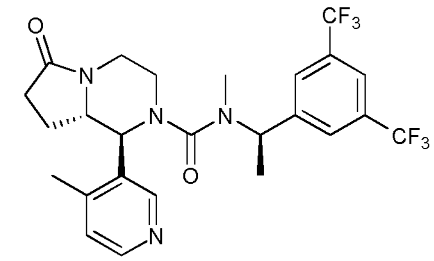

To a solution of triphosgene (73.45 mg, 0.25 mmol) in EtOAc (2 mL) at 0 °C was added solution of Intermediate 91 (115.5 mg, 0.5 mmol), DMAP (6.1 mg, 0.05 mmol) and TEA (151.68 mg, 1.5 mmol) in EtOAc (2 mL). The mixture was stirred for 1.5 h at room temperature, followed by addition of [1-(3,5-Bis-trifluoromethyl-phenyl)-ethyl]-methylamine (163.2 mg, 0.6 mmol) and TEA (151.68 mg, 1.5 mmol) in EtOAc (2 mL). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (120 mg, yield: 39%); m/z (ES+): 529 $[M+H]^+$. $^1H$ NMR (400 MHz, $CDCl_3$) δ 8.63 (d, J = 12.6 Hz, 1H), 8.43 (dd, J = 16.9, 5.2 Hz, 1H), 7.76 (d, J = 29.0 Hz, 1H), 7.56 (s, 1H), 7.46 (d, J = 5.0 Hz, 1H), 7.40 (s, 1H), 5.46 (dd, J = 28.4, 7.0 Hz, 1H), 4.15 (dd, J = 16.8, 11.2 Hz, 2H), 3.77 (s, 1H), 3.32 (d, J = 12.2 Hz, 1H), 3.20 - 3.08 (m, 1H), 2.92 (t, J = 11.8 Hz, 1H), 2.81 - 2.66 (m, 6H), 2.51 - 2.37 (m, 2H), 1.56 (t, J = 14.4 Hz, 2H), 1.43 - 1.37 (m, 3H), 1.25 (s, 1H). m/z (ES+): 529 $[M+H]^+$.

Example 46: Compound 46

(1S,8aS)-N-((R)-1-(3,5-bis(trifluoromethyl)phenyl)ethyl)-N-methyl-1-(3-methylpyridin-4-yl)-6-oxohexahydropyrrolo[1,2-a]pyrazine-2(1H)-carboxamide

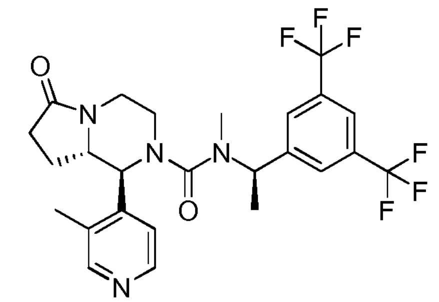

To a solution of triphosgene (58 mg, 0.2 mmol) in EtOAc (2 mL) at 0 °C was added solution of Intermediate 92 (126.8 mg, 0.4 mmol), DMAP (4.7 mg, 0.04 mmol) and TEA (118 mg, 1.2 mmol) in EtOAc (2 mL). The mixture was stirred for 1.5 h at room temperature, followed by addition of (1S,8aS)-1-(3-methylpyridin-4-yl)-hexahydropyrrolo[1,2-a]pyrazin-6(7H)-one (90 mg, 0.4 mmol) in EtOAc (2 mL) and TEA (118 mg, 1.2 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (10 mg, yield: 10%);1H NMR (400 MHz, CDCl3) δ 8.45 (d, J = 13.8 Hz, 2H), 7.80 (s, 1H), 7.57 (s, 2H), 7.22 (s, 1H), 5.56 - 5.41 (m, 1H), 4.18 (d, J = 13.1 Hz, 1H), 4.06 (d, J = 9.7 Hz, 1H), 3.65 (d, J = 9.3 Hz, 1H), 3.29 (d, J = 11.7 Hz, 1H), 3.11 (s, 1H), 3.00 - 2.86 (m, 1H), 2.74 (s, 3H), 2.55 (s, 3H), 2.48 - 2.30 (m, 2H), 1.84 (s, 1H), 1.67 (d, J = 8.3 Hz, 1H), 1.42 (d, J = 6.9 Hz, 3H); m/z (ES+): 529 $[M+H]^+$.

Example 47: Compound 47

(1S,8aS)-N-((R)-1-(3,5-bis(trifluoromethyl)phenyl)ethyl)-N-methyl-1-(2-methylpyridin-3-yl)-6-oxo-hexahydropyrrolo[1,2-a]pyrazine-2(1H)-carboxamide

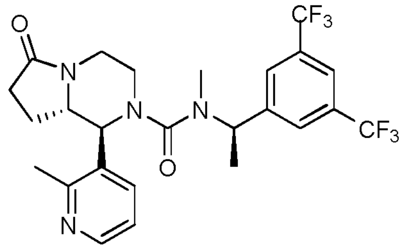

To a solution of triphosgene (73.45 mg, 0.25 mmol) in EtOAc (2 mL) at 0 °C was added solution of Intermediate 93 (115mg, 0.5 mmol), DMAP (6.1 mg, 0.05 mmol) and TEA (151.68 mg, 1.5 mmol) in EtOAc (2 mL). The mixture was stirred for 1.5 h at room temperature, followed by addition of [1-(3,5-Bis-trifluoromethyl-phenyl)-ethyl]-methylamine (139.2 mg, 0.6 mmol) in EtOAc (2 mL) and TEA (151.68 mg, 1.5 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (20 mg, yield: 10%); $^1H$ NMR (400 MHz, $CDCl_3$) δ 8.44 (d, J = 4.5 Hz, 1H), 7.75 (d, J = 24.0 Hz, 1H), 7.60 - 7.51 (m, 3H), 7.10 (dd, J = 7.7, 4.5 Hz, 1H), 5.52 (dt, J = 20.8, 6.9 Hz, 1H), 4.26 - 3.95 (m, 2H), 3.70 (dd, J = 32.6, 8.0 Hz, 1H), 3.33 (dd, J = 28.1, 12.4 Hz, 1H), 3.09 (t, J = 11.3 Hz, 1H), 2.98 (dd, J = 13.2, 10.3 Hz, 1H), 2.76 (t, J = 20.4 Hz, 6H), 2.51 - 2.35 (m, 2H), 1.52 (d, J = 7.2 Hz, 1H), 1.43 (d, J = 6.8 Hz, 3H); m/z (ES+): 529 $[M+H]^+$.

Example 48: Compound 48

(1S,BaS)-N-((R)-1-(3,5-bis,(trifluoromethyl)phenyl)ethyl)-N-methyl-1-(4-ethylthiophen-3-yl)-6-oxohexahydropyrrolo[1,2-a]pyrazine-2(1H)-carboxamide

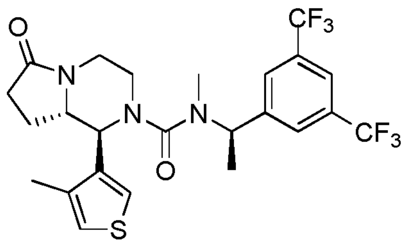

Triphosgene (34.9 mg, 0.119 mmol) was dissolved EtOAc (2 mL), a solution of Intermediate 98 (70 mg, 0.297 mmol), DMAP (4 mg) and TEA (0.13 ml, 0.89 mmol) in EtOAc (2 mL) was added at 0'C. The mixture was stirring for 1.5 h, then a solution (R)-1-(3,5-bis(trifluoromethyl)phenyl)-N-methylethanamine (112.5 mg, 0.415 mmol) and TEA (0.13 ml, 0.89 mmol) in EtOAc (2 mL) was added. The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (20 mg, Yiled: 13%). 1H NMR (400 MHz, CDCl3) δ ppm 7.78 (s, 1H), 7.60 (s, 2H), 6.86 (s, 1H), 6.79 (s, 1H), 5.57-5.62 (m, 1H),4.04-4.14 (m, 2H), 3.66-3.70 (m, 1H), 2.91-3.11 (m, 3H), 2.74 (s, 3H), 2.34-2.45 (m, 2H), 2.22 (s, 3H), 1.99-2.04 (m, 1H), 1.74-1.79 (m, 1H), 1.74(d, J=3.2 Hz, 3H); m/z (ES+): 534 [M+H]+.

Example 49: Compound 49

(1S,8aS)-N-((R)-1-(3-ethyl-5-(trifluoromethyl)phenyl)ethyl)-N-methyl-1-(4-methylthiophen-3-yl)-6-oxohexahydropyrrolo[1,2-a]pyrazine-2(1H)-carboxamide

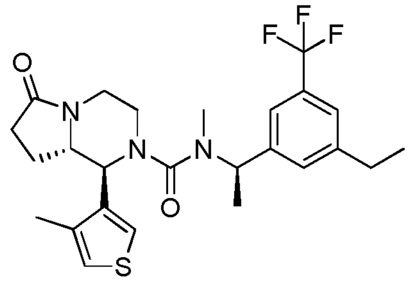

To a solution of triphosgene (63 mg, 0.2 mmol) in EtOAc (2 mL) at 0 °C was added solution of Intermediate 98 (100 mg, 0.42 mmol), DMAP (5 mg, 0.04 mmol) and TEA (127 mg, 1.26 mmol) in EtOAc (2 mL). The mixture was stirred for 1.5 h at room temperature, followed by addition of (R)-1-(3-ethyl-5-(trifluoromethyl)phenyl)-N-methylethanamine (116 mg, 0.5 mmol) in EtOAc (2 mL) and TEA (127 mg, 1.26 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (30 mg, yield: 14%); 1H NMR (400 MHz, CDCl3) δ 7.27 (s, 1H), 7.16 (s, 1H), 7.06 (s, 1H), 6.79 (s, 1H), 6.72 (s, 1H), 5.47 (d, J = 7.0 Hz, 1H), 4.06 - 3.96 (m, 2H), 3.62 - 3.51 (m, 1H), 3.15 - 3.06 (m, 1H), 3.04 - 2.93 (m, 1H), 2.86 (dd, J = 11.7, 3.3 Hz, 1H), 2.63 (s, 3H), 2.61 - 2.54 (m, 2H), 2.44 - 2.23 (m, 2H), 2.15 (d, J = 0.6 Hz, 3H), 1.99 - 1.86 (m, 1H), 1.75 - 1.64 (m, 1H), 1.35 (d, J = 7.0 Hz, 3H), 1.16 (dd, J = 13.5, 5.9 Hz, 3H); m/z (ES+): 494 [M+H]+.

Example 50: Compound 50

(1R,8aS)-N-((R)-1-(3,5-bis(trifluoromethyl)phenyl)ethyl)-N-methyl-1-(3-ethylthiophen-2-yl)-6-oxohexahydropyrrolo[1,2-a]pyrazine-2(1H)-carboxamide

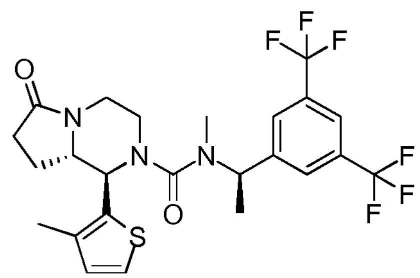

To a solution of triphosgene (63 mg, 0.2 mmol) in EtOAc (2 mL) at 0 °C was added solution of Intermediate 99 (100 mg, 0.42 mmol), DMAP (5 mg, 0.04 mmol) and TEA (127 mg, 1.26 mmol) in EtOAc (2 mL). The mixture was stirred for 1.5 h at room temperature, followed by addition of (R)-1-(3,5-bis(trifluoromethyl)phenyl)-N-methyl ethanamine (135 mg, 0.5 mmol) in EtOAc (2 mL) and TEA (127 mg, 1.26 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (30 mg, yield: 13%); 1H NMR (400 MHz, CDCl3) δ 7.78 (s, 1H), 7.59 (s, 2H), 7.13 (d, J = 5.1 Hz, 1H), 6.79 (d, J = 5.1 Hz, 1H), 5.58 (q, J = 6.9 Hz, 1H), 4.17 - 4.06 (m, 2H), 3.69 (dd, J = 16.9, 7.3 Hz, 1H), 3.19 (d, J = 11.9 Hz, 1H), 3.08 (t, J = 12.3 Hz, 1H), 2.93 (td, J = 11.7, 3.2 Hz, 1H), 2.72 (s, 3H), 2.50 - 2.35 (m, 2H), 2.33 (d, J = 12.2 Hz, 3H), 1.96 (dd, J = 10.3, 6.5 Hz, 1H), 1.76 - 1.66 (m, 1H), 1.46 (d, J = 7.0 Hz, 3H); m/z (ES+): 534 [M+H]+.

Example 51: Compound 51

(1R,8aS)-N-((R)-1-(3-ethyl-5-(trifluoromethyl)phenyl)ethyl)-N-methyl-1-(3-methylthiophen-2-yl)-6-oxohexahydropyrrolo[1,2-a]pyrazine-2(1H)-carboxamide

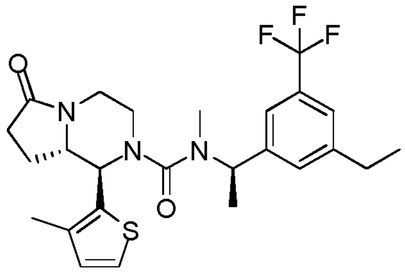

To a solution of triphosgene (63 mg, 0.2 mmol) in EtOAc (2 mL) at 0 °C was added solution of Intermediate 99 (100 mg, 0.42 mmol), DMAP (5 mg, 0.04 mmol) and TEA (127 mg, 1.26 mmol) in EtOAc (2 mL). The mixture was stirred for 1.5 h at room temperature, followed by addition of (R)-1-(3-ethyl-5-(trifluoromethyl)phenyl)-N-methylethanamine (116 mg, 0.5 mmol) in EtOAc (2 mL) and TEA (127 mg, 1.26 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (30 mg, yield: 15.7%); 1H NMR (400 MHz, CDCl3) δ 7.27 (s, 1H), 7.16 (s, 1H), 7.06 (d, J = 4.2 Hz, 2H), 6.72 (d, J = 5.1 Hz, 1H), 5.45 (q, J = 6.9 Hz, 1H), 4.05 (dd, J = 11.2, 6.4 Hz, 2H), 3.61 (dd, J = 16.9, 7.4 Hz, 1H), 3.11 (d, J = 11.9 Hz, 1H), 3.00 (t, J = 12.3 Hz, 1H), 2.83 (td, J = 11.7, 3.1 Hz, 1H), 2.61 (s, 3H), 2.60 - 2.55 (m, 2H), 2.45 - 2.22 (m, 5H), 1.89 (ddd, J = 16.9, 13.1, 3.9 Hz, 1H), 1.73 - 1.64 (m, 1H), 1.32 (d, J = 7.0 Hz, 3H), 1.16 (dd, J = 13.9, 6.3 Hz, 4H); m/z (ES+): 494 [M+H]+.

Example 52: Compound 52

(1R,8aS)-N-methyl-N-((R)-1-(3-methyl-5-(trifluoromethyl)phenyl)ethyl)-1-(3-methylthiophen-2-yl)-6-oxohexahydropyrrolo[1,2-a]pyrazine-2(1H)-carboxamide

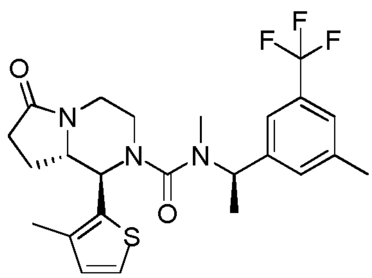

To a solution of triphosgene (63 mg, 0.2 mmol) in EtOAc (2 mL) at 0 °C was added solution of Intermediate 99 (100 mg, 0.42 mmol), DMAP (5 mg, 0.04 mmol) and TEA (127 mg, 1.26 mmol) in EtOAc (2 mL). The mixture was stirred for 1.5 h at room temperature, followed by addition of (R)-N-methyl-1-(3-methyl-5-(trifluoromethyl) phenyl) ethanamine (115.5 mg, 0.5 mmol) in EtOAc (2 mL) and TEA (127 mg, 1.26 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (30 mg, yield: 16.2%); 1H NMR (400 MHz, CDCl3) δ 7.32 (s, 1H), 7.21 (s, 1H), 7.13 (d, J = 5.1 Hz, 1H), 7.10 (s, 1H), 6.80 (d, J = 5.1 Hz, 1H), 5.51 (d, J = 6.9 Hz, 1H), 4.16 - 4.08 (m, 2H), 3.68 (d, J = 9.7 Hz, 1H), 3.19 (d, J = 11.4 Hz, 1H), 3.05 (d, J = 12.4 Hz, 1H), 2.92 (dd, J = 11.8, 3.2 Hz, 1H), 2.68 (s, 3H), 2.49 - 2.28 (m, 8H), 2.02 - 1.90 (m, 1H), 1.69 (d, J = 10.4 Hz, 1H), 1.38 (d, J = 7.0 Hz, 3H); m/z (ES+): 481 [M+H]+.

Example 53: Compound 53

(1R,8aS)-N-((R)-1-(3,5-bis(trifluoromethyl)phenyl)ethyl)-N-methyl-6-oxo-1-(thiophen-2-yl)hexahydropyrrolo[1,2-a]pyrazine-2(1H)-carboxamide

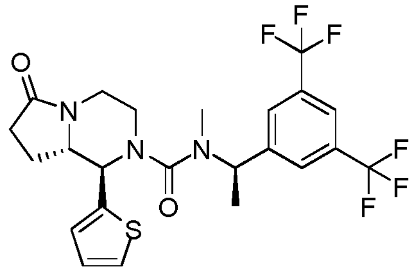

To a solution of triphosgene (46.8 mg, 0.15 mmol) in EtOAc (2 mL) at 0 °C was added solution of Intermediate 100 (70 mg, 0.31 mmol), DMAP (3.8 mg, 0.03 mmol) and TEA (95.5 mg, 0.94 mmol) in EtOAc (2 mL). The mixture was stirred for 1.5 h at room temperature, followed by addition of (R)-1-(3,5-bis(trifluoromethyl)phenyl)-N-methyl ethanamine (102.5 mg, 0.37 mmol) in EtOAc (2 mL) and TEA (95.5 mg, 0.94 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (20 mg, yield: 12.2%); 1H NMR (400 MHz, CDCl3) $\delta$ 7.78 (s, 1H), 7.59 (s, 2H), 7.23 (d, J = 5.1 Hz, 1H), 7.06 (d, J = 3.3 Hz, 1H), 6.97 (dd, J = 5.0, 3.6 Hz, 1H), 5.58 (q, J = 6.8 Hz, 1H), 4.12 (dd, J = 11.1, 7.5 Hz, 2H), 3.71 (dd, J = 17.0, 7.3 Hz, 1H), 3.21 (d, J = 11.9 Hz, 1H), 3.09 (t, J = 12.3 Hz, 1H), 2.96 (td, J = 11.6, 3.2 Hz, 1H), 2.74 (s, 3H), 2.50 - 2.32 (m, 2H), 2.06 - 1.94 (m, 1H), 1.83 - 1.70 (m, 1H), 1.47 (d, J = 7.0 Hz, 3H); m/z (ES+): 520 [M+H]+.

Example 54: Compound 54

(1R,8aS)-N-((R)-1-(3-ethyl-5-(trifluoromethyl)phenyl)ethyl)-N-methyl-6-oxo-1-(thiophen-2-yl)hexahydropyrrolo[1,2-a]pyrazine-2(1H)-carboxamide

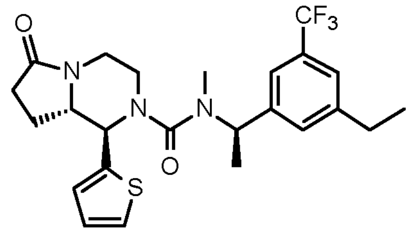

To a solution of triphosgene (46.8 mg, 0.15 mmol) in EtOAc (2 mL) at 0 °C was added solution of Intermediate 100 (70 mg, 0.31 mmol), DMAP (3.8 mg, 0.03 mmol) and TEA (95.5 mg, 0.94 mmol) in EtOAc (2 mL). The mixture was stirred for 1.5 h at room temperature, followed by addition of (R)-1-(3-ethyl-5-(trifluoromethyl)phenyl)-N-methylethanamine (87.3 mg, 0.37 mmol) in EtOAc (2 mL) and TEA (95.5 mg, 0.94 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (20 mg, yield: 13.2%); 1H NMR (400 MHz, CDCl3) $\delta$ 7.27 (s, 1H), 7.16 (d, J = 5.6 Hz, 2H), 7.04 (s, 1H), 7.00 (d, J = 3.0 Hz, 1H), 6.91 (dd, J = 4.8, 3.7 Hz, 1H), 5.46 (q, J = 6.9 Hz, 1H), 4.05 (d, J = 9.9 Hz, 2H), 3.61 (dd, J = 16.8, 7.3 Hz, 1H), 3.12 (d, J = 11.8 Hz, 1H), 3.00 (t, J = 12.2 Hz, 1H), 2.86 (td, J = 11.7, 3.2 Hz, 1H), 2.62 (s, 3H), 2.60 - 2.55 (m, 2H), 2.43 - 2.24 (m, 2H), 1.91 (ddd, J = 17.1, 13.2, 3.9 Hz, 1H), 1.69 (dt, J = 13.4, 9.5 Hz, 1H), 1.34 (d, J = 7.0 Hz, 3H), 1.15 (t, J = 7.6 Hz, 3H); m/z (ES+): 480 [M+H]+.

Example 55: Compound 55

(1S,8aS)-N-methyl-N-((R)-1-(3-methyl-5-(trifluoromethyl)phenyl)ethyl)-1-(2-methylthiophen-3-yl)-6-oxohexahydropyrrolo[1,2-a]pyrazine-2(1H)-carboxamide

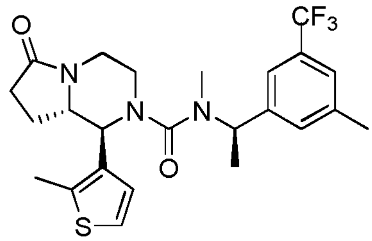

To a solution of triphosgene (47 mg, 0.16 mmol) in EtOAc (10 mL) at 0 °C was added solution of Intermediate 101 (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-1-(3,5-bis(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (70 mg, yield: 31%);$^1H$ NMR (400 MHz, $CDCl_3$) $\delta$ 7.32 (s, 1H), 7.22 (s, 1H), 7.09 (s, 1H), 6.83 (d, $J$ = 3.3 Hz, 1H), 6.61 (d, $J$ = 3.1 Hz, 1H), 5.58 - 5.49 (m, 1H), 4.10 (d, $J$ = 12.7 Hz, 1H), 4.00 (d, $J$ = 9.7 Hz, 1H), 3.65 (dd, $J$ = 17.0, 7.3 Hz, 1H), 3.19 (d, $J$ = 11.9 Hz, 1H), 3.06 (t, $J$ = 12.2 Hz, 1H), 2.97 - 2.85 (m, 1H), 2.68 (s, 3H), 2.49 - 2.41 (m, 4H), 2.40 - 2.31 (m, 4H), 2.05 - 1.91 (m, 1H), 1.82 - 1.71 (m, 1H), 1.42 (dd, $J$ = 17.4, 7.0 Hz, 3H). m/z (ES+):480 $[M+H]^+$.

Example 56: Compound 56

(1S,8aS)-N-((R)-1-(3-ethyl-5-(trifluoromethyl)phenyl)ethyl)-N-methyl-1-(2-methylthiophen-3-yl)-6-oxohexahydropyrrolo[1,2-a]pyrazine-2(1H)-carboxamide

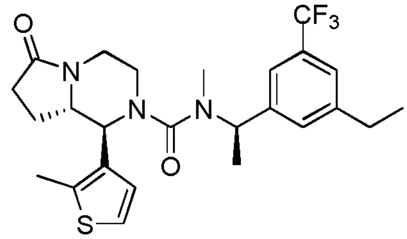

To a solution of triphosgene (47 mg, 0.16 mmol) in EtOAc (10 mL) at 0 °C was added solution of Intermediate 101 (120 mg, 0.35 mmol), DMAP (4 mg, 0.035 mmol) and TEA (107 mg, 1.05 mmol) in EtOAc (2 mL). The mixture was stirred for 1.5 h at room temperature, followed by addition of (R)-1-(3,5-bis(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (70 mg, yield: 31%); $^1H$ NMR (400 MHz, $CDCl_3$) $\delta$ 7.34 (s, 1H), 7.24 (s, 1H), 7.14 (s, 1H), 6.84 (t, $J$ = 4.9 Hz, 1H), 6.60 (d, $J$ = 2.8 Hz, 1H), 5.55 (q, $J$ = 6.9 Hz, 1H), 4.09 (d, $J$ = 12.7 Hz, 1H), 4.00 (d, $J$ = 9.7 Hz, 1H), 3.75 - 3.56 (m, 1H), 3.18 (d, $J$ = 11.9 Hz, 1H), 3.06 (t, $J$ = 12.1 Hz, 1H), 2.96 - 2.83 (m, 1H), 2.72 - 2.58 (m, 5H), 2.50 - 2.28 (m, 5H), 2.00 (ddd, $J$ = 17.1, 13.1, 4.0 Hz, 1H), 1.83 - 1.57 (m, 4H), 1.43 (t, $J$ = 9.6 Hz, 3H), 1.23 (t, $J$ = 7.6 Hz, 4H); m/z (ES+): 494 $[M+H]^+$.

Example 57: Compound 57

(1S,BaS)-N-((R)-1-(3,5-bis(trifluoromethyl)phenyl)ethyl)-N-methyl-1-(2-methylthiophen-3-yl)-6-oxohexahydropyrrolo[1,2-a]pyrazine-2(1H)-carboxamide

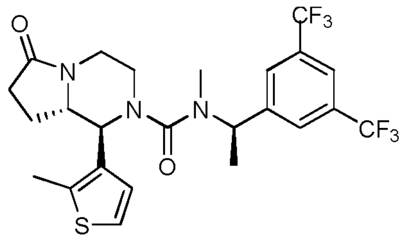

To a solution of triphosgene (29.6 mg, 0.1 mmol) in EtOAc (2 mL) at 0 °C was added solution of Intermediate 101 (49.6 mg, 0.2 mmol), DMAP (2.4 mg, 0.02 mmol) and TEA (61 mg, 0.6 mmol) in EtOAc (2 mL). The mixture was stirred for 1.5 h at room temperature, followed by addition of [1-(3,5-Bis-trifluoromethyl-phenyl)-ethyl]-methylamine (54.2 mg, 0.24 mmol) in EtOAc (2 mL) and TEA (61 mg, 0.6 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (10 mg, yield: 10%); $^1$H NMR (400 MHz, $CDCl_3$) δ 7.79 (s, 1H), 7.61 (s, 2H), 6.82 (d, J = 3.4 Hz, 1H), 6.65 - 6.55 (m, 1H), 5.60 (q, J = 7.0 Hz, 1H), 4.12 (td, J = 10.4, 5.2 Hz, 1H), 4.00 (d, J = 9.7 Hz, 1H), 3.74 - 3.64 (m, 1H), 3.24 - 3.16 (m, 1H), 3.06 (dd, J = 19.6, 7.4 Hz, 1H), 2.94 (td, J = 11.7, 3.3 Hz, 1H), 2.73 (s, 3H), 2.46 - 2.40 (m, 4H), 2.01 (tdd, J = 11.5, 7.6, 3.9 Hz, 1H), 1.77 - 1.66 (m, 2H), 1.47 (dd, J = 25.3, 7.0 Hz, 3H).

Example 58: Compound 58(1R,8aS)-N-((R)-1-(3,5-bis(trifluoromethyl)phenyl)ethyl)-N-methyl-1-(2-methylthiazol-5-yl)-6-oxohexahydropyrrolo[1,2-a]pyrazine-2(1H)-carboxamide

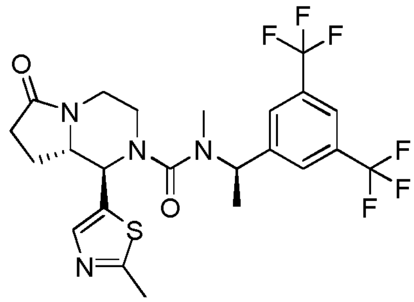

To a solution of triphosgene (63 mg, 0.2 mmol) in EtOAc (2 mL) at 0 °C was added solution of Intermediate 102 (100 mg, 0.42 mmol), DMAP (5 mg, 0.04 mmol) and TEA (127 mg, 1.26 mmol) in EtOAc (2 mL). The mixture was stirred for 1.5 h at room temperature, followed by addition of (R)-1-(3,5-bis(trifluoromethyl)phenyl)-N-methyl ethanamine (135 mg, 0.5 mmol) in EtOAc (2 mL) and TEA (127 mg, 1.26 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by Prep-HPLC to give the title compound (40 mg, yield: 17.7%); 1H NMR (400 MHz, CDCl3) δ 7.78 (d, J = 13.2 Hz, 1H), 7.62 (s, 1H), 7.59 - 7.52 (m, 2H), 5.68 - 5.53 (m, 1H), 4.17 - 4.04 (m, 2H), 3.78 - 3.53 (m, 1H), 3.26 (dd, J = 26.8, 12.2 Hz, 1H), 3.13 - 2.87 (m, 2H), 2.78 (d, J = 35.2 Hz, 3H), 2.67 (d, J = 9.5 Hz, 3H), 2.53 - 2.32 (m, 2H), 2.09 - 1.94 (m, 1H), 1.81 - 1.68 (m, 1H), 1.54 (dd, J = 15.7, 7.1 Hz, 3H). m/z (ES+): 535 [M+H]+.

Example 59: Compound 59

(1R,8aS)-N-((R)-1-(3,5-bis(trifluoromethyl)phenyl)ethyl)-N-methyl-6-oxo-1-(thiazol-5-yl)-hexahydropyrrolo[1,2-a]pyrazine-2(1H)-carboxamide

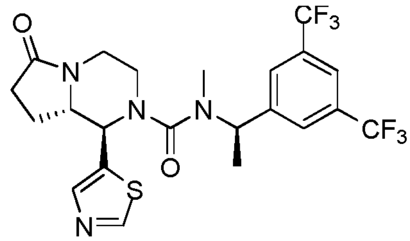

To a solution of triphosgene (33 mg, 0.112 mmol) in EtOAc (2 mL) at 0 °C was added solution of Intermediate 103 (50 mg, 0.224 mmol), DMAP (3 mg, 0.022 mmol) and TEA (68 mg, 0.672 mmol) in EtOAc (2 mL). The mixture was stirred for 1.5 h at room temperature, followed by addition of (R)-1-(3,5-bis(trifluoromethyl)phenyl)-N-methylethanamine (91 mg, 0.336 mmol) in EtOAc (2 mL) and TEA (68 mg, 0.672 mmol). The reaction was stirred at 45 °C for 48 h and quenched with sat. $NH_4Cl$ (aq) solution. The resulting mixture was extracted with EtOAc (2 x 20 mL). The combined organic layers were washed with brine, dried over anhydrous $Na_2SO_4$ and then concentrated in high vacuum. The residue was purified by silica gel chromatography (PE/EtOAc=5/1 to 1/1) to give the title compound (10 mg, yield: 9%); m/z (ES+): 521 $[M+H]^+$. $^1H$ NMR (400 MHz, $CDCl_3$) $\delta$ 8.69 (d, *J* = 9.8 Hz, 1H), 7.81 (d, *J* = 3.0 Hz, 1H), 7.71 (d, *J* = 15.1 Hz, 1H), 7.53 (s, 1H), 7.42 (s, 1H), 5.50 (dt, *J* = 20.9, 7.1 Hz, 1H), 4.11 (dt, *J* = 22.8, 11.3 Hz, 2H), 3.74 - 3.46 (m, 1H), 3.19 (dd, *J* = 27.8, 11.5 Hz, 1H), 3.10 - 2.82 (m, 2H), 2.77 (s, 1H), 2.68 (s, 2H), 2.43 - 2.25 (m, 2H), 2.02 - 1.83 (m, 1H), 1.69 (dd, *J* = 19.3, 10.0 Hz, 1H), 1.45 (dd, *J* = 27.3, 6.8 Hz, 3H).

Example 60: Compound 60

(1S,8aS)-N-((R)-1-(3,5-bis(trifluoromethyl)phenyl)ethyl)-N-methyl-1-(3-methyl-1H-pyrazol-4-yl)-6-oxohexahydropyrrolo[1,2-a]pyrazine-2(1H)-carboxamide

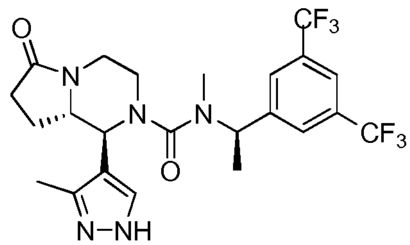

To a solution of Intermediate 110 (60 mg, 0.1 mmol) in MeOH (5 mL) under $N_2$ atmosphere, was added Pd/C (30 mg). The reaction mixture was stirred under $H_2$ atmosphere (4 atm) for 24 h at room temperature. Pd/C was filtered off through a celite pad, and the filtrate was concentrated in high vacuum. The residue was purified by Prep-HPLC to give the title compound (15 mg, yield: 29%); $^1H$ NMR (400 MHz, $CDCl_3$) $\delta$ 7.78 (s, 1H), 7.55 (s, 2H), 7.48 - 7.36 (m, 1H), 5.65 - 5.52 (m, 1H), 4.13 (d, *J* = 12.9 Hz, 1H), 3.77 (dd, *J* = 17.1, 7.3 Hz, 1H), 3.68 (d, *J* = 10.0 Hz, 1H), 3.24 - 3.10 (m, 2H), 3.06 (t, *J* = 10.8 Hz, 2H), 2.91 (td, *J* = 11.8, 3.0 Hz, 2H), 2.69 (s, 4H), 2.46 - 2.32 (m, 6H), 2.03 - 1.89 (m, 1H), 1.61 - 1.50 (m, 2H), 1.46 (d, *J* = 7.0 Hz, 3H).m/z (ES+): 518$[M+H]^+$.

Example 61: Compound 61

(1S,8aS)-N-((R)-1-(3-ethyl-5-(trifluoromethyl)phenyl)ethyl)-N-methyl-1-(3-methyl-1H-pyrazol-4-yl)-6-oxohexahydropyrrolo[1,2-a]pyrazine-2(1H)-carboxamide

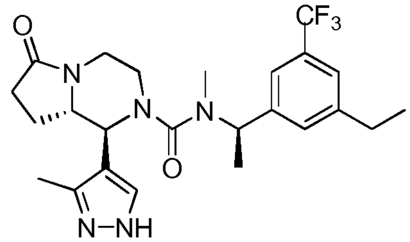

To a solution of Intermediate 111 (60 mg, 0.1 mmol) in MeOH (5 mL) under $N_2$ atmosphere, was added Pd/C (30 mg). The reaction mixture was stirred under $H_2$ atmosphere (4 atm) for 24 h at room temperature. Pd/C was filtered off through a celite pad, and the filtrate was concentrated in high vacuum. The residue was purified by Prep-HPLC to give the title compound (22 mg, yield: 46%); $^1H$ NMR (400 MHz, $CDCl_3$) $\delta$ 7.79 (d, $J$ = 11.6 Hz, 1H), 7.35 (s, 1H), 7.18 (s, 1H), 7.11 (s, 1H), 5.48 (q, $J$ = 6.9 Hz, 1H), 4.14 (d, $J$ = 12.7 Hz, 1H), 3.86 - 3.65 (m, 1H), 3.21 (d, $J$ = 11.9 Hz, 1H), 3.06 (t, $J$ = 12.3 Hz, 1H), 2.87 (dt, $J$ = 11.8, 5.9 Hz, 1H), 2.74 - 2.60 (m, 2H), 2.60 - 2.46 (m, 1H), 2.48 - 2.31 (m, 1H), 2.00 (dd, $J$ = 11.6, 8.3 Hz, 1H), 1.66 - 1.50 (m, 1H), 1.46 (dd, $J$ = 17.5, 7.1 Hz, 1H), 1.29 - 1.15 (m, 1H). m/z (ES+): 478[M+H]$^+$.

Example 62: Compound 62

(1S,8aS)-N-((R)-1-(3-ethyl-5-(trifluoromethyl)phenyl)ethyl)-7,7-bis(2-hydroxyethyl)-N-methyl-1-(3-methy-1H-pyrazol-4-yl)-6-oxohexahydropyrolo[1,2-a]pyrazine-2(1H)-carboxamide

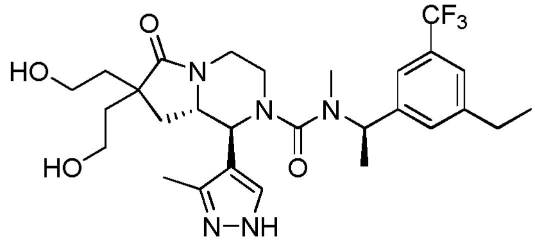

To a solution of Intermediate 115 (40 mg, 0.057 mol) in MeOH (20 mL) under $N_2$ atmosphere, was added Pd/C (30 mg). The reaction mixture was stirred under $H_2$ atmosphere (4 atm) for 24 h at room temperature. Pd/C was filtered off through a celite pad, and the filtrate was concentrated in high vacuum. The residue was purified by pre_HPLC to give the title compound (15 mg, yield: 30%); $^1H$ NMR (400 MHz, $CDCl_3$) $\delta$ 7.41 (s, 1H), 7.34 (s, 1H), 7.18 (s, 1H), 7.08 (s, 1H), 5.53 (d, $J$ = 6.9 Hz, 1H), 4.08 (d, $J$ = 11.7 Hz, 1H), 3.79 (d, $J$ = 9.0 Hz, 3H), 3.71 - 3.59 (m, 3H), 3.19 (d, $J$ = 11.6 Hz, 1H), 3.09 (t, $J$ = 12.1 Hz, 1H), 2.90 (d, $J$ = 10.5 Hz, 1H), 2.71 - 2.65 (m, 2H), 2.62 (s, 3H), 2.36 (s, 3H), 1.96 (dd, $J$ = 13.1, 6.8 Hz, 1H), 1.87 (d, $J$ = 18.3 Hz, 3H), 1.76 - 1.71 (m, 1H), 1.64 - 1.56 (m, 1H), 1.41 (d, $J$ = 6.8 Hz, 3H), 1.26 - 1.20 (m, 5H), 0.87 (d, $J$ = 7.1 Hz, 1H).

Example 63: Compound 63

(1S,8aS)-7,7-bis(2-hydroxyethyl)-N-methyl-1-(3-methyl-1H-pyrazol-4-yl)-N-((R)-1-(3-methyl-5-(trifluoromethyl)phenyl)ethyl)-6-oxo-hexahydropyrrolo[1,2-a]pyrazine-2(1H)-carboxamide

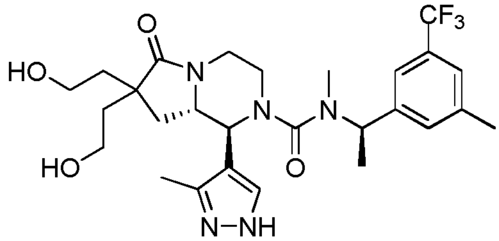

To a solution of Intermediate 117 (40 mg, 0.062 mmol) in MeOH (20 mL) was added Pd/C 5% (100 mg). The reaction was stirred under $H_2$ (5 atm) for 12 h. The catalyst was filtered through a celite pad and the solvent was removed in vacuo. The residue was purified by silica gel chromatography (DCM/MeOH=50/1 to 20/1) to give the title compound (20 mg, yield: 58%). m/z (ES+): 552 $[M+H]^+$. $^1H$ NMR (400 MHz, $CDCl_3$) $\delta$ 7.42 (s, 1H), 7.32 (s, 1H), 7.17 (s, 1H), 7.03 (s, 1H), 5.51 (d, *J* = 6.9 Hz, 1H), 4.09 (d, *J* = 12.5 Hz, 1H), 3.86 - 3.77 (m, 3H), 3.67 (ddd, *J* = 23.1, 11.6, 5.7 Hz, 3H), 3.20 (d, *J* = 12.0 Hz, 1H), 3.08 (t, *J* = 12.2 Hz, 1H), 2.91 (t, *J* = 10.6 Hz, 1H), 2.63 (s, 3H), 2.35 (d, *J* = 12.4 Hz, 6H), 1.92 (dt, *J* = 33.5, 13.5 Hz, 5H), 1.81 - 1.69 (m, 2H), 1.61 (dd, *J* = 13.3, 7.7 Hz, 2H), 1.41 (d, *J* = 6.9 Hz, 3H).

Example 64: Compound 64

(1S,8aS)-N-((R)-1-(3,5-bis(trifluoromethyl)phenyl)ethyl)-1-(1,3-dimethyl-1H-pyrazol-4-yl)-N-methyl-6-oxohexahydropyrrolo[1,2-a]pyrazine-2(1H)-carboxamide

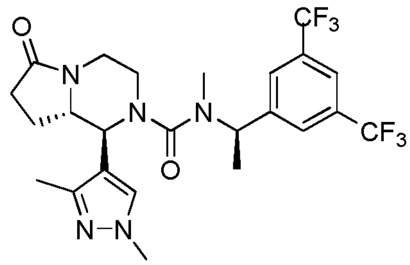

To a suspension of NaH (60%) (20 mg, 0.15 mmol) in THF (4 mL) was dropwisely added compound 59 (80 mg, 0.15 mmol) in THF (10 mL) at 0°C, After stirred at the same temperature for 30 min, MeI (0.1 ml, 0.15 mmol) was added into the reaction mixture at 0°C. The reaction mixture was stirred at 15 °C for 1h and carefully poured into ice-water (200 mL) contained con H2SO4 (6 mL). The resulting mixture was extracted with 30 ml EtOAc, washed with brine (10 mL) and the combined organic layers were dried over anhydrous $Na_2SO_4$ and then concentrated. The residue was purified by Prep-HPLC to give the title compound (20 mg, yield: 25%). $^1H$ NMR (400 MHz, $CDCl_3$) $\delta$ 7.70 (d, *J* = 10.4 Hz, 1H), 7.47 (t, *J* = 17.9 Hz, 2H), 7.25 - 7.09 (m, 1H), 5.51 (q, *J* = 6.6 Hz, 1H), 4.04 (dd, *J* = 11.1, 9.3 Hz, 1H), 3.79 - 3.69 (m, 3H), 3.69 - 3.49 (m, 2H), 3.15 (dd, *J* = 33.3, 21.7 Hz, 1H), 3.09 - 2.65 (m, 3H), 2.65 (d, *J* = 27.2 Hz, 3H), 2.34 (ddd, *J* = 19.4, 11.8, 6.6 Hz, 2H), 2.25 - 2.17 (m, 3H), 1.95 - 1.83 (m, 1H), 1.43 (dd, *J* = 19.7, 12.7 Hz, 3H); m/z (ES+): 532 $[M+H]^+$.

Example 65: Compound 65

(1S,BaS)-1-(2,3-dimethylphenyl)-N-((R)-1-(3-methyl-5-trifluoromethyl)phenyl)ethyl)-N-methyl-6-oxo-hexahydropyrrolo[1,2-a]pyrazine-2(1H)-carboxamide

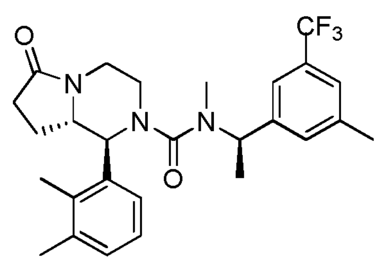

To a solution of triphosgene (73.45 mg, 0.25 mmol) in EtOAc (2 mL) at 0 °C was added solution of Intermediate 85 115.5 mg, 0.5 mmol), DMAP (6.1 mg, 0.05 mmol) and TEA ( 151.68 mg, 1.5 mmol) in EtOAc (2 mL). The mixture was stirred for 1.5 h at room temp erature, followed by addition of (R)-N-methyl-1-(3-methyl-5-(trifluoromethyl) phenyl)et hanamine (163.2 mg, 0.6 mmol) and TEA (151.68 mg, 1.5 mmol) in EtOAc (2 mL). The reaction was stirred at 45 °C for 48 h and quenched with sat. $NH_4Cl$ (aq) solution. The re sulting mixture was extracted with EtOAc (2 x 20 mL). The combined organic layers wer e washed with brine, dried over anhydrous $Na_2SO_4$ and then concentrated in high vacuu

m. The residue was purified by Prep-HPLC to give the title compound (15 mg, yield: 6%). $^1$H NMR (400 MHz, $CDCl_3$) δ 7.31 (s, 1H), 7.18 (s, 1H), 7.11 (s, 1H), 7.03 (d, *J* = 17.3 H z, 3H), 5.48 (s, 1H), 4.14 (d, *J* = 21.7 Hz, 2H), 3.70 (s, 1H), 3.28 (s, 1H), 3.11 (s, 1H), 2.97 (s, 1H), 2.64 (s, 3H), 2.42 (s, 3H), 2.35 (s, 2H), 2.32 (d, *J* = 5.1 Hz, 6H), 1.84 - 1. 78 (m, 1H), 1.67 - 1.63 (m, 1H), 1.57 (s, 3H); m/z (ES+): 488 $[M+H]^+$.

Example 66: Compound 66

(1S,8aS)-N-((R)-1-(3,5-bis(trifluoromethyl)phenyl)ethyl)-1-(3-chloro-2-methylphenyl)-N-methyl-6-oxohexahydropyrrolo[1,2-a]pyrazine-2(1H)-carboxamide

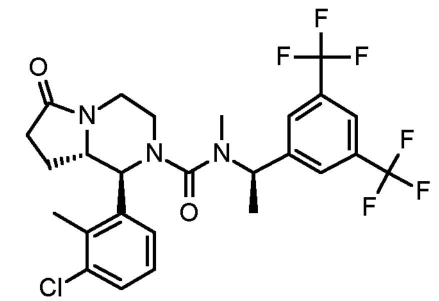

To a solution of triphosgene (244.3 mg, 0.83 mmol) in EtOAc (2 mL) at 0 °C was added solution of intermediate 122 (305 mg, 1.15 mmol), DMAP (17 mg, 0.14 mmol) and TEA (350.5 mg, 3.46 mmol) in EtOAc (2 mL). The mixture was stirred for 1.5 h at RT, followed by addition of (R)-1-(3,5-bis(trifluoromethyl)phenyl)-N-methyl ethanamine (377 mg, 1.38 mmol) in EtOAc (2 mL) and TEA (151.68 mg, 1.5 mmol). The reaction was stirred at 45 °C for 48 h and quenched with $NH_4Cl$ (aq). The resulting mixture was extracted with EtOAc; the combined organic layer was washed with 1 M aqueous HCl solution, dried over $Na_2SO_4$ and then concentrated in high vacuum. The residue was purified by silica gel chromatography to give the title compound (250 mg, yield: 39%); $^1$H-NMR (400 MHz, $CDCl_3$) δ 7.78 (s, 1H), 7.54 (s, 1H), 7.34 - 7.24 (m, 2H), 7.18 (d, *J* = 7.4 Hz, 1H), 7.07 (s, 1H), 5.53 (q, *J* = 7.1 Hz, 1H), 4.14 (dd, *J* = 11.0, 8.4 Hz, 2H), 3.69 (d, *J* = 9.0 Hz, 1H), 3.28 (d, *J* = 11.5 Hz, 1H), 3.08 (dd, *J* = 13.1, 2.2 Hz, 1H), 2.98 (dd, *J* = 12.0, 3.0 Hz, 1H), 2.71 (s, 3H), 2.57 (s, 3H), 2.45 - 2.27 (m, 2H), 1.84 (dd, *J* = 6.5, 2.8 Hz, 1H), 1.71 - 1.48 (m, 1H), 1.43 (d, *J* = 6.9 Hz, 3H);m/z (ES+): 562 $[M+H]^+$.

Example 68 & 69: Compound 68 & 69 (trans-isomers)

N-((R)-1-(3,5-bis(trifluoromethyl)phenyl)ethyl)-1-(2-ethylphenyl)-N-methyl-6-oxohexahydropyrrolo[1,2-a]pyrazine-2(1H)-carboxamide

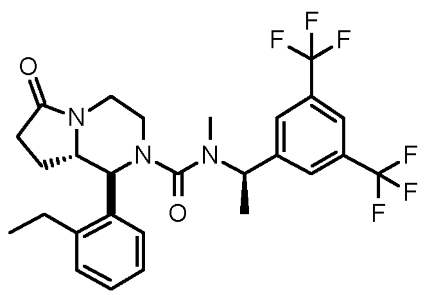

To a solution of triphosgene (73.45 mg, 0.25 mmol) in EtOAc (2 mL) at 0 °C was added solution of intermediate 125 (122 mg, 0.5 mmol), DMAP (6.1 mg, 0.05 mmol) and TEA (151.68 mg, 1.5 mmol) in EtOAc (2 mL). The mixture was stirred for 1.5 h at RT, followed by addition of (R)-1-(3,5-bis(trifluoromethyl)phenyl)-N-methyl ethanamine (163.2 mg, 0.6 mmol) in EtOAc (2 mL) and TEA (151.68 mg, 1.5 mmol). The reaction was stirred at 45 °C for 48 h and quenched with $NH_4Cl$ (aq). The resulting mixture was extracted with ETOAC; the combined organic layer was washed with 1 M

aqueous HCl solution, dried over $Na_2SO_4$ and then concentrated in high vacuum. The residue was purified by silica gel chromatography to give the title compound 68 (40 mg, yield: 15%) and compound 69 (40 mg, yield: 15%).

Compound 68: $^1$H-NMR (400 MHz, $CDCl_3$) δ 7.77 (s, 1H), 7.53 (s, 1H), 7.36 - 7.18 (m, 3H), 7.11 (t, *J* = 7.2 Hz, 1H), 5.53 (q, *J* = 6.8 Hz, 1H), 4.13 (t, *J* = 10.8 Hz, 2H), 3.79 (dd, *J* = 16.3, 7.5 Hz, 1H), 3.26 (d, *J* = 11.8 Hz, 1H), 3.11 (t, *J* = 12.2 Hz, 1H), 2.99 (t, *J* = 11.3 Hz, 2H), 2.95 - 2.76 (m, 1H), 2.7(s, 3H) 2.51 - 2.23 (m, 2H), 1.84 (dd, *J* = 12.4, 8.2 Hz, 1H), 1.39 (d, *J* = 6.9 Hz, 3H), 1.33 (t, *J* = 7.5 Hz, 3H); m/z (ES+): 542 $[M+H]^+$.
Compound 69: 1H-NMR (400 MHz, $CDCl_3$) δ 7.71 (s, 1H), 7.36 (s, 2H), 7.23 (dd, *J* = 7.0, 3.2 Hz, 3H), 7.12 (d, *J* = 7.4 Hz, 1H), 5.57 (q, *J* = 7.1 Hz, 1H), 4.14 (t, *J* = 12.7 Hz, 2H), 3.70 (dd, *J* = 16.9, 7.3 Hz, 1H), 3.31 (d, *J* = 12.1 Hz, 1H), 3.19 - 3.07 (m, 1H), 3.06 - 2.88 (m, 2H), 2.88 - 2.76 (m, 4H), 2.48 - 2.26 (m, 2H), 1.91 - 1.78 (m, 1H), 1.78 - 1.67 (m, 1H), 1.50 (d, *J* = 7.1 Hz, 3H), 1.33 (t, *J* = 7.5 Hz, 3H);m/z (ES+): 542 $[M+H]^+$.

Example 70 & 71: Compound 70 & 71 (trans-isomers)

N-((R)-1-(3-ethyl-5-(trifluoromethyl)phenyl)ethyl)-1-(2-ethylphenyl)-N-methyl-6-oxohexahydropyrrolo[1,2-a]pyrazine-2(1H)-carboxamide

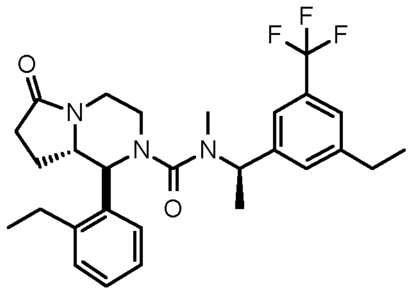

To a solution of triphosgene (73.45 mg, 0.25 mmol) in EtOAc (2 mL) at 0 °C was added solution of intermediate 125 (122 mg, 0.5 mmol), DMAP (6.1 mg, 0.05 mmol) and TEA (151.68 mg, 1.5 mmol) in EtOAc (2 mL). The mixture was stirred for 1.5 h at RT, followed by addition of (R)-1-(3-ethyl-5-(trifluoromethyl)phenyl)-N-methyl ethanamine (138.6 mg, 0.6 mmol) in EtOAc (2 mL) and TEA (151.68 mg, 1.5 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound 70 (40 mg, yield: 16%) and compound 71 (40 mg, yield: 16%).

Compound 70: $^1$H-NMR (400 MHz, $CDCl_3$) δ 7.33 (s, 1H), 7.29 (s, 1H), 7.26 - 7.20 (m, 2H), 7.19 (s, 1H), 7.12 (t, *J* = 7.3 Hz, 1H), 7.04 (s, 1H), 5.48 (q, *J* = 6.9 Hz, 1H), 4.12 (d, *J* = 10.0 Hz, 2H), 3.76 (dd, *J* = 16.8, 7.5 Hz, 1H), 3.27 (d, *J* = 12.1 Hz, 1H), 3.10 (dd, *J* = 20.0, 7.4 Hz, 1H), 3.06 - 2.92 (m, 2H), 2.86 (dd, J = 15.1, 7.5 Hz, 1H), 2.67 - 2.58 (m, 5H), 2.46 - 2.26 (m, 2H), 1.83 (ddd, *J* = 13.2, 12.2, 4.0 Hz, 1H), 1.68 (dd, *J* = 11.2, 5.9 Hz, 1H), 1.34 (t, *J* = 7.5 Hz, 6H), 1.20 (t, *J* = 7.6 Hz, 6H) ; m/z (ES+): 502 $[M+H]^+$.
Compound 71: $^1$H-NMR (400 MHz, $CDCl_3$) δ 7.29 (d, J = 8.8 Hz, 2H), 7.26 - 7.20 (m, 2H), 7.14 (t, *J* = 7.2 Hz, 1H), 7.05 (s, 1H), 6.78 (s, 1H), 5.52 (q, *J* = 7.0 Hz, 1H), 4.13 (t, *J* = 10.7 Hz, 2H), 3.71 (dd, *J* = 16.8, 7.4 Hz, 1H), 3.29 (d, *J* = 12.0 Hz, 1H), 3.16 - 3.06 (m, 1H), 3.02 (dd, *J* = 15.1, 7.6 Hz, 1H), 2.92 (td, *J* = 12.0, 3.1 Hz, 2H), 2.75 (s, 3H), 2.52 (q, *J* = 7.6 Hz, 2H), 2.48 - 2.26 (m, 2H), 1.82 (s, 1H), 1.72 (d, *J* = 7.8 Hz, 1H), 1.44 (d, *J* = 7.1 Hz, 3H), 1.34 (t, *J* = 7.5 Hz, 3H), 1.15 (t, *J* = 7.6 Hz, 3H); m/z (ES+): 502 $[M+H]^+$.

Example 72 & 73: Compound 72 & 73 (trans-isomers)

N-((R)-1-(3,5-bis(trifluoromethyl)phenyl)ethyl)-1-(2-chlorophenyl)-N-methyl-6-oxohexahydropyrrolo[1,2-a]pyrazine-2(1H)-carboxamide

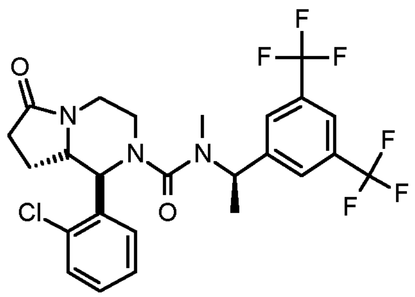

To a solution of triphosgene (73.45 mg, 0.25 mmol) in EtOAc (2 mL) at 0 °C was added solution of intermediate 128 (125 mg, 0.5 mmol), DMAP (6.1 mg, 0.05 mmol) and TEA (151.68 mg, 1.5 mmol) in EtOAc (2 mL). The mixture was stirred for 1.5 h at RT, followed by addition of (R)-1-(3,5-bis(trifluoromethyl)phenyl)-N-methyl ethanamine (163.2 mg, 0.6 mmol) in EtOAc (2 mL) and TEA (151.68 mg, 1.5 mmol). The reaction was stirred at 45 °C for 48 h and quenched with $NH_4Cl$ (aq). The resulting mixture was extracted with EtOAc; the combined organic layer was washed with 1 M aqueous HCl solution, dried over $Na_2SO_4$ and then concentrated in high vacuum. The residue was purified by silica gel chromatography to give the title compound 72 (20 mg, yield: 8%) and compound 73 (20 mg, yield: 8%).

Compound 72: $^1$H NMR (400 MHz, $CDCl_3$) $\delta$ 7.77 (s, 1H), 7.55 (s, 2H), 7.42 (dd, $J$ = 8.2, 4.6 Hz, 2H), 7.31 (dd, $J$ = 5.8, 3.7 Hz, 1H), 7.25 - 7.19 (m, 2H), 5.51 (q, $J$ = 7.0 Hz, 1H), 4.44 (d, $J$ = 10.0 Hz, 1H), 4.14 (d, $J$ = 12.3 Hz, 1H), 3.76 (d, $J$ = 9.5 Hz, 1H), 3.33 (d, $J$ = 11.6 Hz, 1H), 3.07 (dd, $J$ = 19.7, 13.7 Hz, 2H), 2.72 (s, 3H), 2.52 - 2.40 (m, 1H), 2.36 (dd, $J$ = 17.7, 8.9 Hz, 1H), 1.87 (dd, $J$ = 14.1, 6.1 Hz, 2H), 1.45 (d, $J$ = 7.0 Hz, 3H) ; m/z (ES+): 548 $[M+H]^+$.

Compound 73: $^1$H NMR (400 MHz, $CDCl_3$) $\delta$ 7.72 (s, 1H), 7.44 - 7.38 (m, 3H), 7.29 (d, $J$ = 5.2 Hz, 1H), 7.24 - 7.19 (m, 2H), 5.58 (t, $J$ = 7.0 Hz, 1H), 4.45 (d, $J$ = 9.9 Hz, 1H), 4.17 (d, $J$ = 13.0 Hz, 1H), 3.67 (d, $J$ = 9.4 Hz, 1H), 3.39 (d, $J$ = 12.1 Hz, 1H), 3.13 (t, $J$ = 12.1 Hz, 1H), 3.02 - 2.91 (m, 1H), 2.82 (s, 3H), 2.54 - 2.42 (m, 1H), 2.40 - 2.30 (m, 1H), 1.98 (dd, $J$ = 33.8, 9.1 Hz, 2H), 1.85 (dd, $J$ = 12.4, 8.8 Hz, 1H), 1.51 (d, $J$ = 7.2 Hz, 3H); m/z (ES+): 548 $[M+H]^+$.

Example 74: Compound 74

(1S,8aS)-N-((R)-1-(3,5-bis(trifluoromethyl)phenyl)ethyl)-1-(2-(hydroxymethyl)phenyl)-N-methyl-6-oxohexahydropyrrolo[1,2-a]pyrazine-2(1H)-carboxamide

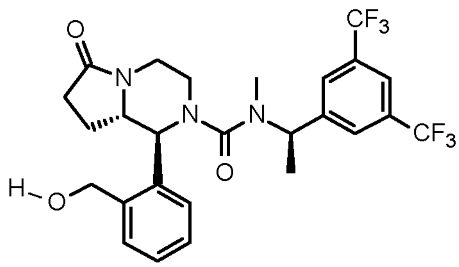

A slow stream of $O_3$ in $O_2$ was passed through a cooled solution of intermediate 134 (150 mg, 0.28mmol) at -78°C in DCM (30mL) until a pale blue colour persisted. Excess of $O_3$ was purged by $N_2$ bubbling, then $NaBH_4$ (105 mg, 2.8 mmol) was added. The solution was allowed to reach 25°C and stirred for 48h. The solvent was removed in vacuo and the crude material was purified by pre_HPLC to give the title compound as white solid (50 mg, yield: 33%). $^1$H NMR (400 MHz, $CDCl_3$) $\delta$ 7.80 (s), 7.55 (s), 7.38 (s), 7.28 (t, $J$ = 3.4 Hz), 5.50 (d, $J$ = 6.8 Hz), 5.11 (d, $J$ = 11.6 Hz), 4.86 (d, $J$ = 8.9 Hz), 4.49 (dd, $J$ = 11.4, 9.2 Hz), 4.21 (dd, $J$ = 26.2, 11.3 Hz), 3.68 (d, $J$ = 9.5 Hz), 3.27 (d, $J$ = 11.7 Hz), 3.14 (s), 3.00 (dd, $J$ = 11.8, 3.0 Hz), 2.72 (s), 2.52 - 2.27 (m), 1.86 - 1.67 (m), 1.40 (d, $J$ = 6.9 Hz); m/z (ES+): 544$[M+H]^+$.

Example 75: Compound 75

(1R,8aS)-N-((R)-1-(3,5-bis(trifluoromethyl)phenyl)ethyl)-1-(3,4-dimethylthiophen-2-yl)-N-methyl-6-oxohexahydropyrrolo[1,2-a]pyrazine-2(1H)-carboxamide

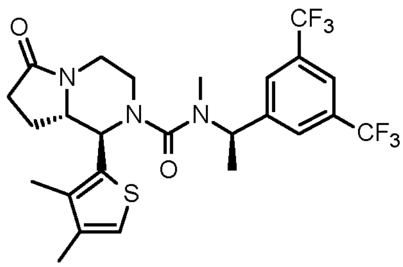

To a solution of triphosgene (47 mg, 0.16 mmol) in EtOAc (10 mL) at 0 °C was added solution of intermediate 138 (120 mg, 0.35 mmol), DMAP (4 mg, 0.035 mmol) and TEA (107 mg, 1.05 mmol) in EtOAc (2 mL). The mixture was stirred for 1.5 h at RT, followed by addition of (R)-1-(3,5-bis(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (70 mg, yield: 31%). $^1$H NMR (400 MHz, $CDCl_3$) $\delta$ 7.78 (s, 1H), 7.59 (s, 2H), 6.78 (s, 1H), 5.59 (q, *J* = 6.9 Hz, 1H), 4.13 (dd, *J* = 11.2, 6.5 Hz, 2H), 3.69 (dd, *J* = 17.0, 7.3 Hz, 1H), 3.19 (d, *J* = 11.8 Hz, 1H), 3.06 (d, *J* = 12.4 Hz, 1H), 2.92 (td, *J* = 11.7, 3.2 Hz, 1H), 2.71 (s, 3H), 2.47 - 2.27 (m, 2H), 2.22 (s, 3H), 2.14 (s, 3H), 2.06 - 1.89 (m, 1H), 1.74 - 1.61 (m, 4H), 1.47 (d, *J* = 7.0 Hz, 3H). m/z (ES+):548 $[M+H]^+$.

Example 76: Compound 76

(1R,8aS)-1-(3,4-dimethylthiophen-2-yl)-N-((R)-1-(3-ethyl-5-(trifluoromethyl)phenyl)ethyl)-N-methyl-6-oxohexahydropyrrolo[1,2-a]pyrazine-2(1H)-carboxamide

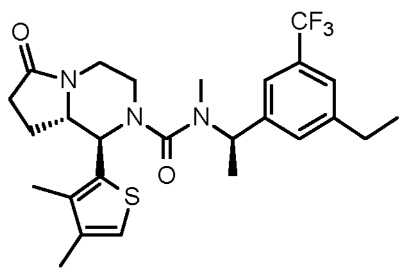

To a solution of triphosgene (47 mg, 0.16 mmol) in EtOAc (10 mL) at 0 °C was added solution of intermediate 138 (120 mg, 0.35 mmol), DMAP (4 mg, 0.035 mmol) and TEA (107 mg, 1.05 mmol) in EtOAc (2 mL). The mixture was stirred for 1.5 h at RT, followed by addition of (R)-1-(3,5-bis(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (70 mg, yield: 31%). $^1$H NMR (400 MHz, CDCl3) $\delta$ 7.34 (s, 1H), 7.23 (s, 1H), 7.13 (s, 1H), 6.79 (s, 1H), 5.53 (q, *J* = 6.9 Hz, 1H), 4.17 - 4.07 (m, 2H), 3.68 (dd, *J* = 17.0, 7.3 Hz, 1H), 3.18 (d, *J* = 11.7 Hz, 1H), 3.06 (t, *J* = 12.2 Hz, 1H), 2.90 (td, *J* = 11.7, 3.3 Hz, 1H), 2.68 - 2.60 (m, 5H), 2.48 - 2.31 (m, 2H), 2.23 (s, 3H), 2.14 (d, *J* = 0.7 Hz, 3H), 2.05 - 1.92 (m, 1H), 1.76 - 1.64 (m, 1H), 1.62 (s, 9H), 1.40 (d, *J* = 7.0 Hz, 3H), 1.23 (dd, *J* = 13.8, 6.2 Hz, 4H); m/z (ES+): 509$[M+H]^+$.

Example 77: Compound 77

(1S,8aS)-N-((R)-1-(3,5-bis(trifluoromethyl)phenyl)ethyl)-1-(3-methoxy-2-methylphenyl)-N-methyl-6-oxohexahydropyrrolo[1,2-a]pyrazine-2(1H)-carboxamide

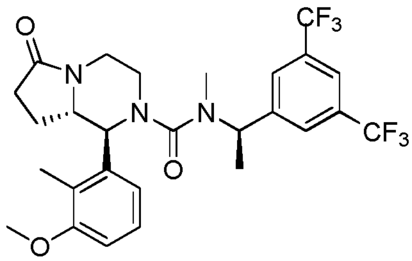

To a solution of triphosgene (47 mg, 0.16 mmol) in EtOAc (10 mL) at 0 °C was added solution of intermediate 143 (120 mg, 0.35 mmol), DMAP (4 mg, 0.035 mmol) and TEA (107 mg, 1.05 mmol) in EtOAc (2 mL). The mixture was stirred for 1.5 h at RT, followed by addition of (R)-1-(3,5-bis(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (70 mg, yield: 31%). $^1H$ NMR (400 MHz, $CDCl_3$) $\delta$ 7.77 (s, 1H), 7.55 (s, 2H), 7.09 (t, *J* = 8.0 Hz, 1H), 6.86 (d, *J* = 7.7 Hz, 1H), 6.86 (d, *J* = 7.7 Hz, 1H), 6.76 (d, *J* = 8.1 Hz, 1H), 5.56 (q, *J* = 6.9 Hz, 1H), 4.22 - 4.11 (m, 2H), 3.83 (s, 3H), 3.70 (dd, *J* = 21.9, 14.5 Hz, 1H), 3.28 (d, *J* = 11.7 Hz, 1H), 3.11 - 3.04 (m, 1H), 2.97 (td, *J* = 11.8, 3.0 Hz, 1H), 2.69 - 2.65 (m, 1H), 2.47 - 2.23 (m, 5H), 1.88 - 1.81 (m, 1H), 1.67 (dd, *J* = 19.6, 10.1 Hz, 3H), 1.44 (d, *J* = 7.0 Hz, 3H), 1.25 (s, 1H); m/z (ES+): 558 $[M+H]^+$.

Example 78: Compound 78

(1S,8aS)-N-((R)-1-(3-isopropyl-5-(trifluoromethyl)phenyl)ethyl)-1-(3-methoxy-2-methylphenyl)-N-methyl-6-oxohexahydropyrrolo[1,2-a]pyrazine-2(1H)-carboxamide

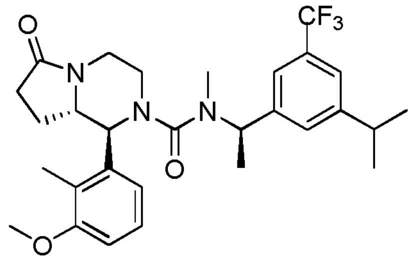

To a solution of triphosgene (47 mg, 0.16 mmol) in EtOAc (10 mL) at 0 °C was added solution of intermediate 143 (120 mg, 0.35 mmol), DMAP (4 mg, 0.035 mmol) and TEA (107 mg, 1.05 mmol) in EtOAc (2 mL). The mixture was stirred for 1.5 h at RT, followed by addition of (R)-1-(3-isopropyl-5-(trifluoromethyl)phenyl)-N-methyl ethanamine (163.2 mg, 0.6 mmol) in EtOAc (2 mL) and TEA (107 mg, 1.05 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (70 mg, yield: 31%). $^1H$ NMR (400 MHz, $CDCl_3$) $\delta$ 7.36 (s, 1H), 7.20 (s, 1H), 7.15 - 7.07 (m, 2H), 6.89 (d, *J* = 7.8 Hz, 1H), 6.75 (d, *J* = 8.1 Hz, 1H), 5.50 (q, *J* = 6.9 Hz, 1H), 4.18 - 4.05 (m, 2H), 3.82 (s, 3H), 3.68 (dd, *J* = 16.7, 7.5 Hz, 1H), 3.26 (d, *J* = 12.2 Hz, 1H), 3.09 (t, *J* = 12.5 Hz, 1H), 3.00 - 2.83 (m, 2H), 2.66 (s, 3H), 2.50 - 2.34 (m, 4H), 2.30 (dd, *J* = 17.2, 8.7 Hz, 1H), 1.83 - 1.75 (m, 1H), 1.72 - 1.65 (m, 1H), 1.44 - 1.35 (m, 3H), 1.33 - 1.14 (m, 7H); m/z (ES+): 532 $[M+H]^+$.

Example 79: Compound 79

(1S,8aS)-N-((R)-1-(3,5-bis(trifluoromethyl)phenyl)ethyl)-1-(2,3-dihydro-1H-inden-4-yl)-N-methyl-6-oxohexahydropyrrolo[1,2-a]pyrazine-2(1H)-carboxamide

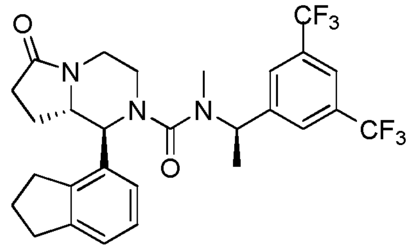

To a solution of triphosgene (47.2 mg, 0.16 mmol) in EtOAc (2 mL) at 0 °C was added solution of intermediate 149 (82 mg, 0.32 mmol), DMAP (3.9 mg, 0.032 mmol) and TEA (97 mg, 0.96 mmol) in EtOAc (2 mL). The mixture was stirred for 1.5 h at RT, followed by addition of (R)-1-(3, 5-bis(trifluoromethyl)phenyl)-N-methylethanamine (119 mg, 0.44 mmol) in EtOAc (2 mL) and TEA (97 mg, 0.96 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (20 mg, yield: 15%); $^1$H-NMR (400 MHz, $CDCl_3$) $\delta$ 7.77 (s, 1H), 7.55 (s, 2H), 7.14 (d, *J* = 7.1 Hz, 1H), 7.04 (dd, *J* = 16.2, 7.3 Hz, 2H), 5.54 (q, *J* = 7.0 Hz, 1H), 4.13 (d, *J* = 12.9 Hz, 1H), 3.93 (d, *J* = 9.9 Hz, 1H), 3.78 - 3.64 (m, 1H), 3.29 - 3.04 (m, 3H), 3.01 - 2.88 (m, 4H), 2.70 (s, 3H), 2.48 - 2.30 (m, 2H), 2.13 - 2.01 (m, 2H), 1.85 (ddd, *J* = 16.8, 13.2, 3.9 Hz, 1H), 1.60 (s, 3H), 1.42 (d, *J* = 7.0 Hz, 3H); m/z (ES+): 554 $[M+H]^+$.

Example 80: Compound 80

(1S,8aS)-1-(2,3-dihydro-1H-inden-4-yl)-N-((R)-1-(3-ethyl-5-(trifluoromethyl)phenyl)ethyl)-N-methyl-6-oxohexahydropyrrolo[1,2-a]pyrazine-2(1H)-carboxamide

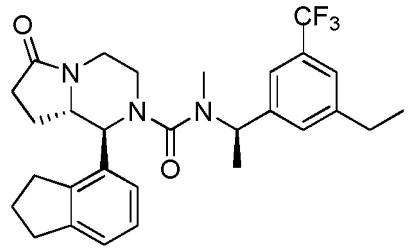

To a solution of triphosgene (47.2 mg, 0.16 mmol) in EtOAc (2 mL) at 0 °C was added solution of intermediate 149 (82 mg, 0.32 mmol), DMAP (3.9 mg, 0.032 mmol) and TEA (97 mg, 0.96 mmol) in EtOAc (2 mL). The mixture was stirred for 1.5 h at RT, followed by addition of (R)-1-(3-ethyl-5-(trifluoromethyl)phenyl)-N-methylethanamine (93 mg, 0.44 mmol) in EtOAc (2 mL) and TEA (97 mg, 0.96 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (18 mg, yield: 14.9%); $^1$H NMR (400 MHz, $CDCl_3$) $\delta$ 7.34 (s, 1H), 7.20 (s, 1H), 7.13 (d, *J* = 6.8 Hz, 1H), 7.05 (dd, *J* = 10.8, 7.1 Hz, 3H), 5.48 (q, *J* = 7.0 Hz, 1H), 4.16 - 4.07 (m, 1H), 3.94 (d, *J* = 9.9 Hz, 1H), 3.69 (dd, *J* = 17.1, 7.3 Hz, 1H), 3.31 - 3.16 (m, 2H), 3.08 (d, *J* = 12.5 Hz, 1H), 2.97 - 2.89 (m, 4H), 2.65 (s, 3H), 2.61 (d, *J* = 7.6 Hz, 1H), 2.44 - 2.31 (m, 2H), 2.15 - 2.03 (m, 2H), 1.69 (s, 2H), 1.20 (t, *J* = 7.6 Hz, 3H); m/z (ES+): 514 $[M+H]^+$.

Example 81: Compound 81

(1S,8aS)-1-(2,3-dihydro-1H-inden-4-yl)-N-((R)-1-(3-isopropyl-5-(trifluoromethyl)phenyl)ethyl)-N-methyl-6-oxohexahydropyrrolo[1,2-a]pyrazine-2(1H)-carboxamide

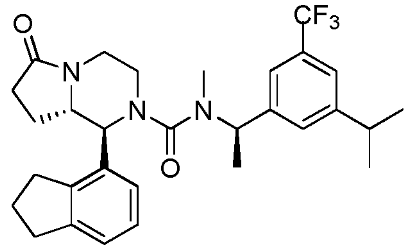

To a solution of triphosgene (47.2 mg, 0.16 mmol) in EtOAc (2 mL) at 0 °C was added solution of intermediate 33 (82 mg, 0.32 mmol), DMAP (3.9 mg, 0.032 mmol) and TEA (97 mg, 0.96 mmol) in EtOAc (2 mL). The mixture was stirred for 1.5 h at RT, followed by addition of (R)-1-(3-isopropyl-5-(trifluoromethyl)phenyl)-N-methylethanamine (101 mg, 0.44 mmol) in EtOAc (2 mL) and TEA (97 mg, 0.96 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (15 mg, yield: 14%); $^1H$ NMR (400 MHz, DMSO) δ 7.36 (s, 1H), 7.20 (s, 1H), 7.12 (t, *J* = 7.7 Hz, 2H), 7.10 - 7.00 (m, 2H), 5.49 (q, *J* = 7.1 Hz, 1H), 4.11 (d, *J* = 12.9 Hz, 1H), 3.93 (d, *J* = 9.9 Hz, 1H), 3.69 (dd, *J* = 17.1, 7.3 Hz, 1H), 3.31 - 3.11 (m, 2H), 3.09 (t, *J* = 12.1 Hz, 1H), 2.98 - 2.84 (m, 5H), 2.66 (s, 3H), 2.46 - 2.29 (m, 2H), 2.27 - 1.97 (m, 3H), 1.87 - 1.78 (m, 1H), 1.74 - 1.66 (m, 2H), 1.36 (d, *J* = 7.0 Hz, 3H), 1.21 (d, *J* = 6.8 Hz, 6H); m/z (ES+): 528 $[M+H]^+$.

Example 82: Compound 82

(1S,8aS)-N-((R)-1-(3,5-bis(trifluoromethyl)phenyl)ethyl)-1-(2-fluoro-6-methylphenyl)-N-methyl-6-oxohexahydropyrrolo[1,2-a]pyrazine-2(1H)-carboxamide

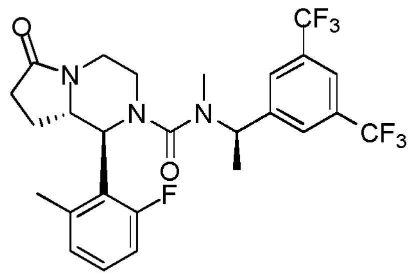

To a solution of triphosgene (25 mg, 0.095 mmol) in EtOAc (2 mL) at 0 °C was added solution of intermediate 154 (50 mg, 0.2 mmol) and TEA (75 mg, 0.72 mmol) in EtOAc (2 mL). The mixture was stirred for 1.5 h at RT, followed by addition of (R)-1-(3,5-bis(trifluoromethyl)phenyl)-N-methylethanamine (60 mg, 0.22 mmol) in EtOAc (2 mL) and TEA (75 mg, 0.72 mmol). The reaction was stirred at 45 °C for 48 h and quenched with $NH_4Cl$ (aq). The resulting mixture was extracted with ETOAC; the combined organic layer was washed with 1 M aqueous HCl solution, dried over $Na_2SO_4$ and then concentrated in high vacuum. The residue was purified by silica gel chromatography to give the title compound (12 mg, yield: 11%); 1H NMR (400 MHz, $CDCl_3$) δ ppm 7.76 (s, 1H), 7.54 (s, 2H), 7.18-7.16 (m, 1H), 7.14-7.12 (m, 1H), 6.98-6.80 (m, 1H), 5.46 - 5.38 (q, 1H), 3.38-36 (d, 1H), 4.18-4.12 (m, 1H), 4.10-3.98 (m, 1H), 3.47-3.43(m, 1H), 3.12-3.04 (m, 1H), 2.65 (s, 3H), 2.55 (s, 3H), 2.47-2.43 (m, 3H),1.93-1.91(m,1H),1.25(d,3H); m/z (ES+): 546 $[M+H]^+$.

Example 83 & 84: Compound 83 & 84 (trans-isomers)

N-((R)-1-(3,5-bis(trifluoromethyl)phenyl)ethyl)-1-(2-chloro-4-fluorophenyl)-N-methyl-6-oxohexahydropyrrolo[1,2-a]pyrazine-2(1H)-carboxamide

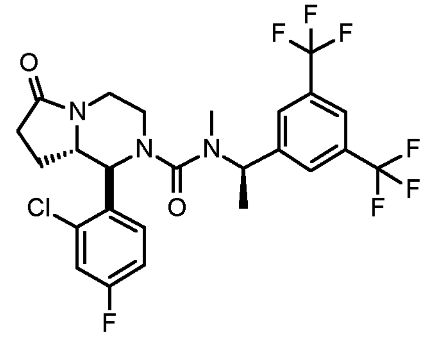

To a solution of triphosgene (41.0 mg, 0.14 mmol) in EtOAc (2 mL) at 0 °C was added a solution of intermediate 157 (94.0 mg, 0.35 mmol), DMAP (4.0 mg, 0.03 mmol) and TEA (106.0 mg, 1.05 mmol) in EtOAc (2 mL). The mixture was stirred for 1.5 h at RT, followed by addition of (R)-1-(3,5-bis(trifluoromethyl)phenyl)-N- methylethanamine (133.0 mg, 0.49 mmol) in EtOAc (2 mL) and TEA (106.0 mg, 1.05 mmol). The reaction was stirred at 40 °C for 16 h and quenched with $NH_4Cl$ (aq). The resulting mixture was extracted with ETOAC; the organic layer was washed with 1 M aqueous HCl solution, dried over $Na_2SO_4$ and then concentrated in high vacuum. The residue was purified by Prep-HPLC to give the compound 83 (10.3 mg, yield: 5%) and the compound 84 (4.3 mg, yield: 2%).

Compound 83: $^1$H NMR (400 MHz, $CDCl_3$) δ ppm 7.78 (s, 1H), 7.55 (s, 2H), 7.31-7.26 (m, 1H), 7.19-7.16 (m, 1H), 6.98-6.93 (m, 1H), 5.50 (q, *J* = 6.8 Hz, 1H), 4.37 (d, *J* = 10.0 Hz, 1H), 4.14 (d, *J* = 12.4 Hz, 1H), 3.76 - 3.74 (m, 1H), 3.32 (d, *J* = 11.6 Hz, 1H), 3.09 - 3.01 (m, 2H), 2.72 (s, 3H), 2.47 - 2.36 (m, 2H), 1.90 - 1.86 (m, 2H), 1.47 (d, *J* = 7.2 Hz, 3H); m/z (ES+): 566 $[M+H]^+$.

Compound 84: $^1$H NMR (400 MHz, $CDCl_3$) δ ppm 7.66 (s, 1H), 7.34 (s, 2H), 7.19-7.17 (m, 1H), 7.12-7.09 (m, 1H), 6.89-6.85 (m, 1H), 5.52 (q, *J* = 7.2 Hz, 1H), 4.34 (d, *J* = 10.0 Hz, 1H), 4.16 -4.09 (m, 1H), 3.58 - 3.56 (m, 1H), 3.32 (d, *J* = 12.0 Hz, 1H), 3.04 - 3.01 (m, 1H), 2.92 - 2.88 (m, 1H), 2.75 (s, 3H), 2.39 - 2.28 (m, 2H), 1.83 - 1.76 (m, 2H), 1.45 (d, *J* = 7.6 Hz, 3H); m/z (ES+): 566 $[M+H]^+$.

Example 85: Compound 85

(1S,8aS)-N-((R)-1-(3,5-bis(trifluoromethyl)phenyl)ethyl)-1-(4-fluorophenyl)-N-methy-6-oxohexahydropyrrolo[1,2-a]pyrazine-2(1H)-carboxamide

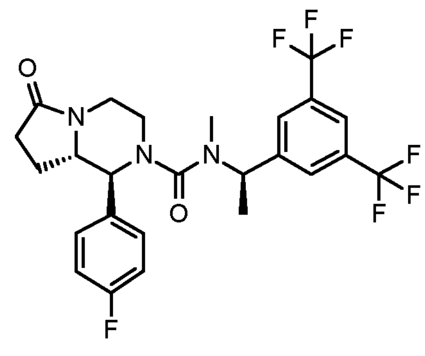

To a solution of triphosgene (25.0 mg, 0.08 mmol) in EtOAc (2 mL) at 0 °C was added a solution of intermediate 162 (50.0 mg, 0.21 mmol), DMAP (4.0 mg, 0.03 mmol) and TEA (64.0 mg, 0.63 mmol) in EtOAc (2 mL). The mixture was stirred for 1.5 h at RT, followed by addition of (R)-1-(3,5-bis(trifluoromethyl)phenyl)-N- methyl ethanamine (80.0

mg, 0.29 mmol) in EtOAc (2 mL) and TEA (64.0 mg, 0.63 mmol). The reaction was stirred at 40 °C for 16 h and quenched with $NH_4Cl$ (aq). The resulting mixture was extracted with ETOAC; the organic layer was washed with 1 M aqueous HCl solution, washed with brine, dried over $Na_2SO_4$ and concentrated in vacuo. The residue was purified by Prep-HPLC to give the tilte compound (7.6 mg, yield: 7%). $^1H$ NMR (400 MHz, $CDCl_3$) δ ppm 7.70 (s, 1H), 7.66 (s, 2H), 7.19-7.13 (m, 2H), 6.97 - 6.93 (m, 2H),5.85 - 5.76 (m, 2H), 5.25 (s, 1H), 3.80 - 3.77 (m, 2H), 3.52 - 3.49 (m, 2H), 2.94 - 2.91 (m, 2H), 2.53 - 2.50 (m, 5H), 1.47 (d, *J* = 7.2 Hz, 3H). m/z ($ES^+$): 532 $[M+H]^+$.

Example 100

Measurement of NK1 receptor mediated intracellular $[Ca^{2+}]$ mobilization in U251 MG cells using FLIPR

U251 MG cells were seeded into black walled clear-bottom 384-well plates (Greiner Bio-One GmbH, Frickenhausen, Germany) at a density of 15,000 cells/well in 50 μl culture medium and cultured overnight in a 37°C 5% CO2 incubator. The cells were then loaded with the calcium sensitive dye Fluo-4 (Invitrogen) at 1 μM in buffer, containing 0.04% Pluronic F-127 (Sigma-Aldrich), and 2.5mM probenecid (Sigma-Aldrich) for 60 min in a humidified atmosphere of 5% $CO_2$. Thereafter, cells were washed three times in washing buffer containing 20mM HEPES and 2.5mM probenecid pH 7.3. Serial dilutions of test compounds in assay buffer containing 2% dimethyl sulfoxide (final concentration in the cell plate is 0.5% DMSO) and/or agonist were then automatically pipetted into each test well, and the peak fluorescence intensity was recorded ($I_{ex}$, 488 nm; $I_{em}$, 540 nm) by the FLIPR instrument (Molecular Devices) for approximately 5 min . To measure antagonist potency, cell plates were first incubated with the test compound and intracellular fluorescence recorded for 5 minutes to check a potential agonist effect of the test compound. Cell plates were quickly removed from the FLIPR instrument and incubated for additional 10 minutes at 37°C before being moved back to the FLIPR instrument for Substance P ($EC_{80}$) addition. The response was measured as the peak relative fluorescence change after agonist addition.

Compound relative %-effect was normalized to the maximal response evoked by 100nM Aprepitant in presence of the $EC_{80}$ of Substance P and the antagonist potency determined by non-linear regression using GraphPad Prism (version 5) or the four-parameter logistic model in XL*fit* (IDBS, Guilford, UK) for Microsoft Excel (Microsoft, Redmond, WA). The IC50 value is defined as the molar concentration of a test compound that produces a response midway between the fitted top and the fitted bottom. $pIC_{50} = -\log IC_{50}$

Formula is listed as follows:

Fit (%Effect calculated) = (A+((B-A)/(1+((C/X)^D))))

A: bottom value
B: top value
C: test compound concentration
X: Relative $IC_{50}$
D: Hill slope coefficient

Ref: Sullivan E, Tucker EM, and Dale IL. Measurement of $[Ca^{2+}]$ using the Fluorometric Imaging Plate Reader (FLIPR). Methods Mol Biol 114:125-133, 1999.

Compounds of the present invention were tested according to example 100.

Results are reported in Table 1.

Example 101

Hepatic Extraction Ratio from Hepatic Intrinsic Clearance following incubation in human liver microsomes

The general procedure consists of an automated incubation system and LC-MS/MS analysis with human liver microsomes. Human liver microsomes are thawed rapidly in a waterbath at 37°C and kept on ice until use. The human liver microsomes are diluted with 50mM potassium phosphate buffer pH 7.4 to a protein concentration of 0.55mg/mL. Test compounds and positive controls (Verapamil and Dextromethorphan, positive controls for CYP3A4 for CYP2D6 isoforms, respectively) are dissolved in methanol (or other appropriate solvent, if necessary) in order to obtain a 5mM solution which is further diluted to the concentration of 50μM. The stock solutions are prepared immediately before the test.

5μL of 50μM test compounds and controls are added to 445 μL-of the 0.55mg/mL microsomes solution and the incubation mixture are pre-warmed at 37°C for 5 minutes. The incubation reactions are initiated by adding 50μL of

pre-warmed NADPH regenerating system to the incubation mixtures. 50μL-aliquots will be taken from the incubation mixtures at: 0, 3, 6, 9, 15 and 30 minutes and the reactions are stopped by adding 100μL of ACN containing Rolipram, used as generic Internal Standard (IS). Samples are then diluted with 120 μL of Milli-Q water and centrifuged at 3000 rpm for 10 minutes, prior the LC-MS/MS analysis.

Intrinsic clearance.

The integrated peak areas for test compounds at the selected time points are divided by the respective peak areas of the IS and the percent of parent remaining is calculated by normalizing the peak area ratio of parent to IS at 0 min. Observed rate constant ($k_{obs}$) for parent degradation is calculated by determining the slope of the line of the graph of the natural log of percentage parent remaining versus time of incubation. From the rate of depletion (min-1) and volume of the incubation (mL), clearance is estimated. This is scaled for the protein in the incubation relative to that in the intact liver, for all species (mL/min/g liver).

$$Clint = Rate/\min * \left(\frac{mL\ incubation}{0.5\ mg\ protein}\right) * \frac{52.5\ mean\ mg\ protein}{g\ liver}$$

Values for the Human in vitro *Clint* are expressed as mL/min/g liver.

Assuming a liver weight of 25.7 g/kg and a liver blood flow of 20.7 mL/min/kg, the hepatic extraction ratio, Eh, can be derived from the Human in vitro *Clint:*

$$Eh = \frac{(Clint * 25.7) * 20.7}{(Clint * 25.7) + 20.7} / 20.7$$

E.g. a Human in vitro *Clint* of 2.4 mL/min/g liver corresponds to a hepatic extraction ratio of 75%.

Some compounds of the present invention were tested according to example 101. Results are reported in Table 1

Table 1

| Compound no. | NK1R mediated intracellular [$Ca^{2+}$] mobilization IC 50 (nM) | Human in vitro Clint ml/min/g |
|---|---|---|
| 36 | 0,811 | 147 |
| 10 | 0,063 | >200 |
| 11 | 0,613 | >200 |
| 14 | 0,094 | >200 |
| 15 | 2,54 | 104 |
| 12 | 0,622 | >200 |
| 13 | 0,034 | >200 |
| 4 | 9,26 | >200 |
| 33 | 0,139 | >200 |
| 1 | 0,154 | >200 |
| 2 | 7,79 | >200 |
| 3 | 0,297 | >200 |
| 6 | 1,59 | >200 |
| 5 | 0,437 | >200 |
| 7 | 0,08 | >200 |
| 8 | 0,096 | >200 |
| 9 | 0,18 | >200 |
| 43 | 2,97 | |

(continued)

| Compound no. | NK1R mediated intracellular [$Ca^{2+}$] mobilization IC 50 (nM) | Human in vitro Clint ml/min/g |
|---|---|---|
| 18 | 1,15 | >200 |
| 19 | 38,8 | >200 |
| 17 | 0,799 | >200 |
| 29 | 0,114 | >200 |
| 16 | 0,243 | >200 |
| 39 | 0,119 | >200 |
| 37 | 0,08 | 30 |
| 45 | 0,772 | <10 |
| 25 | 0,08 | 128 |
| 21 | 0,083 | 36 |
| 38 | 0,118 | 157 |
| 23 | 0,037 | 78 |
| 46 | 0,167 | <10 |
| 20 | 0,137 | 40 |
| 22 | 0,197 | >200 |
| 24 | 0,08 | >200 |
| 27 | 0,064 | >200 |
| 26 | 7,36 | >200 |
| 28 | 0,11 | >200 |
| 60 | 31,8 | - |
| 61 | 59 | >200 |
| 48 | 0,249 | >200 |
| 31 | 0,059 | 61 |
| 30 | 0,116 | 18 |
| 49 | 0,999 | >200 |
| 62 | 1,07 | 115 |
| 34 | 0,054 | >200 |
| 35 | 0,141 | >200 |
| 50 | 0,08 | >200 |
| 51 | 0,055 | >200 |
| 63 | 9,29 | 51 |
| 57 | 0,152 | >200 |
| 54 | 0,514 | >200 |
| 47 | 0,893 | - |
| 53 | 0,43 | >200 |
| 52 | 0,353 | >200 |
| 56 | 1,51 | >200 |
| 55 | 1,58 | >200 |

(continued)

| Compound no. | NK1R mediated intracellular [$Ca^{2+}$] mobilization IC 50 (nM) | Human in vitro Clint ml/min/g |
|---|---|---|
| 58 | 28,6 | >200 |
| 59 | 29,4 | >200 |
| 32 | 0,66 | >200 |
| 41 | 0,111 | >200 |
| 42 | 0,306 | >200 |
| 65 | 0,365 | >200 |
| 64 | 1,23 | 67 |
| 40 | 0,197 | >200 |
| 44 | 100 | >200 |
| 82 | 0,13 | >200 |
| 66 | 0,14 | 51 |
| 84 | 1,15 | - |
| 83 | 0,06 | - |
| 73 | 1,38 | - |
| 72 | 0,02 | - |
| 85 | 100 | >200 |
| 74 | 0,11 | >200 |
| 81 | 3,5 | >200 |
| 71 | 49 | >200 |
| 70 | 0,94 | >200 |
| 69 | 11 | >200 |
| 68 | 0,26 | >200 |
| 76 | 0,26 | >200 |
| 75 | 0,34 | >200 |
| 80 | 0,79 | >200 |
| 79 | 2,57 | >200 |
| 78 | 1,86 | >200 |
| 77 | 0,52 | >200 |

## Claims

1. A compound of general formula A

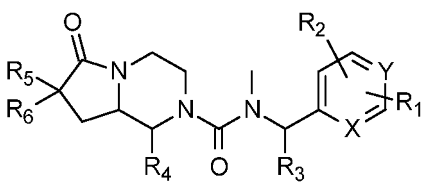

A

wherein

$R_1$ and $R_2$ independently are selected from the group consisting of $(C_{1-4})$alkyl, $(C_{1-4})$haloalkyl, $(C_{1-4})$alkoxy, $CD_3$ or halogen;
$R_3$ is selected from the group consisting of hydrogen, $(C_{1-4})$alkyl, $(C_{1-4})$haloalkyl and $(C_{1-4})$hydroxyalkyl;
$R_4$ is selected from the group consisting of phenyl, 5-membered heteroaryl and 6-membered heteroaryl, said phenyl and 6-membered heteroaryl being substituted with one ore more substituents independently selected from $R_7$, and said 5-membered heteroaryl optionally being substituted with one ore more substituents independently selected from $R_7$;
$R_5$ and $R_6$ are independently selected from the group consisting of hydrogen, $(C_{1-4})$alkyl, $(C_{1-4})$hydroxyalkyl and $(C_{1-4})$haloalkyl;
$R_7$ is selected from the group consisting of halogen, hydroxy, $(C_{1-4})$alkyl, $(C_{1-4})$alkoxy, aryloxy, aryl$(C_{1-4})$alkoxy, cyano, $(C_{1-4})$haloalkyl or $(C_{1-4})$hydroxyalkyl, wherein two adjacent $(C_{1-4})$alkyl's, together with the phenyl, 5-membered heteroaryl or 6-membered heteroaryl to which they are attached, may optionally form a 4-7 membered aliphatic ring;
X and Y are independently selected from the group consisting of CH and N;

or a pharmaceutically acceptable salt or solvate thereof;
with the proviso that when both X and Y are CH, $R_4$ is not 2-methylphenyl.

2. The compound according to claim 1 of general formula A(i)

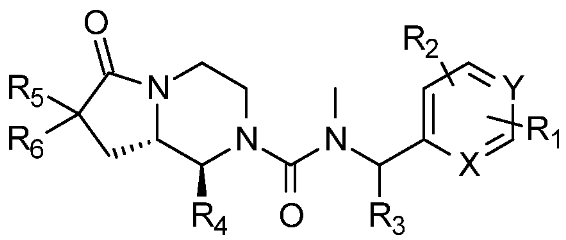

A(i)

wherein $R_1$, $R_2$, $R_3$, $R_4$, $R_5$, $R_6$, X and Y are as indicated in claim 1,
or a pharmaceutically acceptable salt or solvate thereof;
with the proviso that when both X and Y are CH, $R_4$ is not 2-methylphenyl.

3. The compound according to claim 1 of general formula A(ii)

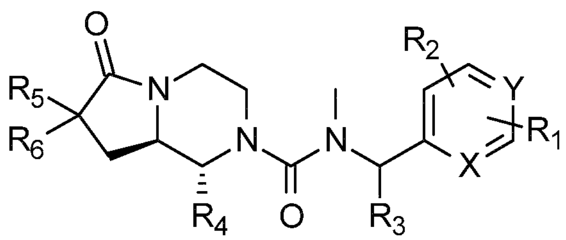

A(ii)

wherein $R_1$, $R_2$, $R_3$, $R_4$, $R_5$, $R_6$, X and Y are as indicated in claim 1,
or a pharmaceutically acceptable salt or solvate thereof;
with the proviso that when both X and Y are CH, $R_4$ is not 2-methylphenyl.

4. The compound according to any one of claims 1-3 wherein $R_1$ and $R_2$ represent $(C_{1-4})$haloalkyl.

5. The compound according to any one of claims 1-3, wherein $R_1$ represents $(C_{1-4})$haloalkyl and $R_2$ represents $(C_{1-4})$alkyl.

6. The compound according to any one of claims 1-5 wherein $R_3$ is selected from the group consisting of hydrogen and $(C_{1-4})$alkyl.

7. The compound according to any one of claims 1-6, wherein $R_5$ and $R_6$ independently are selected from the group consisting of hydrogen and $(C_{1-4})$hydroxyalkyl

8. The compound according to any one of claims 1-7 wherein $R_7$ is selected from the group consisting of halogen and $(C_{1-4})$alkyl.

9. The compound according to any one of claims 1-8 wherein X and Y represent CH.

10. The compound according to any one of claims 1-9 wherein $R_4$ is phenyl, wherein said phenyl is substituted with two substituents independently selected from $R_7$.

11. The compound according to any one of claims 1-10 selected from the group consisting of N-((R)-1-(3,5-bis(trifluoromethyl)phenyl)ethyl)-1-(2,4-dimethylphenyl)-N-methyl-6-oxohexahydropyrrolo[1,2-a]pyrazine-2(1H)-carboxamide, N-(3,5-bis(trifluoromethyl)benzyl)-1-(2,4-dimethylphenyl)-N-methyl-6-oxohexahydropyrrolo[1,2-a]pyrazine-2(1H)-carboxamide, 1-(2,4-dimethylphenyl)-N-methyl-N-((R)-1-(3-methyl-5-(trifluoromethyl)phenyl)ethyl)-6-oxo-hexahydropyrrolo[1,2-a]pyrazine-2(1H)-carboxamide, N-((R)-1-(3,5-bis(trifluoromethyl)phenyl)ethyl)-1-(2,4-dimethylphenyl)-7,7-bis(2-hydroxyethyl)-N-methyl-6-oxohexahydropyrrolo[1,2-a]pyrazine-2(1H)-carboxamide, 1-(2,4-dimethylphenyl)-7,7-bis(2-hydroxyethyl)-N-methyl-N-((R)-1-(3-methyl-5-(trifluoromethyl)phenyl)ethyl)-6-oxo-hexahydropyrrolo[1,2-a]pyrazine-2(1H)-carboxamide, N-(3,5-bis(trifluoromethyl)benzyl)-1-(2,4-dimethylphenyl)-7,7-bis(2-hydroxyethyl)-N-methyl-6-oxohexahydropyrrolo[1,2-a]pyrazine-2(1H)-carboxamide, *N*-((R)-1-(3-chloro-5-(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide, 1-(4-fluoro-2-methylphenyl)-7,7-bis(2-hydroxyethyl)-*N*-methyl-*N*-((R)-1-(3-methyl-5-(trifluoromethyl)phenyl)ethyl)-6-oxohexahydropyrrolo[1,2-a]pyrazine-2(1*H*)-carboxamide, *N*-((R)-1-(3,5-bis(trifluoromethyl)phenyl)ethyl)-1-(4-fluoro-2-methylphenyl)-7,7-bis(2-hydroxyethyl)-*N*-methyl-6-oxohexahydropyrrolo[1,2-a]pyrazine-2(1*H*)-carboxamide, N-((R)-1-(3,5-bis(trifluoromethyl)phenyl)ethyl)-7,7-bis(2-hydroxyethyl)-1-(4-methoxy-2-methylphenyl)-N-methyl-6-oxohexahydropyrrolo[1,2-a]pyrazine-2(1H)-carboxamide, (1S,8aS)-7,7-bis(2-hydroxyethyl)-1-(4-methoxy-2-methylphenyl)-N-methyl-N-((R)-1-(3-methyl-5-(trifluoromethyl)phenyl)ethyl)-6-oxohexahydropyrrolo[1,2-a]pyrazine-2(1H)-carboxamide, *N*-((R)-1-(3,5-bis(trifluoromethyl)phenyl)ethyl)-1-(4-methoxy-2-methylphenyl)-*N*-methyl-6-oxohexahydropyrrolo[1,2-a]pyrazine-2(1*H*)-carboxamide, (1S,8aS)-N-((R)-1-(3,5-bis(trifluoromethyl)phenyl)ethyl)-1-(4-fluoro-2-methylphenyl)-N-methyl-6-oxohexahydropyrrolo[1,2-a]pyrazine-2(1H)-carboxamide, (1S,8aS)-1-(4-fluoro-2-methylphenyl)-N-methyl-N-((R)-1-(3-methyl-5-(trifluoromethyl) phenyl)ethyl)-6-oxohexahydropyrrolo[1,2-a]pyrazine-2(1H)-carboxamide, (1S,8aS)-1-(4-fluoro-2-methylphenyl)-N-(1-(3-isopropyl-5-(trifluoromethyl)phenyl)ethyl)-N-methyl-6-oxohexahydropyrrolo[1,2-a]pyrazine-2(1H)-carboxamide, (1S,8aS)-N-((R)-1-(3-ethyl-5-(trifluoromethyl)phenyl)ethyl)-1-(4-fluoro-2-methylphenyl)-N-methyl-6-oxohexahydropyrrolo[1,2-a]pyrazine-2(1H)-carboxamide, (1S,8aS)-N-((R)-1-(3-ethyl-5-(trifluoromethyl)phenyl)ethyl)-1-(4-fluoro-2-methylphenyl)-7,7-bis(2-hydroxyethyl)-N-methyl-6-oxohexahydropyrrolo[1,2-a]pyrazine-2(1H)-carboxamide, (1S,8aS)-1-(4-fluoro-2-methylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide, (1S,8aS)-1-(4-(benzyloxy)-2-methylphenyl)-N-((R)-1-(3,5-bis(trifluoromethyl)phenyl) ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(4-hydroxy-2-methylphenyl)-N-methyl-6-oxohexahydropyrrolo[1,2-a]pyrazine-2(1H)-carboxamide, (1S,8aS)-N-((R)-1-(3-ethyl-5-(trifluoromethyl)phenyl)ethyl)-1-(4-hydroxy-2-methylphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-7,7-bis(2-hydroxyethyl)-1-(4-methoxy-2-methylphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(4-hydroxy-2-methylphenyl)-7,7-bis(2-hydroxyethyl)-N-methyl-6-oxo-hexahydropyrrolo[1,2-a]pyrazine-2(1H)-carboxamide, (1S,8aS)-N-((R)-1-(3-ethyl-5-(trifluoromethyl)phenyl)ethyl)-1-(4-hydroxy-2-methylphenyl)-7,7-bis(2-hydroxyethyl)-N-methyl-6-oxohexahydropyrrolo[1,2-a]pyrazine-2(1H)-carboxamide, (1S,8aS)-N-((R)-1-(4-ethyl-6-(trifluoromethyl)pyridin-2-yl)ethyl)-N-methyl-6-oxo-1-o-tolylhexahydropyrrolo[1,2-a]pyrazine-2(1H)-carboxamide,

(1S,8aS)-7,7-bis(2-hydroxyethyl)-N-methyl-N-((4-methyl-6-(trifluoromethyl)pyridin-2-yl)methyl)-6-oxo-1-o-tolylhexahydropyrrolo[1,2-a]pyrazine-2(1H)-carboxamide, (1S,8aS)-N-((R)-1-(3-chloro-5-(trifluoromethyl)phenyl)ethyl)-1-(2,4-dimethylphenyl)-7,7-bis(2-hydroxyethyl)-N-methyl-6-oxo-hexahydropyrrolo[1,2-a]pyrazine-2(1H)-carboxamide,
(1S,8aS)-1-(2,4-dimethylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-7,7-bis(2-hydroxyethyl)-1-(2-methoxyphenyl)-N-methyl-6-oxo-hexahydropyrrolo[1,2-a]pyrazine-2(1H)-carboxamide, (1S,8aS)-N-((R)-1-(3,5-bis(trifluoromethyl)phenyl)ethyl)-1-(2,5-dimethylphenyl)-7,7-bis(2-hydroxyethyl)-N-methyl-6-oxo-hexahydropyrrolo[1,2-a]pyrazine-2(1H)-carboxamide,
(1S,8aS)-1-(2,5-dimethylphenyl)-7,7-bis(2-hydroxyethyl)-N-((R)-1-(3-isopropyl-5-(trifluoromethyl)phenyl)ethyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2,3-dimethylphenyl)-7,7-bis(2-hydroxyethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(2,3-dimethylphenyl)-N-methyl-6-oxo-hexa hyd ropyrrolo[1,2-a]pyrazine-2(1H)-carboxamide, (1S,8aS)-1-(2,3-dimethylphenyl)-N-((R)-1-(3-ethyl-5-(trifluoromethyl)phenyl)ethyl)-N-methyl-6-oxo-hexahydropyrrolo[1,2-a]pyrazine-2(1H)-carboxamide,
(1S,8aS)-1-(2,3-dimethylphenyl)-N-((R)-1-(3-isopropyl-5-trifluoromethyl)phenyl)ethyl)-N-methyl-6-oxo-hexahydropyrrolo[1,2-a]pyrazine-2(1H)-carboxamide, (1R,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide,
(1S,8aS)-N-((R)-1-(3,5-bis(trifluoromethyl)phenyl)ethyl)-N-methyl-1-(3-methylpyridin-2-yl)-6-oxo-hexahydropyrrolo[1,2-a]pyrazine-2(1H)-carboxamide,
(1S,8aS)-N-((R)-1-(3,5-bis(trifluoromethyl)phenyl)ethyl)-N-methyl-1-(4-methylpyridin-3-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(3-methylpyridin-4-yl)-6-oxohexahydropyrrolo[1,2-a]pyrazine-2(1H)-carboxamide,
(1S,8aS)-N-((R)-1-(3,5-bis(trifluoromethyl)phenyl)ethyl)-N-methyl-1-(2-methylpyridin-3-yl)-6-oxo-hexahydropyrrolo[1,2-a]pyrazine-2(1H)-carboxamide, (1S,8aS)-N-((R)-1-(3,5-bis(trifluoromethyl)phenyl)ethyl)-N-methyl-1-(4-ethylthiophen-3-yl)-6-oxohexahydropyrrolo[1,2-a]pyrazine-2(1H)-carboxamide,
(1S,8aS)-N-((R)-1-(3-ethyl-5-(trifluoromethyl)phenyl)ethyl)-N-methyl-1-(4-methylthiophen-3-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-1-(3-ethylthiophen-2-yl)-6-oxohexahydropyrrolo[1,2-a]pyrazine-2(1H)-carboxamide, (1R,8aS)-N-((R)-1-(3-ethyl-5-(trifluoromethyl)phenyl)ethyl)-N-methyl-1-(3-methylthiophen-2-yl)-6-oxohexahydropyrrolo[1,2-a]pyrazine-2(1H)-carboxamide, (1R,8aS)-N-methyl-N-((R)-1-(3-methyl-5-(trifluoromethyl)phenyl)ethyl)-1-(3-methylthiophen-2-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-6-oxo-1-(thiophen-2-yl)hexahydropyrrolo[1,2-a]pyrazine-2(1H)-carboxamide,
(1R,8aS)-N-((R)-1-(3-ethyl-5-(trifluoromethyl)phenyl)ethyl)-N-methyl-6-oxo-1-(thiophen-2-yl)hexahydropyrrolo[1,2-a]pyrazine-2(1H)-carboxamide (1S,8aS)-N-methyl-N-((R)-1-(3-methyl-5-(trifluoromethyl)phenyl)ethyl)-1-(2-methylthiophen-3-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-N-methyl-1-(2-methylthiophen-3-yl)-6-oxohexahydropyrrolo[1,2-a]pyrazine-2(1H)-carboxamide, (1S,8aS)-N-((R)-1-(3,5-bis(trifluoromethyl)phenyl)ethyl)-N-methyl-1-(2-methylthiophen-3-yl)-6-oxohexahydropyrrolo[1,2-a]pyrazine-2(1H)-carboxamide, (1R,8aS)-N-((R)-1-(3,5-bis(trifluoromethyl)phenyl)ethyl)-N-methyl-1-(2-methylthiazol-5-yl)-6-oxohexahydropyrrolo[1,2-a]pyrazine-2(1H)-carboxamide,
(1R,8aS)-N-((R)-1-(3,5-bis(trifluoromethyl)phenyl)ethyl)-N-methyl-6-oxo-1-(thiazol-5-yl)-hexahydropyrrolo[1,2-a]pyrazine-2(1H)-carboxamide,
(1S,8aS)-N-((R)-1-(3,5-bis(trifluoromethyl)phenyl)ethyl)-N-methyl-1-(3-methyl-1H-pyrazol-4-yl)-6-oxohexahydropyrrolo[1,2-a]pyrazine-2(1H)-carboxamide (1S,8aS)-N-((R)-1-(3-ethyl-5-(trifluoromethyl)phenyl)ethyl)-N-methyl-1-(3-methyl-1H-pyrazol-4-yl)-6-oxohexahydropyrrolo[1,2-a]pyrazine-2(1H)-carboxamide, (1S,8aS)-N-((R)-1-(3-ethyl-5-(trifluoromethyl)phenyl)ethyl)-7,7-bis(2-hydroxyethyl)-N-methyl-1-(3-methyl-1H-pyrazol-4-yl)-6-oxohexahydropyrrolo[1,2-a]pyrazine-2(1H)-carboxamide,
(1S,8aS)-7,7-bis(2-hydroxyethyl)-N-methyl-1-(3-methyl-1H-pyrazol-4-yl)-N-((R)-1-(3-methyl-5-(trifluoromethyl)phenyl)ethyl)-6-oxo-hexahydropyrrolo[1,2-a]pyrazine-2(1H)-carboxamide,
(1S,8aS)-N-((R)-1-(3,5-bis(trifluoromethyl)phenyl)ethyl)-1-(1,3-dimethyl-1H-pyrazol-4-yl)-N-methyl-6-oxohexahydropyrrolo[1,2-a]pyrazine-2(1H)-carboxamide, (1S,8aS)-1-(2,3-dimethylphenyl)-N-((R)-1-(3-methyl-5-trifluoromethyl)phenyl)ethyl)-N-methyl-6-oxo-hexahydropyrrolo[1,2-a]pyrazine-2(1H)-carboxamide,
(1S,8aS)-N-((R)-1-(3,5-bis(trifluoromethyl)phenyl)ethyl)-1-(3-chloro-2-methylphenyl)-N-methyl-6-oxohexahydropyrrolo[1,2-a]pyrazine-2(1H)-carboxamide, N-((R)-1-(3,5-bis(trifluoromethyl)phenyl)ethyl)-1-(2-ethylphenyl)-N-methyl-6-oxohexahydropyrrolo[1,2-a]pyrazine-2(1H)-carboxamide,
25N-((R)-1-(3-ethyl-5-(trifluoromethyl)phenyl)ethyl)-1-(2-ethylphenyl)-N-methyl-6-oxohexahydropyrrolo[1,2-a]pyrazine-2(1H)-carboxamide,

N-((R)-1-(3,5-bis(trifluoromethyl)phenyl)ethyl)-1-(2-chlorophenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2-(hydroxymethyl)phenyl)-N-methyl-6-oxohexahydropyrrolo[1,2-a]pyrazine-2(1H)-carboxamide, (1R,8aS)-N-((R)-1-(3,5-bis(trifluoromethyl)phenyl)ethyl)-1-(3,4-dimethylthiophen-2-yl)-N-methyl-6-oxohexahydropyrrolo[1,2-a]pyrazine-2(1H)-carboxamide, (1R,8aS)-1-(3,4-dimethylthiophen-2-yl)-N-((R)-1-(3-ethyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(3-methoxy-2-methylphenyl)-N-methyl-6-oxohexahydropyrrolo[1,2-a]pyrazine-2(1H)-carboxamide, (1S,8aS)-N-((R)-1-(3-isopropyl-5-(trifluoromethyl)phenyl)ethyl)-1-(3-methoxy-2-methylphenyl)-N-methyl-6-oxohexahydropyrrolo[1,2-a]pyrazine-2(1H)-carboxamide, (1S,8aS)-N-((R)-1-(3,5-bis(trifluoromethyl)phenyl)ethyl)-1-(2,3-dihydro-1H-inden-4-yl)-N-methyl-6-oxohexahydropyrrolo[1,2-a]pyrazine-2(1H)-carboxamide, (1S,8aS)-1-(2,3-dihydro-1H-inden-4-yl)-N-((R)-1-(3-ethyl-5-(trifluoromethyl)phenyl)ethyl)-N-methyl-6-oxohexahydropyrrolo[1,2-a]pyrazine-2(1H)-carboxamide,
(1S,8aS)-1-(2,3-dihydro-1H-inden-4-yl)-N-((R)-1-(3-isopropyl-5-(trifluoromethyl)phenyl)ethyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(2-fluoro-6-methylphenyl)-N-methyl-6-oxohexahydropyrrolo[1,2-a]pyrazine-2(1H)-carboxamide, N-((R)-1-(3,5-bis(trifluoromethyl)phenyl)ethyl)-1-(2-chloro-4-fluorophenyl)-N-methyl-6-oxohexahydropyrrolo[1,2-a]pyrazine-2(1H)-carboxamide,
(1S,8aS)-N-((R)-1-(3,5-bis(trifluoromethyl)phenyl)ethyl)-1-(4-fluorophenyl)-N-methyl-6-oxohexahydropyrrolo[1,2-a]pyrazine-2(1H)-carboxamide
or a pharmaceutically acceptable salt or solvate thereof.

**12.** A compound according to any one of claims 1-11 for use in therapy.

**13.** A compound according to claims 1-11 for use in the prevention, treatment or amelioration of pruritic skin conditions , e.g. acute pruritus in any condition; chronic pruritus on diseased skin, such as inflammatory, infectious, or autoimmune cutaneous diseases, genodermatoses, drug reactions, dermatoses of pregnancy and skin lymphomas, prurigo , lichen planus, atopic dermatitis, eczema, contact dermatitis, allergic dermatitis, nummular dermatitis, lichen simplex, psoriasis, Sézary syndrome, cutaneous lymphomas, bullous pemphigoid, alopecia areata, scabies, vitiligo, urticaria and drug-induced pruritus; pruritic diseases on non-diseased skin of systemic, neurological or psychosomatic/psychiatric origin, including endocrine and metabolic disorders, infections, haematological and lymphoproliferative diseases, solid neoplasms and drug-induced pruritus; mastocytosis; pruritus of unknown cause; pruritus with chronic secondary scratch lesions, such as prurigo nodularis, and all types of prurigo; or any other dermal disease or condition **characterized by** pruritus.

**14.** The compound according to claim 13 wherein the pruritic skin condition is selected from prurigo , lichen planus, atopic dermatitis, eczema, contact dermatitis, allergic dermatitis, nummular dermatitis, lichen simplex, psoriasis, Sézary syndrome, cutaneous lymphomas, urticaria, mastocytosis and pruritus with chronic secondary scratch lesions.

**15.** A pharmaceutical composition comprising, as a therapeutically active ingredient, a compound according to any one of claims 1-11 and a pharmaceutically acceptable carrier or vehicle.

**16.** The pharmaceutical composition according to claim 15 together with one or more other therapeutically active compound(s).

**17.** The pharmaceutical composition according to claims 15-16 suitable for topical administration.

**18.** A compound selected from the list consisting of
1-(2,4-dimethylphenyl)hexahydropyrrolo[1,2-a]pyrazin-6(7H)-one, 7,7-diallyl-1-(2,4-dimethylphenyl)hexahydropyrrolo[1,2-a]pyrazin-6(7H)-one, 7,7-diallyl-1-(4-fluoro-2-methylphenyl)hexahydropyrrolo[1,2-a]pyrazin-6(7H)-one, 1-(4-methoxy-2-methylphenyl)hexahydropyrrolo[1,2-a]pyrazin-6(7H)-one, 1-(4-fluoro-2-methylphenyl)hexahydropyrrolo[1,2-a]pyrazin-6(7*H*)-one, 1-(4-methoxy-2-methylphenyl)hexahydropyrrolo[1,2-a]pyrazin-6(7*H*)-one, 7,7-diallyl-1-(4-methoxy-2-methylphenyl)hexahydropyrrolo[1,2-a]pyrazin-6(7H)-one, 1-(4-(benzyloxy)-2-methylphenyl)hexahydropyrrolo[1,2-a]pyrazin-6(7*H*)-one, 7,7-diallyl-1-(4-(benzyloxy)-2-methylphenyl)hexahydropyrrolo[1,2-a]pyrazin-6(7H)-one, tert-butyl7,7-diallyl-1-(2,5-dimethylphenyl)-6-oxo-hexahydropyrrolo[1,2-a]pyrazine-2(1H)-carboxylate,
7,7-diallyl-1-(2,3-dimethylphenyl)hexahydropyrrolo[1,2-a]pyrazin-6(7H)-one, 1-(2,3-dimethylphenyl)-hexahydropyrrolo[1,2-a]pyrazin-6(7H)-one, 1-(3-methylpyridin-2-yl)-hexahydropyrrolo[1,2-a]pyrazin-6(7H)-one, 1-(4-methylpyridin-3-yl)hexahydropyrrolo[1,2-a]pyrazin-6(7H)-one, 1-(3-methylpyridin-4-yl)hexahydropyrrolo[1,2-a]pyrazin-6(7H)-one, 1-(2-methylpyridin-3-yl)hexahydropyrrolo[1,2-a]pyrazin-6(7H)-one, 1-(4-methylthiophen-3-yl)hexahydropyrrolo[1,2-a]pyrazin-6(7H)-one, 1-(3-methylthiophen-2-yl)hexahydropyrrolo[1,2-a]pyrazin-6(7H)-one, 1-(thiophen-2-yl)hexahydropyrrolo[1,2-a]pyrazin-6(7H)-one, 1-(2-methylthiophen-3-yl)hexahydropyrrolo[1,2-a]pyrazin-6(7H)-one, 1-(2-methylthiazol-5-yl)hexahydropyrrolo[1,2-a]pyrazin-6(7H)-one, 1-(thiazol-5-yl)hexahydropyrrolo[1,2-a]pyrazin-6(7H)-one, 1-(1-benzyl-3-methyl-1H-pyrazol-4-yl)hexahydropyrrolo[1,2-a]pyrazin-6(7H)-one, 7,7-diallyl-1-(1-benzyl-3-methyl-1H-pyrazol-4-yl)hexahydropyrrolo[1,2-a]pyrazin-6(7H)-one,
(1S,8aS)-1-(3-chloro-2-methylphenyl)hexahydropyrrolo[1,2-a]pyrazin-6(2H)-one, 1-(2-ethylphenyl)hexahydropyrrolo[1,2-a]pyrazin-6(7H)-one, 1-(2-chlorophenyl)hexahydropyrrolo[1,2-a]pyrazin-6(7H)-one,
(1S, 8aS)-1-(2-vinylphenyl)hexahydropyrrolo[1,2-a]pyrazin-6(7H)-one, (8aS)-1-(3,4-dimethylthiophen-2-yl)hexahydropyrrolo[1,2-a]pyrazin-6(7H)-one, (1S,8aS)-1-(3-methoxy-2-methylphenyl)hexahydropyrrolo[1,2-a]pyrazin-6(7H)-one,
(1S,8aS)-1-(2,3-dihydro-1H-inden-4-yl)hexahydropyrrolo[1,2-a]pyrazin-6(7H)-one, (1S,8aS)-7,7-diallyl-1-(2,3-dimethylphenyl)hexahydropyrrolo[1,2-a]pyrazin-6(7H)-one, 1-(2-chloro-4-fluorophenyl)hexahydropyrrolo[1,2-a]pyrazin-6(7H)-one, (1S,8aS)-1-(4-fluorophenyl)hexahydropyrrolo[1,2-a]pyrazin-6(7H)-one.

## Patentansprüche

**1.** Verbindung der allgemeinen Formel A

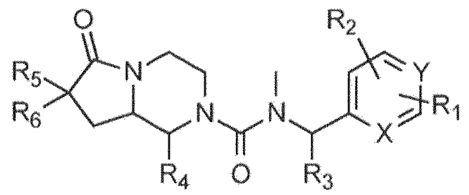

A

wobei

$R_1$ und $R_2$ unabhängig ausgewählt sind aus der Gruppe bestehend aus $(C_{1-4})$-Alkyl, $(C_{1-4})$-Haloalkyl, $(C_{1-4})$-Alkoxy, $CD_3$ oder Halogen;
$R_3$ ausgewählt ist aus der Gruppe bestehend aus Wasserstoff, $(C_{1-4})$-Alkyl, $(C_{1-4})$-Haloalkyl und $(C_{1-4})$-Hydroxyalkyl;
$R_4$ ausgewählt ist aus der Gruppe bestehend aus Phenyl, 5-gliedrigem Heteroaryl und 6-gliedrigem Heteroaryl, wobei das Phenyl und 6-gliedrige Heteroaryl mit einem oder mehreren Substituenten substituiert sind, die unabhängig aus $R_7$ ausgewählt sind, und das 5-gliedrige Heteroaryl optional mit einem oder mehreren Substituenten substituiert ist, die unabhängig aus $R_7$ ausgewählt sind; $R_5$ und $R_6$ unabhängig ausgewählt sind aus der Gruppe bestehend aus Wasserstoff, $(C_{1-4})$-Alkyl, $(C_{1-4})$-Hydroxyalkyl und $(C_{1-4})$-Haloalkyl;
$R_7$ ausgewählt ist aus der Gruppe bestehend aus Halogen, Hydroxy, $(C_{1-4})$-Alkyl, $(C_{1-4})$-Alkoxy, Aryloxy, Aryl-$(C_{1-4})$-alkoxy, Cyano, $(C_{1-4})$-Haloalkyl oder $(C_{1-4})$-Hydroxyalkyl, wobei zwei benachbarte $(C_{1-4})$-Alkyle gemeinsam mit dem Phenyl, 5-gliedrigen Heteroaryl oder 6-gliedrigen Heteroaryl, an das sie gebunden sind, optional einen 4- bis 7-gliedrigen aliphatischen Ring bilden können;
X und Y unabhängig ausgewählt sind aus der Gruppe bestehend aus CH und N;

oder pharmazeutisch unbedenkliches Salz oder Solvat davon;
mit der Maßgabe, dass dann, wenn sowohl X als auch Y CH sind, $R_4$ nicht 2-Methylphenyl ist.

**2.** Verbindung nach Anspruch 1 der allgemeinen Formel A(i)

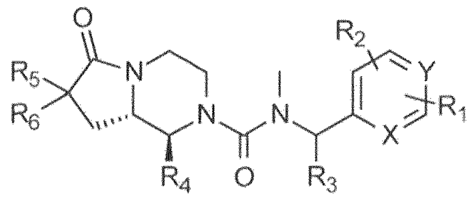

A(i)

wobei $R_1$, $R_2$, $R_3$, $R_4$, $R_5$, $R_6$, X und Y wie in Anspruch 1 angegeben sind, oder pharmazeutisch unbedenkliches Salz oder Solvat davon;
mit der Maßgabe, dass dann, wenn sowohl X als auch Y CH sind, $R_4$ nicht 2-Methylphenyl ist.

**3.** Verbindung nach Anspruch 1 der allgemeinen Formel A(ii)

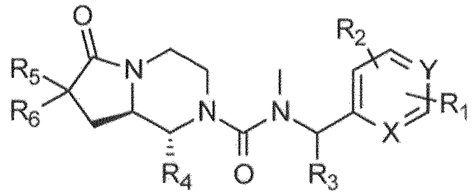

A(ii)

wobei $R_1$, $R_2$, $R_3$, $R_4$, $R_5$, $R_6$, X und Y wie in Anspruch 1 angegeben sind,
oder pharmazeutisch unbedenkliches Salz oder Solvat davon;
mit der Maßgabe, dass dann, wenn sowohl X als auch Y CH sind, $R_4$ nicht 2-Methylphenyl ist.

**4.** Verbindung nach einem der Ansprüche 1-3, wobei $R_1$ und $R_2$ für $(C_{1-4})$-Haloalkyl stehen.

**5.** Verbindung nach einem der Ansprüche 1-3, wobei $R_1$ für $(C_{1-4})$-Haloalkyl steht und $R_2$ für $(C_{1-4})$-Alkyl steht.

**6.** Verbindung nach einem der Ansprüche 1-5, wobei $R_3$ ausgewählt ist aus der Gruppe bestehend aus Wasserstoff und $(C_{1-4})$-Alkyl.

**7.** Verbindung nach einem der Ansprüche 1-6, wobei $R_5$ und $R_6$ unabhängig ausgewählt sind aus der Gruppe bestehend aus Wasserstoff und $(C_{1-4})$-Hydroxyalkyl

**8.** Verbindung nach einem der Ansprüche 1-7, wobei $R_7$ ausgewählt ist aus der Gruppe bestehend aus Halogen und $(C_{1-4})$-Alkyl.

**9.** Verbindung nach einem der Ansprüche 1-8, wobei X und Y für CH stehen.

**10.** Verbindung nach einem der Ansprüche 1-9, wobei $R_4$ Phenyl ist, wobei das Phenyl mit zwei Substituenten substituiert ist, die unabhängig aus $R_7$ ausgewählt sind.

**11.** Verbindung nach einem der Ansprüche 1-10, ausgewählt aus der Gruppe bestehend aus N-((R)-1-(3,5-Bis(trifluormethyl)phenyl)ethyl)-1-(2,4-dimethylphenyl)-N-methyl-6-oxohexahyd ropyrrolo[1,2-a]pyrazin-2(1H)-carboxamid,
N-(3,5-Bis(trifluormethyl)benzyl)-1-(2,4-dimethylphenyl)-N-methyl-6-oxohexahydropyrrolo[1,2 -a]pyrazin-2(1H)-carboxamid,
1-(2,4-Dimethylphenyl)-N-methyl-N-((R)-1-(3-methyl-5-(trifluormethyl)phenyl)ethyl)-6-oxohex ahydropyrrolo[1,2-a]pyrazin-2(1H)-carboxamid,
N-((R)-1-(3,5-Bis(trifluormethyl)phenyl)ethyl)-1-(2,4-dimethylphenyl)-7,7-bis(2-hydroxyethyl)-N-methyl-6-oxohexahydropyrrolo[1,2-a]pyrazin-2(1H)-carboxamid,
1-(2,4-Dimethylphenyl)-7,7-bis(2-hydroxyethyl)-N-methyl-N-((R)-1-(3-methyl-5-(trifluormethyl )phenyl)ethyl)-6-oxo-hexahydropyrrolo[1,2-a]pyrazin-2(1H)-carboxamid, N-(3,5-Bis(trifluormethyl)benzyl)-1-(2,4-dimethylphenyl)-7,7-bis(2-hydroxyethyl)-N-methyl-6-oxohexahydropyrrolo[1,2-a]pyrazin-2(1H)-carboxamid,
*N*-((R)-1-(3-Chlor-5-(trifluormethyl)phenyl)ethyl)-1-(4-fluor-2-methylphenyl)-7,7-bis(2-hydrox yethyl)-*N*-methyl-6-oxohexahydropyrrolo[1,2-a]pyrazin-2(1*H*)-carboxamid, 1-(4-Fluor-2-methylphenyl)-7,7-bis(2-hydroxyethyl)-*N*-methyl-*N*-((R)-1-(3-methyl-5-(trifluorme thyl)phenyl)ethyl)-6-oxohexahydropyrrolo[1,2-a]pyrazin-2(1*H*)-carboxamid,
*N*-((R)-1-(3,5-Bis(trifluoromethyl)phenyl)ethyl)-1-(4-fluor-2-methylphenyl)-7,7-bis(2-hydroxyet hyl)-*N*-methyl-6-oxo-hexahydropyrrolo[1,2-a]pyrazin-2(1*H*)-carboxamid,
N-((R)-1-(3,5-Bis(trifluormethyl)phenyl)ethyl)-7,7-bis(2-hydroxyethyl)-1-(4-methoxy-2-methyl phenyl)-N-methyl-6-oxohexahydropyrrolo[1,2-a]pyrazin-2(1H)-carboxamid, (1 S,8aS)-7,7-Bis(2-hydroxyethyl)-1-(4-methoxy-2-methylphenyl)-N-methyl-N-((R)-1-(3-methyl -5-(trifluormethyl)phenyl)ethyl)-6-oxohexahydropyrrolo[1,2-a]pyrazin-2(1H)-carboxamid,
*N*-((R)-1-(3,5-Bis(trifluormethyl)phenyl)ethyl)-1-(4-methoxy-2-methylphenyl)-*N*-methyl-6-oxoh exahydropyrrolo[11,2-a]pyrazin-2(1*H*)-carboxamid, (1S,8aS)-N-((R)-1-(3,5-Bis(trifluormethyl)phenyl)ethyl)-1-(4-fluor-2-methylphenyl)-N-methyl-6 -oxohexahydropyrrolo[1,2-a]pyrazin-2(1H)-carboxamid,
(1S,8aS)-1-(4-Fluor-2-methylphenyl)-N-methyl-N-((R)-1-(3-methyl-5-(trifluormethyl)phenyl)et hyl)-6-oxohexahydropyrrolo[1,2-a]pyrazin-2(1H)-carboxamid, (1S,8aS)-1-(4-Fluor-2-methylphenyl)-N-(1-(3-isopropyl-5-(trifluormethyl)phenyl)ethyl)-N-meth yl-6-oxohexahydropyrrolo[1,2-a]pyrazin-2(1H)-carboxamid,
(1S,8aS)-N-((R)-1-(3-Ethyl-5-(trifluormethyl)phenyl)ethyl)-1-(4-fluor-2-methylphenyl)-N-meth yl-6-oxohexahydropyrrolo[1,2-a]pyrazin-2(1 H)-carboxamid,
(1S,8aS)-N-((R)-1-(3-Ethyl-5-(trifluormethyl)phenyl)ethyl)-1-(4-fluor-2-methylphenyl)-7,7-bis( 2-hydroxyethyl)-N-methyl-6-oxohexahydropyrrolo[1,2-a]pyrazin-2(1H)-carboxamid,
(1S,8aS)-1-(4-Fluor-2-methylphenyl)-7,7-bis(2-hydroxyethyl)-N-((R)-1-(3-isopropyl-5-(trifluor methyl)phenyl)ethyl)-N-methyl-6-oxohexahydropyrrolo[1,2-a]pyrazin-2(1H)-carboxamid, (1S,8aS)-1-(4-(Benzyloxy)-2-methylphenyl)-N-((R)-1-(3,5-bis(trifluormethyl)phenyl)ethyl)-N-m ethyl-6-oxohexahydropyrrolo[1,2-a]pyrazin-2(1H)-carboxamid, (1S,8aS)-N-((R)-1-(3,5-Bis(trifluormethyl)phenyl)ethyl)-1-(4-hydroxy-2-methylphenyl)-N-meth yl-6-oxohexahydropyrrolo[1,2-a]pyrazin-2(1H)-carboxamid,
(1S,8aS)-N-((R)-1-(3-Ethyl-5-(trifluormethyl)phenyl)ethyl)-1-(4-hydroxy-2-methylphenyl)-N-m ethyl-6-oxohexahydropyrrolo[1,2-a]pyrazin-2(1H)-carboxamid,
(1 S,8aS)-N-((R)-1-(3,5-Bis(trifluormethyl)phenyl)ethyl)-7,7-bis(2-hydroxyethyl)-1-(4-methoxy-2-methylphenyl)-N-methyl-6-oxohexahydropyrrolo[1,2-a]pyrazin-2(1H)-carboxamid, (1S,8aS)-N-((R)-1-(3,5-Bis(trifluormethyl)phenyl)ethyl)-1-(4-hydroxy-2-methylphenyl)-7,7-bis( 2-hydroxyethyl)-N-methyl-6-oxo-hexahydropyrrolo[ 1,2-a]pyrazin-2(1H)-carboxamid, (1S,8aS)-N-((R)-1-(3-Ethyl-5-(trifluormethyl)phenyl)ethyl)-1-(4-hydroxy-2-methylphenyl)-7,7-b is(2-hydroxyethyl)-N-methyl-6-oxohexahydropyrrolo[1,2-a]pyrazin-2(1H)-carboxamid, (1S,8aS)-N-((R)-1-(4-Ethyl-6-(trifluormethyl)pyridin-2-yl)ethyl)-N-methyl-6-oxo-1-o-tolylhexa hydropyrrolo[1,2-a]pyrazin-2(1H)-carboxamid, (1S,8aS)-7,7-Bis(2-hydroxyethyl)-N-methyl-N-((4-methyl-6-(trifluormethyl)pyridin-2-yl)methyl )-6-oxo-1-o-tolylhexahydropyrrolo[1,2-a]pyrazin-2(1H)-carboxamid,
(1S,8aS)-N-((R)-1-(3-Chlor-5-(trifluormethyl)phenyl)ethyl)-1-(2,4-dimethylphenyl)-7,7-bis(2-hy droxyethyl)-N-methyl-6-oxo-hexahydropyrrolo[1,2-a]pyrazin-2(1H)-carboxamid, (1S,8aS)-1-(2,4-Dimethylphenyl)-7,7-bis(2-hydroxyethyl)-N-((R)-1-(3-isopropyl-5-(trifluormeth yl)phenyl)ethyl)-N-methyl-6-oxohexahydropyrrolo[1,2-a]pyrazin-2(1H)-carboxamid, (1S,8aS)-N-((R)-1-(3,5-Bis(trifluormethyl)phenyl)ethyl)-7,7-bis(2-hydroxyethyl)-1-(2-methoxyp henyl)-N-methyl-6-oxo-hexahydropyrrolo[1,2-a]pyrazin-2(1H)-carboxamid, (1S,8aS)-N-((R)-1-(3,5-Bis(trifluormethyl)phenyl)ethyl)-1-(2,5-dimethylphenyl)-7,7-bis(2-hydro xyethyl)-N-methyl-6-oxo-hexahydropyrrolo[1,2-a]pyrazin-2(1H)-carboxamid, (1S,8aS)-1-(2,5-Dimethylphenyl)-7,7-bis(2-hydroxyethyl)-N-((R)-1-(3-isopropyl-5-(trifluormeth yl)phenyl)ethyl)-N-methyl-6-oxohexahydropyrrolo[1,2-a]pyrazin-2(1H)-carboxamid, (1S,8aS)-N-((R)-1-(3,5-Bis(trifluormethyl)phenyl)ethyl)-1-(2,3-dimethylphenyl)-7,7-bis(2-hydro xyethyl)-N-methyl-6-oxohexahydropyrrolo[1,2-a]pyrazin-2(1H)-carboxamid,
(1S,8aS)-N-((R)-1-(3,5-Bis(trifluormethyl)phenyl)ethyl)-1-(2,3-dimethylphenyl)-N-methyl-6-ox o-hexahydropyrrolo[1,2-a]pyrazin-2(1H)-carboxamid,
(1S,8aS)-1-(2,3-Dimethylphenyl)-N-((R)-1-(3-ethyl-5-(trifluormethyl)phenyl)ethyl)-N-methyl-6-oxo-hexahydropyrrolo[1,2-a]pyrazin-2(1H)-carboxamid,
(1S,8aS)-1-(2,3-Dimethylphenyl)-N-((R)-1-(3-isopropyl-5-trifluormethyl)phenyl)ethyl)-N-methy 1-6-oxo-hexahydropyrrolo[1,2-a]pyrazin-2(1H)-carboxamid,
(1R,8aS)-N-((R)-1-(3,5-Bis(trifluormethyl)phenyl)ethyl)-N-methyl-1-(3-methylpyridin-2-yl)-6-o xo-hexahydropyrrolo[1,2-a]pyrazin-2(1H)-carboxamid, (1S,8aS)-N-((R)-1-(3,5-Bis(trifluormethyl)phenyl)ethyl)-N-methyl-1-(3-methylpyridin-2-yl)-6-o xo-hexahydropyrrolo[1,2-a]pyrazin-2(1H)-carboxamid, (1S,8aS)-N-((R)-1-(3,5-Bis(trifluormethyl)phenyl)ethyl)-N-methyl-1-(4-methylpyridin-3-yl)-6-o xo-hexahydropyrrolo[1,2-a]pyrazin-2(1H)-carboxamid, (1S,8aS)-N-((R)-1-(3,5-Bis(trifluormethyl)phenyl)ethyl)-N-methyl-1-(3-methylpyridin-4-yl)-6-o xohexahydropyrrolo[1,2-a]pyrazin-2(1H)-carboxamid, (1S,8aS)-N-((R)-1-(3,5-Bis(trifluormethyl)phenyl)ethyl)-N-methyl-1-(2-methylpyridin-3-yl)-6-o xo-hexahydropyrrolo[1,2-a]pyrazin-2(1H)-carboxamid, (1S,8aS)-N-((R)-1-(3,5-Bis(trifluormethyl)phenyl)ethyl)-N-methyl-1-(4-ethylthiophen-3-yl)-6-o xohexahydropyrrolo[1,2-a]pyrazin-2(1H)-carboxamid, (1S,8aS)-N-((R)-1-(3-Ethyl-5-(trifluormethyl)phenyl)ethyl)-N-methyl-1-(4-methylthiophen-3-yl) -6-oxohexahydropyrrolo[1,2-a]pyrazin-2(1H)-carboxamid,
(1R,8aS)-N-((R)-1-(3,5-Bis(trifluormethyl)phenyl)ethyl)-N-methyl-1-(3-ethylthiophen-2-yl)-6-o xohexahydropyrrolo[1,2-a]pyrazin-2(1H)-carboxamid,
(1R,8aS)-N-((R)-1-(3-Ethyl-5-(trifluormethyl)phenyl)ethyl)-N-methyl-1-(3-methylthiophen-2-yl )-6-oxohexahydropyrrolo[1,2-a]pyrazin-2(1H)-carboxamid, (1R,8aS)-N-Methyl-N-((R)-1-(3-methyl-5-(trifluormethyl)phenyl)ethyl)-1-(3-methylthiophen-2-y])-6-oxohexahydropyrrolo[1,2-a]pyrazin-2(1H)-carboxamid, (1R,8aS)-N-((R)-1-(3,5-Bis(trifluormethyl)phenyl)ethyl)-N-methyl-6-oxo-1-(thiophen-2-yl)hexa hydropyrrolo[1,2-a]pyrazin-2(1H)-carboxamid, (1R,8aS)-N-((R)-1-(3-Ethyl-5-(trifluormethyl)phenyl)ethyl)-N-methyl-6-oxo-1-(thiophen-2-yl)h exahydropyrrolo[1,2-a]pyrazin-2(1H)-carboxamid
(1S,8aS)-N-Methyl-N-((R)-1-(3-methyl-5-(trifluormethyl)phenyl)ethyl)-1-(2-methylthiophen-3-yl)-6-oxohexahydropyrrolo[1,2-a]pyrazin-2(1H)-carboxamid,
(1 S,8aS)-N-((R)-1-(3-Ethyl-5-(trifluormethyl)phenyl)ethyl)-N-methyl-1-(2-methylthiophen-3-yl) -6-oxohexahydropyrrolo[1,2-a]pyrazin-2(1H)-carboxamid,
(1S,8aS)-N-((R)-1-(3,5-Bis(trifluormethyl)phenyl)ethyl)-N-methyl-1-(2-methylthiophen-3-yl)-6-oxohexahydropyrrolo[1,2-a]pyrazin-2(1H)-carboxamid, (1R,8aS)-N-((R)-1-(3,5-Bis(trifluormethyl)phenyl)ethyl)-N-methyl-1-(2-methylthiazol-5-yl)-6-o xohexahydropyrrolo[1,2-a]pyrazin-2(1H)-carboxamid, (1R,8aS)-N-((R)-1-(3,5-Bis(trifluormethyl)phenyl)ethyl)-N-methyl-6-oxo-1-(thiazol-5-yl)-hexah ydropyrrolo[1,2-a]pyrazin-2(1 H)-carboxamid,
(1S,8aS)-N-((R)-1-(3,5-Bis(trifluormethyl)phenyl)ethyl)-N-methyl-1-(3-methyl-1H-pyrazol-4-yl )-6-oxohexahydropyrrolo [1,2-a]pyrazin-2(1H)-carboxamid (1S,8aS)-N-((R)-1-(3-Ethyl-5-(trifluormethyl)phenyl)ethyl)-N-methyl-1-(3-methyl-1H-pyrazol-4 -yl)-6-oxohexahydropyrrolo[1,2-a]pyrazin-2(1H)-carboxamid,
(1S,8aS)-N-((R)-1-(3-Ethyl-5-(trifluormethyl)phenyl)ethyl)-7,7-bis(2-hydroxyethyl)-N-methyl-1 -(3-methyl-1H-pyrazol-4-yl)-6-oxohexahydropyrrolo[1,2-a]pyrazin-2(1H)-carboxamid,
(11S,8aS)-7,7-Bis(2-hydroxyethyl)-N-methyl-1-(3-methyl-1H-pyrazol-4-yl)-N-((R)-1-(3-methyl-5-(trifluormethyl)phenyl)ethyl)-6-oxo-hexahydropyrrolo[1,2-a]pyrazin-2(1H)-carboxamid, (1S,8aS)-N-((R)-1-(3,5-Bis(trifluormethyl)phenyl)ethyl)-1-(1,3-dimethyl-1H-pyrazol-4-yl)-N-m ethyl-6-oxohexahydropyrrolo[1,2-a]pyrazin-2(1H)-carboxamid,
(1S,8aS)-1-(2,3-Dimethylphenyl)-N-((R)-1-(3-methyl-5-trifluormethyl)phenyl)ethyl)-N-methyl-6-oxo-hexahydropyrrolo[1,2-a]pyrazin-2(1H)-carboxamid,
(1S,8aS)-N-((R)-1-(3,5-Bis(trifluormethyl)phenyl)ethyl)-1-(3-chlor-2-methylphenyl)-N-methyl-6-oxohexahydropyrrolo[1,2-a]pyrazin-2(1H)-carboxamid, N-((R)-1-(3,5-Bis(trifluormethyl)phenyl)ethyl)-1-(2-ethylphenyl)-N-methyl-6-oxohexahydropyrr olo[1,2-a]pyrazin-2(1H)-carboxamid,
25N-((R)-1-(3-Ethyl-5-(trifluormethyl)phenyl)ethyl)-1-(2-ethylphenyl)-N-methyl-6-oxohexahyd ropyrrolo[1,2-a]pyrazin-2(1H)-carboxamid,
N-((R)-1-(3,5-Bis(trifluormethyl)phenyl)ethyl)-1-(2-chlorphenyl)-N-methyl-6-oxohexahydropyr rolo[1,2-a]pyrazin-2(1H)-carboxamid,
(1S,8aS)-N-((R)-1-(3,5-Bis(trifluormethyl)phenyl)ethyl)-1-(2-(hydroxymethyl)phenyl)-N-methyl -6-oxohexahydropyrrolo[1,2-a]pyrazin-2(1H)-carboxamid,
(1R,8aS)-N-((R)-1-(3,5-Bis(trifluormethyl)phenyl)ethyl)-1-(3,4-dimethylthiophen-2-yl)-N-meth yl-6-oxohexahydropyrrolo[1,2-a]pyrazin-2(1H)-carboxamid, (1R,8aS)-1-(3,4-Dimethylthiophen-2-yl)-N-((R)-1-(3-ethyl-5-(trifluormethyl)phenyl)ethyl)-N-m ethyl-6-oxohexahydropyrrolo[1,2-a]pyrazin-2(1H)-carboxamid,
(1S,8aS)-N-((R)-1-(3,5-Bis(trifluormethyl)phenyl)ethyl)-1-(3-methoxy-2-methylphenyl)-N-meth yl-6-oxohexahydropyrrolo[1,2-a]pyrazin-2(1H)-carboxamid,
(1S,8aS)-N-((R)-1-(3-Isopropyl-5-(trifluormethyl)phenyl)ethyl)-1-(3-methoxy-2-methylphenyl)-N-methyl-6-oxohexahydropyrrolo[1,2-a]pyrazin-2(1H)-carboxamid,
(1S,8aS)-N-((R)-1-(3,5-Bis(trifluormethyl)phenyl)ethyl)-1-(2,3-dihydro-1H-inden-4-yl)-N-meth yl-6-oxohexahydropyrrolo[1,2-a]pyrazin-2(1H)-carboxamid,
(1S,8aS)-1-(2,3-Dihydro-1H-inden-4-yl)-N-((R)-1-(3-ethyl-5-(trifluormethyl)phenyl)ethyl)-N-m ethyl-6-oxohexahydropyrrolo[1,2-a]pyrazin-2(1H)-carboxamid,
(1S,8aS)-1-(2,3-Dihydro-1H-inden-4-yl)-N-((R)-1-(3-isopropyl-5-(trifluormethyl)phenyl)ethyl)-N-methyl-6-oxohexahydropyrrolo[1,2-a]pyrazin-2(1H)-carboxamid,
(1 S,8aS)-N-((R)-1-(3,5-Bis(trifluormethyl)phenyl)ethyl)-1-(2-fluor-6-methylphenyl)-N-methyl-6 -oxohexahydropyrrolo[1,2-a]pyrazin-2(1H)-carboxamid,
N-((R)-1-(3,5-Bis(trifluormethyl)phenyl)ethyl)-1-(2-chlor-4-fluorphenyl)-N-methyl-6-oxohexah ydropyrrolo[1,2-a]pyrazin-2(1H)-carboxamid, (1S,8aS)-N-((R)-1-(3,5-Bis(trifluormethyl)phenyl)ethyl)-1-(4-fluorphenyl)-N-methyl-6-oxohexa hydropyrrolo[1,2-a]pyrazin-2(1H)-carboxamid oder pharmazeutisch unbedenkliches Salz oder Solvat davon.

**12.** Verbindung nach einem der Ansprüche 1-11 zur Verwendung bei der Therapie.

**13.** Verbindung nach Ansprüchen 1-11 zur Verwendung bei der Vorbeugung, Behandlung oder Linderung von pruriginösen Hautzuständen, z. B. akutem Pruritus in jedem Zustand; chronischem Pruritus an erkrankter Haut, wie etwa entzündlichen, infektiösen, oder autoimmunen Hautkrankheiten, Genodermatosen, Reaktionen auf Arzneimittel, Dermatosen in der Schwangerschaft und bei Hautlymphomen, Prurigo, Lichen planus, atopischer Dermatitis, Ekzem, Kontaktdermatitis, allergischer Dermatitis, nummulärer Dermatitis, Lichen simplex, Psoriasis, Sézary-Syndrom, kutanen Lymphomen, bullösem Pemphigoid, Alopecia areata, Krätze, Vitiligo, Urtikaria und arzneimittelinduziertem Pruritus; pruriginösen Erkrankungen an nicht erkrankter Haut systemischen, neurologischen oder psychosomatischen/psychiatrischen Ursprungs, einschließlich endokriner und metabolischer Störungen, Infektionen, hämatologischen und lymphoproliferativen Erkrankungen, soliden Neoplasmen und arzneimittelinduziertem Pruritus; Mastozytose; Pruritus unbekannter Ursache; Pruritus mit chronischen sekundären Kratzläsionen, wie etwa Prurigo nodularis, und allen Arten von Prurigo; oder jeder anderen Hauterkrankung oder eines Zustands, der durch Pruritus gekennzeichnet ist.

**14.** Verbindung nach Anspruch 13, wobei der pruriginöse Hautzustand ausgewählt ist aus Prurigo, Lichen planus, atopischer Dermatitis, Ekzem, Kontaktdermatitis, allergischer Dermatitis, nummulärer Dermatitis, Lichen simplex, Psoriasis, Sézary-Syndrom, kutanen Lymphomen, Urtikaria, Mastozytose und Prurigo mit chronischen sekundären Kratzläsionen.

**15.** Pharmazeutische Zusammensetzung, die als therapeutisch wirksamen Inhaltsstoff eine Verbindung nach einem der Ansprüche 1-11 und einen pharmazeutisch unbedenklichen Träger oder ein pharmazeutisch unbedenkliches Vehikel umfasst.

**16.** Pharmazeutische Zusammensetzung nach Anspruch 15 gemeinsam mit einer oder mehreren anderen therapeutisch wirksamen Verbindung(en).

**17.** Pharmazeutische Zusammensetzung nach Ansprüchen 15-16, die zur topischen Verabreichung geeignet ist.

**18.** Verbindung, ausgewählt aus der Liste bestehend aus
1-(2,4-Dimethylphenyl)hexahydropyrrolo[1,2-a]pyrazin-6(7H)-on, 7,7-Diallyl-1-(2,4-dimethylphenyl)hexahydropyrrolo[1,2-a]pyrazin-6(7H)-on, 7,7-Diallyl-1-(4-fluor-2-methylphenyl)hexahydropyrrolo[1,2-a]pyrazin-6(7H)-on, 1-(4-Methoxy-2-methylphenyl)hexahydropyrrolo[1,2-a]pyrazin-6(7H)-on, 1-(4-Fluor-2-methylphenyl)hexahydropyrrolo[1,2-a]pyrazin-6(7*H*)-on, 1-(4-Methoxy-2-methylphenyl)hexahydropyrrolo[1,2-a]pyrazin-6(7*H*)-on, 7,7-Diallyl-1-(4-methoxy-2-methylphenyl)hexahydropyrrolo[1,2-a]pyrazin-6(7H)-on, 1-(4-(Benzyloxy)-2-methylphenyl)hexahydropyrrolo[1,2-a]pyrazin-6(7*H*)-on, 7,7-Diallyl-1-(4-(benzyloxy)-2-methylphenyl)hexahydropyrrolo[1,2-a]pyrazin-6(7H)-on, tert-Butyl-7,7-diallyl-1-(2,5-dimethylphenyl)-6-oxo-hexahydropyrrolo[1,2-a]pyrazin-2(1H)-carb oxylat,
7,7-Diallyl-1-(2,3-dimethylphenyl)hexahydropyrrolo[1,2-a]pyrazin-6(7H)-on, 1-(2,3-Dimethylphenyl)-hexahydropyrrolo[1,2-a]pyrazin-6(7H)-on, 1-(3-Methylpyridin-2-yl)-hexahydropyrrolo[1,2-a]pyrazin-6(7H)-on, 1-(4-Methylpyridin-3-yl)hexahydropyrrolo[1,2-a]pyrazin-6(7H)-on, 1-(3-Methylpyridin-4-yl)hexahydropyrrolo[1,2-a]pyrazin-6(7H)-on, 1-(2-Methylpyridin-3-yl)hexahydropyrrolo[1,2-a]pyrazin-6(7H)-on, 1-(4-Methylthiophen-3-yl)hexahydropyrrolo[1,2-a]pyrazin-6(7H)-on, 1-(3-Methylthiophen-2-yl)hexahydropyrrolo[1,2-a]pyrazin-6(7H)-on, 1-(Thiophen-2-yl)hexahydropyrrolo[1,2-a]pyrazin-6(7H)-on, 1-(2-Methylthiophen-3-yl)hexahydropyrrolo[1,2-a]pyrazin-6(7H)-on, 1-(2-Methylthiazol-5-yl)hexahydropyrrolo[1,2-a]pyrazin-6(7H)-on, 1-(Thiazol-5-yl)hexahydropyrrolo[1,2-a]pyrazin-6(7H)-on,
1-(1-Benzyl-3-methyl-1H-pyrazol-4-yl)hexahydropyrrolo[1,2-a]pyrazin-6(7H)-on, 7,7-Diallyl-1-(1-benzyl-3-methyl-1H-pyrazol-4-yl)hexahydropyrrolo[1,2-a]pyrazin-6(7H)-on, (1S,8aS)-1-(3-Chlor-2-methylphenyl)hexahydropyrrolo[1,2-a]pyrazin-6(2H)-on, 1-(2-Ethylphenyl)hexahydropyrrolo[1,2-a]pyrazin-6(7H)-on, 1-(2-Chlorphenyl)hexahydropyrrolo[1,2-a]pyrazin-6(7H)-on,
(1S,8aS)-1-(2-Vinylphenyl)hexahydropyrrolo[1,2-a]pyrazin-6(7H)-on, (8aS)-1-(3,4-Dimethylthiophen-2-yl)hexahydropyrrolo[1,2-a]pyrazin-6(7H)-on, (1S,8aS)-1-(3-Methoxy-2-methylphenyl)hexahydropyrrolo[1,2-a]pyrazin-6(7H)-on, (1S,8aS)-1-(2,3-Dihydro-1H-inden-4-yl)hexahydropyrrolo[1,2-a]pyrazin-6(7H)-on, (1S,8aS)-7,7-Diallyl-1-(2,3-dimethylphenyl)hexahydropyrrolo[1,2-a]pyrazin-6(7H)-on, 1-(2-Chlor-4-fluorphenyl)hexahydropyrrolo[1,2-a]pyrazin-6(7H)-on,
(1S,8aS)-1-(4-Fluorphenyl)hexahydropyrrolo[1,2-a]pyrazin-6(7H)-on.

## Revendications

**1.** Composé de formule générale A

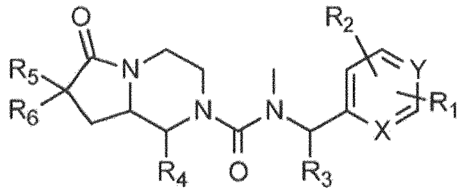

A

dans laquelle

$R_1$ et $R_2$ sont choisis indépendamment parmi le groupe constitué par alkyle en ($C_{1-4}$), haloalkyle en ($C_{1-4}$), alcoxy en ($C_{1-4}$), $CD_3$ ou halogène ;
$R_3$ est choisi parmi le groupe constitué par hydrogène, alkyle en ($C_{1-4}$), haloalkyle en ($C_{1-4}$) et hydroxyalkyle en ($C_{1-4}$) ;
$R_4$ est choisi parmi le groupe constitué par phényle, hétéroaryle à 5 membres et hétéroaryle à 6 membres, lesdits phényle et hétéroaryle à 6 membres étant substitués par un ou plusieurs substituants choisis indépendamment parmi $R_7$, et ledit hétéroaryle à 5 membres étant facultativement substitué par un ou plusieurs substituants choisis indépendamment parmi $R_7$ ;
$R_5$ et $R_6$ sont choisis indépendamment parmi le groupe constitué par hydrogène, alkyle en ($C_{1-4}$), hydroxyalkyle en ($C_{1-4}$) et haloalkyle en ($C_{1-4}$);
$R_7$ est choisi parmi le groupe constitué par halogène, hydroxy, alkyle en ($C_{1-4}$), alcoxy en ($C_{1-4}$), aryloxy, alcoxy aryle en ($C_{1-4}$), cyano, haloalkyle en ($C_{1-4}$) ou hydroxyalkyle en ($C_{1-4}$), deux alkyles en ($C_{1-4}$) adjacents, conjointement avec le phényle, l'hétéroaryle à 5 membres ou l'hétéroaryle à 6 membres auquel ils sont attachés pouvant facultativement former un cycle aliphatique à 4 à 7 membres ;
X et Y sont choisis indépendamment parmi le groupe constitué par CH et N ; ou sel ou solvate pharmaceutiquement acceptable de celui-ci ;
à la condition que lorsque X et Y sont conjointement CH, $R_4$ ne soit pas le 2-méthylphényle.

**2.** Composé selon la revendication 1 de formule générale A(i)

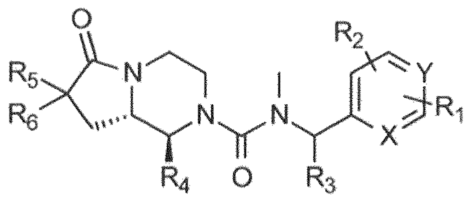

A(i)

dans laquelle $R_1$, $R_2$, $R_3$, $R_4$, $R_5$, $R_6$, X et Y sont tels qu'indiqués dans la revendication 1,
ou sel ou solvate pharmaceutiquement acceptable de celui-ci ;
à la condition que lorsque X et Y sont conjointement CH, $R_4$ ne soit pas le 2-méthylphényle.

**3.** Composé selon la revendication 1 de formule générale A(ii)

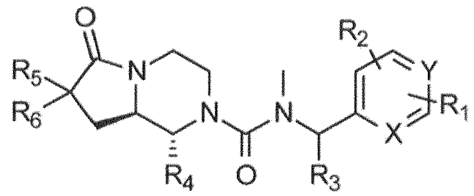

A(ii)

dans laquelle $R_1$, $R_2$, $R_3$, $R_4$, $R_5$, $R_6$. X et Y sont tels qu'indiqués dans la revendication 1, ou sel ou solvate pharmaceutiquement acceptable de celui-ci ; à la condition que lorsque X et Y sont conjointement CH, $R_4$ ne soit pas le 2-méthylphényle.

**4.** Composé selon l'une quelconque des revendications 1 à 3 dans lequel $R_1$ et $R_2$ représentent un haloalkyle en ($C_{1-4}$).

**5.** Composé selon l'une quelconque des revendications 1 à 3 dans lequel $R_1$ représente un haloalkyle en ($C_{1-4}$) et $R_2$ représente un alkyle en ($C_{1-4}$).

**6.** Composé selon l'une quelconque des revendications 1 à 5 dans lequel $R_3$ est choisi parmi le groupe constitué par hydrogène et alkyle en ($C_{1-4}$).

**7.** Composé selon l'une quelconque des revendications 1 à 6 dans lequel $R_5$ et $R_6$ sont choisis indépendamment parmi le groupe constitué par hydrogène et hydroxyalkyle en ($C_{1-4}$)

**8.** Composé selon l'une quelconque des revendications 1 à 7 dans lequel $R_7$ est choisi parmi le groupe constitué par halogène et alkyle en ($C_{1-4}$).

**9.** Composé selon l'une quelconque des revendications 1 à 8 dans lequel X et Y représentent CH.

**10.** Composé selon l'une quelconque des revendications 1 à 9 dans lequel $R_4$ est le phényle, ledit phényle étant substitué par deux substituants choisis indépendamment parmi $R_7$.

**11.** Composé selon l'une quelconque des revendications 1 à 10 choisi parmi le groupe constitué par
N-((R)-1-(3,5-bis(trifluorométhyl)phényl)éthyl)-1-(2,4-diméthylphényl)-N-méthyl -6-oxohexahydropyrrolo[1,2-a]pyrazine-2(1H)-carboxamide,
N-(3,5-bis(trifluorométhyl)benzyl)-1-(2,4-diméthylphényl)-N-méthyl-6-oxohexahydropyrrolo[1, 2-a]pyrazine-2(1H)-carboxamide,
1-(2,4-diméthylphényl)-N-méthyl-N-((R)-1-(3-méhyl-5-(trifluorométhyl)phényl)éthyl)-6-oxohexahydropyrrolo[1,2-a]pyrazine-2(1H)-carboxamide, N-((R)-1-(3,5-bis(trifluorométhyl)phényl)éthyl)-1-(2,4-diméthylphényl)-7,7-bis(2-hydroxyéthyl) -N-méthyl-6-oxohexahydropyrrolo[1,2-a]pyrazine-2(1H)-carboxamide,
1-(2,4-diméthylphényl)-7,7-bis(2-hydroxyéthyl)-N-méthyl-N-((R)-1-(3-méthyl-5-(trifluorométhy 1)phényl)éthyl)-6-oxo-hexahydropyrrolo[1,2-a]pyrazine-2(1H)-carboxamide, N-(3,5-bis(trifluorométhyl)benzyl)-1-(2,4-diméthylphényl)-7,7-bis(2-hydroxyéthyl)-N-méthyl-6-oxohexahydropyrrolo[1,2-a]pyrazine-2(1H)-carboxamide,
N-((R)-1-(3-chloro-5-(trifluorométhyl)phényl)éthyl)-1-(4-fluoro-2-méthylphényl)-7,7-bis(2-hydr oxyéthyl)-N-méthyl-6-oxohexahydropyrrolo[1,2-a]pyrazine-2(1H)-carboxamide, 1-(4-fluoro-2-méthylphényl)-7,7-bis(2-hydroxyéthyl)-N-méthyl-N-((R)-1-(3-méthyl-5-(trifluoro méthyl)phényl)éthyl)-6-oxohexahydropyrrolo[1,2-a]pyrazine-2(1H)-carboxamide,
N-((R)-1-(3,5-bis(trifluorométhyl)phényl)éthyl)-1-(4-fluoro-2-méthylphényl)-7,7-bis(2-hydroxyé thyl)-N-méthyl-6-oxohexahydropyrrolo[1,2-a]pyrazine-2(1H)-carboxamide,
N-((R)-1-(3,5-bis(trifluorométhyl)phényl)éthyl)-7,7-bis(2-hydroxyéthyl)-1-(4-méthoxy-2-méthyl phényl)-N-méthyl-6-oxohexahydropyrrolo[1,2-a]pyrazine-2(1H)-carboxamide,
(1S,8aS)-7,7-bis(2-hydroxyéthyl)-1-(4-méthoxy-2-méthylphényl)-N-méthyl-N-((R)-1-(3-méthyl-5-(trifluorométhyl)phényl)éthyl)-6-oxohexahydropyrrolo[1,2-a]pyrazine-2(1H)-carboxamide, N-((R)-1-(3,5-bis(trifluorométhyl)phényl)éthyl)-1-(4-méthoxy-2-méthylphényl)-N-méthyl-6-oxo hexahydropyrrolo[1,2-a]pyrazine-2(1 H)-carboxamide,
(1S,8aS)-N-((R)-1-(3,5-bis(trifluorométhyl)phényl)éthyl)-1-(4-fluoro-2-méthylphényl)-N-méthyl -6-oxohexahydropyrrolo[1,2-a]pyrazine-2(1H)-carboxamide, (1S,8aS)-1-(4-fluoro-2-méthylphényl)-N-méthyl-N-((R)-1-(3-méthyl-5-(trifluorométhyl)phényl) éthyl)-6-oxohexahydropyrrolo[1,2-a]pyrazine-2(1H)-carboxamide, (1S,8aS)-1-(4-fluoro-2-méthylphényl)-N-(1-(3-isopropyl-5-(trifluorométhyl)phényl)éthyl) -N-méthyl-6-oxohexahydropyrrolo[1,2-a]pyrazine-2(1H)-carboxamide,
(1S,8aS)-N-((R)-1-(3-éthyl-5-(trifluorométhyl)phényl)éthyl)-1-(4-fluoro-2-méthylphényl)-N-mét hyl-6-oxohexahydropyrrolo[1,2-a]pyrazine-2(1H)-carboxamide;
(1S,8aS)-N-((R)-1-(3-éthyl-5-(trifluorométhyl)phényl)éthyl)-1-(4-fluoro-2-méthylphényl)-7,7-bi s(2-hydroxyéthyl)-N-méthyl-6-oxohexahydropyrrolo[1,2-a]pyrazine-2(1H)-carboxamide, (1S,8aS)-1-(4-fluoro-2-méthylphényl)-7,7-bis(2-hydroxyéthyl)-N-((R)-1-(3-isopropyl-5-(trifluor ométhyl)phényl)éthyl)-N-méthyl-6-oxohexahydropyrrolo[1,2-a]pyrazine-2(1H)-carboxamide, (1S,8aS)-1-(4-(benzyloxy)-2-méthylphényl)-N-((R)-1-(3,5-bis(trifluorométhyl)phényl)éthyl)-N-méthyl-6-oxohexahydropyrrolo[1,2-a]pyrazine-2(1H)-carboxamide,
(1S,8aS)-N-((R)-1-(3,5-bis(trifluorométhyl)phényl)éthyl)-1-(4-hydroxy-2-méthylphényl)-N-méthyl-6-oxohexahydropyrrolo[1,2-a]pyrazine-2(1H)-carboxamide, (1S,8aS)-N-((R)-1-(3-éthyl-5-(trifluorométhyl)phényl)éthyl)-1-(4-hydroxy-2-méthylphényl)-N-méthyl-6-oxohexahydropyrrolo[1,2-a]pyrazine-2(1H)-carboxamide,
(1S,8aS)-N-((R)-1-(3,5-bis(trifluorométhyl)phényl)éthyl)-7,7-bis(2-hydroxyéthyl)-1-(4-méthoxy -2-méthylphényl)-N-méthyl-6-oxohexahydropyrrolo[1,2-a]pyrazine-2(1H)-carboxamide, (1 S,8aS)-N-((R)-1-(3,5-bis(trifluorométhyl)phényl)éthyl)-1-(4-hydroxy-2-méthylphényl)-7,7-bis(2-hydroxyéthyl)-N-méthyl-6-oxo-hexahydropyrrolo[1,2-a]pyrazine-2(1H) -carboxamide,
(1S,8aS)-N-((R)-1-(3-éthyl-5-(trifluorométhyl)phényl)éthyl)-1-(4-hydroxyl-méthyl phényl)-7,7-bis(2-hydroxyéthyl)-N-méthyl-6-oxohexahydropyrrolo[1,2-a]pyrazine-2(1H)-carbox amide,
(1S,8aS)-N-((R)-1-(4-éthyl-6-(trifluorométhyl)pyridin-2-yl)éthyl)-N-méthyl-6-oxo-1-o-tolylhexa hydropyrrolo[1,2-a]pyrazine-2(1H)-carboxamide,
(1S,8aS)-7,7-bis(2-hydroxyéthyl)-N-méthyl-N-((4-méthyl-6-(trifluorométhyl)pyrid in-2-yl)méthyl)-6-oxo-1-o-tolylhexahydropyrrolo[1,2-a]pyrazine-2(1H)-carboxamide, (1 S,8aS)-N-((R)-1-(3-chloro-5-(trifluorométhyl)phényl)éthyl)-1-(2,4-diméthyl phényl)-7,7-bis(2-hydroxyéthyl)-N-méthyl-6-oxo-hexahydropyrrolo[1,2-a]pyrazine-2(1H)-carbo xamide,
(1S,8aS)-1-(2,4-diméthylphényl)-7,7-bis(2-hydroxyéthyl)-N-((R)-1-(3-isopropyl-5-(trifluorométhyl)phényl)éthyl)-N-méthyl-6-oxohexahydropyrrolo[1,2-a]pyrazine-2(1H)-carboxamide, (1S,8aS)-N-((R)-1-(3,5-bis(trifluorométhyl)phényl)éthyl)-7,7-bis(2-hydroxyéthyl)-1-(2-méthoxy phényl)-N-méthyl-6-oxo-hexahydropyrrolo[1,2-a]pyrazine-2(1H)-carboxamide, (1S,8aS)-N-((R)-1-(3,5-bis(trifluorométhyl)phényl)éthyl)-1-(2,5-diméthylphényl)-7,7-bis(2-hydr oxyéthyl)-N-méthyl-6-oxo-hexahydropyrrolo[1,2-a]pyrazine-2(1H)-carboxamide, (1S,8aS)-1-(2,5-diméthylphényl)-7,7-bis(2-hydroxyéthyl)-N-((R)-1-(3-isopropyl-5-(trifluorométhyl)phényl)éthyl)-N-méthyl-6-oxohexahydropyrrolo[1,2-a]pyrazine-2(1H)-carboxamide, (1S,8aS)-N-((R)-1-(3,5-bis(trifluorométhyl)phényl)éthyl)-1-(2,3-diméthylphényl)-7,7-bis(2-hydr oxyéthyl)-N-méthyl-6-oxohexahydropyrrolo[1,2-a]pyrazine-2(1H)-carboxamide,
(1S,8aS)-N-((R)-1-(3,5-bis(trifluorométhyl)phényl)éthyl)-1-(2,3-diméthylphényl)-N-méthyl-6-o xo-hexahydropyrrolo[1,2-a]pyrazine-2(1H)-carboxamide,
(1S,8aS)-1-(2,3-diméthylphényl)-N-((R)-1-(3-éthyl-5-(trifluorométhyl)phényl)éthyl)-N-méthyl-6 -oxo-hexahydropyrrolo[1,2-a]pyrazine-2(1H)-carboxamide,
(1S,8aS)-1-(2,3-diméthylphényl)-N-((R)-1-(3-isopropyl-5-trifluorométhyl)phényl)éthyl)-N-méth yl-6-oxo-hexahydropyrrolo[1,2-a]pyrazine-2(1H)-carboxamide,
(1R,8aS)-N-((R)-1-(3,5-bis(trifluorométhyl)phényl)éthyl)-N-méthyl-1-(3-méthylpyridin-2-yl)-6-oxo-hexahydropyrrolo[1,2-a]pyrazine-2(1H)-carboxamide, (1S,8aS)-N-((R)-1-(3,5-bis(trifluorométhyl)phényl)éthyl)-N-méthyl-1-(3-méthylpyridin-2-yl)-6-oxo-hexahydropyrrolo[1,2-a]pyrazine-2(1H)-carboxamide,
(1S,8aS)-N-((R)-1-(3,5-bis(trifluorométhyl)phényl)éthyl)-N-méthyl-1-(4-méthylpyridin-3-yl)-6-oxo-hexahydropyrrolo[1,2-a]pyrazine-2(1H)-carboxamide, (1S,8aS)-N-((R)-1-(3,5-bis(trifluorométhyl)phényl)éthyl)-N-méthyl-1-(3-méthylpyridin-4-yl)-6-oxohexahydropyrrolo[1,2-a]pyrazine-2(1H)-carboxamide,
(S,8aS)-N-((R)-1-(3,5-bis(trifluorométhyl)phényl)éthyl)-N-méthyl-1-(2-méthylpyridin-3-yl)-6-oxo-hexahydropyrrolo[1,2-a]pyrazine-2(1H)-carboxamide,
(1S,8aS)-N-((R)-1-(3,5-bis(trifluorométhyl)phényl)éthyl)-N-méthyl-1-(4-éthylthiophén-3-yl)-6-o xohexahydropyrrolo[1,2-a]pyrazine-2(1H)-carboxamide,
(1S,8aS)-N-((R)-1-(3-éthyl-5-(trifluorométhyl)phényl)éthyl)-N-méthyl-1-(4-méthylthiophén-3-yl )-6-oxohexahydropyrrolo[1,2-a]pyrazine-2(1H)-carboxamide,
(1R,8aS)-N-((R)-1-(3,5-bis(trifluorométhyl)phényl)éthyl)-N-méthyl-1-(3-éthylthiophén-2-yl)-6-oxohexahydropyrrolo[1,2-a]pyrazine-2(1H)-carboxamide,
(1R,8aS)-N-((R)-1-(3-éthyl-5-(trifluorométhyl)phényl)éthyl)-N-méthyl-1-(3-méthylthiophén-2-y l)-6-oxohexahydropyrrolo[1,2-a]pyrazine-2(1H)-carboxamide,
(1R,8aS)-N-méthyl-N-((R)-1-(3-méthyl-5-(trifluorométhyl)phényl)éthyl)-1-(3-méthylthiophén-2 -yl)-6-oxohexahydropyrrolo[1,2-a]pyrazine-2(1H)-carboxamide,

(1R,8aS)-N-((R)-1-(3,5-bis(trifluorométhyl)phényl)éthyl)-N-méthyl-6-oxo-1-(thiophén-2-yl)hex ahydropyrrolo[1,2-a]pyrazine-2(1H)-carboxamide,
(1R,8aS)-N-(R)-1-(3-éthyl-5-(trifluorométhyl)phényl)éthyl)-N-méthyl-6-oxo-1-(thiophén-2-yl)h exahydropyrrolo[1,2-a]pyrazine-2(1H)-carboxamide
(1S,8aS)-N-méthyl-N-((R)-1-(3-méthyl-5-(trifluorométhyl)phényl)éthyl)-1-(2-méthylthiophén-3-yl)-6-oxohexahydropyrrolo[1,2-a]pyrazine-2(1H)-carboxamide,

(1S,8aS)-N-((R)-1-(3-éthyl-5-(trifluorométhyl)phényl)éthyl)-N-méthyl-1-(2-méthylthiophén-3-yl )-6-oxohexahydropyrrolo[1,2-a]pyrazine-2(1H)-carboxamide,
(1S,8aS)-N-((R)-1-(3,5-bis(trifluorométhyl)phényl)éthyl)-N-méthyl-1-(2-méthylthiophén-3-yl)-6 -oxohexahydropyrrolo[1,2-a]pyrazine-2(1H)-carboxamide,
(1R,8aS)-N-((R)-1-(3,5-bis(trifluorométhyl)phényl)éthyl)-N-méthyl-1-(2-méthylthiazol-5-yl)-6-oxohexahydropyrrolo[1,2-a]pyrazine-2(1H)-carboxamide,
(1R,8aS)-N-((R)-1-(3,5-bis(trifluorométhyl)phényl)éthyl)-N-méthyl-6-oxo-1-(thiazol-5-yl)-hexa hydropyrrolo[1,2-a]pyrazine-2(1H)-carboxamide,
(1S,8aS)-N-((R)-1-(3,5-bis(trifluorométhyl)phényl)éthyl)-N-méthyl-1-(3-méthyl-1H-pyrazol-4-y l)-6-oxohexahydropyrrolo[1,2-a]pyrazine-2(1H)-carboxamide
(1S,8aS)-N-((R)-1-(3-éthyl-5-(trifluorométhyl)phényl)éthyl)-N-méthyl-1-(3-méthyl-1H-pyrazol-4-yl)-6-oxohexahydropyrrolo[1,2-a]pyrazine-2(1H)-carboxamide,
(1S,8aS)-N-((R)-1-(3-éthyl-5-(trifluorométhyl)phényl)éthyl)-7,7-bis(2-hydroxyéthyl)-N-méthyl-1 -(3-méthyl-1H-pyrazol-4-yl)-6-oxohexahydropyrrolo[1,2-a]pyrazine-2(1H)-carboxamide, (1S,8aS)-7,7-bis(2-hydroxyéthyl)-N-méthyl-1-(3-méthyl-1H-pyrazol-4-yl)-N-((R)-1-(3-méthyl-5 -(trifluorométhyl)phényl)éthyl)-6-oxo-hexahydropyrrolo[1,2-a]pyrazine-2(1H)-carboxamide, (1S,8aS)-N-((R)-1-(3,5-bis(trifluorométhyl)phényl)éthyl)-1-(1,3-diméthyl-1H-pyrazol-4-yl)-N-m éthyl-6-oxohexahydropyrrolo[1,2-a]pyrazine-2(1H)-carboxamide,
(1S,8aS)-1-(2,3-diméthylphényl)-N-((R)-1-(3-méthyl-5-trifluorométhyl)phényl)éthyl)-N-méthyl-6-oxo-hexahydro-pyrrolo[1,2-a]pyrazine-2(1H)-carboxamide,
(1S,8aS)-N-((R)-1-(3,5-bis(trifluorométhyl)phényl)éthyl)-1-(3-chloro-2-méthylphényl)-N-méthy 1-6-oxohexahydropyrrolo[1,2-a]pyrazine-2(1H)-carboxamide,
N-((R)-1-(3,5-bis(trifluorométhyl)phényl)éthyl)-1-(2-éthylphényl)-N-méthyl-6-oxohexahydropyr rolo[1,2-a]pyrazine-2(1H)-carboxamide,
25N-((R)-1-(3-éthyl-5-(trifluorométhyl)phényl)éthyl)-1-(2-éthylphényl)-N-méthyl-6-oxohexahy dropyrrolo[1,2-a]pyrazine-2(1H)-carboxamide,
N-((R)-1-(3,5-bis(trifluorométhyl)phényl)éthyl)-1-(2-chlorophényl)-N-méthyl-6-oxohexahydrop yrrolo[1,2-a]pyrazine-2(1H)-carboxamide,
(1S,8aS)-N-((R)-1-(3,5-bis(trifluorométhyl)phényl)éthyl)-1-(2-(hydroxyméthyl)phényl)-N-méthyl-6-oxohexahydropyrrolo[1,2-a]pyrazine-2(1H)-carboxamide,
(1R,8aS)-N-((R)-1-(3,5-bis(trifluorométhyl)phényl)éthyl)-1-(3,4-diméthylthiophén-2-yl)-N-méth yl-6-oxohexahydropyrrolo[1,2-a]pyrazine-2(1H)-carboxamide,
(1R,8aS)-1-(3,4-diméthylthiophén-2-yl)-N-((R)-1-(3-éthyl-5-(trifluorométhyl)phényl)éthyl)-N-méthyl-6-oxohexahydropyrrolo[1,2-a]pyrazine-2(1H)-carboxa mide,
(1S,8aS)-N-((R)-1-(3,5-bis(trifluorométhyl)phényl)éthyl)-1-(3-méthoxy-2-méthylphényl)-N-mét hyl-6-oxohexahydropyrrolo[1,2-a]pyrazine-2(1H)-carboxamide,
(1S,8aS)-N-((R)-1-(3-isopropyl-5-(trifluorométhyl)phényl)éthyl)-1-(3-méthoxy-2-méthylphényl) -N-méthyl-6-oxo-hexahydropyrrolo[1,2-a]pyrazine-2(1H)-carboxamide,
(1S,8aS)-N-((R)-1-(3,5-bis(trifluorométhyl)phényl)éthyl)-1-(2,3-dihydro-1H-indén-4-yl)-N-méth yl-6-oxohexahydropyrrolo[1,2-a]pyrazine-2(1H)-carboxamide,
(1S,8aS)-1-(2,3-dihydro-1H-indén-4-yl)-N-((R)-1-(3-éthyl-5-(trifluorométhyl)phényl)éthyl)-N-m éthyl-6-oxohexahydropyrrolo[1,2-a]pyrazine-2(1H)-carboxamide, (1S,8aS)-1-(2,3-dihydro-1H-indén-4-yl)N-((R)-1-(3-isopropyl-5-(trifluorométhyl)phényl)éthyl)-N-méthyl-6-oxohexahydropyrrolo[1,2-a]pyrazine-2(1H)-carboxa mide,
(1S,8aS)-N-((R)-1-(3,5-bis(trifluorométhyl)phényl)éthyl)-1-(2-fluoro-6-méthylphényl)-N-méthyl -6-oxohexahydropyrrolo[1,2-a]pyrazine-2(1H)-carboxamide, N-((R)-1-(3,5-bis(trifluorométhyl)phényl)éthyl)-1-(2-chloro-4-fluorophényl)-N-méthyl-6-oxohe xahydropyrrolo[1,2-a]pyrazine-2(1H)-carboxamide,
(1S,8aS)-N-((R)-1-(3,5-bis(trifluorométhyl)phényl)éthyl)-1-(4-fluorophényl)-N-méthyl-6-oxohe xahydropyrrolo[1,2-a]pyrazine-2(1H)-carboxamide
ou sel ou solvate pharmaceutiquement acceptable de celui-ci.

**12.** Composé selon l'une quelconque des revendications 1 à 11 pour utilisation thérapeutique.

**13.** Composé selon les revendications 1 à 11 pour utilisation dans la prévention, le traitement ou l'amélioration des pathologies cutanées prurigineuses, p. ex. prurit aigu dans toute pathologie ; prurit chronique sur peau pathologique, telles que maladies cutanées inflammatoires, infectieuses ou autoimmunes, génodermatoses, réactions médicamenteuses, dermatoses de la grossesse et lymphomes cutanés, prurigo, lichen plan, dermatite atopique, eczéma, dermatite de contact, dermatite allergique, dermatite nummulaire, lichen simplex, psoriasis, syndrome de Sezary, lymphomes cutanés, pemphigorde bulleuse, alopécie en aires, gale, vitiligo, urticaire et prurit iatrogène ; maladies prurigineuses sur peau saine d'origine systémique, neurologique ou psychosomatique/psychiatrique, notamment troubles endocriniens et métaboliques, infections, maladies hématologiques et lymphoprolifératives, néoplasies solides et prurit iatrogène ; mastocytose ; prurit de cause inconnue ; prurit avec lésions de grattage secondaires, tel que prurigo nodulaire, et tous les types de prurigo ; ou toute autre maladie ou pathologie cutanée **caractérisée par** un prurit.

**14.** Composé selon la revendication 13, la pathologie cutanée prurigineuse étant choisie parmi prurigo, lichen plan, dermatite atopique, eczéma, dermatite de contact, dermatite allergique, dermatite nummulaire, lichen simplex, psoriasis, syndrome de Sezary, lymphomes cutanés, urticaire, mastocytose et prurit avec lésions secondaires chroniques de grattage.

**15.** Composition pharmaceutique comprenant comme ingrédient thérapeutiquement actif un composé selon l'une quelconque des revendications 1 à 11 et un véhicule pharmaceutiquement acceptable.

**16.** Composition pharmaceutique selon la revendication 15 conjointement avec un ou plusieurs autres composés thérapeutiquement actifs.

**17.** Composition pharmaceutique selon les revendications 15-16 convenant pour l'administration topique.

**18.** Composé choisi parmi la liste constituée par
1-(2,4-diméthyl phényl)hexahydropyrrolo[1,2-a]pyrazin-6(7H)-one, 7,7-diallyl-1-(2,4-diméthylphényl)hexahydropyrrolo[1,2-a]pyrazin-6(7H)-one, 7,7-diallyl-1-(4-fluoro-2-méthylphényl)hexahydropyrrolo[1,2-a]pyrazin-6(7H)-one, 1-{4-méthoxy-2-méthylphényl)hexahydropyrrolo[1,2-a]pyrazin-6(7H)-one, 1-(4-fluoro-2-méthylphényl)hexahydropyrrolo[1,2-a]pyrazin-6(7H)-one, 1-(4-méthoxy-2-méthylphényl)hexahydropyrrolo[1,2-a]pyrazin-6(7H)-one, 7,7-diallyl-1-(4-méthoxy-2-méthylphényl)hexahydropyrrolo[1,2-a]pyrazin-6(7H)-one, 1-(4-(benzyloxy)-2-méthylphényl)hexahydropyrrolo[1,2-a]pyrazin-6(7H)-one, 7,7-dial]yl-1-(4-(benzyloxy)-2-méthylphényl)hexahydropyrrolo[1,2-a]pyrazin-6(7H)-one, tert-butyl 7,7-diallyl-1-(2,5-diméthylphényl)-6-oxo-hexahydropyrrolo[1,2-a]pyrazine-2(1H)-carboxylate,

7,7-diallyl-1-(2,3-di méthylphényl)hexahydropyrrolo[1,2-a]pyrazin-6(7H)-one, 1-(2,3-diméthylphényl)-hexahydropyrrolo[1,2-a]pyrazin-6(7H)-one,
1-(3-méthylpyridin-2-yl)-hexahydropyrrolo[1,2-a]pyrazin-6(7H)-one,
1-(4-méthylpyridin-3-yl)hexahydropyrrolo[1,2-a]pyrazin-6(7H)-one,
1-(3-méthylpyridin-4-yl)hexahydropyrrolo[1,2-a]pyrazin-6(7H)-one,
1-(2-méthylpyridin-3-yl)hexahydropyrrolo[1,2-a]pyrazin-6(7H)-one,
1-(4-méthylthiophén-3-yl) hexahydropyrrolo[1,2-a]pyrazin-6(7H)-one,
1-(3-méthylthiophén-2-yl)hexahydropyrrolo[1,2-a]pyrazin-6(7H)-one,
1-(thiophén-2-yl)hexahydropyrrolo[1,2-a]pyrazin-6(7H)-one,
1-(2-méthylthiophén-3-yl)hexahydropyrrolo[1,2-a]pyrazin-6(7H)-one, 1-(2-méthylthiazol-5-yl)hexahydropyrrolo[1,2-a]pyrazin-6(7H)-one,
1-(thiazol-5-yl)hexahydropyrrolo[1,2-a]pyrazin-6(7H)-one,
1-(1-benzyl-3-méthyl-1H-pyrazol-4-yl)hexahydropyrrolo[1,2-a]pyrazin-6(7H)-one, 7,7-diallyl-1-(1-benzyl-3-méthyl-1H-pyrazol-4-yl)hexahydropyrrolo[1,2-a]pyrazin-6(7H)-one, (1S,8aS)-1-(3-chloro-2-méthylphényl)hexahydropyrrolo[1,2-a]pyrazine-6(2H)-one,
1-(2-éthylphényl)hexahydropyrrolo[1,2-a]pyrazin-6(7H)-one,
1-(2-chlorophényl)hexahydropyrrolo [1,2-a]pyrazin-6(7H)-one,
(1S,8aS)-1-(2-vinylphényl)hexahydropyrrolo[1,2-a]pyrazin-6(7H)-one, (8aS)-1-(3,4-diméthylthiophén-2-yl)hexahydropyrrolo[1,2-a]pyrazin-6(7H)-one, (1S,8aS)-1-(3-méthoxy-2-méthylphényl)hexahydropyrrolo[1,2-a]pyrazin-6(7H)-one, (1S,8aS)-1-(2,3-dihydro-1H-indén-4-yl)hexahydropyrrolo[1,2-a]pyrazin-6(7H)-one, (1S,8aS)-7,7-diallyl-1-(2,3-diméthylphényl)hexahydropyrrolo[1,2-a]pyrazin-6(7H)-one, 1-(2-chloro-4-fluorophényl)hexahydropyrrolo[1,2-a]pyrazin-6(7H)-one, (1S,8aS)-1-(4-fluorophényl)hexahydropyrrolo[1,2-a]pyrazin-6(7H)-one.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0125219 A
- WO 0232867 A
- WO 02081457 A
- WO 2009002770 A
- WO 2013124286 A


### Non-patent literature cited in the description


- **MW GREAVES.** *Curr. Allergy Asthma Rep.,* 2010, vol. 10, 236-242
- **U. RAAP et al.** *Curr. Opin. Allergy Clin. Immunol.,* 2011, vol. 11, 420-427
- **J. SALOMON ; E. BARAN.** *JEADV,* 2008, vol. 22, 223-228
- **S-E. CHANG et al.** *Br. J. Dermatol.,* 2007, vol. 156, 1272-1277
- **PATEL ; YOSIPOVITCH.** *Expert. Opin. Pharmacother,* 2010, vol. 11 (10), 1673-1682
- **DUVAL ; DUBERTRET.** *New Engl. J. Med.,* 2009, vol. 361, 1415-1416
- **S. STANDER et al.** *Plos One,* 2010, 5
- **KENAKIN, T.** *J. Pharm. Exp. Ther.,* 2006, vol. 319, 710-723
- Goodman & Gilman's The Pharmacological Basis of Therapeutics. McGraw-Hill, 1995
- **REMINGTON.** The Science and Practice of Pharmacy,. 2000
- *Encyclopedia of Pharmaceutical Technology,* 1994, vol. 9
- Encyclopedia of Pharmaceutical Technology. 1989, vol. 2
- Modern Pharmaceutics. Marcel Dekker, 427-432
- Modern Pharmaceutics. Marcel Dekker, 618-619, 718-721
- Encyclopedia of Pharmaceutical Technology. Marcel Dekker, vol. 10, 191-221
- *Methods Mol Biol,* 1999, vol. 114, 125-133